(19)

Europäisches
Patentamt
European
Patent Office
Office européen
des brevets

(11) **EP 4 764 000 A2**

(12) **EUROPEAN PATENT APPLICATION**

(43) Date of publication:
**24.06.2026 Bulletin 2026/26**

(21) Application number: **26160972.1**

(22) Date of filing: **04.05.2022**

(51) International Patent Classification (IPC):
***C12Q 1/6888*** *(2018.01)*

(52) Cooperative Patent Classification (CPC):
**G16H 50/70; C12Q 1/6888; G06N 3/0499;**
**G06N 3/09; G06N 3/0985; G16B 20/20;**
**G16H 10/40; G16H 50/20; G16H 50/30;**
C12Q 2600/158; Y02A 90/10

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **04.05.2021 US 202163183927 P**

(62) Document number(s) of the earlier application(s) in
accordance with Art. 76 EPC:
**22799481.1 / 4 334 474**

(71) Applicant: **Inflammatix, Inc.**
**Sunnyvale, CA 94085 (US)**

(72) Inventors:
• **LUETHY, Roland**
**Sunnyvale, CA 94085 (US)**

• **BUTUROVIC, Ljubomir**
**Sunnyvale, CA 94085 (US)**
• **SWEENEY, Timothy**
**Sunnyvale, CA 94085 (US)**

(74) Representative: **Mathys & Squire**
**32 London Bridge Street**
**The Shard**
**London SE1 9SG (GB)**

Remarks:
•This application was filed on 26-02-2026 as a
divisional application to the application mentioned
under INID code 62.
•Claims filed after the date of filing of the application /
after the date of receipt of the divisonal application
(Rule 68(4) EPC).

(54) **SYSTEMS AND METHODS FOR ASSESSING A BACTERIAL OR VIRAL STATUS OF A SAMPLE**

(57)    Systems and methods for determining infectious disease states are provided. An ensemble classifier is obtained using a training dataset including labels and attribute values for a plurality of genes including at least 20 genes selected from one or more of Table 1, Table 2, Table 8, and Table 9. For each of a plurality of random seeds, initial classifiers with pseudo-randomly assigned hyperparameters are binned and downsampled using evaluation scores obtained from one or more iterations of K-fold cross-validation. The ensemble classifier is formed from initial classifiers with the best score for each random seed. Infectious disease states are determined for a test subject by inputting attribute values for the plurality of genes to a trained ensemble classifier. Compositions and kits for determining infectious disease states, including amplification primers for the plurality of genes, are further provided.

Fig. 1

**Description**

**CROSS-REFERENCES TO RELATED APPLICATIONS**

This application claims priority to U.S. Provisional Application No. 63/183,927, filed May 4, 2021, the disclosure of which is hereby incorporated by reference in its entirety for all purposes.

**TECHNICAL FIELD**

This specification relates generally to methods for diagnosis of bacterial and viral infections. In particular, the invention relates to the use of biomarkers that can distinguish whether a patient has a bacterial infection, viral infection, or no infection.

**BACKGROUND**

Early and accurate diagnosis of infection is key to improving patient outcomes and reducing antibiotic resistance. The mortality rate of bacterial sepsis increases 8% for each hour by which antibiotics are delayed; however, giving antibiotics to patients without bacterial infections increases rates of morbidity and antimicrobial resistance. The rate of inappropriate antibiotic prescriptions in the hospital setting is estimated at 30-50%, and would be aided by improved diagnostics.

Strikingly, close to 95% of patients given antibiotics for suspected enteric fever have negative cultures. There is currently no gold-standard point of care diagnostic that can broadly determine the presence and type of infection. The National Action Plan for Combating AntibioticResistant Bacteria, for example, calls for "point-of-need diagnostic tests to distinguish rapidly between bacterial and viral infections." While new PCR-based molecular diagnostics can profile pathogens directly from a blood culture, such methods rely on the presence of adequate numbers of pathogens in the blood, which may not be reliably present at point-of-care monitoring and testing, or during acute or early stages of infection. Moreover, PCR-based molecular diagnostics are limited to detecting a discrete range of pathogens. As a result, there is growing interest in molecular diagnostics that profile the host gene response. These include diagnostics that can distinguish the presence of infection as compared to inflamed but non-infected patients.

Currently available methods focus on gene sets that can distinguish between types of infections, such as bacterial versus viral infections. Other conventional methods utilize models that distinguish among three classes of infection (*e.g.,* non-infected patients, patients with bacterial illness, and patients with viral illness), but which require additional laboratory preparation and processing workflows (*e.g.,* detection and measurement of probes) or rely on large probe sets and/or gene panels that lead to unwieldy and computationally-intensive analysis pipelines and have limited clinical application due to the difficulty of interpreting such large datasets. Overall, while great promise has been shown in this field, no host gene expression infection diagnostic has yet made it into clinical practice.

**SUMMARY**

Given the above background, there is a need in the art for improved approaches for using molecular diagnostic methods (*e.g.,* analysis of biomarkers) to distinguish between infectious disease states (*e.g.,* bacterial infections, viral infections, and/or non-infections). For example, there is a need in the art for improved selection of biomarkers that are sensitive and specific and can be readily interpreted, thus providing clinical utility during point-of-care applications. Further, there is a need in the art for improved methods of analyzing biomarker data (*e.g.,* gene expression data) for the rapid and accurate identification of infectious disease states, which can in turn benefit downstream applications such as diagnosis, monitoring, and therapy.

In some aspects, the present disclosure addresses the shortcomings identified in the background by providing systems and methods of obtaining and using ensemble classifiers for determining an infectious disease state of a subject, *e.g.,* for distinguishing between at least bacterial etiologies and viral etiologies. In some embodiments, an ensemble classifier is obtained using a training dataset including labels (*e.g.,* known infectious disease states for training subjects) and attribute values (*e.g.,* gene expression data, *e.g.,* mRNA abundance values) for a plurality of genes. For each random seed in a plurality of random seeds, initial classifiers are pseudo-randomly assigned hyperparameters. Initial classifiers are then binned, and an outer loop is performed over the plurality of bins. Each bin is, in turn, used to perform an inner loop including ranking the initial classifiers based on K-fold cross-validation evaluation scores and selecting the best-performing classifiers based on a downsampling rate parameter. For example, each round in the inner loop comprises, for each initial classifier in the respective bin, training the classifier specified by the hyperparameters using a given number of iterations, in a K-fold cross-validation setting, obtaining the cross-validation evaluation scores, and downsampling the set of initial classifiers in the respective bin, based on the obtained evaluation scores and the downsampling rate. In each successive round within the inner loop, the set of initial classifiers are trained for increasing numbers of iterations. The ensemble classifier is formed by selecting the initial classifier with the best score across the plurality of bins for each random seed (*e.g.,* within the outer loop), and combining the plurality of best-scored classifiers from each of the random seeds. A trained ensemble classifier is used to determine infectious disease states, by inputting attribute values for the plurality of genes to a trained ensemble classifier.

In some aspects, the present disclosure addresses the shortcomings identified in the background by providing biomarker sets for determining infectious disease states (*e.g.,* at least 20 genes selected from Table 1, at least 20 genes selected from Table 2, and/or at least 20 genes from Table 9). Additionally, compositions and kits for determining infectious disease states, including amplification primers for the plurality of genes, are provided.

The systems, methods, and compositions disclosed herein thus improve upon the need for biomarkers that are sensitive, specific, and readily interpretable by providing a plurality of genes (*e.g.,* in Table 1, Table 2, and Table 9) that can be used to distinguish between infectious disease states based on attribute values (*e.g.,* mRNA abundance). Furthermore, the systems and methods disclosed herein improve upon the need for more rapid and accurate determination of infectious disease states, by providing methods for obtaining classifiers (*e.g.,* with optimized hyperparameters), methods for training classifiers (*e.g.,* with labeled training datasets), and/or methods for using classifiers (*e.g.,* with test datasets) to obtain indications of infectious disease states (*e.g.,* bacterial infection, viral infection, and/or non-infection) in subjects.

Accordingly, one aspect of the present disclosure provides a method for obtaining (*e.g.,* training) an ensemble classifier for determining an infectious disease state of a subject, *e.g.,* for distinguishing between at least bacterial etiologies and viral etiologies. The method includes obtaining a training dataset, where the training dataset comprises, in electronic form, for each respective training subject in a plurality of training subjects (i) a corresponding label for the infectious disease state of the respective training subject and (ii) a respective attribute value for each corresponding gene in a plurality of genes obtained from a biological sample of the respective training subject, where the plurality of training subjects is 100 training subjects or more.

For each respective random seed in a plurality of random seeds, a corresponding instance of an outer loop is performed, where each corresponding instance of the outer loop is characterized by a respective downsampling rate and a respective maximum iteration rate.

The corresponding instance of the outer loop includes, for each respective initial classifier in a plurality of initial classifiers, using the random seed to pseudo-randomly assign values for each respective hyperparameter in a plurality of hyperparameters for the respective initial classifier (*e.g.,* pseudo-randomly obtaining hyperparameter configurations for each initial classifier). Each respective hyperparameter in the plurality of hyperparameters has a respective value selected from a respective plurality of candidate values for the respective hyperparameter, and each respective initial classifier in the plurality of initial classifiers has a corresponding plurality of parameters (*e.g.,* weights), where the corresponding plurality of parameters comprises more than 500 parameters (*e.g.,* weights). The outer loop further includes binning the plurality of initial classifiers into a plurality of bins, where each bin in the plurality of bins is characterized by a respective initial number of initial classifiers in the plurality of initial classifiers, a respective initial number of iterations, and the downsampling rate.

For each respective bin in the plurality of bins, a corresponding inner loop is performed in which an iteration count is initially set to the respective initial number of iterations.

The corresponding inner loop includes, for a number of iterations equal to the iteration count, training each initial classifier in the respective bin in a K-fold cross-validation context, where the K-fold cross-validation comprises refining each initial classifier in the respective bin against the training dataset using the values assigned for each respective hyperparameter in the plurality of hyperparameters for the respective initial classifier. Based on the K-fold cross-validation, a corresponding evaluation score is determined for each initial classifier in the respective bin, and a subset of initial classifiers is removed from the respective bin in accordance with the downsampling rate and the corresponding evaluation score for each initial classifier in the respective bin.

The iteration count is increased as a function of an inverse of the downsampling rate, and the inner loop (*e.g.,* the performing, determining, removing, and increasing) is repeated for a number of repetitions that is determined based on a corresponding identity for the respective bin.

Referring again to the outer loop, the method comprises selecting, from among all initial classifiers in the plurality of initial classifiers (*e.g.,* from across all bins in the plurality of bins in the corresponding instance of the outer loop), a corresponding classifier that has the best corresponding evaluation score as representative of the respective random seed in the plurality of random seeds. The ensemble classifier is formed from the corresponding classifier selected for each respective random seed in the plurality of random seeds (*e.g.,* the ensemble classifier comprises a plurality of classifiers, each classifier having the best score for its respective random seed).

In some embodiments, the method further includes obtaining a test dataset comprising, in electronic form, a respective attribute value for each corresponding gene in the plurality of genes obtained from a biological sample of a test subject, and using the ensemble classifier to determine the infectious disease state of the test subject, based on at least the plurality of attribute values for the plurality of genes.

In some embodiments, the method further includes, when the infectious disease state determined for the test subject indicates the presence of an infection, administering a first therapeutic regimen tailored for treatment of the subject in the presence of the infection; and when the infectious disease state determined for the test subject indicates the absence of an infection, administering a second therapeutic regimen tailored for treatment of the subject in the absence of the infection.

In some embodiments, the plurality of genes comprises at least 20 genes selected from Table 1, at least 20 genes selected from Table 2, and/or at least 20 genes selected from Table 9. In some embodiments, the plurality of genes comprises at least 29 genes selected from Table 1, at least 29 genes selected from Table 2, and/or at least 29 genes selected from Table 9. In some embodiments, the plurality of genes comprises no more than 1000 genes. In some embodiments, the plurality of genes comprises no more than 200 genes.

Another aspect of the present disclosure provides a method for determining an infectious disease state of a test subject, the method including obtaining, in electronic form, a dataset comprising a respective attribute value for each corresponding gene in a plurality of genes obtained from a biological sample of the test subject, thereby obtaining a plurality of attribute values, where the plurality of genes comprises at least 20 genes selected from Table 1, at least 20 genes selected from Table 2, and/or at least 20 genes selected from Table 9. Responsive to inputting the plurality of attribute values to a trained classifier, the method further includes obtaining, as output from the trained classifier, a determination as to whether the test subject has an infectious disease state, *e.g.,* distinguishing between at least bacterial etiologies and viral etiologies.

In some embodiments, the method further includes, when the infectious disease state determined for the test subject indicates the presence of an infection, administering a first therapeutic regimen tailored for treatment of the subject in the presence of the infection; and when the infectious disease state determined for the test subject indicates the absence of an infection, administering a second therapeutic regimen tailored for treatment of the subject in the absence of the infection.

In some embodiments, the trained classifier is obtained by a method including obtaining a training dataset, where the training dataset comprises, in electronic form, for each respective training subject in a plurality of training subjects (i) a corresponding label for the infectious disease state of the respective training subject and (ii) a respective attribute value for each corresponding gene in the plurality of genes obtained from a biological sample of the respective training subject, wherein the plurality of training subjects is 100 training subjects or more. For each respective random seed in a plurality of random seeds, a corresponding instance of an outer loop is performed, where each corresponding instance of the outer loop is characterized by a respective downsampling rate and a respective maximum iteration rate. The corresponding instance of the outer loop includes, for each respective initial classifier in a plurality of initial classifiers, using the random seed to pseudo-randomly assign values for each respective hyperparameter in a plurality of hyperparameters for the respective initial classifier (*e.g.,* pseudo-randomly obtaining hyperparameter configurations for each initial classifier). Each respective hyperparameter in the plurality of hyperparameters has a respective value selected from a respective plurality of candidate values for the respective hyperparameter, and each respective initial classifier in the plurality of initial classifiers has a corresponding plurality of parameters (*e.g.,* weights), where the corresponding plurality of parameters comprises more than 500 parameters (*e.g.,* weights). The outer loop further includes binning the plurality of initial classifiers into a plurality of bins, where each bin in the plurality of bins is characterized by a respective initial number of initial classifiers in the plurality of initial classifiers, a respective initial number of iterations, and the downsampling rate.

For each respective bin in the plurality of bins, a corresponding inner loop is performed in which an iteration count is initially set to the respective initial number of iterations. For a number of iterations equal to the iteration count, each initial classifier in the respective bin is trained in a K-fold cross-validation context, where the K-fold cross-validation comprises refining each initial classifier in the respective bin against the training dataset using the values assigned for each respective hyperparameter in the plurality of hyperparameters for the respective initial classifier. Based on the K-fold cross-validation, a corresponding evaluation score is determined for each initial classifier in the respective bin, and a subset of initial classifiers is removed from the respective bin in accordance with the downsampling rate and the corresponding evaluation score for each initial classifier in the respective bin. The iteration count is increased as a function of an inverse of the downsampling rate, and inner loop (*e.g.,* the performing, determining, removing, and increasing) is repeated for a number of repetitions that is determined based on a corresponding identity for the respective bin.

Referring again to the outer loop, the method comprises selecting, from among all initial classifiers in the plurality of initial classifiers (*e.g.,* from across all bins in the plurality of bins in the corresponding instance of the outer loop), a corresponding classifier that has the best corresponding evaluation score as representative of the respective random seed in the plurality of random seeds. The ensemble classifier is formed from the corresponding classifier selected for each respective random seed in the plurality of random seeds (*e.g.,* the ensemble classifier comprises a plurality of classifiers, each classifier having the best score for its respective random seed).

Another aspect of the present disclosure provides a method for determining an infectious disease state of a subject. The method comprises at a computer system comprising at least one processor and a memory storing at least one program for execution by the at least one processor, the at least one program comprising instructions for: (i) obtaining, in electronic form, a dataset comprising respective attribute values for at least two genes selected from Table 8, wherein the attribute value is obtained from a biological sample of the subject; (ii) responsive to inputting the attribute values to a trained classifier, obtaining, as output from the trained classifier, a determination as to whether the subject has an infectious disease state selected from: infected with a bacteria, infected with a virus, and not-infected.

In some embodiments, the at least two genes are selected from LY6E, IRF9, ITGAM, and PSTPIP2. In some embodiments, the method comprises obtaining, in electronic form, a dataset comprising respective attribute values for at least three genes selected from Table 8. In some embodiments, the at least three genes are selected from LY6E, IRF9, ITGAM, and PSTPIP2. In some embodiments, the method comprises obtaining, in electronic form, a dataset comprising respective attribute values for at least four genes selected from Table 8. In some embodiments, the at least four genes comprise LY6E, IRF9, ITGAM, and PSTPIP2. In some embodiments, the dataset comprises an attribute value for one additional gene that is not LY6E, IRF9, ITGAM, and PSTPIP2. This additional gene, in some cases, is another gene selected from Table 8.

In some embodiments, the biological sample is a blood sample of the subject. In some embodiments, the biological sample comprises blood, whole blood, plasma, serum, urine, cerebrospinal fluid, fecal, saliva, sweat, tears, pleural fluid, pericardial fluid, peritoneal fluid, nasal swabs, nasopharyngeal swabs, or oropharyngeal swabs of the subject.

In some embodiments, the attribute value is mRNA abundance data. In some embodiments, the attribute value is obtained using real-time polymerase chain reaction (RT-PCR), quantitative RT-PCR (qRT-PCR), or real-time quantitative isothermal amplification on one or more nucleic acid molecules in the biological sample of the subject. In some embodiments, the real-time quantitative isothermal amplification is real-time quantitative loop-mediated isothermal amplification (LAMP).

Another aspect of the disclosure provides a method for diagnosing a subject suspected of having a bacterial or viral infection, the method comprising: receiving a biological sample obtained from the subject; measuring the expression levels of at least two genes selected from Table 8; determining whether the subject has a bacterial infection or viral infection using the expression levels in a classification model which has been validated in multiple independent cohorts, wherein the classification model has an area under the receiver operating characteristic (ROC) curve of at least 0.65 in at least one validation cohort.

In some embodiments, the at least two genes are selected from LY6E, IRF9, ITGAM, and PSTPIP2. In some embodiments, the method comprises measuring the expression levels of at least three genes selected from Table 8. In some embodiments, the at least three genes are selected from LY6E, IRF9, ITGAM, and PSTPIP2. In some embodiments, the method comprises measuring the expression levels of at least four genes selected from Table 8. In some embodiments, the at least four genes comprise LY6E, IRF9, ITGAM, and PSTPIP2. In some embodiments, the method comprises measuring the expression levels of at least five genes selected from Table 8.

In some embodiments, the classification model has an ROC curve of at least 0.7 in at least one validation cohort. In some embodiments, the classification model has an ROC curve of at least 0.75 in at least one validation cohort. In some embodiments, the classification model has an ROC curve of at least 0.8 in at least one validation cohort.

In some embodiments, the biological sample is a blood sample of the subject. In some embodiments, the biological sample comprises blood, whole blood, plasma, serum, urine, cerebrospinal fluid, fecal, saliva, sweat, tears, pleural fluid, pericardial fluid, peritoneal fluid, nasal swabs, nasopharyngeal swabs, or oropharyngeal swabs of the test subject.

In some embodiments, the expression levels are obtained using real-time polymerase chain reaction (RT-PCR), quantitative RT-PCR (qRT-PCR), or real-time quantitative isothermal amplification on one or more nucleic acid molecules in the biological sample of the subject. In some embodiments, the real-time quantitative isothermal amplification is real-time quantitative loop-mediated isothermal amplification (LAMP).

In some embodiments, the method further comprises administering an antibiotic to the subject if the subject is determined to have a bacterial infection. In some embodiments, the method further comprises administering an anti-viral agent to the subject if the subject is determined to have a viral infection.

Another aspect of the present disclosure provides compositions comprising a plurality of amplification primers for determining an infectious disease state of a subject, the plurality of amplification primers comprising, for each respective gene in a plurality of genes comprising at least 20 genes selected from Table 1, at least 20 genes selected from Table 2, and/or at least 20 genes selected from Table 9, a respective forward amplification primer and a respective reverse amplification primer. The respective forward amplification primer comprises a 3' binding region and a 5' auxiliary region, where the 3' binding region consists of from 10 to 50 nucleotides and has a sequence that is complementary to a first target sequence in a first strand of the respective gene or a transcript thereof, and the 5' auxiliary region has a sequence that is not complementary to the sequence of the first strand of the respective gene or a transcript thereof. The respective reverse amplification primer comprises a binding region, where the binding region consists of from 10 to 50 nucleotides and has a sequence that is complementary to a second target sequence in the second strand of the respective gene or a transcript thereof. In some embodiments, the plurality of genes comprises at least 29 genes selected from Table 1, at least 29 genes selected from Table 2, and/or at least 29 genes selected from Table 9. In some embodiments, the plurality of genes comprises no more than 1000 genes. In some embodiments, the plurality of genes comprises no more than 200 genes.

Another aspect of the present disclosure provides kits comprising agents for determining an infectious disease state of a subject. The kit comprises a plurality of amplification primers comprising, for each respective gene in a plurality of genes comprising at least 20 genes selected from Table 1, at least 20 genes selected from Table 2, and/or at least 20 genes selected from Table 9, a respective forward amplification primer and a respective reverse amplification primer. The respective forward amplification primer comprises a 3' binding region and a 5' auxiliary region, where the 3' binding region consists of from 10 to 50 nucleotides and has a sequence that is complementary to a first target sequence in a first strand of the respective gene or a transcript thereof, and the 5' auxiliary region has a sequence that is not complementary to the sequence of the first strand of the respective gene or a transcript thereof. The respective reverse amplification primer comprises a binding region, where the binding region consists of from 10 to 50 nucleotides and has a sequence that is complementary to a second target sequence in the second strand of the respective gene or a transcript thereof. In some embodiments, the kit further includes information, in electronic or paper form, comprising instructions for measuring attributes of the plurality of genes in a biological sample of the subject, thus obtaining a plurality of attribute values for the plurality of genes. In some embodiments, the kit further includes information, in electronic or paper form, comprising instructions for using the plurality of attribute values with a trained classifier to determine an infectious disease state of the subject, *e.g.,* for distinguishing between at least bacterial etiologies and viral etiologies. In some embodiments, the plurality of genes comprises at least 29 genes selected from Table 1, at least 29 genes selected from Table 2, and/or at least 29 genes from Table 9. In some embodiments, the plurality of genes comprises no more than 1000 genes. In some embodiments, the plurality of genes comprises no more than 200 genes.

Another aspect of the present disclosure provides a plurality of conjugated nucleic acid probes for determining an infectious disease state of a subject. The plurality of conjugated nucleic acid probes comprises, for each respective gene in a plurality of genes comprising at least 20 genes selected from Table 1, at least 20 genes selected from Table 2, and/or at least 20 genes selected from Table 9, a respective nucleic acid probe comprising a respective nucleic acid conjugated to a non-nucleic acid detection moiety, where the respective nucleic acid is complementary to the respective gene. In some embodiments, the plurality of genes comprises at least 29 genes selected from Table 1, at least 29 genes selected from Table 2, at least 29 genes selected from Table 9. In some embodiments, the plurality of genes comprises no more than 1000 genes. In some embodiments, the plurality of genes comprises no more than 200 genes.

Another aspect of the present disclosure provides computer systems comprising at least one processor and a memory storing at least one program including instructions for execution by the at least one processor, for performing any of the methods and embodiments disclosed herein, and/or any combinations thereof as will be apparent to one skilled in the art. In some embodiments, the at least one program is configured for execution by a computer.

Another aspect of the present disclosure provides a non-transitory computer-readable storage medium having stored thereon program code instructions that, when executed by a processor, cause the processor to perform any of the methods and embodiments disclosed herein, and/or any combinations thereof as will be apparent to one skilled in the art. In some embodiments, the program code instructions are configured for execution by a computer.

## INCORPORATION BY REFERENCE

All publications, patents, and patent applications herein are incorporated by reference in their entireties. In the event of a conflict between a term herein and a term in an incorporated reference, the term herein controls.

## BRIEF DESCRIPTION OF THE DRAWINGS

The patent or application file contains at least one drawing executed in color. Copies of this patent or patent application publication with color drawing(s) will be provided by the Office upon request and payment of the necessary fee.

The implementations disclosed herein are illustrated by way of example, and not by way of limitation, in the figures of the accompanying drawings. Like reference numerals refer to corresponding parts throughout the several views of the drawings.

Figure 1 is a block diagram illustrating an example of a computing system in accordance with some embodiments of the present disclosure.

Figures 2A and 2B collectively illustrate an example of a flowchart of a method for determining an infectious disease state of a subject, in which dashed boxes represent optional portions of the method, in accordance with some embodiments of the present disclosure.

Figure 3 illustrates an example of a flowchart of a method for determining an infectious disease state of a subject, in which dashed boxes represent optional portions of the method, in accordance with some embodiments of the present disclosure.

Figure 4 illustrates an example schematic of methods and compositions for obtaining attribute values for a plurality of genes, in accordance with some embodiments of the present disclosure.

Figure 5 illustrates an example system for determining infectious disease states in a subject, in accordance with some embodiments of the present disclosure.

Figure 6 illustrates an example performance measure for a method of obtaining attribute values for a plurality of genes compared to a reference technology, in accordance with some embodiments of the present disclosure.

Figure 7 illustrates an example performance measure for a method of obtaining attribute values for a plurality of genes compared to a reference technology, in accordance with some embodiments of the present disclosure.

Figure 8 illustrates an example performance measure for a method of obtaining attribute values for a plurality of genes compared to a reference technology, in accordance with some embodiments of the present disclosure.

Figure 9 illustrates the results of an example process for gene selection, in accordance with some embodiments of the present disclosure.

Figure 10 illustrates an example of a method for obtaining ensemble classifier for determining an infectious disease state of a subject, in accordance with some embodiments of the present disclosure.

Figure 11 illustrates an example output for an infectious disease state of a test subject, in accordance with some embodiments of the present disclosure.

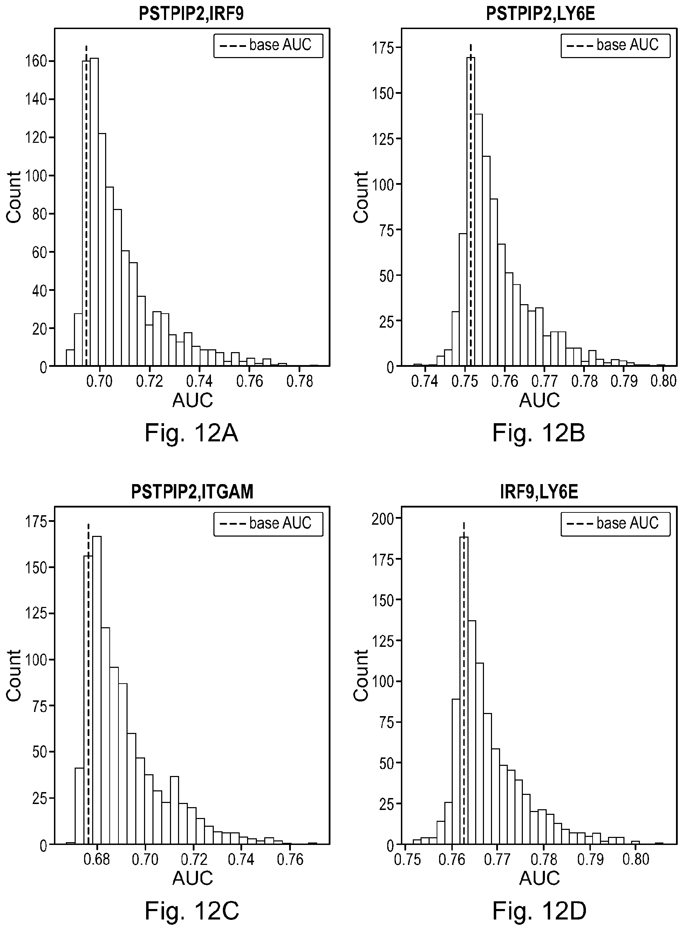

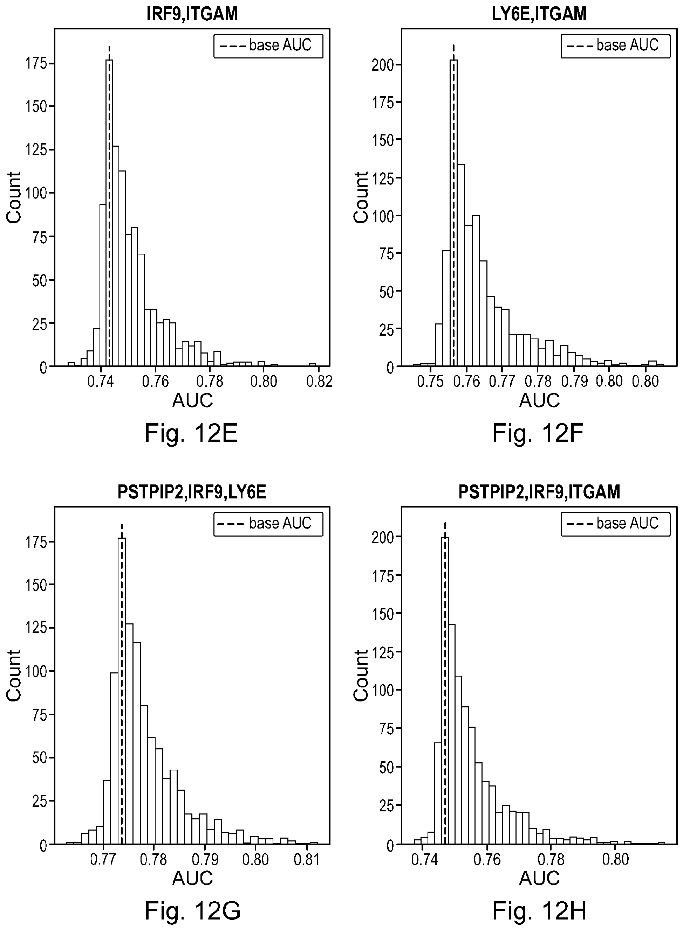

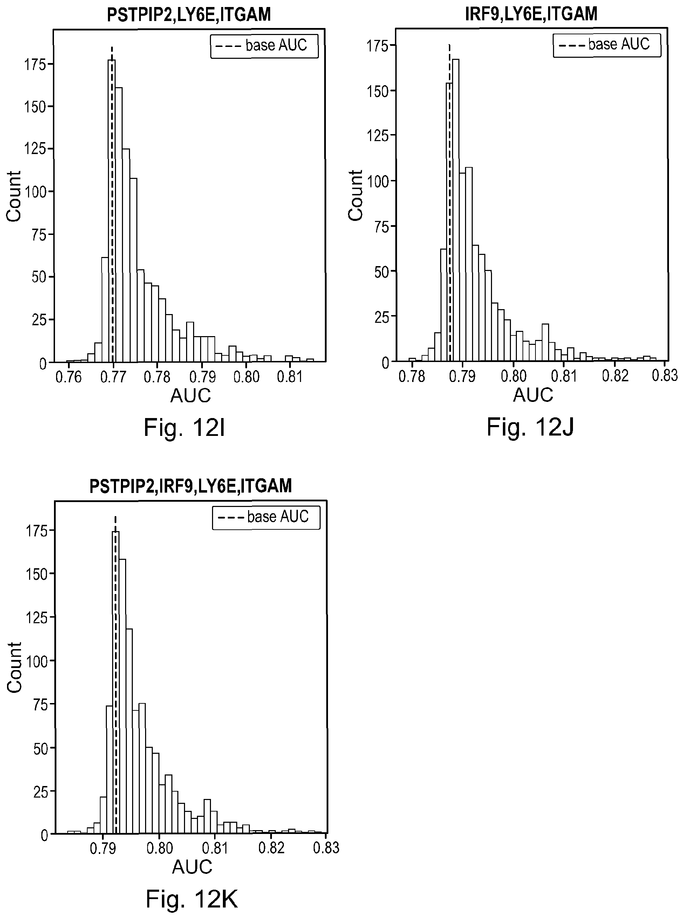

Figures 12A-12K illustrates AUCs of 1000 3 gene classification models comprising PSTPIP2, IRF9, and one random gene (Figure 12A); 1000 3 gene classification models comprising PSTPIP2, LY6E, and one random gene (Figure 12B); 1000 3 gene classification models comprising PSTPIP2, ITGAM, and one random gene (Figure 12C); 1000 3 gene classification models comprising IRF9, LY6E, and one random gene (Figure 12D); 1000 3 gene classification models comprising IRF9, ITGAM, and one random gene (Figure 12E); 1000 3 gene classification models comprising LY6E, ITGAM, and one random gene (Figure 12F); 1000 4 gene classification models comprising PSTPIP2, IRF9, LY6E, and one random gene (Figure 12G); 1000 4 gene classification models comprising PSTPIP2, IRF9, ITGAM, and one random gene (Figure 12H); 1000 4 gene classification models comprising PSTPIP2, LY6E, ITGAM, and one random gene (Figure 12I); 1000 4 gene classification models comprising IRF9, LY6E, ITGAM, and one random gene (Figure 12J); and 1000 5 gene classification models comprising PSTPIP2, IRF9, LY6E, ITGAM, and one random gene (Figure 12K).

Figures 13A-13C illustrates the ranges of AUCs from 1000 classification models of 3 random genes (Figure 13A); 1000 classification models of 4 random genes (Figure 13B); and 1000 classification models of 5 random genes (Figure 13C).

Figures 14A-14K illustrates, for each classification model of Table 10, the base AUC, the AUCs of 1000 augmented classification models (e.g., plus one random gene), and the AUCs of 1000 classification models of with the corresponding number of random genes.

## DETAILED DESCRIPTION

### Introduction

Point-of-care treatments are increasingly important to the timely diagnosis and treatment of disease conditions and to the improvement of patient outcomes. Recent technologies allow for the profiling of pathogens directly from patient samples or blood cultures. Together with such technologies, the analysis of mRNA signatures provides a powerful tool for measuring immune responses, such as in infectious and inflammatory diseases. For instance, mRNA signatures can be used for studying a variety of disease and health conditions, including, but not limited to, infectious disease (*e.g.,* acute bacterial/viral diseases, sepsis, tuberculosis, dengue; malaria, and/or vaccine response); autoimmunity and fibrosis (*e.g.,* lupus, scleroderma, COPD, organ transplant, and/or pulmonary hypertension); therapy response (*e.g.,* biologics in ulcerative colitis and/or Crohn's, TCA cycle in cancer, immune modulators in infections, and/or acute respiratory distress syndrome); and/or oncology (*e.g.,* lung adenocarcinoma, RAS-driven cancers, and/or pan-cancer diagnoses).

As an example, the rapid and accurate detection and diagnosis of sepsis is a huge unmet need in terms of both human lives and dollars. For instance, sepsis-related complications result in at least 50% of all hospital deaths and at least 40% of all intensive care unit (ICU) costs totaling more than $USD 40 billion. Underlying causes for sepsis can be bloodstream infections, non-bloodstream infections, and/or a number of other pathologies. Conventional methods, however, are limited to identifying sepsis in specific sample types or only for specific pathogens or infection types, such as bacterial infections only found in the blood stream (*e.g.,* T2, BioFire, GenMark, Accelerate, *etc.*), or viral infections found only in plasma (*e.g.,* Karius). Other traditional methods require the administration of one or more additional assays in conjunction with molecular diagnostics in order to obtain a reliable diagnosis, including, but not limited to, vitals, physical exams, complete blood count (CBC), lactate, procalcitonin (PCT), rapid microbial testing, imaging, and/or serologies.

Furthermore, as detailed above, conventional methods for detection and diagnosis of infections (*e.g.,* bacterial and/or viral infections) suffer from difficulties in interpreting and applying molecular diagnostic data to obtain meaningful conclusions. For example, some conventional methods use a single biomarker such as procalcitonin (PCT) as an indicator for infection in a patient (see, *e.g.,* Huang et al., N Engl J Med (2018); 379:236-249, which is hereby incorporated herein by reference in its entirety). Typically, a biomarker can be used to indicate the presence or absence of an infection or to indicate whether an infection is severe or not severe (*e.g.,* via detection of a presence or absence of the respective biomarker and/or via a high or low abundance of the biomarker). However, single biomarkers cannot both determine infection and predict severity, as the observation of a presence and/or a high abundance of a biomarker could indicate either infection, severity, or both, but would fail to discriminate between the three possibilities. Results obtained in such fashion are usually not actionable and thus would result in limited clinical utility and/or misdiagnoses. For instance, the improper prescription of antibiotics can occur where a medical practitioner cannot determine which method of treatment is best, based on ambiguity with respect to the identity of an infection type, pathogen, and/or severity.

Alternatively, some conventional methods use large biomarker panels, such as large probe sets and gene panels that lead to unwieldy and computationally-intensive analysis pipelines. Such traditional methods also have limited clinical utility and poor applicability, due to the difficulty of interpreting such large datasets.

Notably, the use of biomarker panels to assay host gene expression for the detection and determination of infectious disease states is largely untapped. Thus, there is a need in the art for systems and methods that overcome the above limitations of the conventional art and provide rapid, accurate, accessible, and easily interpretable data that can be used to inform downstream applications such as clinical diagnoses, monitoring, and/or treatment of infectious disease, including, but not limited to, bacterial infections, viral infections, and non-infections.

Advantageously, in some embodiments, the present disclosure provides systems, methods, and compositions for an expression-based framework that provides at least an indication of whether inflammation in a subject is associated with a viral etiology or a bacterial etiology with high specificity and high sensitivity. Further, in some embodiments, the expression-based test provides an indication of the severity of the condition of the subject, *e.g.,* a prognosis for whether the subject will develop sepsis. For instance, Example 3 describes a model, in accordance with some implementations of the present disclosure, that classifies bacterial and viral etiologies with high performance during both training and validation testing, as presented in Table 6 (*e.g.,* validation: mAUC > 0.88; bacterial sensitivity > 98%; bacterial specificity > 95%; viral specificity > 96%).

Furthermore, in some embodiments, the systems, methods, and compositions described herein provide very rapid prognosis, enabling faster medical responses associated with improved clinical outcomes. For instance, Example 1 describes a test, in accordance with some implementations of the present disclosure, that provides accurate diagnosis of bacterial and viral infections, and accurate prognosis for the severity of the subject's condition within 30 minutes using a single blood sample from the patient.

In some aspects, one or more of these advantages are realized, at least in part, by the identification of a limited set of mRNA biomarkers, isolated from patient blood, that provide diagnostic and power when quantified using rapid isothermal amplification techniques. For example, Table 2 provides a set of 29 genes that are differentially expressed in leukocytes that, when measured using an isothermal amplification technique, such as qRT-LAMP, provide diagnostic and prognostic power for the tests described herein.

In some aspects, one or more of the advantages described herein are realized, at least in part, by use of a hyperband methodology of hyperparameter tuning for improved training of a classifier (*e.g.,* an ensemble of neural networks) providing accurate diagnosis of bacterial etiologies and viral etiologies and/or accurate prognosis for the condition of the subject (*e.g.,* a prognosis for whether the subject will develop sepsis).

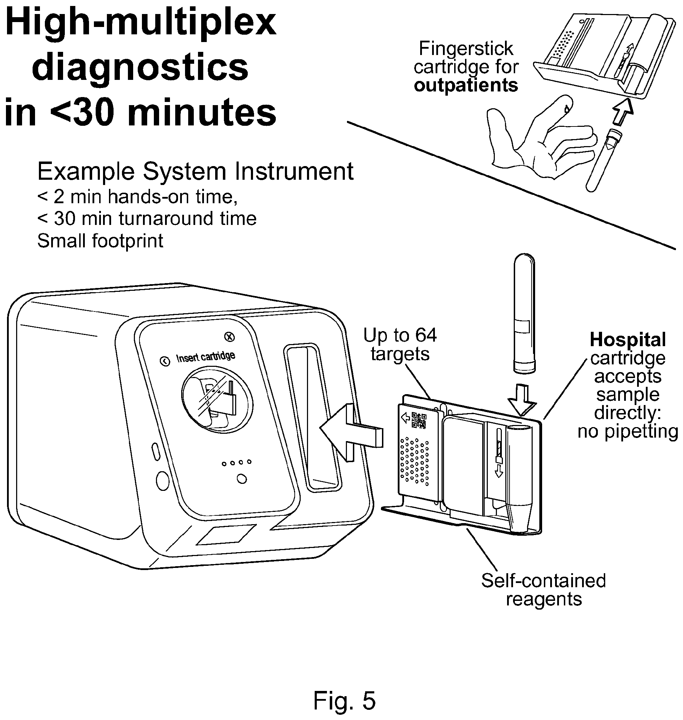

In an example implementation, the systems and methods disclosed herein "read" the immune response by analyzing and interpreting patterns of mRNA from white blood cells obtained from a host subject (*e.g.,* a human patient). In particular, the method uses circulating white blood cells that encode rich information about local infections. In such a manner, an infectious disease state is determined, where the infectious disease state includes, but is not limited to, a presence or absence of infection (*e.g.,* detection of bloodstream infections and/or non-bloodstream infections), an identity of an infection type (*e.g.,* differentiation between infection types), a presence, absence, or likelihood of sepsis (*e.g.,* risk-stratification of sepsis), a prediction of therapy response, and/or a prognosis (*e.g.,* a severity and/or mortality). Another example implementation of the systems and methods disclosed herein includes a high-multiplex diagnostics system that can provide results in less than 30 minutes and is additionally easy for both practitioners and patients to use (*e.g.,* via easy-insert cartridges and/or fingerstick cartridges that accept samples directly without the need for pipetting or multiple transfers). See, for example, an embodiment of a system for determining infectious disease states described in Example 1, below, and illustrated in Figure 5.

Furthermore, in some aspects of the present disclosure, systems and methods are provided for the development of classifiers used for accurate determination of infectious disease states. Accurate classifiers are obtained using a selection process (*e.g.,* a multi-layer perceptron classifier combined with the Hyperband method for hyperparameter search) that generates initial classifiers with pseudo-randomly assigned hyperparameter configurations and iteratively evaluates (for example, via cross-validation), and downsamples the initial classifiers using a training dataset (*e.g.,* including gene expression values and infectious disease state labels). Selection of classifiers with high-performing hyperparameters is based on the evaluation scores after completion of the iterations. In contrast to conventional methods for obtaining classifiers, the systems and methods provided herein avoid lengthy and computationally-intensive methods for selection of classification models and optimization of classifier hyperparameters, which typically require fallible trial-and-error attempts and/or tuning and optimization of classifier parameters (*e.g.,* weights) by adjustment (*e.g.,* via an empirically determined learning rate for neural networks and/or a number of trees for, e.g., XGBoost).

In particular, the systems and methods provided herein disclose use of the selection process to pseudo-randomly generate and then search for the best combination of hyperparameters, without the need for extensive trial-and-error or tuning. Furthermore, the iterative nature of the selection process, coupled with downsampling, provides a means for successively validating and evaluating top-performing initial classifiers with increasing depths while conserving computational power during each iteration. Additionally, the method employs a "hedging" strategy, such that initial hyperparameter configurations are evaluated across a variety of combinations of depth and breadth. An ensemble architecture, where the generated classifier is formed from multiple classifiers selected using the presently disclosed methods, adds additional layers of classification and predictive power to the final model. Thus, the method allows for selection and optimization of highly accurate classifiers for the determination of infectious disease states with greater efficiency and lower processing requirements.

Advantageously, the systems and methods disclosed herein address an unmet need for novel, rapid testing in hospitals and clinics, which uniquely bring together three growth frontiers, including rapid and point-of-care testing, blood and immune sampling for studying, profiling, or diagnosing disease, and the improved use of data and machine learning for more accurate and actionable diagnosis and determination of clinically actionable results.

Reference will now be made in detail to embodiments, examples of which are illustrated in the accompanying drawings. In the following detailed description, numerous specific details are set forth in order to provide a thorough understanding of the present disclosure. However, it will be apparent to one of ordinary skill in the art that the present disclosure may be practiced without these specific details. In other instances, well-known methods, procedures, components, circuits, and networks have not been described in detail so as not to unnecessarily obscure aspects of the embodiments.

The implementations described herein provide various technical solutions for training and using a classifier to distinguish between infectious disease states (*e.g.,* bacterial infections, viral infections, and/or non-infections) in a subject.

*Definitions*

As used herein, the terms "about" or "approximately" refer to an acceptable error range for a particular value as determined by one of ordinary skill in the art, which can depend in part on how the value is measured or determined, *e.g.,* the limitations of the measurement system. For example, "about" can mean within 1 or more than 1 standard deviation, per the practice in the art. "About" can mean a range of $\pm20\%$, $\pm10\%$, $\pm5\%$, or $\pm1\%$ of a given value. The term "about" or "approximately" can mean within an order of magnitude, within 5-fold, or within 2-fold, of a value. Where particular values are described in the application and claims, unless otherwise stated the term "about" meaning within an acceptable error range for the particular value should be assumed. The term "about" can have the meaning as commonly understood by one of ordinary skill in the art. The term "about" can refer to $\pm10\%$. The term "about" can refer to $\pm5\%$.

As used herein, the term "between" used in a range is intended to include the recited endpoints. For example, a number "between X and Y" can be X, Y, or any value from X to Y.

As used herein, the terms "sample," "biological sample," or "patient sample," refer to any sample taken from a subject, which can reflect a biological state associated with the subject, and that includes cell-free DNA. Examples of biological samples include, but are not limited to, blood, whole blood, plasma, serum, urine, cerebrospinal fluid, fecal, saliva, sweat, tears, pleural fluid, pericardial fluid, peritoneal fluid, nasal swabs, nasopharyngeal swabs, or oropharyngeal swabs of the subject. A biological sample can include any tissue or material derived from a living or dead subject. A biological sample can be a cell-free sample. A biological sample can comprise a nucleic acid (*e.g.,* DNA or RNA) or a fragment thereof. The term "nucleic acid" can refer to deoxyribonucleic acid (DNA), ribonucleic acid (RNA) or any hybrid or fragment thereof. The nucleic acid in the sample can be a cell-free nucleic acid. A sample can be a liquid sample or a solid sample (*e.g.,* a cell or tissue sample). A biological sample can be a bodily fluid, such as blood, plasma, serum, urine, vaginal fluid, fluid from a hydrocele (*e.g.,* of the testis), vaginal flushing fluids, pleural fluid, ascitic fluid, cerebrospinal fluid, saliva, sweat, tears, sputum, bronchoalveolar lavage fluid, discharge fluid from the nipple, aspiration fluid from different parts of the body (*e.g.,* thyroid, breast), *etc.* A biological sample can be a stool sample. In various embodiments, the majority of DNA in a biological sample that has been enriched for cell-free DNA (*e.g.,* a plasma sample obtained via a centrifugation protocol) can be cell-free (*e.g.,* greater than 50%, 60%, 70%, 80%, 90%, 95%, or 99% of the DNA can be cell-free). A biological sample can be treated to physically disrupt tissue or cell structure (*e.g.,* centrifugation and/or cell lysis), thus releasing intracellular components into a solution which can further contain enzymes, buffers, salts, detergents, and the like which can be used to prepare the sample for analysis.

As used herein, the terms "infectious disease state" or "status of infection" refer to a condition of a sample relative to infection, including a characteristic and/or measure of the condition. For example, a sample can have an infectious disease state that is "infected" or "not infected." An "infected" sample can additionally be infected with one or more infectious agents, including but not limited to bacteria, viruses, fungi, protozoa, and/or helminths. Accordingly, an infectious disease state can be one or more of "infected with a bacteria," "infected with a virus," "infected with a protozoan,", and/or "infected with a helminth," among others. An infectious disease state can include a primary site of infection, such as bloodstream infections, tissue infections, organ infections, and the like. An infectious disease state can be a condition and/or symptom associated with infection, including sepsis, inflammation, co-infections, fever, and/or other physiological manifestations of chronic or acute infections. An infectious disease state can be a metric and/or one or more clinical features associated with an infection, including a quantity of a pathogen within a subject or a tissue thereof (*e.g.,* a concentration, burden, titer, and/or load), a severity (*e.g.,* of sepsis, inflammation, fever, shock, necrosis, *etc.),* a prognosis (*e.g.,* hospitalization, fatality, *etc.*), and/or a site of infection (*e.g.,* disseminated, systemic, migration into deep tissues, *etc.*). An infectious disease state can further be a presence, absence, or likelihood of any of the metrics and/or features described herein, such as a presence, absence or likelihood of sepsis, a presence, absence or likelihood of inflammation, and/or a severe or non-severe infection. An infectious disease state can be a stage of infection, such as acute or chronic. An infectious disease state can also be a survival metric, which can be a predetermined likelihood of survival for a predetermined period of time. Multiple samples from a single subject can have different infectious disease states or the same infectious disease state. Multiple subjects can have different infectious disease states or the same infectious disease state.

As used herein, the term "Systemic inflammatory response syndrome," or "SIRS," refers to a clinical response to a variety of severe clinical insults, as manifested by two or more of the following conditions within a 24-hour period:

body temperature greater than 38 °C. (100.4 °F.) or less than 36 °C. (96.8°F.);

heart rate (HR) greater than 90 beats/minute;

respiratory rate (RR) greater than 20 breaths/minute, or

$P_{CO2}$ less than 32 mmHg, or requiring mechanical ventilation; and

white blood cell count (WBC) either greater than $12.0 \times 10^9$/L or less than $4.0 \times 10^9$/L.

These symptoms of SIRS represent a consensus definition of SIRS that can be modified or supplanted by other definitions in the future. The present definition is used to clarify current clinical practice and does not represent a critical aspect of the invention (see, *e.g.,* American College of Chest Physicians/Society of Critical Care Medicine Consensus Conference: Definitions for Sepsis and Organ Failure and Guidelines for the Use of Innovative Therapies in Sepsis, 1992, Crit. Care. Med. 20, 864-874, the entire contents of which are herein incorporated by reference).

As used herein, in some embodiments the term "sepsis" refers to a systemic host response to infection with SIRS plus a documented infection (*e.g.,* a subsequent laboratory confirmation of a clinically significant infection such as a positive culture for an organism). Thus, in some embodiments, sepsis refers to the systemic inflammatory response to a documented infection (see, *e.g.,* American College of Chest Physicians Society of Critical Care Medicine, Chest, 1997, 101:1644-1655, the entire contents of which are herein incorporated by reference). As used herein, "sepsis" includes all stages of sepsis including, but not limited to, the onset of sepsis, severe sepsis, septic shock and multiple organ dysfunction ("MOD") associated with the end stages of sepsis.

In some embodiments, the term "sepsis" refers to a physiological response to infection in a subject, often resulting in injury to the organs and/or tissues of the subject. Non-limiting examples of physiological responses that can occur as a result of sepsis include fever, low body temperature, increased heart rate, increased breathing rate, confusion, and edema. Early signs of sepsis can include decreased urination and high blood sugar, while signs of established sepsis can include metabolic acidosis, low blood pressure, and disorders in blood clotting leading to organ failure. In some instances, sepsis may be accompanied by symptoms related to specific infections, such as a cough with pneumonia or painful urination with a kidney infection. Sepsis can be caused by a number of organisms, including bacteria, viruses, parasites, and fungi. Sepsis can vary in severity and may be life-threatening. As used herein, sepsis is understood to include any definition of sepsis as determined using systemic inflammatory response syndrome (SIRS) criteria (*e.g.,* abnormal body temperature, heart rate, respiratory rate or blood gas, and white blood cell count). For instance, in some embodiments, sepsis is determined by the presence of two or more SIRS criteria in response to an infectious process. In some embodiments, sepsis includes severe sepsis and septic shock. As used herein, sepsis is further understood to include any definition of sepsis as determined using the sequential organ failure assessment (SOFA) score and the abbreviated version (qSOFA). The three criteria for the qSOFA score include a respiratory rate greater than or equal to 22 breaths per minute, systolic blood pressure 100 mmHg or less and altered mental status. For instance, in some embodiments, sepsis is determined by the presence of two or more of the qSOFA criteria in a subject.

The "onset of sepsis" refers to an early stage of sepsis, *e.g.,* prior to a stage when conventional clinical manifestations are sufficient to support a clinical suspicion of sepsis. The exact mechanism by which a subject becomes septic is not a critical aspect of the invention. The methods of the present invention can detect the onset of sepsis independent of the origin of the infectious process.

"Severe sepsis" can refer to sepsis (*e.g.,* defined using SIRS criteria) with sepsis-induced organ dysfunction or tissue hypoperfusion, or sepsis-induced hypotension. Hypoperfusion abnormalities include, but are not limited to, lactic acidosis, oliguria, or an acute alteration in mental status. In some embodiments, severe sepsis is an infectious disease state associated with multiple organ dysfunction syndrome (MODS).

In some embodiments, "septic shock" refers to severe sepsis with persistently low blood pressure (*e.g.,* despite the administration of intravenous fluids). In some embodiments, "septic shock" refers to sepsis-induced hypotension that is not responsive to adequate intravenous fluid challenge and with manifestations of peripheral hypoperfusion.

As used herein, the term "classification" refers to any number(s) or other characters(s) that are associated with a particular property of a sample. For example, the term "classification" can refer to an infectious disease state in the subject and/or sample, such as "infected with a bacteria," "infected with a virus," and/or "not infected." Classification can refer to a presence, absence, and/or likelihood of infection, a presence, absence, and/or likelihood of inflammation, a presence, absence, and/or likelihood of sepsis, a presence, absence, and/or likelihood of severe infection, an identity of one or more infecting agents, an identity of a type of infecting agent (*e.g.,* bacteria, virus, fungi, protozoa, and/or helminths), a stage of the infection in the subject (*e.g.,* acute and/or chronic), a pathogen load in the subject and/or sample, and/or a site or dissemination of infection in the subject. The classification can be binary (*e.g.,* positive or negative, yes or no, likely or not likely, presence or absence) or multi-class. In some embodiments, classification comprises outputting predicted class labels and/or probabilities.

As used herein, the term "cell-free nucleic acids" refers to nucleic acid molecules that can be found outside cells, in bodily fluids such as blood, whole blood, plasma, serum, urine, cerebrospinal fluid, fecal, saliva, sweat, sweat, tears, pleural fluid, pericardial fluid, peritoneal fluid, nasal swabs, nasopharyngeal swabs, or oropharyngeal swabs of a subject. Cell-free nucleic acids can originate from one or more healthy cells and/or from one or more diseased cells. Cell-free nucleic acids are used interchangeably as circulating nucleic acids. Examples of the cell-free nucleic acids include but are not limited to RNA, mitochondrial DNA, or genomic DNA.

As used herein, the terms "control," "control sample," "reference," "reference sample," "normal," or "normal sample" describe a sample from a subject that does not have a particular condition or is otherwise healthy. In an example, a method as disclosed herein can be performed on a subject having an infection, where the reference sample is a sample taken from a healthy tissue of the subject. A reference sample can be obtained from the subject, or from a database. A reference sample can include one or more samples corresponding to a respective one or more subjects from a cohort of healthy subjects. A reference sample can include data from a reference dataset, such as a data repository, including one or more attribute values for a respective one or more target nucleotide sequences (*e.g.,* genes) in a reference sequence. The reference sequence can be, for example, a complete or incomplete reference genome, including a haploid or diploid genome. For example, a reference sample can include data obtained from a gene expression databases (*e.g.,* NIH Gene Expression Omnibus (GEO) and/or EBI ArrayExpress) for one or more genes of interest, where the gene expression data is obtained from one or more healthy subjects in a plurality of healthy subjects. Other databases include genomic sequence databases, protein databases, antimicrobial resistance marker databases, biomarker databases, mRNA databases, and the like. As used herein, the phrase "healthy," refers to a subject possessing good health. A healthy subject can demonstrate an absence of any infectious disease. A "healthy individual" can have other diseases or conditions, unrelated to the infection condition being assayed, which can normally not be considered "healthy."

As used herein, the terms "nucleic acid" or "nucleic acid molecule" refer to nucleic acids of any composition form, such as deoxyribonucleic acid (DNA, *e.g.,* complementary DNA (cDNA), genomic DNA (gDNA) and the like), DNA analogs (*e.g.,* containing base analogs, sugar analogs and/or a non-native backbone and the like), and ribonucleic acid (RNA, *e.g.,*

messenger RNA (mRNA), ribosomal RNA (rRNA), transfer RNA (tRNA), small nuclear RNA (snRNA), and the like, including total RNA), which may be present in single- or double-stranded form. Unless otherwise limited, a nucleic acid can comprise known analogs of natural nucleotides, some of which can function in a similar manner as naturally occurring nucleotides. A nucleic acid can be in any form useful for conducting processes herein (*e.g.*, linear, circular, supercoiled, single-stranded, double-stranded and the like). A nucleic acid in some embodiments can be from a single chromosome or fragment thereof (*e.g.*, a nucleic acid sample may be from one chromosome of a sample obtained from a diploid organism). In certain embodiments, nucleic acids comprise nucleosomes, fragments or parts of nucleosomes or nucleosome-like structures. Nucleic acids sometimes comprise protein (*e.g.*, histones, DNA binding proteins, and the like). Nucleic acids analyzed by processes described herein sometimes are substantially isolated and are not substantially associated with protein or other molecules. Nucleic acids also include derivatives, variants and analogs of DNA or RNA synthesized, replicated or amplified from single-stranded ("sense" or "antisense," "plus" strand or "minus" strand, "forward" reading frame or "reverse" reading frame) and double-stranded polynucleotides. A nucleic acid may be prepared using a nucleic acid obtained from a subject as a template. Nucleic acids can be fragmented (*e.g.*, by physical shearing, enzymatic digestion, or chemical fragmentation, generating nucleic acid fragments (*e.g.*, DNA and/or RNA fragments). The terms "polynucleotide" or "oligonucleotide" are used herein to include a polymeric form of nucleotides of any length, either ribonucleotides or deoxyribonucleotides. Generally, this term refers to the primary structure of the molecule and thus includes triple-, double- and single-stranded DNA, as well as triple-, double- and single-stranded RNA. It also includes modifications, such as by methylation and/or by capping, and unmodified forms of the polynucleotide. More particularly, the terms "polynucleotide," and "oligonucleotide," include polydeoxyribonucleotides (containing 2-deoxy-D-ribose), polyribonucleotides (containing D-ribose), and any other type of polynucleotide which is an N- or C-glycoside of a purine or pyrimidine base. There is no intended distinction in length between the terms "polynucleotide," "oligonucleotide," "nucleic acid" and "nucleic acid molecule," and these terms are used interchangeably.

As used herein, the term "differentially expressed" refers to differences in the quantity and/or the frequency of a biomarker present in a sample taken from patients having, for example, an infection (*e.g.*, viral infection or bacterial infection) as compared to a control subject or non-infected subject. For example, a biomarker can be a polynucleotide which is present at an elevated level or at a decreased level in samples of patients with an infection (*e.g.*, viral infection or bacterial infection) compared to samples of control subjects. Alternatively, a biomarker can be a polynucleotide which is detected at a higher frequency or at a lower frequency in samples of patients with an infection (*e.g.*, viral infection or bacterial infection) compared to samples of control subjects. A biomarker can be differentially present in terms of quantity, frequency or both. A polynucleotide is differentially expressed between two samples if the amount of the polynucleotide in one sample is statistically significantly different from the amount of the polynucleotide in the other sample. For example, a polynucleotide is differentially expressed in two samples if it is present at least about 120%, at least about 130%, at least about 150%, at least about 180%, at least about 200%, at least about 300%, at least about 500%, at least about 700%, at least about 900%, or at least about 1000% greater than it is present in the other sample, or if it is detectable in one sample and not detectable in the other. In some instances, a polynucleotide is differentially expressed in two sets of samples if the frequency of detecting the polynucleotide in a first subset of samples (*e.g.*, samples of patients suffering from sepsis) is statistically significantly higher or lower than in control samples. For example, a polynucleotide is differentially expressed in two sets of samples if it is detected at least about 120%, at least about 130%, at least about 150%, at least about 180%, at least about 200%, at least about 300%, at least about 500%, at least about 700%, at least about 900%, or at least about 1000% more frequently or less frequently observed in one set of samples than the other set of samples.

As used herein, the term "similarity value" refers to a representation of the degree of similarity between two things being compared. For example, a similarity value can be a number that indicates the overall similarity between a patient's expression profile using specific phenotype-related biomarkers and reference value ranges for the biomarkers in one or more control samples or a reference expression profile (*e.g.*, the similarity to a "viral infection" expression profile or a "bacterial infection" expression profile). The similarity value may be expressed as a similarity metric, such as a correlation coefficient, or may simply be expressed as the expression level difference, or the aggregate of the expression level differences, between levels of biomarkers in a patient sample and a control sample or reference expression profile.

As used herein, the terms "polypeptide" or "protein" refer to a polymer of amino acid residues and are not limited to a minimum length. Thus, peptides, oligopeptides, dimers, multimers, and the like, are included within the definition. Both full-length proteins and fragments thereof are encompassed by the definition. The terms also include post-expression modifications of the polypeptide, for example, glycosylation, acetylation, phosphorylation, hydroxylation, oxidation, and the like.

As used herein, the terms "detection moiety," "detectable moiety," and "detectable label" refer to a molecule, typically conjugated to or having affinity for (directly or indirectly) an analyte that is used for detection and/or identification of the analyte. Detection moieties contemplated for use in the present disclosure include, but are not limited to, radioisotopes, fluorescent dyes such as fluorescein, phycoerythrin, Cy-3, Cy-5, allophycocyanin, DAPI, Texas Red, rhodamine, Oregon green, Lucifer yellow, and the like, green fluorescent protein (GFP), red fluorescent protein (DsRed), Cyan Fluorescent Protein (CFP), Yellow Fluorescent Protein (YFP), Cerianthus Orange Fluorescent Protein (cOFP), alkaline phosphatase (AP), beta-lactamase, chloramphenicol acetyltransferase (CAT), adenosine deaminase (ADA), aminoglycoside phosphotransferase (neor, G418r) dihydrofolate reductase (DHFR), hygromycin-B-phosphotransferase (HPH), thymidine kinase (TK), lacZ (encoding β-galactosidase), and xanthine guanine phosphoribosyltransferase (XGPRT), beta-glucuronidase (gus), Placental Alkaline Phosphatase (PLAP), Secreted Embryonic alkaline phosphatase (SEAP), or firefly or bacterial luciferase (LUC). Enzyme tags are used with their cognate substrate. The terms also include color-coded microspheres of known fluorescent light intensities (see *e.g.*, microspheres with xMAP technology produced by Luminex (Austin, Tex.); microspheres containing quantum dot nanocrystals, for example, containing different ratios and combinations of quantum dot colors (*e.g.,* Qdot nanocrystals produced by Life Technologies (Carlsbad, Calif.); glass coated metal nanoparticles (see *e.g.,* SERS nanotags produced by Nanoplex Technologies, Inc. (Mountain View, Calif.); barcode materials (see *e.g.,* sub-micron sized striped metallic rods such as Nanobarcodes produced by Nanoplex Technologies, Inc.), encoded microparticles with colored bar codes (see *e.g.,* CellCard produced by Vitra Bioscience, vitrabio.com), and glass microparticles with digital holographic code images (see *e.g.,* CyVera microbeads produced by Illumina (San Diego, Calif.). As with many of the standard procedures associated with the practice of the invention, skilled artisans will be aware of additional labels that can be used.

As used herein, the term "biomarker" refers to a biological compound that indicates a presence, absence, and/or likelihood of a biological or physiological state, such as a disease state (*e.g.*, an infectious disease state or condition). A biomarker can be a biological compound, such as a polynucleotide, which is differentially expressed in a sample taken from one or more subjects having a first infectious disease state (*e.g.,* a patient with an infection, including a bacterial or viral infection) as compared to a comparable sample taken from one or more subjects having a second infectious disease state (*e.g.,* a control subject, a subject with a negative diagnosis, a normal or healthy subject, and/or a non-infected subject). A biomarker can be a nucleic acid, a fragment of a nucleic acid, a polynucleotide, or an oligonucleotide that can be detected and/or quantified. Biomarkers include polynucleotides comprising nucleotide sequences from genes or RNA transcripts of genes, including but not limited to, viral response genes, bacterial response genes, and/or sepsis response genes. Biomarkers can further include markers (*e.g.,* indicators) of sepsis subtypes, markers for diagnosis of sepsis, markers for diagnosis of bacterial and/or viral infections, markers for identification of bacterial and/or viral pathogens, markers for use in prognosis, markers for inflammation, markers for severity (*e.g.,* mortality), and/or any other disease condition or combination thereof as will be apparent to one skilled in the art. Specific examples of biomarkers useful in the methods and systems described herein are provided in Tables 1, 2, and 9. Other examples of biomarkers that are generally useful for resolving bacterial infections, viral infections, and/or condition severity (*e.g.,* prognostic for sepsis development) are described in U.S. Patent Application No. US16/096,261, Publication No. US20190144943A1, filed on June 5, 2017; PCT Application No. US2016/022233, Publication No. WO2016145426A1, filed on March 12, 2016; PCT Application No. US2017/036003, Publication No. WO2017214061A1, filed on June 5, 2017; PCT Application No. US2017/029468, Publication No. WO2018004806A1, filed on April 25, 2017; and PCT Application No. US2019/015462, Publication No. WO2019168622A1, filed on January 28, 2019, each of which is hereby incorporated herein by reference in its entirety for all purposes, and specifically for their disclosures of diagnostic and prognostic biomarkers.

As used herein, the term "reference genome" refers to any particular known, sequenced or characterized genome, whether partial or complete, of any organism or virus that may be used to reference identified sequences from a subject. Exemplary reference genomes used for human subjects as well as many other organisms are provided in the online genome browser hosted by the National Center for Biotechnology Information ("NCBI") or the University of California, Santa Cruz (UCSC). A "genome" refers to the complete genetic information of an organism or virus, expressed in nucleic acid sequences. As used herein, a reference sequence or reference genome often is an assembled or partially assembled genomic sequence from an individual or multiple individuals. In some embodiments, a reference genome is an assembled or partially assembled genomic sequence from one or more human individuals. The reference genome can be viewed as a representative example of a species' set of genes. In some embodiments, a reference genome comprises sequences assigned to chromosomes. Exemplary human reference genomes include but are not limited to NCBI build 34 (UCSC equivalent: hg16), NCBI build 35 (UCSC equivalent: hg17), NCBI build 36.1 (UCSC equivalent: hg18), GRCh37 (UCSC equivalent: hg19), and GRCh38 (UCSC equivalent: hg38).

As used herein, the term "subject" refers to any living or non-living organism, including but not limited to a human (*e.g.,* a male human, female human, fetus, pregnant female, child, or the like), a non-human animal, a plant, a bacterium, a fungus or a protist. Any human or non-human animal can serve as a subject, including but not limited to mammal, reptile, avian, amphibian, fish, ungulate, ruminant, bovine (*e.g.,* cattle), equine (*e.g.,* horse), caprine and ovine (*e.g.,* sheep, goat), swine (*e.g.,* pig), camelid (*e.g.,* camel, llama, alpaca), monkey, ape (*e.g.,* gorilla, chimpanzee), ursid (*e.g.,* bear), poultry, dog, cat, mouse, rat, fish, dolphin, whale, and shark. In some embodiments, a subject is a male or female of any stage (*e.g.,* a man, a woman or a child). A subject from whom a sample is taken or who is treated by any of the methods or compositions described herein can be of any age and can be an adult, infant or child. In some cases, the subject, *e.g.,* patient is 0, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, or 99 years old, or within a range therein (*e.g.,* between about 2 and about 20 years old, between about 20 and about 40 years old, or between about 40 and about 90 years old). A particular class of subjects, *e.g.,* patients that can benefit from a method of the present disclosure is subjects, *e.g.,* patients over the age of 40.

As used herein, the term "tissue" refers to a group of cells that group together as a functional unit. More than one type of cell can be found in a single tissue. Different types of tissue may consist of different types of cells (*e.g.,* hepatocytes, alveolar cells or blood cells), but also can correspond to tissue from different organisms (mother vs. fetus) or to healthy cells vs. tumor cells. The term "tissue" can generally refer to any group of cells found in the human body (*e.g.,* heart tissue, lung tissue, kidney tissue, nasopharyngeal tissue, oropharyngeal tissue). In some aspects, the term "tissue" or "tissue type" can be used to refer to a tissue from which a cell-free nucleic acid originates. In one example, viral nucleic acid fragments can be derived from blood tissue. In another example, viral nucleic acid fragments can be derived from tumor tissue.

As used herein, the term "diagnosis" refers to a determination as to whether a subject is likely affected by a given disease, disorder or dysfunction. The skilled artisan will appreciate that a diagnosis can be made on the basis of one or more diagnostic indicators, *e.g.,* a biomarker, the presence, absence, or amount of which is indicative of the presence or absence of the disease, disorder or dysfunction.

As used herein, the term "prognosis" refers to a prediction of the probable course and outcome of a clinical condition or disease. A prognosis of a patient is usually made by evaluating factors or symptoms of a disease that are indicative of a favorable or unfavorable course or outcome of the disease. It is understood that the term "prognosis" does not necessarily refer to the ability to predict the course or outcome of a condition with 100% accuracy. The skilled artisan will understand that the term "prognosis" refers to an increased probability that a certain course or outcome will occur; that is, that a course or outcome is more likely to occur in a patient exhibiting a given condition, when compared to those individuals not exhibiting the condition.

As used herein, the term "random seed" refers to a number or vector that is used to initialize a pseudo-random number generation. For example, in some embodiments, a value of a random seed can be used as input to a pseudo-random number generator to generate a plurality of values that follow a probability distribution in a pseudo-random manner. Input of a random seed into a pseudo-random number generator will consistently produce the same sequence of values, thus allowing reproducibility of the respective configuration. Further details regarding pseudo-random assignment of values to hyperparameters for generation of pseudo-random hyperparameter configurations are disclosed below (see, *e.g.,* the section entitled "Classifiers and Hyperparameters").

As used interchangeably herein, the term "neuron," "node," "unit," "hidden neuron," "hidden unit," or the like, refers to a unit of a neural network that accepts input and provides an output via an activation function and one or more coefficients (*e.g.,* weights). For example, a hidden neuron can accept one or more inputs from a prior layer and provide an output that serves as an input for a subsequent layer. In some embodiments, a neural network comprises only one output neuron. In some embodiments, a neural network comprises a plurality of output neurons are possible. Generally, the output is a prediction value, such as a probability, a binary determination (*e.g.,* a presence or absence, a positive or negative result), and/or a label (*e.g.,* a classification) of a condition of interest such as an infectious disease state. For single-class classification models, the output can be a probability of an input dataset (*e.g.,* of a biological sample and/or subject) having a condition (*e.g.,* a label or class). For multi-class classification models, multiple prediction values can be generated, with each prediction value indicating the probability of an input dataset for each condition of interest.

As used herein, the term "parameter" refers to any coefficient or, similarly, any value of an internal or external element (*e.g.,* a weight and/or a hyperparameter) in an algorithm, model, and/or classifier that can affect (*e.g.,* modify, tailor, and/or adjust) one or more inputs, outputs, and/or functions in the algorithm, model, and/or classifier. For example, in some embodiments, a parameter refers to any coefficient, weight, and/or hyperparameter that can be used to control, modify, tailor, and/or adjust the behavior, learning and/or performance of a model. In some instances, a parameter is used to increase or decrease the influence of an input (*e.g.,* a feature) to a model. In some instances, a parameter is used to increase or decrease the influence of a node (*e.g.,* of a neural network), where the node comprises one or more activation functions. Assignment of parameters to specific inputs, outputs, and/or functions is not limited to any one paradigm for a given model but can be used in any suitable model architecture for a desired performance. In some embodiments, a parameter has a fixed value. In some embodiments, a value of a parameter is manually and/or automatically adjustable. In some embodiments, a value of a parameter is modified by a validation and/or training process for a model (*e.g.,* by error minimization and/or backpropagation methods, as described elsewhere herein).

As used herein, the term "initial classifier" refers to a machine learning model or algorithm that is pseudo-randomly assigned values for each respective parameter in a plurality of parameters associated with the model or algorithm. In some embodiments, each pseudo-randomly assigned parameter in the plurality of parameters is a pseudo-randomly assigned hyperparameter. Generally, initial classifiers are untrained or partially untrained (*e.g.,* have not been trained on a training dataset). As used herein, the term "downsampling" refers to reducing a plurality of elements to a subset of the plurality of elements. For instance, a set of initial classifiers can be downsampled by selecting a subset of the set of initial classifiers and removing the unselected classifiers from the set of initial classifiers. In some embodiments, the proportion of the plurality of elements (*e.g.,* initial classifiers) that are retained in (and/or alternately, removed from) the plurality of elements is determined by a downsampling rate. For example, a downsampling rate of 2 indicates that the number of elements in the set will be reduced by a factor of 2 after downsampling (*e.g.,* half of the elements will remain in the set after downsampling). Similarly, a downsampling rate of 3 indicates that the number of elements in the set will be reduced by a factor of 3 after downsampling (*e.g.,* one-third of the elements will remain in the set after downsampling). In some embodiments, the downsampling rate is a parameter. In some embodiments, the downsampling rate is predefined (*e.g.,* by a user and/or practitioner). In some embodiments, the downsampling rate is randomly or pseudo-randomly generated. In some embodiments, the downsampling rate is determined from an optimization or tuning method (*e.g.,* hyperparameter selection).

The terminology used herein is for the purpose of describing particular cases only and is not intended to be limiting. As used herein, the singular forms "a," "an" and "the" are intended to include the plural forms as well, unless the context clearly indicates otherwise. Furthermore, to the extent that the terms "including," "includes," "having," "has," "with," or variants thereof are used in either the detailed description and/or the claims, such terms are intended to be inclusive in a manner similar to the term "comprising."

Several aspects are described below with reference to example applications for illustration. It should be understood that numerous specific details, relationships, and methods are set forth to provide a full understanding of the features described herein. One having ordinary skill in the relevant art, however, will readily recognize that the features described herein can be practiced without one or more of the specific details or with other methods. The features described herein are not limited by the illustrated ordering of acts or events, as some acts can occur in different orders and/or concurrently with other acts or events. Furthermore, not all illustrated acts or events are required to implement a methodology in accordance with the features described herein.

*Exemplary System Embodiments*

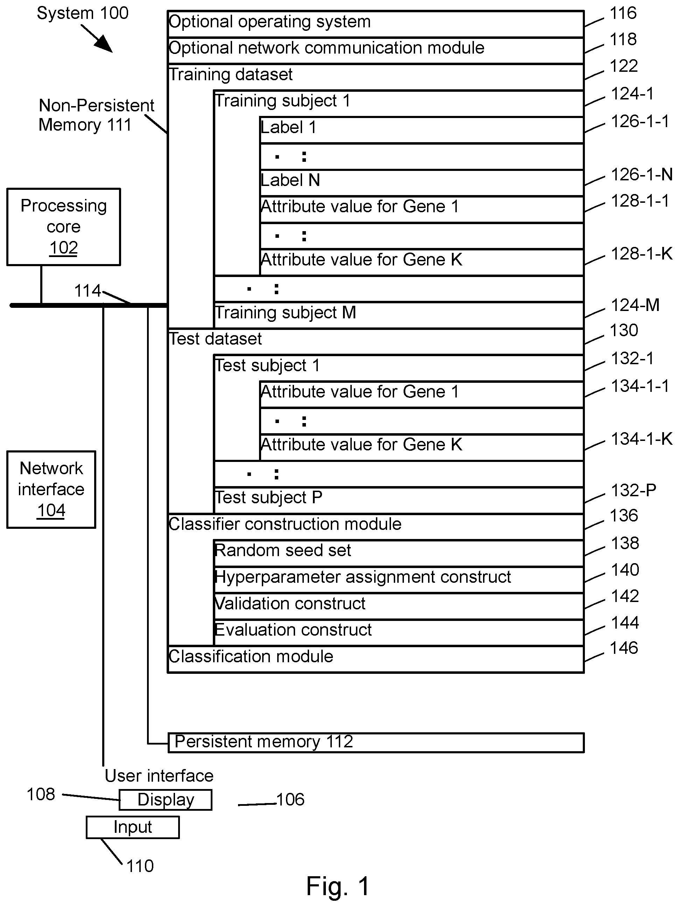

Details of an exemplary system are now described in conjunction with Figure 1. Figure 1 is a block diagram illustrating a system 100 in accordance with some implementations. The device 100 in some implementations includes at least one or more processing units CPU(s) 102 (also referred to as processors), one or more network interfaces 104, a display 106 having a user interface 108, an input device 110, a memory 111, and one or more communication buses 114 for interconnecting these components. The one or more communication buses 114 optionally include circuitry (sometimes called a chipset) that interconnects and controls communications between system components. The memory 111 may be a non-persistent memory, a persistent memory 112, or any combination thereof. The non-persistent memory typically includes high-speed random access memory, such as DRAM, SRAM, DDR RAM, ROM, EEPROM, flash memory, whereas the persistent memory typically includes CD-ROM, digital versatile disks (DVD) or other optical storage, magnetic cassettes, magnetic tape, magnetic disk storage or other magnetic storage devices, magnetic disk storage devices, optical disk storage devices, flash memory devices, or other non-volatile solid-state storage devices. Regardless of its specific implementation, memory 111 comprises at least one non-transitory computer-readable storage medium, and it stores thereon computer-executable executable instructions which can be in the form of programs, modules, and data structures.

In some embodiments, as shown in Figure 1, the memory 111 stores the following:

- an optional operating system 116, which includes procedures for handling various basic system services and for performing hardware-dependent tasks;

- an optional network communication module (or instructions) 118 for connecting the system 100 with other devices and/or to a communication network;

- a training dataset 122 comprising, for each respective training subject 124 in a plurality of training subjects (*e.g.,* 124-1,... 124-M), a corresponding label 126 (*e.g.,* 126-1-1,...,126-1-N) for the infectious disease state of the respective training subject and a respective attribute value 128 for each corresponding gene in a plurality of genes (*e.g.,* 128-1-1,...,128-1-K) obtained from a biological sample of the respective training subject;

- an optional test dataset 130 comprising, for each respective test subject 132 in a plurality of training subjects (*e.g.,* 132-1,... 132-P), a respective attribute value 134 for each corresponding gene in a plurality of genes (*e.g.,* 134-1-1,...,134-1-K) obtained from a biological sample of the respective test subject;

- a classifier construction module 136 comprising:

  - a random seed set 138, each random seed in the random seed set corresponding to a respective instance of an outer loop characterized by a respective downsampling rate and a respective maximum iteration rate;

- a hyperparameter assignment construct 140 that uses the random seed to pseudo-randomly assign values to each respective hyperparameter in a plurality of hyperparameters for each respective initial classifier in a plurality of initial classifiers;

- a validation construct 142 that performs classifier training for a given number of iterations, in the K-fold cross-validation context, for each respective bin in a plurality of bins of initial classifiers, comprising refining each initial classifier in the respective bin against the training dataset using the assigned hyperparameter values for the respective initial classifier; and

- an evaluation construct 144 that determines, based on the K-fold cross-validation, a corresponding evaluation score for each initial classifier in the respective bin and removes a subset of initial classifiers from the respective bin in accordance with the downsampling rate and the corresponding evaluation score;

- wherein the steps performed by the validation construct 142 and the evaluation construct 144 are optionally repeated, for each round in a respective total number of rounds for each respective bin in the plurality of bins, after a downsampling of the set of initial classifiers in the respective bin, and wherein, for each respective round in the total number of rounds, the number of iterations performed by the validation construct 142 is increased from the previous round; and

- a classification module 146 comprising an ensemble classifier including, for each respective seed in the random seed set 138, a corresponding classifier that has the best corresponding evaluation score as representative of the respective seed.

In various implementations, one or more of the above-identified elements are stored in one or more of the previously mentioned memory devices and correspond to a set of instructions for performing various methods described herein. The above-identified modules, data, or programs *(e.g.,* sets of instructions) need not be implemented as separate software programs, procedures, datasets, or modules, and thus various subsets of these modules and data may be combined or otherwise re-arranged in various implementations. In some implementations, the memory 111 optionally stores a subset of the modules and data structures identified above. Furthermore, in some embodiments, the memory stores additional modules and data structures not described above. In some embodiments, one or more of the above-identified elements is stored in a computer system, other than that of the system 100, that is addressable by the system 100 so that the system 100 may retrieve all or a portion of such data when needed.

Although Figure 1 depicts a "system 100," the figure is intended more as a functional description of the various features which may be present in computer systems than as a structural schematic of the implementations described herein. In practice, and as recognized by those of ordinary skill in the art, items shown separately could be combined and some items can be separate. Moreover, although Figure 1 depicts certain data and modules in the memory 111 (which can be non-persistent or persistent memory), it should be appreciated that these data and modules, or portion(s) thereof, may be stored in more than one memory.

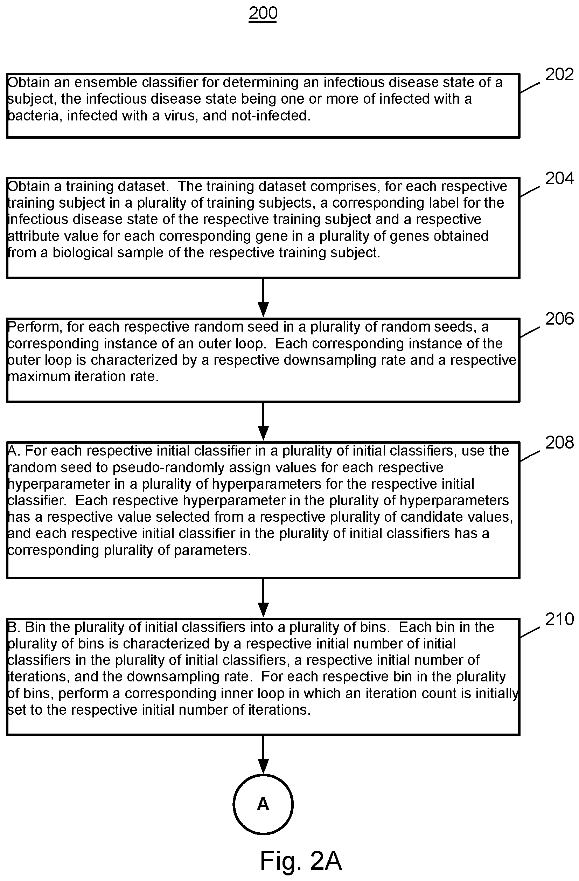

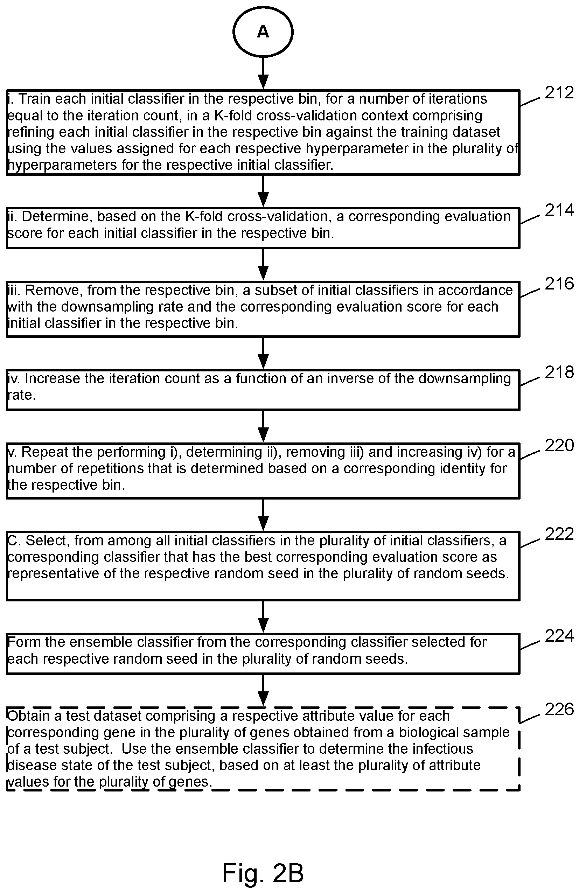

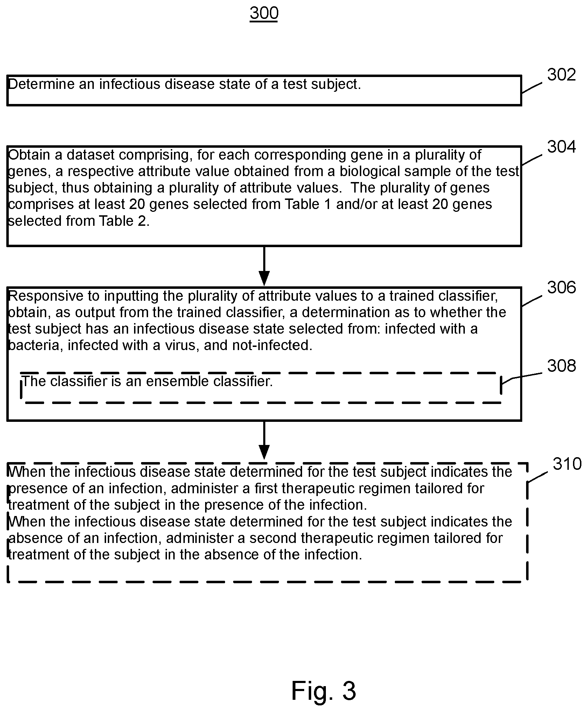

While a system in accordance with the present disclosure has been disclosed with reference to Figure 1, methods in accordance with the present disclosure are now detailed with reference to Figures 2A, 2B, and 3. Any of the methods in accordance with embodiments of the present disclosure can make use of any of the assays, algorithms, techniques, biomarkers, compositions, kits, and/or any combinations thereof, disclosed in U.S. Patent Application No. US16/096,261, Publication No. US20190144943, filed June 5, 2017, the content of which is hereby incorporated herein by reference in its entirety, in order to distinguish between infectious disease states *(e.g.,* bacterial infections, viral infections, and/or non-infections).

*Specific Embodiments of the Disclosure*

Referring to Block 202 of Figure 2A, one aspect of the present disclosure provides a method 200 for obtaining an ensemble classifier for determining an infectious disease state of a subject, the infectious disease state being one or more of infected with a bacteria, infected with a virus, and not-infected, at a computer system comprising at least one processor and a memory storing at least one program for execution by the at least one processor.

*Subjects and Samples*

Referring to Block 204, the method comprises obtaining a training dataset *(e.g.,* a training dataset 122, as illustrated in Figure 1). The training dataset comprises, in electronic form, for each respective training subject *(e.g.,* training subjects 124 in training dataset 122) in a plurality of training subjects *(e.g.,* 100 training subjects or more), (i) a corresponding label for the infectious disease state of the respective training subject (*e.g.,* labels 126) and (ii) a respective attribute value for each corresponding gene in a plurality of genes (*e.g.,* attribute values 128) obtained from a biological sample of the respective training subject.

In some embodiments, a training subject is a subject that is used to train an untrained or partially untrained model (*e.g.,* a machine learning algorithm, a neural network, and/or a downstream classifier). For example, in some embodiments, training the untrained or partially untrained model using one or more training subjects comprises inputting one or more datasets (*e.g.,* training datasets) for each respective training subject into the untrained or partially untrained model. In some such embodiments, training the untrained or partially untrained model further comprises inputting a corresponding label (*e.g.,* an infectious disease state and/or a disease condition) for each respective training subject into the model.

In some embodiments, the plurality of training subjects comprises at least 20, at least 30, at least 40, at least 50, at least 60, at least 70, at least 80, at least 90, at least 100, at least 200, at least 300, at least 400, or at least 500 subjects. In some embodiments, the plurality of training subjects comprises at least 100, at least 500, at least 800, at least 1000, at least 2000, at least 3000, at least 4000, at least 5000, at least 6000, at least 7000, at least 8000, at least 9000, at least 10,000, or at least 20,000 subjects. In some embodiments, the plurality of training subjects comprises no more than 20,000, no more than 10,000, no more than 5000, no more than 4000, no more than 3000, no more than 2000, no more than 1000, no more than 900, no more than 800, no more than 700, no more than 600, no more than 500, no more than 400, no more than 300, or no more than 200 subjects. In some embodiments, the plurality of training subjects comprises between 20 and 500, between 100 and 800, between 50 and 1000, between 500 and 2000, between 1000 and 5000, or between 5000 and 10,000 subjects. In some embodiments, the plurality of training subjects falls within another range starting no lower than 20 subjects and ending no higher than 20,000 subjects.

In some embodiments, the biological sample is a blood sample of the respective training subject. In some embodiments, the biological sample comprises blood, whole blood, plasma, serum, urine, cerebrospinal fluid, fecal, saliva, sweat, tears, pleural fluid, pericardial fluid, peritoneal fluid, nasal swabs, nasopharyngeal swabs, or oropharyngeal swabs of the respective training subject.

In some embodiments, the biological sample obtained from the subject is whole blood, buffy coat, plasma, serum, or blood cells (*e.g.,* leukocytes, peripheral blood mononucleated cells (PBMCS), band cells, neutrophils, monocytes, or T cells). In some embodiments, the biological sample is any sample from bodily fluids, tissue or cells that contain the expressed biomarkers. A biological sample can be obtained from a subject by any conventional technique known in the art. For example, blood can be obtained by venipuncture, and solid tissue samples can be obtained by surgical techniques according to methods well known in the art. In some embodiments, the biological sample is processed to extract biological materials (*e.g.,* nucleic acids) in preparation for measurement of biomarkers, using any suitable means known in the art.

In some embodiments, the biological sample is a control sample. As defined above, in some embodiments, a control sample comprises bodily fluid, tissue, or cells that has an infectious disease state other than an infectious disease state of interest. In some embodiments, where the disease state of interest is "infected," then the control sample is not infected, without precluding the possibility that the control sample has a disease condition other than an infection. That is, the control sample is obtained from a normal (*e.g.,* healthy) subject, a non-infected subject (*e.g.,* an individual known to not have a viral infection, bacterial infection, sepsis, or inflammation), and/or a non-infected subject that has a disease condition other than an infectious disease. In some embodiments, where the disease state of interest is "infected with a bacteria," then the control sample is any sample obtained from a tissue or subject that is not infected with a bacteria, without precluding the possibility that the control sample has an infection other than a bacterial infection. Thus, in some such embodiments, the control sample is obtained from a normal (*e.g.,* healthy) subject, a non-infected subject, a non-infected subject that has a disease condition other than an infectious disease, and/or an infected subject that has a type of infection other than a bacterial infection (*e.g.,* a viral infection).

In some embodiments, each respective training subject and/or the biological sample from the respective training subject has an infectious disease state. For example, in some embodiments, the infectious disease state is absence or presence of infection. In some embodiments, the infectious disease state is absence or presence of a type of infection (*e.g.,* bacterial infection and/or viral infection). In some embodiments, the infectious disease state is an identity of an infectious agent (e.g., bacteria, viruses, fungi, protozoa, and/or helminths). In some embodiments, the infectious disease state is absence or presence of sepsis. In some embodiments, the infectious disease state is absence or presence of inflammation. In some embodiments, the infectious disease state is absence or presence of a severity (*e.g.,* a severe disease and/or a non-severe disease). In some embodiments, the infectious disease state is a diagnosis and/or a prognosis.

In some embodiments, the infectious disease state is a likelihood of infection, a likelihood of a type of infection, a likelihood of infection by an infectious agent, a likelihood of sepsis, a likelihood of inflammation, a likelihood of severity, a likelihood of a diagnosis, and/or a likelihood of a prognosis. In some embodiments, the infectious disease state is any of the embodiments described herein, and/or any substitutions, modifications, additions, deletions, and/or combinations thereof, as will be apparent to one skilled in the art (see, Definitions, "Infectious Disease States," above).

Accordingly, in some embodiments, the corresponding label for the infectious disease state of the respective training subject comprises an indication of any one of more of the infectious disease states disclosed herein. In some embodiments, the corresponding label for the infectious disease state further comprises a covariate, where the covariate is one or more features of the subject and/or sample, including sample type, sample processing features, clinical history, and/or subject demographics. In some embodiments, the corresponding label for the infectious disease state of the respective training subject comprises an indication of one or more of: infected with a bacteria, infected with a virus, not-infected, a sepsis status, a severity, an inflammation status, and/or an outcome. In some embodiments, the corresponding label for the infectious disease state further comprises a covariate selected from the group consisting of: a sample type (*e.g.,* whole blood, buffy coat, plasma, serum, or blood cells (*e.g.,* leukocytes)), a sample processing feature, a clinical history, and a subject demographic feature.

In some embodiments, a first subject in the plurality of training subjects has the same or different infectious disease state as a second subject in the plurality of training subjects. In some embodiments, the plurality of training subjects has at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 30, at least 40, at least 50, at least 60, at least 70, at least 80, at least 90, or at least 100 subjects having an infectious disease state of "infected with a bacteria." In some embodiments, the plurality of training subjects has at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 30, at least 40, at least 50, at least 60, at least 70, at least 80, at least 90, or at least 100 subjects having an infectious disease state of "infected with a virus." In some embodiments, the plurality of training subjects has at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 30, at least 40, at least 50, at least 60, at least 70, at least 80, at least 90, or at least 100 subjects having an infectious disease state of "not infected."

*Biomarkers*

In some embodiments, the plurality of genes comprises at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, at least 45, at least 46, at least 47, or at least 48 genes. In some embodiments, the plurality of genes comprises at least 10, at least 20, at least 30, at least 40, at least 50, at least 60, at least 70, at least 80, at least 90, or at least 100 genes. In some embodiments, the plurality of genes comprises at least 100, at least 200, at least 300, at least 400, at least 500, at least 600, at least 700, at least 800, at least 900, or at least 1000 genes.

In some embodiments, the plurality of genes comprises no more than 2000, no more than 1000, no more than 900, no more than 800, no more than 700, no more than 600, no more than 500, no more than 400, no more than 300, no more than 200, no more than 100, no more than 90, no more than 80, no more than 70, no more than 60, no more than 50, no more than 40, or no more than 30 genes. In some embodiments, the plurality of genes comprises between 5 and 10, between 2 and 50, between 10 and 200, between 20 and 500, between 10 and 80, between 30 and 100, between 100 and 1000, between 300 and 2000, or between 1000 and 2000 genes. In some embodiments, the plurality of genes includes between 15 genes and 50 genes. In some embodiments, the plurality of genes includes between 15 genes and 40 genes. In some embodiments, the plurality of genes includes between 15 genes and 30 genes. In some embodiments, the plurality of genes includes between 20 genes and 50 genes. In some embodiments, the plurality of genes includes between 20 genes and 40 genes. In some embodiments, the plurality of genes includes between 20 genes and 30 genes. In some embodiments, the plurality of genes includes between 25 genes and 50 genes. In some embodiments, the plurality of genes includes between 25 genes and 40 genes. In some embodiments, the plurality of genes includes between 25 genes and 35 genes. In some embodiments, the plurality of genes includes between 25 genes and 30 genes. In some embodiments, the plurality of genes falls within another range starting no lower than 10 genes and ending no higher than 2000 genes.

Biomarkers of the aspects provided herein may comprise one or more of ARG1, CTLA4, FURIN, HLA-DMB, KCNJ2, MTCH1, PSMB9, SMARCD3, BATF, CTSB, GADD45A, HLA-DPB1, KIAA1370, OASL, RAPGEF1, TGFBI, C3AR1, CTSL1, GNA15, ICAM1, LAX1, OLFM4, RELB, TMEM19, C9orf95, DDX6, HAL, IFI27, LCN2, PDE4B, RGS1, TNIP1, CD163, DEFA4, HIF1A, ISG15, LTF, PER1, S100A12, ZBTB33, CEACAM1, FCER1A, HK3, JUP, LY86, PLEKH01, SAMSN1, and ZDHHC19 (shown in Table 1).

Biomarkers of the aspects provided herein may comprise one or more of ARG1, CTSB, HK3, KIAA1370, PSMB9, BATF, CTSL1, HLA-DMB, LY86, RAPGEF1, C3AR1, DEFA4, IFI27, OASL, S100A12, C9orf95, FURIN, ISG15, OLFM4, TGFBI, CD163, GADD45A, JUP, PDE4B, ZDHHC19, CEACAM1, GNA15, KCNJ2, and PER1 (shown in Table 2).

Biomarkers of the aspects provided herein may comprise one or more of ARG1, DDX6, HIF1A, JUP, PER1, SMARCD3, BATF, DEFA4, HK3, KCNJ2, PLEKH01, TCN1, C3AR1, FAM89A, HLA-DMB, KIAA1370, PSMB9, TDRD9, C9orf95, FCER1A, HLA-DPB1, LAX1, RAPGEF1, TGFBI, CD63, FURIN, ICAM1, LCN2, RELB, TMEM19, CD163, GADD45A, IFI27, LTF, RETN, TNIP1, CEACAM1, GNA15, IFI44, LY86, RGS1, XAF1, CLEC5A, GNLY, IFI44L, MTCH1, RSAD2, ZBTB33, CTLA4, HAL, IFI6, OASL, S100A12, ZDHHC19, CTSB, HERC5, IL1R2, OLFM4, SAMSN1, CTSL1, HERC6, ISG15, PDE4B, and SIGLEC1 (shown in Table 9).

In some embodiments, the plurality of genes comprises at least 10 genes selected from Table 1. In some embodiments, the plurality of genes comprises at least 10 genes selected from Table 2. In some embodiments, the plurality of genes comprises at least 10 genes selected from Table 9. In some embodiments, the plurality of genes comprises at least 20 genes selected from Table 1. In some embodiments, the plurality of genes comprises at least 20 genes selected from Table 2. In some embodiments, the plurality of genes comprises at least 20 genes selected from Table 9. In some embodiments, the plurality of genes comprises at least 29 genes selected from Table 1. In some embodiments, the plurality of genes comprises at least 29 genes selected from Table 2. In some embodiments, the plurality of genes comprises at least 29 genes selected from Table 9. In some embodiments, the plurality of genes comprises at least 2, at least 3, at least 4, at least 5, at least 6, at least 7, at least 8, at least 9, at least 10, at least 20, at least 30, at least 40, at least 50, at least 75, at least 100, or more genes selected from Table 8, as described below in the section entitled "Additional Biomarkers." In some embodiments, the plurality of genes comprises any number of genes selected from Table 8, as described below in the section entitled "Additional Biomarkers."

In some embodiments, the plurality of genes comprises at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, at least 45, at least 46, at least 47, or at least 48 genes selected from Table 1.

In some embodiments, the plurality of genes comprises at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, or at least 29 genes selected from Table 2.

In some embodiments, the plurality of genes comprises at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, at least 45, at least 46, at least 47, at least 48, at least 49, at least 50, at least 51, at least 52, at least 53, at least 54, at least 55, at least 56, at least 57, at least 58, at least 59, at least 60, at least 61, at least 62, at least 63, or at least 64 genes selected from Table 9.

In some embodiments, all of the genes are selected from Table 1. That is, in some embodiments, the plurality of genes consists of 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, or 48 genes selected from Table 1. In some embodiments, the plurality of genes consists of from 5 to 20, from 10 to 30, from 20 to 40, from 15 to 48, or from 10 to 48 genes selected from Table 1. In some embodiments, the plurality of genes falls within another range starting no lower than 5 genes and ending no higher than 48 genes from Table 1.

In some embodiments, all of the genes are selected from Table 2. That is, in some embodiments, the plurality of genes consists of 2, 3, 4, 5, 6, 7, 8, 9, 10, 11, 12, 13, 14, 15, 16, 17, 18, 19, 20, 21, 22, 23, 24, 25, 26, 27, 28, or 29 genes selected from Table 2. In some embodiments, the plurality of genes consists of from 10 to 15, from 10 to 25, from 5 to 20, from 10 to 29, or from 15 to 29 genes selected from Table 2. In some embodiments, the plurality of genes falls within another range starting no lower than 5 genes and ending no higher than 29 genes from Table 2.

In some embodiments, all of the genes are selected from Table 9. That is, in some embodiments, the plurality of genes consists of 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, or 64 genes selected from Table 9. In some embodiments, the plurality of genes consists of from 5 to 20, from 10 to 30, from 20 to 40, from 30 to 50, or from 40 to 60 genes selected from Table 9. In some embodiments, the plurality of genes falls within another range starting no lower than 5 genes and ending no higher than 64 genes from Table 9.

Table 1. Genes for Determining Infectious Disease States

| ARG1 | CTLA4 | FURIN | HLA-DMB | KCNJ2 | MTCH1 | PSMB9 | SMARCD3 |
|---|---|---|---|---|---|---|---|
| BATF | CTSB | GADD45A | HLA-DPB1 | KIAA1370 | OASL | RAPGEF1 | TGFBI |
| C3AR1 | CTSL1 | GNA15 | ICAM1 | LAX1 | OLFM4 | RELB | TMEM19 |
| C9orf95 | DDX6 | HAL | IFI27 | LCN2 | PDE4B | RGSI | TNIP1 |

(continued)

| CD163 | DEFA4 | HIF1A | ISG15 | LTF | PERI | S100A12 | ZBTB33 |
| CEACAM1 | FCER1A | HK3 | JUP | LY86 | PLEKH01 | SAMSN1 | ZDHHC19 |

Table 2. Genes for Determining Infectious Disease States

| ARG1 | CTSB | HK3 | KIAA1370 | PSMB9 |
| BATF | CTSL1 | HLA-DMB | LY86 | RAPGEF1 |
| C3AR1 | DEFA4 | IFI27 | OASL | S100A12 |
| C9orf95 | FURIN | ISG15 | OLFM4 | TGFBI |
| CD163 | GADD45A | JUP | PDE4B | ZDHHC19 |
| CEACAM1 | | KCNJ2 | PER1 | |

Table 9. Genes for Determining Infectious Disease States

| ARG1 | DDX6 | HIF1A | JUP | PER1 | SMARCD3 |
| BATF | DEFA4 | HK3 | KCNJ2 | PLEKH01 | TCN1 |
| C3AR1 | FAM89A | HLA-DMB | KIAA1370 | PSMB9 | TDRD9 |
| C9orf95 | FCERIA | HLA-DPB1 | LAX1 | RAPGEF1 | TGFBI |
| CD63 | FURIN | ICAM1 | LCN2 | RELB | TMEM19 |
| CD163 | 3ADD45A | IFI27 | LTF | RETN | TNIP1 |
| CEACAM1 | GNA15 | IFI44 | LY86 | RGS1 | XAF1 |
| CLEC5A | GNLY | IFI44L | MTCH1 | RSAD2 | ZBTB33 |
| CTLA4 | HAL | IFI6 | OASL | S100A12 | ZDHHC19 |
| CTSB | HERC5 | IL1R2 | OLFM4 | SAMSN1 | |
| CTSL1 | HERC6 | ISG15 | PDE4B | SIGLEC1 | |

Additional details on Table 1 and Table 2, including methods of selecting genes for inclusion in Tables 1 and 2, are further described below in the Examples (see, Examples 2 and 3).

In some embodiments, each gene in the plurality of genes is selected for use in a biomarker panel (*e.g.,* via detection of an mRNA transcript for the gene). In some embodiments, the plurality of genes is a panel of genes selected for use in a biomarker panel (*e.g.,* via detection of mRNA transcripts for the panel of genes).

In some embodiments, biomarkers are target nucleic acid sequences or genes. In some embodiments, biomarkers include host and/or pathogen targets (*e.g.,* bacterial, viral, fungal, and/or parasitic). In some embodiments, biomarkers include one or more targets obtained from published lists of nucleic acid and/or amino acid target sequences. In some embodiments, biomarkers include nucleic acid and/or amino acid target sequences deposited for further study in public databases such as NIH Gene Expression Omnibus (GEO) and EBI ArrayExpress. In some embodiments, biomarkers include publicly and/or commercially available gene sets. In some embodiments, biomarkers include gene panels designed for specific disease conditions (*e.g.,* bacterial, viral, fungal, and/or parasitic infections, inflammation, immunology, and/or sepsis). In some embodiments, a biomarker is any of the embodiments described herein, and/or any substitutions, modifications, additions, deletions, and/or combinations thereof, as will be apparent to one skilled in the art (see, Definitions, "Biomarkers," above).

In some embodiments, a panel of biomarkers is used for diagnosis of an infection. For example, in some embodiments, biomarker panels of any size are suitable for use in the presently disclosed systems and methods. In some embodiments, a biomarker panel includes at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, at least 45, at least 46, at least 47, or at least 48 biomarkers. In some embodiments, a biomarker panel includes at least 10, at least 20, at least 30, at least 40, at least 50, at least 60, at least 70, at least 80, at least 90, or at least 100 biomarkers. In some embodiments, a biomarker panel includes at least 100, at least 200, at least 300, at least 400, at least 500, at least 600, at least 700, at least 800, at least 900, or at least 1000 biomarkers.

In some embodiments, a biomarker panel includes no more than 2000, no more than 1000, no more than 900, no more than 800, no more than 700, no more than 600, no more than 500, no more than 400, no more than 300, no more than 200, or no more than 100 biomarkers. In some embodiments, a biomarker panel includes no more than 100, no more than 90, no more than 80, no more than 70, no more than 60, no more than 50, no more than 40, no more than 30, or no more than 20 biomarkers. In some embodiments, a biomarker panel includes between 5 and 10, between 2 and 50, between 10 and 200, between 20 and 500, between 10 and 80, between 30 and 100, between 100 and 1000, between 300 and 2000, or between 1000 and 2000 biomarkers. In some embodiments, a biomarker panel falls within another range starting no lower than 10 biomarkers and ending no higher than 2000 biomarkers. Although, in some instances, smaller biomarker panels are generally more economical, larger biomarker panels (*e.g.,* greater than 30 biomarkers) may have the advantage of providing more detailed information and can also be used in the practice of the invention.

In some embodiments, the plurality of genes comprises one or more genes selected for detection of biomarkers (*e.g.,* mRNA transcripts for the one or more genes) specific to viral infections, bacterial infections, and/or non-infections, as described herein, in combination with one or more additional biomarkers that are capable of determining (*e.g.,* detecting, identifying, and/or distinguishing) one or more additional infectious disease states (*e.g.,* sepsis, inflammation, severity, *etc.*). For example, the one or more additional biomarkers can be used to distinguish whether inflammation in a subject is caused by an infection or a noninfectious source of inflammation (*e.g.,* traumatic injury, surgery, autoimmune disease, thrombosis, or systemic inflammatory response syndrome (SIRS)). In some embodiments, a first set of biomarkers is used to determine whether the acute inflammation is caused by an infectious or non-infectious source, and if the source of inflammation is an infection, a second set of biomarkers is used to determine whether the infection is a viral infection or a bacterial infection. In some embodiments, the use of specialized sets of biomarkers with different purposes provides information that can be used in downstream applications, such as generating therapy recommendations (*e.g.,* whether a subject will benefit from treatment with either antiviral agents or antibiotics, respectively).

In some embodiments, each gene (*e.g.,* biomarker) in the plurality of genes used for determining an infectious disease state in a subject is selected based on one or more selection criteria. For example, in some embodiments, each gene in the plurality of genes is selected based on a minimum gene expression abundance and/or based on a minimum dynamic range.

In some embodiments, each gene in the plurality of genes has an abundance that satisfies an abundance threshold, where the abundance threshold is determined based on a threshold limit of quantitation (*e.g.,* a limit of quantification (LOQ)) for the respective gene. In some such embodiments, the threshold limit of quantitation is determined, for each respective gene in the plurality of genes, based on one or more corresponding methods of measurement used to obtain the attribute value for the respective gene. For example, as defined below, the LOQ is defined as the lowest total amount of analyte input per assay well that will produce a fluorescent signal with a threshold time that exhibits a target precision and falls within a target range. In some such embodiments, when the attribute value for each gene in the plurality of genes is obtained using LAMP, the threshold limit of quantitation is between 10 and 500 copies per 150 ng total RNA load. In some embodiments, the threshold limit of quantitation is at least 2, at least 5, at least 10, at least 20, at least 30, at least 40, at least 50, at least 60, at least 70, at least 80, at least 90, at least 100, at least 200, at least 300, at least 400, at least 500, at least 600, at least 700, at least 800, at least 900, or at least 1000 copies per 150 ng total RNA load. In some embodiments, the threshold limit of quantitation is no more than 1000, no more than 900, no more than 800, no more than 700, no more than 600, no more than 500, no more than 400, no more than 300, or no more than 200 copies per 150 ng total RNA load.

In some embodiments, each gene in the plurality of genes has a dynamic range that satisfies a dynamic range threshold. In some embodiments, the dynamic range threshold is determined, for each respective gene in the plurality of genes, based on one or more corresponding methods of measurement used to obtain the attribute value for the respective gene. For example, the counts (*e.g.,* measures of abundance) for a respective gene obtained from a first method of measurement can differ from the counts for the respective gene obtained from a second method of measurement. In some embodiments, the dynamic range threshold can be determined either from known assay parameters or from optimization assays. Thus, in some embodiments, when the attribute value for each gene in the plurality of genes is mRNA abundance data, the dynamic range threshold is determined based on a fold difference of abundance values for the respective gene, measured across a plurality of samples obtained from a reference cohort. In some embodiments, the dynamic range of a gene (*e.g.,* a biomarker) is determined as the fold difference between the 95th and 5th percentiles of attribute values (*e.g.,* counts and/or mRNA abundances) for the respective gene, as measured across a plurality of samples. In some such embodiments, the measurement is performed using any method of measuring attribute values (described below, see, "Measurement of Biomarkers"). In some embodiments, the plurality of samples includes any cohort of samples (*e.g.,* reference samples) obtained from healthy and/or diseased subjects, used for optimization of assay parameters. In some embodiments, the dynamic range threshold is between 2-fold and 40-fold. In some embodiments, the dynamic range threshold is at least 2, at least 3, at least 4, at least 5, at least 6, at least 7, at least 8, at least 9, at least 10, at least 15, at least 20, at least 25, at least 30, at least 35, at least 40, at least 45, or at least 50-fold. In some embodiments, the dynamic range threshold is no more than 50, no more than 40, no more than 30, no more than 20, or no more than 10-fold.

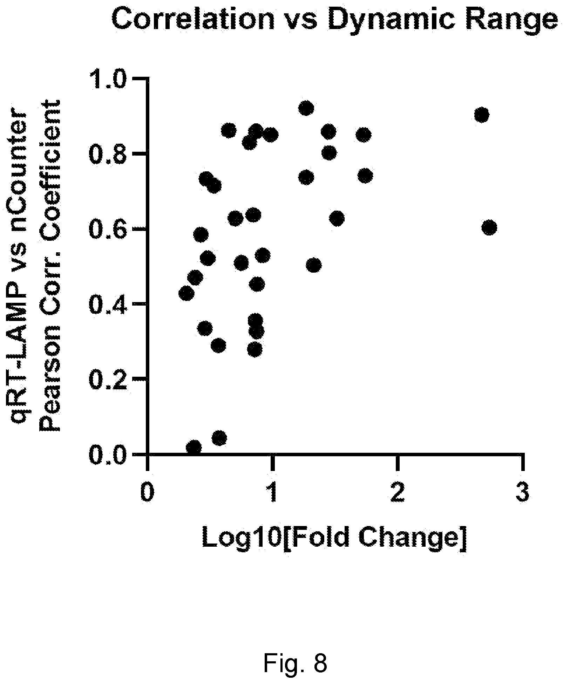

Additional details on selection criteria for genes (*e.g.,* biomarkers) are provided below (see, Examples 2 and 3 and discussion of Figure 8, below).

*Measurement of Biomarkers*

In some embodiments, the attribute value for each corresponding gene in the plurality of genes is a measurement of one or more nucleic acid molecules for the corresponding genes. For example, in some embodiments, the attribute value for each gene is determined from an abundance, a nucleotide sequence, a copy number, a methylation state, a sequence variation (e.g., SNPs, SNVs), and/or any other attribute or characteristic of one or more nucleic acid molecules for the respective gene.

In some embodiments, measuring attribute values for the plurality of genes comprises performing one or more methods including microarray analysis via fluorescence, chemiluminescence, or electric signal detection, polymerase chain reaction (PCR), reverse transcriptase polymerase chain reaction (RT-PCR), digital droplet PCR (ddPCR), solid-state nanopore detection, RNA switch activation, a Northern blot, and/or a serial analysis of gene expression (SAGE).

In some embodiments, the attribute value is a measure of gene expression from mRNA molecules of the respective gene. In some embodiments, the attribute value is absolute abundance or relative abundance. In some embodiments, the attribute value for each corresponding gene in the plurality of genes is mRNA abundance data.

For example, in some embodiments, expression levels of each gene in the plurality of genes are determined by measuring polynucleotide levels of one or more nucleic acid molecules corresponding to the respective gene. The levels of transcripts of specific biomarker genes can be determined from the amount of mRNA, or polynucleotides derived therefom, present in a biological sample. Polynucleotides can be detected and quantitated by a variety of methods including, but not limited to, microarray analysis, polymerase chain reaction (PCR), reverse transcriptase polymerase chain reaction (RT-PCR), Northern blot, serial analysis of gene expression (SAGE), RNA switches, and solid-state nanopore detection. See, *e.g.,* Draghici, Data Analysis Tools for DNA Microarrays, Chapman and Hall/CRC, 2003; Simon et al., Design and Analysis of DNA Microarray Investigations, Springer, 2004; Real-Time PCR: Current Technology and Applications, Logan, Edwards, and Saunders eds., Caister Academic Press, 2009; Bustin A-Z of Quantitative PCR (IUL Biotechnology, No. 5), International University Line, 2004; Velculescu et al. (1995) Science 270: 484-487; Matsumura et al. (2005) Cell. Microbiol. 7: 11-18; Serial Analysis of Gene Expression (SAGE): Methods and Protocols (Methods in Molecular Biology), Humana Press, 2008; each of which is hereby incorporated herein by reference in its entirety.

In some embodiments, attribute values (*e.g.,* mRNA abundance values) are obtained from expressed RNA or a nucleic acid derived therefom *(e.g.,* cDNA or amplified RNA derived from cDNA that incorporates an RNA polymerase promoter) from the biological sample of the respective subject, including naturally occurring nucleic acid molecules, as well as synthetic nucleic acid molecules. Thus, in some embodiments, the one or more nucleic acid molecules corresponding to the respective gene or biomarker comprise RNA, including, but by no means limited to, total cellular RNA, poly(A)+ messenger RNA (mRNA) or a fraction thereof, cytoplasmic mRNA, or RNA transcribed from cDNA (*e.g.,,* cRNA; see, *e.g.,* Linsley & Schelter, U.S. patent application Ser. No. 09/411,074, filed Oct. 4, 1999, or U.S. Pat. No. 5,545,522, 5,891,636, or 5,716,785). Methods for preparing total and poly(A)+ RNA are well known in the art, and are described generally, *e.g.,* in Sambrook, et al., Molecular Cloning: A Laboratory Manual (3rd Edition, 2001). RNA can be extracted from a cell of interest using guanidinium thiocyanate lysis followed by CsCl centrifugation (Chirgwin et al., 1979, Biochemistry 18:5294-5299), a silica gel-based column (*e.g.,* RNeasy (Qiagen, Valencia, Calif.) or StrataPrep (Stratagene, La Jolla, Calif.)), or using phenol and chloroform, as described in Ausubel et al., eds., 1989, Current Protocols In Molecular Biology, Vol. III, Green Publishing Associates, Inc., John Wiley & Sons, Inc., New York, at pp. 13.12.1-13.12.5). Poly(A)+ RNA can be selected, *e.g.,* by selection with oligo-dT cellulose or, alternatively, by oligo-dT primed reverse transcription of total cellular RNA. RNA can be fragmented by methods known in the art, *e.g.,* by incubation with ZnCl2, to generate fragments of RNA.

In some embodiments, total RNA, mRNA, or nucleic acids derived therefom, are isolated from a sample taken from a subject having an infection or inflammation. For example, in some embodiments, total RNA, mRNA, or nucleic acids derived therefom, are isolated from a sample taken from a subject having a bacterial infection and/or a viral infection. In some implementations, a biological sample is further enriched using normalization techniques (*e.g.,* where biomarker polynucleotides are poorly expressed in particular cells) (see, *e.g.,* Bonaldo et al., 1996, Genome Res. 6:791-806).

As described above, in some embodiments, the one or more nucleic acid molecules corresponding to a gene in the plurality of genes can be detectably labeled at one or more nucleotides. Any method known in the art can be used to label the target polynucleotides. In some implementations, this labeling incorporates the label uniformly along the length of the target polynucleotides (*e.g.,* RNA), and in some embodiments, the labeling is carried out at a high degree of efficiency. For example, polynucleotides can be labeled by oligo-dT primed reverse transcription. Random primers (*e.g.,* 9-mers) can be used in reverse transcription to uniformly incorporate labeled nucleotides over the full length of the polynucleotides. Alternatively, or in addition, random primers can be used in conjunction with PCR methods or T7 promoter-based in vitro transcription methods in order to amplify polynucleotides.

The detectable label can be a luminescent label. For example, fluorescent labels, bioluminescent labels, chemiluminescent labels, and colorimetric labels can be used in the practice of the invention. Fluorescent labels that can be used include, but are not limited to, fluorescein, a phosphor, a rhodamine, or a polymethine dye derivative. Chemiluminescent labels that can be used include, but are not limited to, luminol. Additionally, commercially available fluorescent labels including, but not limited to, fluorescent phosphoramidites such as FluorePrime (Amersham Pharmacia, Piscataway, N.J.), Fluoredite (Millipore, Bedford, Mass.), FAM (ABI, Foster City, Calif.), and Cy3 or Cy5 (Amersham Pharmacia, Piscataway, N.J.) can be used. Alternatively, the detectable label can be a radiolabeled nucleotide.

In one embodiment, the one or more nucleic acid molecules corresponding to a gene in the plurality of genes from a biological sample of a first subject having a first infectious disease state (*e.g.,* a training subject having an infection) are labeled differentially from the corresponding nucleic acid molecules of a reference sample (*e.g.,* from a healthy reference cohort and/or a second subject having a second infectious disease state). For instance, the reference sample can comprise polynucleotide molecules from a normal biological sample (*e.g.,* a control sample such as blood or PBMCs from a subject not having an infection or inflammation) or from a reference biological sample, (e.g., blood or PBMCs from a subject having a viral infection or bacterial infection).

In some embodiments, attribute values for the plurality of genes are measured using microarrays. An advantage of microarray analysis is that the expression of each of the genes can be measured simultaneously, and microarrays can be specifically designed to provide a diagnostic expression profile for a particular disease or condition (*e.g.,* sepsis).

Generally, microarrays are prepared by selecting probes which comprise a polynucleotide sequence, and then immobilizing such probes to a solid support or surface. For example, the probes can comprise DNA sequences, RNA sequences, or copolymer sequences of DNA and RNA. The polynucleotide sequences of the probes can also comprise DNA and/or RNA analogues, or combinations thereof. For example, the polynucleotide sequences of the probes can be full or partial fragments of genomic DNA. The polynucleotide sequences of the probes can also be synthesized nucleotide sequences, such as synthetic oligonucleotide sequences. The probe sequences can be synthesized either enzymatically in vivo, enzymatically in vitro (*e.g.,* by PCR), or non-enzymatically in vitro.

Probes used in the methods of the present disclosure are preferably immobilized to a solid support which can be either porous or non-porous. For example, the probes can be polynucleotide sequences which are attached to a nitrocellulose or nylon membrane or filter covalently at either the 3' or the 5' end of the polynucleotide. Such hybridization probes are well known in the art (see, *e.g.,* Sambrook, et al., Molecular Cloning: A Laboratory Manual (3rd Edition, 2001). Alternatively, the solid support or surface can be a glass, silicon, or plastic surface. In one embodiment, hybridization levels are measured to microarrays of probes consisting of a solid phase on the surface of which are immobilized a population of polynucleotides, such as a population of DNA or DNA mimics, or, alternatively, a population of RNA or RNA mimics. The solid phase can be a nonporous or, optionally, a porous material such as a gel, or a porous wafer such as a TipChip (Axela, Ontario, Canada).

As noted above, in some embodiments, the "probe" to which a particular polynucleotide molecule specifically hybridizes contains a complementary polynucleotide sequence (*e.g.,* of a respective target gene in the plurality of genes). The probes of the microarray typically consist of nucleotide sequences of no more than 1,000 nucleotides. In some embodiments, the probes of the array consist of nucleotide sequences of 10 to 1,000 nucleotides. In one embodiment, the nucleotide sequences of the probes are in the range of 10-200 nucleotides in length and are genomic sequences of one species of organism, such that a plurality of different probes is present, with sequences complementary and thus capable of hybridizing to the genome of such a species of organism, sequentially tiled across all or a portion of the genome. In other embodiments, the probes are in the range of 10-30 nucleotides in length, in the range of 10-40 nucleotides in length, in the range of 20-50 nucleotides in length, in the range of 40-80 nucleotides in length, in the range of 50-150 nucleotides in length, in the range of 80-120 nucleotides in length, or are 60 nucleotides in length.

In some embodiments, the probes comprise DNA or DNA "mimics" (*e.g.,* derivatives and analogues) corresponding to a portion of an organism's genome. In some embodiments, the probes of the microarray are complementary RNA or RNA mimics. DNA mimics are polymers composed of subunits capable of specific, Watson-Crick-like hybridization with DNA, or of specific hybridization with RNA. The nucleic acids can be modified at the base moiety, at the sugar moiety, or at the phosphate backbone (*e.g.,* phosphorothioates).

In some embodiments, attribute values for the plurality of genes are measured and/or analyzed by other methods including, but not limited to, northern blotting, nuclease protection assays, RNA fingerprinting, polymerase chain reaction, ligase chain reaction, Qbeta replicase, isothermal amplification method, strand displacement amplification, transcription based amplification systems, nuclease protection (Si nuclease or RNAse protection assays), SAGE as well as methods disclosed in International Publication Nos. WO 88/10315 and WO 89/06700, and International Applications Nos. PCT/US87/00880 and PCT/US89/01025; herein incorporated by reference in their entireties.

A standard Northern blot assay can be used to ascertain an RNA transcript size, identify alternatively spliced RNA transcripts, and the relative amounts of mRNA in a sample, in accordance with conventional Northern hybridization techniques known to those persons of ordinary skill in the art. In Northern blots, RNA samples are first separated by size by electrophoresis in an agarose gel under denaturing conditions. The RNA is then transferred to a membrane, cross-linked, and hybridized with a labeled probe. Nonisotopic or high specific activity radiolabeled probes can be used, including random-primed, nick-translated, or PCR-generated DNA probes, in vitro transcribed RNA probes, and oligonucleotides. Additionally, sequences with only partial homology (e.g., cDNA from a different species or genomic DNA fragments that might contain an exon) can be used as probes. The labeled probe, *e.g.,* a radiolabeled cDNA, either containing the full-length, single stranded DNA or a fragment of that DNA sequence may be at least 20, at least 30, at least 50, or at least 100 consecutive nucleotides in length. The probe can be labeled by any of the many different methods known to those skilled in this art. The labels most commonly employed for these studies are radioactive elements, enzymes, chemicals that fluoresce when exposed to ultraviolet light, and others. A number of fluorescent materials are known and can be utilized as labels. These include, but are not limited to, fluorescein, rhodamine, auramine, Texas Red, AMCA blue and Lucifer Yellow. A particular detecting material is anti-rabbit antibody prepared in goats and conjugated with fluorescein through an isothiocyanate. Proteins can also be labeled with a radioactive element or with an enzyme. The radioactive label can be detected by any of the currently available counting procedures. Isotopes that can be used include, but are not limited to, 3H, 14C, 32P, 35S, 36Cl, 35Cr, 57Co, 58Co, 59Fe, 90Y, 125I, 131I, and 186Re. Enzyme labels are likewise useful and can be detected by any of the presently utilized colorimetric, spectrophotometric, fluorospectrophotometric, amperometric or gasometric techniques. The enzyme is conjugated to the selected particle by reaction with bridging molecules such as carbodiimides, diisocyanates, glutaraldehyde and the like. Any enzymes known to one of skill in the art can be utilized. Examples of such enzymes include, but are not limited to, peroxidase, beta-D-galactosidase, urease, glucose oxidase plus peroxidase and alkaline phosphatase. U.S. Pat. Nos. 3,654,090, 3,850,752, and 4,016,043 are referred to by way of example for their disclosure of alternate labeling material and methods.

Nuclease protection assays (including both ribonuclease protection assays and S1 nuclease assays) can be used to detect and quantitate specific mRNAs. In nuclease protection assays, an antisense probe (labeled with, *e.g.,* radiolabeled or nonisotopic) hybridizes in solution to an RNA sample. Following hybridization, single-stranded, unhybridized probe and RNA are degraded by nucleases. An acrylamide gel is used to separate the remaining protected fragments. Typically, solution hybridization is more efficient than membrane-based hybridization, and it can accommodate up to 100 μg of sample RNA, compared with the 20-30 μg maximum of blot hybridizations.

The ribonuclease protection assay, which is the most common type of nuclease protection assay, requires the use of RNA probes. Oligonucleotides and other single-stranded DNA probes can be used in assays containing Si nuclease. The single-stranded, antisense probe is typically completely homologous to target RNA to prevent cleavage of the probe:target hybrid by nuclease.

Serial Analysis Gene Expression (SAGE) can also be used to determine RNA abundances in a cell sample. See, *e.g.,* Velculescu et al., 1995, Science 270:484-7; Carulli, et al., 1998, Journal of Cellular Biochemistry Supplements 30/31:286-96; herein incorporated by reference in their entireties. SAGE analysis does not require a special device for detection and is one of the preferable analytical methods for simultaneously detecting the expression of a large number of transcription products. First, poly A+ RNA is extracted from cells. Next, the RNA is converted into cDNA using a biotinylated oligo (dT) primer and treated with a four-base recognizing restriction enzyme (Anchoring Enzyme: AE) resulting in AE-treated fragments containing a biotin group at their 3' terminus. Next, the AE-treated fragments are incubated with streptavidin for binding. The bound cDNA is divided into two fractions, and each fraction is then linked to a different double-stranded oligonucleotide adapter (linker) A or B. These linkers are composed of: (1) a protruding single strand portion having a sequence complementary to the sequence of the protruding portion formed by the action of the anchoring enzyme, (2) a 5' nucleotide recognizing sequence of the IIS-type restriction enzyme (cleaves at a predetermined location no more than 20 bp away from the recognition site) serving as a tagging enzyme (TE), and (3) an additional sequence of sufficient length for constructing a PCR-specific primer. The linker-linked cDNA is cleaved using the tagging enzyme, and only the linker-linked cDNA sequence portion remains, which is present in the form of a short-strand sequence tag. Next, pools of short-strand sequence tags from the two different types of linkers are linked to each other, followed by PCR amplification using primers specific to linkers A and B. As a result, the amplification product is obtained as a mixture comprising myriad sequences of two adjacent sequence tags (ditags) bound to linkers A and B. The amplification product is treated with the anchoring enzyme, and the free ditag portions are linked into strands in a standard linkage reaction. The amplification product is then cloned. Determination of the clone's nucleotide sequence can be used to obtain a read-out of consecutive ditags of constant length. The presence of mRNA corresponding to each tag can then be identified from the nucleotide sequence of the clone and information on the sequence tags.

Quantitative reverse transcriptase PCR (qRT-PCR) can also be used to determine the expression profiles of biomarkers (see, *e.g.,* U.S. Patent Application Publication No. 2005/0048542A1; herein incorporated by reference in its entirety). The first step in gene expression profiling by RT-PCR is the reverse transcription of the RNA template into cDNA, followed by its exponential amplification in a PCR reaction. For instance, two commonly used reverse transcriptases that can be used in the presently disclosed methods are avilo myeloblastosis virus reverse transcriptase (AMV-RT) and Moloney murine leukemia virus reverse transcriptase (MLV-RT). The reverse transcription step is typically primed using specific primers, random hexamers, or oligo-dT primers, depending on the circumstances and the goal of expression profiling. For example, extracted RNA can be reverse-transcribed using a GeneAmp RNA PCR kit (Perkin Elmer, Calif., USA), following the manufacturer's instructions. The derived cDNA can then be used as a template in the subsequent PCR reaction.

Although the PCR step can use a variety of thermostable DNA-dependent DNA polymerases, in some embodiments, it employs the Taq DNA polymerase, which has a 5'-3' nuclease activity but lacks a 3'-5' proofreading endonuclease activity. Thus, TAQMAN PCR typically utilizes the 5'-nuclease activity of Taq or Tth polymerase to hydrolyze a hybridization probe bound to its target amplicon, but any enzyme with equivalent 5' nuclease activity can be used. Two oligonucleotide primers are used to generate an amplicon typical of a PCR reaction. A third oligonucleotide, or probe, is designed to detect nucleotide sequence located between the two PCR primers. The probe is non-extendible by Taq DNA polymerase enzyme and is labeled with a reporter fluorescent dye and a quencher fluorescent dye. Any laser-induced emission from the reporter dye is quenched by the quenching dye when the two dyes are located close together as they are on the probe. During the amplification reaction, the Taq DNA polymerase enzyme cleaves the probe in a template-dependent manner. The resultant probe fragments disassociate in solution, and signal from the released reporter dye is free from the quenching effect of the second fluorophore. One molecule of reporter dye is liberated for each new molecule synthesized, and detection of the unquenched reporter dye provides the basis for quantitative interpretation of the data.

TAQMAN RT-PCR can be performed using commercially available equipment, such as, for example, ABI PRISM 7700 sequence detection system (Perkin-Elmer-Applied Biosystems, Foster City, Calif., USA), or Lightcycler (Roche Molecular Biochemicals, Mannheim, Germany). Alternatives include, but are not limited to, sample-to-answer point-of-need devices such as cobas Liat (Roche Molecular Diagnostics, Pleasanton, Calif., USA) or GeneXpert systems (Cepheid, Sunnyvale, Calif., USA). One of ordinary skill will appreciate that the invention is not limited to the listed devices, and that other devices can be used for TAQMAN-PCR. In a preferred embodiment, the 5' nuclease procedure is run on a real-time quantitative PCR device such as the ABI PRISM 7700 sequence detection system. The system consists of a thermocycler, laser, charge-coupled device (CCD), camera and computer. The system includes software for running the instrument and for analyzing the data. 5'-Nuclease assay data are initially expressed as Ct, or the threshold cycle. Fluorescence values are recorded during every cycle and represent the amount of product amplified to that point in the amplification reaction. The point when the fluorescent signal is first recorded as statistically significant is the threshold cycle (Ct). Alternatives to standard thermal cycling include, but are not limited to, amplification by continuous thermal gradient, or isothermal amplification with endpoint detection and other known devices to those of ordinary skill. To minimize errors and the effect of sample-to-sample variation, RT-PCR can be performed using an internal standard. The ideal internal standard is expressed at a constant level among different tissues and is unaffected by the experimental treatment. In some implementations, RNAs used to normalize patterns of gene expression include mRNAs for the housekeeping genes glyceraldehyde-3-phosphate-dehydrogenase (GAPDH) and beta-actin.

A more recent variation of the RT-PCR technique is the real time quantitative PCR, which measures PCR product accumulation through a dual-labeled fluorigenic probe (*e.g.,* TAQMAN probe). Real time PCR is compatible both with quantitative competitive PCR, where internal competitor for each target sequence is used for normalization, and with quantitative comparative PCR using a normalization gene contained within the sample, or a housekeeping gene for RT-PCR. For further details see, *e.g.,* Held et al., Genome Research 6:986-994 (1996).

An alternative is the detection of PCR products using digital counting methods. These include, but are not limited to, digital droplet PCR and solid-state nanopore detection of PCR products. In these methods the counts of the products of interests can be normalized to the counts of housekeeping genes. Other methods of PCR detection known to those of ordinary skill can be used, and the invention is not limited to the listed methods.

Other methods for measuring attribute values for genes and/or biomarkers, including microarray analysis, polymerase chain reaction (PCR), reverse transcriptase polymerase chain reaction (RT-PCR), digital droplet PCR (ddPCR), solid-state nanopore detection, RNA switch activation, a Northern blot, and/or a serial analysis of gene expression (SAGE), are further described in U.S. Patent Application No. US16/096,261, Publication No. US20190144943A1, filed on June 5, 2017; PCT Application No. US2016/022233, Publication No. WO2016145426A1, filed on March 12, 2016; PCT Application No. US2017/036003, Publication No. WO2017214061A1, filed on June 5, 2017; PCT Application No. US2017/029468, Publication No. WO2018004806A1, filed on April 25, 2017; and PCT Application No. US2019/015462, Publication No. WO2019168622A1, filed on January 28, 2019, each of which is hereby incorporated herein by reference in its entirety. Methods for measuring attribute values further include any of the embodiments described herein, and/or any substitutions, modifications, additions, deletions, and/or combinations thereof, as will be apparent to one skilled in the art.

In some embodiments, the attribute value for each corresponding gene in the plurality of genes is obtained using real-time quantitative isothermal amplification on one or more nucleic acid molecules in the biological sample of the respective training subject.

In some embodiments, the quantitative real-time isothermal amplification comprises strand displacement amplification (SDA), transcription mediated amplification (IMA), nucleic acid sequence based amplification (NASBA), recombinase polymerase amplification (RPA), rolling circle amplification (RCA), ramification amplification, helicase-dependent isothermal DNA amplification (HD A), nicking enzyme amplification reaction (NEAR) and loop mediated isothermal amplification (LAMP) (see, *e.g.,* Notomi et al., (2000) Nucleic Acids Research, 28(12)E63, incorporated herein by reference).

In some embodiments, the real-time quantitative isothermal amplification is real-time quantitative loop-mediated isothermal amplification (LAMP).

For example, LAMP offers selectivity and employs a polymerase and a set of specially designed primers that recognize distinct sequences in the target nucleic acid (see, *e.g.,* Nixon et al., (2014) Bimolecular Detection and Quantitation, 2:4-10; Schuler et al., (2016) Anal Methods., 8:2750-2755; and Schoepp et al., (2017) Set. Transl. Med. 9:eaal3693). Unlike methods for PCR, LAMP performs amplification of target nucleic acid molecules at a constant temperature (*e.g.*, 60-65 °C) using multiple inner and outer primers and a polymerase having strand displacement activity. In some instances, an inner primer pair containing a nucleic acid sequence complementary to a portion of die sense and antisense strands of the target nucleic acid initiate LAMP. Following strand displacement synthesis by the inner primers, strand displacement synthesis primed by an outer primer pair can cause release of a single-stranded amplicon. The single-stranded amplicon can serve as a template for further synthesis primed by a second inner and second outer primer that hybridize to the other end of the target nucleic acid and produce a stem-loop nucleic acid structure. In subsequent LAMP cycling, one inner primer hybridizes to the loop on the product and initiates displacement and target nucleic acid synthesis, yielding the original stem-loop product and a new stem-loop product with a stem twice as long. Additionally, the 3' terminus of an amplicon loop structure serves as initiation site for self-templating strand synthesis, yielding a hairpin-like amplicon that forms an additional loop structure to prime subsequent rounds of self-templated amplification. The amplification continues with accumulation of many copies of the target nucleic acid. The final products of the LAMP process are stem-loop nucleic acids with concatenated repeats of the target nucleic acid in cauliflower-like structures with multiple loops formed by annealing between alternately inverted repeats of a target nucleic acid sequence in the same strand.

In some embodiments, the isothermal amplification assay comprises a digital reverse-transcription loop-mediate isothermal amplification (dRT-LAMP) reaction for quantifying the target nucleic acid. Typically, LAMP assays produce a detectable signal (*e.g.,* fluorescence) during the amplification reaction. In some embodiments, the method comprises detecting and/or quantifying a detectable signal (*e.g.,* fluorescence) produced during the LAMP assay. Any suitable method for detecting and quantifying florescence can be used. In some instances, a device such as Applied Biosystem's QuantStudio can be used to detect and quantify fluorescence from the isothermal amplification assay.

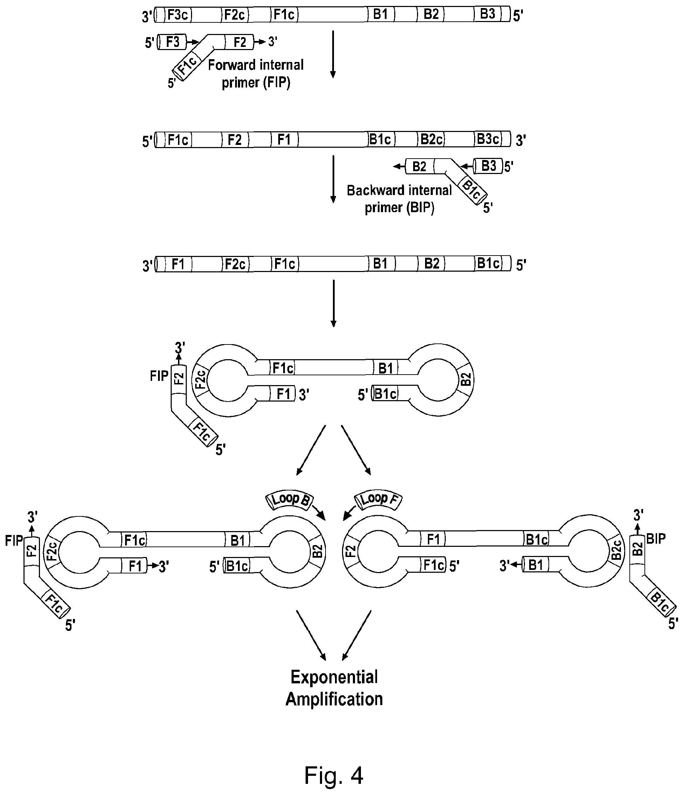

Figure 4 illustrates a schematic mechanism of loop-mediated isothermal amplification. In the first stage of the mechanism, FIP and BIP primers invade a duplex nucleic acid to initiate a primary round replication that generates a copy with a non-uniform 3' terminus; this amplicon is separated from the original template by strand-displacement replication primed by either the F3 or B3 primers. The free amplicon then serves as a template for amplification from the opposing FIP/BIP primer *(e.g.,* a FIP template is copied by a BIP primer or vice versa). Amplicons containing both FIP and BIP primer sequences fold back on themselves as sequences at the termini bind complimentary sequences within the transcript, creating a dumbbell structure. This dumbbell amplicon serves as the primary template for exponential amplification, enabling additional rounds of replication primed at 3 sites within the molecule.

In some embodiments, LAMP primers, solutions, and/or other reagents are designed in order to optimize or improve performance, or to tailor assay results to achieve one or more desired outcomes (e.g., linearity and reportable range, performance of synthetic control materials, assay efficiency, limit of quantitation (LOQ), limit of detection (LOD), limit of blank (LOB), analytical precision, *etc.).* Further details on loop-mediated isothermal amplification (LAMP) are provided herein (see, *e.g.*, Examples 2 and 3, below), and in PCT Application No. US2019/051765, Publication No. WO2020061217A1, filed September 18, 2019; and "Loop-Mediated Isothermal Amplification," NEB, available online at neb.com/applications/dna-amplification-pcr-and-qper/isothermal-amplification/loop-mediated-isothermal-amplification-lamp, each of which is hereby incorporated herein by reference in its entirety.

*Selection of Configurations*

As described above, in some embodiments, the present disclosure provides methods for obtaining an ensemble model *(e.g.,* using a classifier construction module 136, as illustrated in Figure 1), by selecting a set of classifiers from a plurality of initial classifiers with pseudo-randomly assigned hyperparameter configurations.

Generally, selection and/or optimization of parameters (e.g., hyperparameters) is used in model building to create models with improved performance in one or more desired tasks *(e.g.,* providing predictive probabilities of infectious disease states based on mRNA abundance data). As used herein, a parameter can refer to an element in a model, or a value thereof *(e.g.,* a coefficient, weight, and/or hyperparameter), that can be used to control, modify, tailor, and/or adjust the behavior, learning and/or performance of a model. In some embodiments, a parameter is a hyperparameter. In some embodiments, a parameter is a fixed value. In some embodiments, a parameter is manually and/or automatically adjustable. In some embodiments, a parameter can be used to control, modify, tailor, and/or adjust one or more functions in the model *(e.g.,* input or output values for one or more activation functions). Classifiers and hyperparameters are further detailed below (see, *e.g.,* the section entitled "Classifiers and Hyperparameters").

In some embodiments, any suitable method for selecting and/or optimizing hyperparameters for classifiers are contemplated. For example, in some embodiments, hyperparameter selection is performed using random search, K-fold cross-validation, leave-one-out, and/or Bayesian optimization methods. Generally, while random search methods have been reported to have superior performance and faster speeds compared to traditional Bayesian optimization methods, random search can also be inefficient (see, Jamieson et al., "Hyperband: A Novel Bandit-Based Approach to Hyperparameter Optimization," available online at arxiv.org/abs/1603.06560, which is hereby incorporated herein by reference in its entirety).

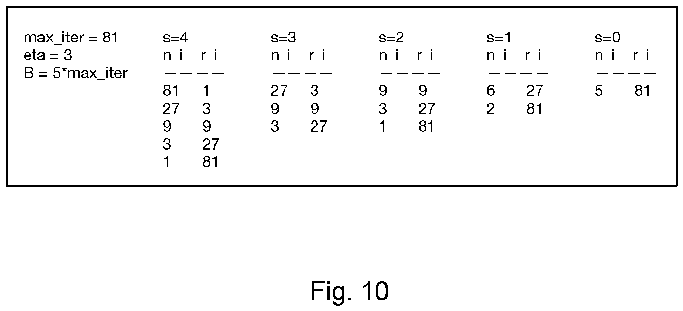

Given the above limitations, selection of hyperparameters can be performed using a Hyperband method. Generally, the Hyperband method provides a faster selection process that also outperforms traditional Bayesian and random search methods. As will be described in more detail herein, the method comprises obtaining a plurality of initial classifiers with pseudo-randomly generated hyperparameter configurations and successively downsampling the number of initial classifiers over sequential rounds of selection. Furthermore, in some embodiments, selection of hyperparameters further comprises successively deeper iterations of validation and evaluation of hyperparameter configurations, using K-fold cross-validation, prior to each round of downsampling. Example methods for hyperparameter selection, *e.g.*, as performed within classifier construction module 136, will be further described with reference to Block 206-224 and Figure 10.

Accordingly, referring to Block 206, the method comprises, for each respective random seed in a plurality of random seeds *(e.g.,* a random seed set 138), performing a corresponding instance of an outer loop, where each corresponding instance of the outer loop is characterized by a respective downsampling rate and a respective maximum iteration rate.

In some embodiments, the downsampling rate determines the rate at which a plurality of initial classifiers (e.g., pseudo-randomly generated hyperparameter configurations) will be reduced during the hyperparameter selection process. For example, a downsampling rate of 2 indicates that the number of initial classifiers will be reduced by a factor of 2 (such that half of the classifiers will remain after each successive round of downsampling). As another example, a downsampling rate of 3 indicates that the number of initial classifiers will be reduced by a factor of 3 (such that one-third of the classifiers will remain after each successive round of downsampling).

In some embodiments, the respective downsampling rate for each corresponding instance of the outer loop is between 1.5 and 6. In some embodiments, the downsampling rate is between 1.2 and 20. In some embodiments, the downsampling rate is between 1.2 and 5, between 2 and 10, between 5 and 15, or between 10 and 20. In some embodiments, the downsampling rate is about 1.2, about 1.5, about 2, about 2.5, about 3, about 3.5, about 4, about 4.5, about 5, about 5.5, about 6, about 6.5, about 7, about 7.5, about 8, about 8.5, about 9, about 9.5, or about 10. In some embodiments, the downsampling rate is 2, 3, 4, 5, 6, 7, 8, 9, or 10.

In some embodiments, the maximum iteration rate indicates the maximum number of times that a respective initial classifier (e.g., hyperparameter configuration) in the plurality of initial classifiers will be validated and/or evaluated. In some embodiments, the iteration rate can also be considered as a validation depth.

In some embodiments, the maximum iteration rate for each corresponding instance of the outer loop is at least 2, at least 3, at least 4, at least 5, at least 6, at least 7, at least 8, at least 9, at least 10, at least 20, at least 30, at least 40, at least 50, at least 60, at least 70, at least 80, at least 90, at least 100, at least 200, at least 300, at least 400, at least 500, at least 1000, at least 2000, at least 2500, at least 3000, or at least 5000. In some embodiments, the maximum iteration rate is no more than 3000, no more than 2500, no more than 2000, no more than 1000, no more than 500, no more than 400, no more than 300, no more than 200, no more than 100, or no more than 50. In some embodiments, the maximum iteration rate for each corresponding instance of the outer loop is between 20 and 1000. In some embodiments, the maximum iteration rate for each corresponding instance of the outer loop is between 2 and 5000, between 5 and 2000, between 50 and 2500, between 10 and 1000, between 1000 and 5000, between 500 and 2000, between 100 and 800, between 50 and 3000, between 20 and 500, between 30 and 200, or between 50 and 100. In some embodiments, the maximum iteration rate falls within another range starting no lower than 5 and ending no higher than 5000.

In some embodiments, the downsampling rate and/or the maximum iteration rate is a hyperparameter that is predefined *(e.g.,* by a user and/or practitioner). In some embodiments, the downsampling rate and/or the maximum iteration rate is randomly or pseudo-randomly generated. In some embodiments, the downsampling rate and/or the maximum iteration rate is determined from a hyperparameter optimization or tuning method.

Referring to Block 208, the corresponding instance of the outer loop comprises, for each respective initial classifier in a plurality of initial classifiers, using the random seed to pseudo-randomly assign values for each respective hyperparameter in a plurality of hyperparameters for the respective initial classifier (e.g., where pseudo-random assignment of values is performed using a hyperparameter assignment construct 140). Each respective hyperparameter in the plurality of hyperparameters has a respective value selected from a respective plurality of candidate values for the respective hyperparameter, and each respective initial classifier in the plurality of initial classifiers has a corresponding plurality of parameters (e.g., weights), where the corresponding plurality of parameters comprises more than 500 parameters (e.g., weights).

Thus, each corresponding instance of the outer loop is associated with a respective random seed in the plurality of random seeds, and each initial classifier in the plurality of initial classifiers for the respective instance of the outer loop has a plurality of hyperparameters that is further pseudo-randomly assigned by the respective random seed *(e.g.,* thus generating a plurality of hyperparameter configurations).

More generally, in some embodiments, the corresponding instance of the outer loop comprises, for each respective initial classifier in a plurality of initial classifiers, using the random seed to pseudo-randomly assign values for each respective parameter in a plurality of parameters for the respective initial classifier. In some such embodiments, each respective parameter in the plurality of parameters has a respective value selected from a plurality of candidate values for the respective parameter.

As described above, in some embodiments, a parameter in the corresponding plurality of parameters is any coefficient or, similarly, any value of an internal or external element (e.g., a weight and/or a hyperparameter) in a model that can affect (e.g., modify, tailor, and/or adjust) one or more inputs, outputs, and/or functions in the model. For example, in some embodiments, a parameter refers to any coefficient, weight, and/or hyperparameter that can be used to control, modify, tailor, and/or adjust the behavior, learning and/or performance of a model. In some embodiments, a parameter is a fixed value. In some embodiments, a parameter is manually and/or automatically adjustable. In some embodiments, a value of a parameter is modified by a classifier validation and/or training process (e.g., by error minimization and/or backpropagation methods, as described herein).

In some embodiments, the plurality of random seeds comprises at least 2, at least 3, at least 4, at least 5, at least 6, at least 7, at least 8, at least 9, at least 10, at least 20, at least 30, at least 40, at least 50, at least 60, at least 70, at least 80, at least 90, at least 100, at least 200, at least 300, at least 400, or at least 500 random seeds. In some embodiments, the plurality of random seeds comprises no more than 500, no more than 400, no more than 300, no more than 200, or no more than 100 random seeds. In some embodiments, the plurality of random seeds comprises no more than 100, no more than 50, no more than 40, no more than 30, or no more than 20 random seeds. In some embodiments, the plurality of random seeds comprises between 1 and 50, between 2 and 20, between 5 and 50, between 10 and 80, between 5 and 15, between 3 and 30, between 10 and 500, between 2 and 100, or between 50 and 100 random seeds. In some embodiments, the plurality of random seeds falls within another range starting no lower than 1 and ending no higher than 500.

In some embodiments, the value for each random seed in the plurality of random seeds is selected from a range of values from 1 to 50,000, from 10 to 30,000, from 50 to 20,000, from 100 to 15,000, from 10 to 10,000, or from 1000 to 10,000. In some embodiments, the value for each random seed in the plurality of random seeds is selected from a range of values from 1 to 500, from 10 to 1000, from 100 to 2000, from 1000 to 5000, from 1000 to 9999, or from 2000 to 50,000. In some embodiments, the value for each random seed in the plurality of random seeds falls within another range starting no lower than 1 and ending no higher than 50,000.

In some embodiments, the value of each random seed in the plurality of random seeds is a hyperparameter that is predefined (e.g., by a user and/or practitioner). In some embodiments, the value of each random seed in the plurality of random seeds is randomly or pseudo-randomly generated (e.g., initialized). In some embodiments, the value of each random seed in the plurality of random seeds is determined from a hyperparameter optimization or tuning method.

In some embodiments, the plurality of initial classifiers comprises at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 30, at least 40, at least 50, at least 60, at least 70, at least 80, at least 90, at least 100, at least 200, at least 300, at least 400, or at least 500 initial classifiers. In some embodiments, the plurality of initial classifiers comprises at least 100, at least 500, at least 800, at least 1000, at least 2000, at least 3000, at least 4000, at least 5000, at least 6000, at least 7000, at least 8000, at least 9000, at least 10,000, or at least 20,000 initial classifiers. In some embodiments, the plurality of initial classifiers comprises no more than 20,000, no more than 10,000, no more than 5000, no more than 4000, no more than 3000, no more than 2000, no more than 1000, no more than 900, no more than 800, no more than 700, no more than 600, no more than 500, no more than 400, no more than 300, no more than 200, no more than 100, no more than 50, or no more than 10 initial classifiers. In some embodiments, the plurality of initial classifiers comprises between 10 and 50, between 10 and 200, between 20 and 500, between 100 and 800, between 50 and 1000, between 500 and 2000, between 1000 and 5000, or between 5000 and 10,000 initial classifiers. In some embodiments, the plurality of initial classifiers falls within another range starting no lower than 10 and ending no higher than 20,000.

In some embodiments, the corresponding plurality of parameters for each respective initial classifier in the plurality of initial classifiers comprises at least 2, at least 3, at least 4, at least 5, at least 6, at least 7, at least 8, at least 9, at least 10, at least 50, at least 100, at least 200, at least 300, at least 400, at least 500, or at least 1000 parameters. In some embodiments, the plurality of parameters comprises at least 2000, at least 3000, at least 4000, at least 5000, at least 6000, at least 7000, at least 8000, at least 9000, at least 10,000, at least 20,000, at least 30,000, at least 40,000, at least 50,000, at least 60,000, at least 70,000, at least 80,000, at least 90,000, or at least 100,000 parameters. In some embodiments, the plurality of parameters comprises no more than 100,000, no more than 50,000, no more than 10,000, no more than 5000, no more than 4000, no more than 3000, no more than 2000, no more than 1000, no more than 900, no more than 800, no more than 700, no more than 600, or no more than 500 parameters. In some embodiments, the plurality of parameters comprises between 10 and 50, between 50 and 200, between 200 and 5000, between 1000 and 8000, between 5000 and 10,000, between 5000 and 20,000, between 10,000 and 50,000, or between 50,000 and 100,000 parameters. In some embodiments, the plurality of parameters falls within another range starting no lower than 500 and ending no higher than 100,000.

In some embodiments, candidate values for hyperparameters (or, generally, parameters) are pseudo-randomly assigned based on, *e.g.,* the respective random seed. Candidate values for hyperparameters (or, generally, parameters) and assignment of corresponding values are described in further detail below (see, *e.g.,* the section entitled "Classifiers and Hyperparameters").

Referring to Block 210, the corresponding instance of the outer loop further comprises binning the plurality of initial classifiers into a plurality of bins. Each bin in the plurality of bins is characterized by a respective initial number of initial classifiers (e.g., Figure 10; "n_i") in the plurality of initial classifiers, a respective initial number of iterations (e.g., Figure 10; "r_i"), and the downsampling rate *(e.g.,* Figure 10; "eta"). The method includes, for each respective bin in the plurality of bins, performing a corresponding inner loop in which an iteration count is initially set to the respective initial number of iterations.

In some embodiments, the number of bins is between 3 and 25. In some embodiments, the number of bins is at least 2, at least 3, at least 4, at least 5, at least 6, at least 7, at least 8, at least 9, at least 10, at least 20, at least 30, at least 40, at least 50, at least 60, at least 70, at least 80, at least 90, at least 100, at least 200, at least 300, at least 400, or at least 500 bins. In some embodiments, the number of bins is no more than 100, no more than 50, no more than 40, no more than 30, no more than 20, or no more than 10 bins. In some embodiments, the plurality of bins comprises between 1 and 50, between 2 and 20, between 5 and 50, between 10 and 80, between 5 and 15, between 3 and 30, between 10 and 500, between 2 and 100, or between 50 and 100 bins. In some embodiments, the plurality of bins falls within another range starting no lower than 2 and ending no higher than 500.

In some embodiments, the number of bins is defined as s_max+1, where s_max is a positive integer. Thus, for example, as illustrated in Figure 10, where s_max = 4, then the number of bins is 5. In some embodiments, s_max is a hyperparameter. In some embodiments, s_max is predefined *(e.g.,* by a user and/or practitioner). In some embodiments, s_max is randomly or pseudo-randomly generated (e.g., initialized). In some embodiments, s_max is determined from a hyperparameter optimization or tuning method.

In some embodiments, each respective bin in the plurality of bins corresponds to a respective round (e.g., pass) of the corresponding instance of the outer loop. Bins are further represented in Figure 10 as columns indicated by different identifying values of s from 0 to s_max and comprising a different respective group of initial classifiers.

As described above with reference to Block 210, each corresponding bin (e.g., column) is characterized by an initial number of initial classifiers (n_i), obtained from the plurality of initial classifiers for the respective instance of the outer loop, and an initial number of iterations (r_i). In some embodiments, the initial number of initial classifiers for each corresponding bin is less than or equal to the number of initial classifiers in the plurality of initial classifiers. In some embodiments, the initial number of initial classifiers for each corresponding bin is different for each respective bin in the plurality of bins. In some embodiments, the initial number of iterations for each corresponding bin is less than or equal to the maximum iteration rate. In some embodiments, the initial number of iterations for each corresponding bin is different for each respective bin in the plurality of bins.

In some embodiments, for each corresponding instance of the outer loop, the respective initial number of initial classifiers binned into each respective bin in the plurality of bins is determined based on the number of bins, the maximum iteration rate *(e.g.,* s_max+1), the downsampling rate *(e.g.,* eta), and the corresponding identity for the respective bin *(e.g.,* s). In some embodiments, the maximum initial number of initial classifiers is determined based on the maximum iteration rate for the corresponding instance of the outer loop. In some embodiments, the maximum initial number of initial classifiers is equal to the maximum iteration rate for the corresponding instance of the outer loop. In some embodiments, a first bin with a larger initial number of initial classifiers will have a corresponding smaller initial number of iterations, and a second bin with a smaller initial number of initial classifiers than the first bin will have a corresponding larger initial number of iterations compared to the first bin.

Thus, as illustrated in Figure 10, for each bin in a plurality of 5 bins, the maximum initial number of initial classifiers is equal to the maximum iteration rate, where the maximum number of initial classifiers is indicated in the top row of the left-most column *(e.g.,* n_i = 81), the maximum initial number of iterations is indicated in the top row of the right-most column *(e.g.,* r_i = 81), and each subsequent bin (s=5, s=4, s=3, s=2, s=1, s=0) comprises successively smaller initial numbers of initial classifiers *(e.g.,* 81, 27, 9, 6, 5) and successively larger initial numbers of iterations *(e.g.,* 1, 3, 9, 27, 81).

Thus, in some embodiments, the outer loop describes the hedging strategy alluded to above (see, "Introduction") and the inner loop describes the early-stopping procedure that considers multiple hyperparameter configurations in parallel and terminates poor performing configurations leaving more resources for more promising configurations. For instance, certain hyperparameters will exhibit poor performance for a small number of iterations but high performance after a larger number of iterations (e.g., learning rate; step size). Configurations containing these hyperparameters would thus be removed after a first pass of downsampling where the initial iteration rate is small *(e.g.,* 1 or 3; see Figure 10 at columns s=4 and s=3), and therefore potentially high performing initial classifiers would be lost at an early stage of the hyperparameter selection process. The outer loop hedges over varying degrees of aggressiveness, balancing a breadth-based versus a depth-based search. For example, Figure 10 shows that each instance of the outer loop *(e.g.,* each of the 5 columns: s=4, s=3, s=2, s=1, and s=0) employs a different balance of breadth *(e.g.,* number of classifiers) and depth *(e.g.,* number of iterations), with some instances characterized by high breadth and low depth *(e.g.,* column s=4; initial number of classifiers=81; initial number of iterations =1) and some instances characterized by low breadth and high depth *(e.g.,* column s=0; initial number of classifiers=5; initial number of iterations =81).

In some embodiments, the initial number of initial classifiers binned into each respective bin is defined as $(eta)^s$ and is modified by a scaling factor that accounts for smaller values of s. In some embodiments, this is an integer factor obtained as $int((s\_max+1)/(s+1))$. For example, referring to Figure 10, s_max = 4 and eta = 3. Then, for column s = 4, the initial number of initial classifiers for the respective column is $(3^4) = 81$ and the scaling factor is 5/5 = 1, such that no scaling is applied to n_i = 81. Similar calculations can be performed for columns s=3 and s=2 (n_i = 27 and 9 with no modification, respectively). In contrast, for small values of s *(e.g.,* 1 and 0), the scaling factors become int(5/2) = 2 and int(5/1) = 5, respectively, such that for s = 1, n_i is $(3^1)*2 = 6$ and for s = 0, n_i is $(3^0)*5 = 5$, respectively. In some embodiments, the initial number of initial classifiers in each respective bin is not modified by a scaling factor.

Additional details regarding initial numbers of initial classifiers, initial numbers of iterations, and determination of the same, are provided in Jamieson et al., "Hyperband: A Novel Bandit-Based Approach to Hyperparameter Optimization," available online at arxiv.org/abs/1603.06560, which is hereby incorporated herein by reference in its entirety.

Referring again to Block 210, each round of the outer loop *(e.g.,* each bin) in turn performs a corresponding instance of an inner loop. Thus, as illustrated in Figure 10, for each respective bin in the plurality of bins *(e.g.,* each column), the number of classifiers remaining in the bin after each round *(e.g.,* each pass) of the inner loop is indicated on the left side *(e.g.,* n), and the number of iterations to be performed in each round *(e.g.,* each pass) of the inner loop is indicated on the right side *(e.g.,* r).

In some embodiments, the inner loop repeats the validation, evaluation, and downsampling of initial classifiers in the bin for a number of repeats determined based on a value of s, with the number of classifiers tested decreasing at each pass of the inner loop until the loop is complete.

Blocks 212 to 220 describe the process covered by the inner loop, for a respective bin in the plurality of bins *(e.g.,* a respective round or hedge of the outer loop).

Referring to Block 212, the inner loop comprises, i) for a number of iterations equal to the iteration count, training each initial classifier in the respective bin in a K-fold cross-validation context, where the K-fold cross-validation comprises refining each initial classifier in the respective bin against the training dataset using the values assigned for each respective hyperparameter in the plurality of hyperparameters for the respective initial classifier. For example, as illustrated in Figure 1, the method comprises performing validation for the initial classifiers in the respective bin using a validation construct 142 in classifier construction module 136.

In some embodiments, the method comprises performing any other suitable method for validation, including but not limited to advanced cross-validation, random cross-validation, grouped cross-validation *(e.g.*, K-fold grouped cross-validation), bootstrap bias corrected cross-validation, random search, and/or Bayesian hyperparameter optimization.

In some embodiments, the K-fold cross-validation is performed by training the classifiers on a training subset obtained from the training dataset *(e.g.*, via a K-fold training/testing split), and evaluating the performance of each initial classifier against a testing subset that is different from the training subset. In some such embodiments, the cross-validation is performed K times, for each training/testing split.

In some such embodiments, a training dataset is divided into K bins. For each fold of training, one bin in the plurality of K bins is left out of the training dataset and the classifier is trained on the remaining K-1 bins. Performance of the trained or partially trained classifier is then evaluated on the $K^{th}$ bin that was removed from the training. This process is repeated K times, until each bin has been used once for validation. In some embodiments, K is 2, 3, 4, 5, 6, 7, 8, 9, 10, 11, 12, 13, 14, 15, 16, 17, 18, 19, 20, or more than 20. In some embodiments, the K-fold cross-validation is performed with a value for K that is between 2 and 20. In some embodiments, the K-fold cross-validation is performed with a value for K that is between 3 and 8. In some embodiments, K is between 1 and 10, between 10 and 20, between 20 and 30, between 30 and 40, or between 40 and 50. In some embodiments, K is between 3 and 10. In some embodiments, training is performed using K-fold cross-validation with shuffling. In some such embodiments, K-fold cross-validation is repeated by shuffling the training dataset and performing a second K-fold cross-validation training. The shuffling is performed so that each bin in the plurality of K bins in the second K-fold cross-validation is populated with a different (e.g., shuffled) subset of training data. In some such embodiments, the training comprises shuffling the training dataset 1, 2, 3, 4, 5, 6, 7, 8, 9, 10, or more than 10 times. For example, in some embodiments, performing multiple iterations of validation comprises performing K-fold cross-validation with shuffling before each subsequent iteration.

In some embodiments, the performing K-fold cross-validation further comprises, for each initial classifier in the respective bin, obtaining one or more cross-validation scores based on a performance measure of the respective initial classifier after training. In some embodiments, a cross-validation score is an area under curve (AUC), area under receiver operator curve (AUROC), pooled AUC, mean AUC (mAUC), and/or an error. For example, in some embodiments, the corresponding cross-validation score is an error computed using an error function *(e.g.,* a loss function). In some embodiments, the loss function is mean square error, quadratic loss, mean absolute error, mean bias error, hinge, multi-class support vector machine, and/or cross-entropy. In some embodiments, the error is computed in accordance with a gradient descent algorithm and/or a minimization function. In some embodiments, the corresponding cross-validation score is a loss calculated from expected and predicted probability outputs on the test subset of the training dataset *(e.g.,* the subset of the training dataset).

In some embodiments, the corresponding cross-validation score is obtained by averaging (e.g., averaging AUROC scores over folds). In some embodiments, the corresponding evaluation score is averaged over a plurality of repeated cross-validations (e.g., a plurality of cross-validation scores obtained from a respective plurality of repeats of K-fold cross-validation, each time using different shuffling of training data to obtain folds).

Referring to Block 214, the inner loop further comprises ii) determining, based on the K-fold cross-validation, a corresponding evaluation score for each initial classifier in the respective bin. For example, as illustrated in Figure 1, the method comprises determining the evaluation score for the initial classifiers in the respective bin using an evaluation construct 144 in classifier construction module 136.

In some embodiments, the corresponding evaluation score is an area under curve (AUC), area under receiver operator curve (AUROC), pooled AUC, mean AUC (mAUC), and/or an error. For example, in some embodiments, the corresponding evaluation score is an error computed using an error function *(e.g.,* a loss function). In some embodiments, the loss function is mean square error, quadratic loss, mean absolute error, mean bias error, hinge, multi-class support vector machine, and/or cross-entropy. In some embodiments, the error is computed in accordance with a gradient descent algorithm and/or a minimization function. In some embodiments, the corresponding evaluation score is a loss calculated from expected and predicted probability outputs on a test subset of the training dataset *(e.g.,* a hold-out test subset of the training dataset).

In some embodiments, the performing K-fold cross-validation further comprises, for each initial classifier in the respective bin, obtaining one or more cross-validation scores based on a performance measure of the respective initial classifier, and the determining a corresponding evaluation score for the respective initial classifier is determined from the one or more cross-validation scores obtained from the K-fold cross-validation.

In some embodiments, the corresponding evaluation score is a combined score obtained from a plurality of folds *(e.g.,* a pool of K evaluation scores) and/or a plurality of iterations *(e.g.,* splits of averaged or separate cross-validation scores). In some embodiments, the corresponding evaluation score is averaged over a plurality of splits (e.g., one or more cross-validation scores obtained from a respective one or more iterations of K-fold cross-validation with shuffling).

In some embodiments, the corresponding evaluation score comprises any of the methods disclosed herein (see, for example, the section entitled "Training Classifiers," below), and/or any substitutions, modifications, additions, deletions, and/or combinations thereof, as will be apparent to one skilled in the art.

Referring to Block 216, the inner loop further comprises iii) removing, from the respective bin, a subset of initial classifiers in accordance with the downsampling rate and the corresponding evaluation score for each initial classifier in the respective bin.

In some embodiments, the removing further comprises ranking each initial classifier in the respective bin based on the corresponding evaluation score and removing a number of lowest ranked initial classifiers in accordance with the downsampling rate. Thus, for example, the initial classifiers retained in the bin are the highest ranked classifiers for the respective round of the inner loop, and the number of initial classifiers remaining after downsampling is the number of initial classifiers currently in the bin divided by the downsampling rate. The number of classifiers in the respective bin will further decrease in accordance with the downsampling rate after each repetition *(e.g.,* each round) of the inner loop.

Referring to Block 218, the inner loop further comprises iv) increasing the iteration count as a function of an inverse of the downsampling rate.

For example, referring to Figure 10, where the downsampling rate is 3, then the number of classifiers in the bin will be reduced by a factor of 3 and the number of iterations for the subsequent round will be increased by a factor of 3. Thus, a first round of an inner loop comprising 81 classifiers and an initial iteration rate of 1 will progress to a second round comprising 81/3 = 27 classifiers and an iteration rate of 1*3 = 3, a third round comprising 27/3 = 9 classifiers and an iteration rate of 3*3 = 9, and so on.

Referring to Block 220, the inner loop further comprises v) repeating the performing i), determining ii), removing iii) and increasing iv) for a number of repetitions that is determined based on a corresponding identity for the respective bin.

In some embodiments, the number of repetitions is the same for each bin in the plurality of bins. In some embodiments, the number of repetitions is different for each bin in the plurality of bins. In some embodiments, the number of repetitions in the repeating v) is s+1, wherein s is the identifying value assigned to the respective bin. Thus, in some such embodiments, for each bin with a corresponding identifying value s, the performing i), determining ii), removing iii) and increasing iv) is repeated s+1 times.

For example, Figure 10 illustrates the number of repetitions of the inner loop, for each bin in the plurality of bins. Each round of the inner loop is repeated for each i in s+1, such that for the bin denoted by s = 4, the inner loop is repeated 5 times. Similarly, for the bin denoted by s=0, the inner loop is performed once *(e.g.,* no repetitions).

In some embodiments, the final number of initial classifiers obtained at the completion of the inner loop, for each respective bin in the plurality of bins, is 1. In some embodiments, the final number of initial classifiers obtained at the completion of the inner loop is more than 1. In some such embodiments, the final number of initial classifiers obtained at the completion of the inner loop depends on the initial number of initial classifiers *(e.g.,* n_i), the number of repetitions *(e.g.,* s+1), and the downsampling rate. Thus, any change in the values for any one or more of these hyperparameters can affect the final number of initial classifiers.

Referring to Block 222, at the conclusion of each round *(e.g.,* each column in Figure 10) of the outer loop, the corresponding instance of the outer loop further comprises selecting, from among all initial classifiers in the plurality of initial classifiers, a corresponding classifier that has the best corresponding evaluation score as representative of the respective random seed in the plurality of random seeds.

In some embodiments, the corresponding classifier that has the best corresponding evaluation score is selected from any one of the bins in the plurality of bins. In some embodiments, the corresponding classifier that has the best corresponding evaluation score is obtained from the final round of downsampling in any one of the bins in the plurality of bins. In some embodiments, corresponding classifier that has the best corresponding evaluation score is not obtained from the final round of downsampling, but from an intermediate round of downsampling. In some embodiments, the corresponding classifier that has the best corresponding evaluation score is a plurality of initial classifiers.

In some embodiments, the selected classifier indicates the best hyperparameter configuration pseudo-randomly generated by the respective random seed, for each respective random seed in the plurality of random seeds.

Referring to Block 224, the method includes forming the ensemble classifier from the corresponding classifier selected by the selecting (e.g., as referred to in Block 222), for each respective random seed in the plurality of random seeds.

For example, an ensemble classifier may allow for improved performance in determining infectious disease states, due to the combined predictive power of multiple classifiers over a single classifier.

In some such embodiments, the ensemble classifier is formed after performing the outer loop detailed above in Blocks 206-222 for each random seed in a plurality of random seeds and selecting the corresponding best classifier for the respective random seed. Thus, if the method comprises 10 random seeds, then the best classifier for each random seed will be selected for a total of 10 classifiers, and the ensemble classifier will be formed from at least the 10 corresponding best classifiers.

In some embodiments, the ensemble classifier is formed from a plurality of selected classifiers. In some embodiments, the number of selected classifiers in the ensemble classifier is equal to the number of random seeds in the plurality of random seeds. In some embodiments, the number of selected classifiers in the ensemble classifier is more or less than the number of random seeds in the plurality of random seeds. In some embodiments, the ensemble classifier comprises at least 2, at least 3, at least 4, at least 5, at least 6, at least 7, at least 8, at least 9, at least 10, at least 20, at least 30, at least 40, at least 50, at least 60, at least 70, at least 80, at least 90, at least 100, at least 200, at least 300, at least 400, or at least 500 classifiers. In some embodiments, the ensemble classifier comprises no more than 500, no more than 400, no more than 300, no more than 200, or no more than 100 classifiers. In some embodiments, the ensemble classifier comprises no more than 100, no more than 50, no more than 40, no more than 30, or no more than 20 classifiers. In some embodiments, the ensemble classifier comprises between 1 and 50, between 2 and 20, between 5 and 50, between 10 and 80, between 5 and 15, between 3 and 30, between 10 and 500, between 2 and 100, or between 50 and 100 classifiers. In some embodiments, the plurality of selected classifiers that forms the ensemble classifier falls within another range starting no lower than 1 and ending no higher than 500.

In some embodiments, the ensemble classifier is formed by combining a plurality of outputs obtained from the plurality of classifiers selected by the selecting of the best classifier.

For example, in some embodiments, each classifier in the ensemble classifier provides an output for the determination of an infectious disease state. In some embodiments, an output is a predicted probability of an infectious disease state, a class label for one or more infectious disease states, a binary indication of an infectious disease state, and/or any other embodiment of a classifier output and/or infectious disease state as disclosed herein (see, for example, the sections entitled "Training Classifiers," and "Determining Infectious Disease States," below).

In some embodiments, the plurality of outputs from the classifiers is combined using any measure of central tendency known in the art, including but not limited to a mean, median, mode, a weighted mean, weighted median, weighted mode, *etc.* In some such embodiments, the final determination from the ensemble classifier (e.g., the final determination of the infectious disease state) is obtained based on the average of the outputs across all classifiers in the ensemble classifier.

For example, in some embodiments, the plurality of outputs from the classifiers is combined for the ensemble classifier by averaging the outputs (e.g., averaging the predicted probabilities obtained from each individual model in the ensemble classifier) and determining the final outputted infectious disease state for the subject using the average of the outputs.

In some embodiments, the plurality of outputs is combined using a voting method. For example, in some embodiments, the plurality of outputs is combined by tallying the number of outputs, from each classifier in the ensemble classifier, that indicate a respective infectious disease state. In some such embodiments, the final determination of the infectious disease state is obtained based on the count of votes for each respective outputted infectious disease state in a plurality of possible outputted infectious disease states. In some embodiments, the plurality of outputs from the classifiers is combined using a majority vote (e.g., such that the output with the highest count is selected for the final determination). In some embodiments, the plurality of outputs from the classifiers is combined by selecting, from the plurality of possible outputted infectious disease states, the output that has a tally that is greater than a voting threshold. In some embodiments, the voting threshold is at least 50% of total votes from the plurality of classifiers in the ensemble classifier. In some embodiments, the voting threshold is at least 20%, at least 30%, at least 40%, at least 50%, at least 60%, at least 70%, at least 80%, at least 90%, or at least 95% of total votes from the plurality of classifiers in the ensemble classifier.

In some embodiments, each classifier in the ensemble classifier is unweighted (e.g., each classifier has one vote in the ensemble model). In some embodiments, one or more classifiers in the ensemble classifier is further weighted (e.g., has greater than 1 vote in the ensemble model).

In some embodiments, the method comprises obtaining a single ensemble model.

In some embodiments, the ensemble model provides, as output, a plurality of scores (e.g., probability, label, and/or other indication) for a plurality of different infectious disease states. For example, in some embodiments, the ensemble model provides a first score indicating a first infectious disease state (e.g., infected with a bacteria or not infected with a bacteria), and a second score indicating a second infectious disease state other than the first infectious disease state (e.g., infected with a virus or not infected with a virus). In some embodiments, the ensemble model provides a third score indicating a third infectious disease state (e.g., not infected). In some embodiments, the first score is an indication of bacterial infection, the second score is an indication of viral infection, and the third score is an indication of non-infection. In some such embodiments, a score is not reported if it can be derived from another score (e.g., where a negative indication for non-infection can be inferred from a positive indication for a bacterial infection and/or a viral infection). In some embodiments, the ensemble model provides additional scores indicating one or more additional infectious disease states (e.g., severity, inflammation, and/or sepsis). In some embodiments, the one or more additional infectious disease states are provided by an additional classification model separate from the ensemble model (e.g., a logistic regression model).

In some embodiments, the ensemble model comprises a plurality of sets of single-label component classifiers, each respective set of classifiers corresponding to a respective different infectious disease state (e.g., a first set of single-label component classifiers corresponding to outputs for bacterial infection, a second set of single-label component classifiers corresponding to outputs for viral infection, and a third set of single-label component classifiers corresponding to outputs for non-infection). In some such embodiments, each single-label classifier in a respective set of single-label component classifiers provides a score for the respective infectious disease state, and the ensemble model is formed by combining the plurality of scores, from each respective set of single-label component classifiers, to provide a combined output. Thus, for example, in some such embodiments, the ensemble model is formed by combining a first set of scores from a first set of component classifiers, a second set of scores from a second set of component classifiers, and a third set of scores from a third set of component classifiers, where each respective set of scores indicates a respective different infectious disease state.

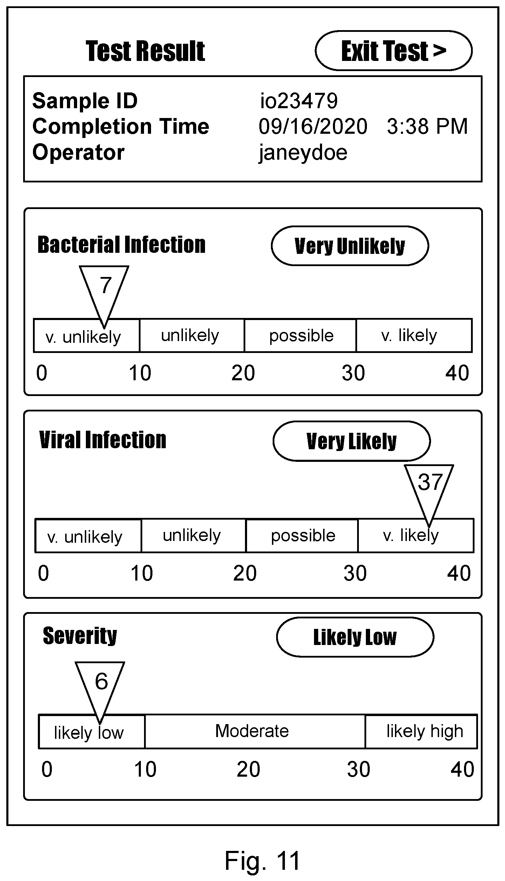

For example, referring to Figure 11, in an example embodiment of a determination of an infectious disease state, an output is provided that includes three scores for a respective subject: (i) a probability score for a bacterial etiology, (ii) a probability score for a viral etiology, and (iii) a score for the severity of the subject's condition. An example system for determining three scores for the respective subject is further described in Example 1 and illustrated in Figure 5. Thus, in some embodiments, the single ensemble model provides a plurality of scores by combining (i) a first set of bacterial etiology scores provided by a first set of bacterial etiology classifiers, and (ii) a second set of viral etiology scores provided by a second set of viral etiology classifiers. In some embodiments, as illustrated in Figure 11, a third score is provided for a severity, where the third score is obtained from an additional classification model separate from the ensemble model (e.g., a logistic regression model).

In some embodiments, the ensemble model provides at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 25, at least 30, at least 35, at least 40, at least 45, or at least 50 outputs. In some embodiments, the ensemble model provides no more than 50, no more than 40, no more than 30, no more than 20, no more than 15, or no more than 10 outputs. In some embodiments, the ensemble model provides between 2 and 10, between 5 and 15, between 5 and 20, between 2 and 8, or between 10 and 50 outputs. In some embodiments, the ensemble model comprises at least as many component classifiers as desired outputs (e.g., for different infectious disease states). In some embodiments, the ensemble model comprises the same number of component classifiers as desired outputs.

In some embodiments, the ensemble model comprises a plurality of multi-label component classifiers, each respective multi-label component classifier providing, as output, a plurality of scores (e.g., probability, label, and/or other indication) for a plurality of different infectious disease states. For example, in some embodiments, each component classifier in the ensemble model provides a first score indicating a first infectious disease state (e.g., infected with a bacteria) and a second score indicating a second infectious disease state (e.g., infected with a virus). In some embodiments, each component classifier in the ensemble model further provides a third score indicating a third infectious disease state (e.g., not infected). In some embodiments, each component classifier in the ensemble of classifiers computes three scores: a first score indicating bacterial infection, a second score indicating viral infection, and a third score indicating not infected. In some such embodiments, a score is not reported if it can be derived from another score (e.g., where a negative indication for not infected can be inferred from a positive indication for a bacterial infection and/or a viral infection). In some embodiments, each classifier in the ensemble of classifiers provides additional scores indicating one or more additional infectious disease states (e.g., severity, inflammation, and/or sepsis). In some embodiments, the ensemble model provides a plurality of scores for a respective plurality of infectious disease states (e.g., a bacterial score, a viral score, and/or a non-infection score), where each score in the plurality of scores is formed by combining the set of scores for each infectious disease state obtained from the set of multi-class classifiers in the ensemble classifier. Thus, for example, in some implementations, each multi-class classifier provides a bacterial infection score and a viral infection score, the bacterial infection score from each classifier is combined into a set of bacterial infection scores, and the viral infection score from each classifier is combined into a set of viral infection scores. In some embodiments, a final score is determined, for each respective infectious disease state in the plurality of infectious disease states, by averaging the scores in each respective set of scores for the infectious disease state. The averaged scores from the ensemble classifier provides a final bacterial infection score and a final viral infection score.

Thus, for example, in some such embodiments, the ensemble model is formed by combining, for each respective multi-class classifier in the plurality of multi-class classifiers, a plurality of scores for a respective plurality of different infectious disease states, thus obtaining a final plurality of scores from the ensemble model.

In some embodiments, the ensemble model comprising a plurality of multi-class classifiers provides additional scores indicating one or more additional infectious disease states (e.g., severity, inflammation, and/or sepsis). In some embodiments, the one or more additional infectious disease states are provided by an additional classification model separate from the ensemble model (e.g., a logistic regression model).

In some embodiments, each multi-class component classifier in the ensemble model provides at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 25, at least 30, at least 35, at least 40, at least 45, or at least 50 outputs. In some embodiments, each multi-class component classifier in the ensemble model provides no more than 50, no more than 40, no more than 30, no more than 20, no more than 15, or no more than 10 outputs. In some embodiments, each multi-class component classifier in the ensemble model provides between 2 and 10, between 5 and 15, between 5 and 20, between 2 and 8, or between 10 and 50 outputs.

Thus, referring again to Figure 11, in some embodiments, the single ensemble model provides three scores by combining (i) a plurality of bacterial etiology scores and (ii) a plurality of viral etiology scores, and (iii) a plurality of severity scores, where the bacterial, viral, and severity scores are obtained from each respective component classifier in the ensemble model. In some embodiments, a third score is provided for a severity, where the third score is obtained from an additional classification model separate from the ensemble model (e.g., a logistic regression model).

In some embodiments, the method comprises obtaining a plurality of ensemble models. For example, in some embodiments, the plurality of ensemble models comprises at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 25, at least 30, at least 35, at least 40, at least 45, or at least 50 ensemble models. In some embodiments, the plurality of ensemble models comprises no more than 50, no more than 40, no more than 30, no more than 20, no more than 15, or no more than 10 ensemble models. In some embodiments, the plurality of ensemble models comprises between 2 and 10, between 5 and 15, between 5 and 20, between 2 and 8, or between 10 and 50 ensemble models. In some embodiments, the plurality of ensemble models falls within another range starting no lower than 2 ensemble models and ending no higher than 50 ensemble models. In some embodiments, the plurality of ensemble models comprises at least as many ensemble models as desired outputs (e.g., for different infectious disease states). In some embodiments, the plurality of ensemble models comprises the same number of ensemble models as desired outputs.

In some embodiments, each ensemble model in the plurality of ensemble models provides, as output, an indication of a different infectious disease state. For example, in some embodiments, a first ensemble model provides an output indicating a first infectious disease state (e.g., infected with a bacteria or not infected with a bacteria), and a second ensemble model provides an output indicating a second infectious disease state other than the first infectious disease state (e.g., infected with a virus or not infected with a virus). In some such embodiments, a third ensemble model provides an output indicating a third infectious disease state (e.g., not infected). In some embodiments, each ensemble model in the plurality of ensemble models comprises a respective plurality of selected (e.g., component) classifiers, where each classifier in the plurality of component classifiers in the respective ensemble model similarly provides an output indicating the respective infectious disease state. Thus, for example, in some such embodiments, a respective first ensemble model is formed by combining a plurality of outputs from a plurality of component classifiers, where each output from each respective component classifier is for a respective first infectious disease state, and the combined output from the first ensemble model is for the respective first infectious disease state.

Thus, referring again to Figure 11, in some embodiments, (i) the bacterial etiology score is provided by a first ensemble classifier comprising a plurality of component classifiers, each component classifier providing a component bacterial etiology score and (ii) the viral etiology score is provided by a second ensemble classifier comprising a plurality of component classifiers, each component classifier providing a component viral etiology score. In some embodiments, a third score is provided for a severity, where the third score is obtained from an additional classification model separate from the ensemble model (e.g., a logistic regression model).

Any architecture known in the art is contemplated for the ensemble classifier, including bagging architectures (e.g., random forest, extra tree algorithms) and boosting architectures (e.g., gradient boosting, XGBoost). Furthermore, other methods of selecting initial classifiers from corresponding instances of the outer loop are possible, as will be apparent to one skilled in the art. For example, in some embodiments, the method comprises selecting more than one "best" initial classifier (e.g., with a corresponding best evaluation score) from an instance of the outer loop. Thus, in some such embodiments, two or more "best" classifiers would be selected as representative of the corresponding random seed. Similarly, in some embodiments, at least 2, at least 3, at least 4, at least 5, at least 6, at least 7, at least 8, at least 9, at least 10, or more "best" classifiers are selected from each corresponding instance of the outer loop (e.g., for each random seed in the plurality of random seeds). In some embodiments, each random seed is represented in the ensemble model at least once. In some embodiments, at least one random seed is not represented in the ensemble model (e.g., where no initial classifier was selected from the corresponding instance of the outer loop to be included in the ensemble classifier).

*Classifiers and Hyperparameters*

Any suitable model for use in the obtaining of the ensemble classifier is contemplated, as disclosed herein.

In some embodiments, each respective initial classifier in a plurality of initial classifiers is a neural network algorithm (e.g., a multi-layer perceptron, a fully connected neural network, a partially connected neural network, *etc.*), a support vector machine algorithm, a Naive Bayes algorithm, a nearest neighbor algorithm, a boosted trees algorithm (e.g., XGBoost, LightGBM), a random forest algorithm, a decision tree algorithm, a multinomial logistic regression algorithm, a linear model, or a linear regression algorithm.

In some embodiments, each initial classifier in the plurality of initial classifiers is the same type of classifier. In some embodiments, the plurality of initial classifiers comprises two or more different types of classifiers.

In some embodiments, a classifier in the plurality of initial classifiers is a multi-layer perceptron neural network. In some embodiments, a classifier is logistic regression. In some embodiments, a classifier is a neural network algorithm, a support vector machine algorithm, a Naive Bayes algorithm, a nearest neighbor algorithm, a boosted trees algorithm, a random forest algorithm, a decision tree algorithm, a multinomial logistic regression algorithm, a linear model, or a linear regression algorithm. In some embodiments, a classifier is a 2-stage stochastic gradient descent (SGD) model. In some embodiments, a classifier is a deep neural network (e.g., a deep- and-wide sample-level classifier).

Logistic regression algorithms are disclosed in Agresti, An Introduction to Categorical Data Analysis, 1996, Chapter 5, pp. 103-144, John Wiley & Son, New York, which is hereby incorporated by reference.

Neural network algorithms, including convolutional neural network algorithms, are disclosed in *See,* Vincent et al., 2010, "Stacked denoising autoencoders: Learning useful representations in a deep network with a local denoising criterion," J Mach Learn Res 11, pp. 3371-3408; Larochelle et al., 2009, "Exploring strategies for training deep neural networks," J Mach Learn Res 10, pp. 1-40; and Hassoun, 1995, Fundamentals of Artificial Neural Networks, Massachusetts Institute of Technology, each of which is hereby incorporated by reference.

SVM algorithms are described in Cristianini and Shawe-Taylor, 2000, "An Introduction to Support Vector Machines," Cambridge University Press, Cambridge; Boser et al., 1992, "A training algorithm for optimal margin classifiers," in Proceedings of the 5th Annual ACM Workshop on Computational Learning Theory, ACM Press, Pittsburgh, Pa., pp. 142-152; Vapnik, 1998, Statistical Learning Theory, Wiley, New York; Mount, 2001, Bioinformatics: sequence and genome analysis, Cold Spring Harbor Laboratory Press, Cold Spring Harbor, N.Y.; Duda, Pattern Classification, Second Edition, 2001, John Wiley & Sons, Inc., pp. 259, 262-265; and Hastie, 2001, The Elements of Statistical Learning, Springer, New York; and Furey et al., 2000, Bioinformatics 16, 906-914, each of which is hereby incorporated by reference in its entirety. When used for classification, SVMs separate a given set of binary labeled data training set with a hyper-plane that is maximally distant from the labeled data. For cases in which no linear separation is possible, SVMs can work in combination with the technique of "kernels," which automatically realizes a non-linear mapping to a feature space. The hyper-plane found by the SVM in feature space corresponds to a non-linear decision boundary in the input space.

Decision trees are described generally by Duda, 2001, Pattern Classification, John Wiley & Sons, Inc., New York, pp. 395-396, which is hereby incorporated by reference. Tree-based methods partition the feature space into a set of rectangles, and then fit a model (like a constant) in each one. In some embodiments, the decision tree is random forest regression. One specific algorithm that can be used is a classification and regression tree (CART). Other specific decision tree algorithms include, but are not limited to, ID3, C4.5, MART, and Random Forests. CART, ID3, and C4.5 are described in Duda, 2001, Pattern Classification, John Wiley & Sons, Inc., New York, pp. 396-408 and pp. 411-412, which is hereby incorporated by reference. CART, MART, and C4.5 are described in Hastie et al., 2001, The Elements of Statistical Learning, Springer-Verlag, New York, Chapter 9, which is hereby incorporated by reference in its entirety. Random Forests are described in Breiman, 1999, "Random Forests--Random Features," Technical Report 567, Statistics Department, U.C. Berkeley, September 1999, which is hereby incorporated by reference in its entirety.

Clustering is described at pages 211-256 of Duda and Hart, Pattern Classification and Scene Analysis, 1973, John Wiley & Sons, Inc., New York, (hereinafter "Duda 1973") which is hereby incorporated by reference in its entirety. As described in Section 6.7 of Duda 1973, the clustering problem is described as one of finding natural groupings in a dataset. To identify natural groupings, two issues are addressed. First, a way to measure similarity (or dissimilarity) between two samples is determined. This metric (similarity measure) is used to ensure that the samples in one cluster are more like one another than they are to samples in other clusters. Second, a mechanism for partitioning the data into clusters using the similarity measure is determined.

Similarity measures are discussed in Section 6.7 of Duda 1973, where it is stated that one way to begin a clustering investigation is to define a distance function and to compute the matrix of distances between all pairs of samples in the training set. If distance is a good measure of similarity, then the distance between reference entities in the same cluster will be significantly less than the distance between the reference entities in different clusters. However, as stated on page 215 of Duda 1973, clustering does not require the use of a distance metric. For example, a nonmetric similarity function s(x, x') can be used to compare two vectors x and x'. Conventionally, s(x, x') is a symmetric function whose value is large when x and x' are somehow "similar." An example of a nonmetric similarity function s(x, x') is provided on page 218 of Duda 1973.

Alternatively, or in addition to the methods disclosed in the preceding sections, any suitable model for use in hyperparameter selection (or, generally, parameter selection) is also contemplated (e.g., random search and/or Bayesian hyperparameter optimization methods).

As described above, parameters refer generally to the elements in a model, or the values thereof (e.g., coefficients, hyperparameters, and/or weights), that can be used to modify, tailor, and/or adjust the behavior, learning or performance of a model. In some embodiments, each hyperparameter (or, generally, each parameter) in a respective classifier is assigned a value from a plurality of candidate values. In some such embodiments, the assigning of values is performed manually (e.g., by a user or practitioner), automatically (e.g., by tuning or optimization processes), and/or pseudo-randomly (e.g., via a random search and/or hyperband method). Referring again to Block 208, for each respective classifier in the plurality of initial classifiers, each hyperparameter in the respective classifier is pseudo-randomly assigned a value from a plurality of candidate values (e.g., based on a pseudo-random sequence of values determined by a random seed and a random number generator). Candidate values for hyperparameters will be further discussed herein.

For example, in some embodiments, each respective classifier in the plurality of initial classifiers is a neural network (e.g., a multi-layer perceptron) that comprises a corresponding plurality of inputs, wherein each input in the corresponding plurality of inputs is for an attribute value for a gene (e.g., an abundance of an mRNA biomarker) in the plurality of genes. The neural network further includes a corresponding first hidden layer comprising a corresponding plurality of hidden neurons. Each hidden neuron in the corresponding plurality of hidden neurons is (i) fully or partially connected to each input in the plurality of inputs, (ii) associated with a first activation function type, and (iii) associated with a corresponding parameter in the corresponding plurality of parameters (e.g., a corresponding weight in the corresponding plurality of weights) for the respective neural network. The neural network further comprises one or more corresponding neural network outputs, where each respective neural network output in the corresponding one or more neural network outputs (i) directly or indirectly receives, as input, an output of each hidden neuron in the corresponding plurality of hidden neurons, and (ii) is associated with a second activation function type.

In some embodiments, the first activation function type (e.g., for a respective node in a corresponding hidden layer) is pseudo-randomly assigned (e.g., by using a random seed) from the group consisting of all or a combination of tanh, sigmoid, softmax, logistic, Gaussian, Boltzmann-weighted averaging, absolute value, linear, rectified linear unit (ReLU), leaky ReLU, exponential linear unit (eLU), bounded rectified linear, soft rectified linear, parameterized rectified linear, average, max, min, sign, square, square root, multiquadric, inverse quadratic, inverse multiquadric, polyharmonic spline, and thin-plate spline.

In some embodiments, the second activation function type (e.g., for a respective node in a corresponding hidden layer) is pseudo-randomly assigned (e.g., by using a random seed) from the group consisting of all or a combination of tanh, sigmoid, softmax, logistic, Gaussian, Boltzmann-weighted averaging, absolute value, linear, rectified linear unit (ReLU), leaky ReLU, exponential linear unit (eLU), bounded rectified linear, soft rectified linear, parameterized rectified linear, average, max, min, sign, square, square root, multiquadric, inverse quadratic, inverse multiquadric, polyharmonic spline, and thin-plate spline.

In some embodiments, the second activation function type is the same as the first activation function type (*e.g.,* for a respective node in a corresponding hidden layer). In some embodiments, the second activation function type is different from the first activation function type (*e.g.,* for a respective node in a corresponding hidden layer).

In some embodiments, each hidden neuron (*e.g.,* in a respective hidden layer in a respective classifier) is associated with an activation function that performs a function on the input data (*e.g.,* a linear or non-linear function). Generally, the purpose of the activation function is to introduce nonlinearity into the data such that the neural network is trained on representations of the original data and can subsequently "fit" or generate additional representations of new (*e.g.,* previously unseen) data. Selection of activation functions is dependent on the use case of the neural network, as certain activation functions can lead to saturation at the extreme ends of a dataset (*e.g.,* tanh and/or sigmoid functions).

In some embodiments, each hidden neuron (*e.g.,* in a respective hidden layer in a respective classifier) is further associated with a parameter (*e.g.,* weight) that contributes to the output of the neural network, determined based on the activation function. In some embodiments, the hidden neuron is initialized with arbitrary parameters (*e.g.,* randomized weights). In some alternative embodiments, the hidden neuron is initialized with a predetermined set of parameters.

In some embodiments, each hidden neuron (*e.g.,* in a respective hidden layer in a respective classifier) is associated with a corresponding parameter in the corresponding plurality of parameters (*e.g.,* at least 500 weights) for the corresponding classifier (*e.g.,* multi-layer perceptron neural network). In some alternative embodiments, one or more hidden neurons are not associated with a corresponding parameter in the corresponding plurality of parameters for the corresponding classifier. In some embodiments, the corresponding plurality of parameters further comprises a plurality of bias values.

In some embodiments, the corresponding plurality of hidden neurons (*e.g.,* in a respective classifier, *e.g.*, across one or more hidden layers) is pseudo-randomly assigned by the using the random seed to be between 2 and 500 neurons. In some embodiments, the corresponding plurality of hidden neurons is pseudo-randomly assigned by the using the random seed to be between 2 and 300 neurons.

In some embodiments, the corresponding plurality of hidden neurons in a respective classifier in the plurality of classifiers (*e.g.,* across one or more hidden layers) is pseudo-randomly assigned by the using the random seed to be at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 30, at least 40, at least 50, at least 60, at least 70, at least 80, at least 90, at least 100, at least 200, at least 300, at least 400, or at least 500 neurons. In some embodiments, the corresponding plurality of hidden neurons in a respective classifier in the plurality of classifiers is pseudo-randomly assigned by the using the random seed to be at least 100, at least 500, at least 800, at least 1000, at least 2000, at least 3000, at least 4000, at least 5000, at least 6000, at least 7000, at least 8000, at least 9000, at least 10,000, at least 15,000, at least 20,000, or at least 30,000 neurons. In some embodiments, the corresponding plurality of hidden neurons is pseudo-randomly assigned by the using the random seed to be no more than 30,000, no more than 20,000, no more than 15,000, no more than 10,000, no more than 9000, no more than 8000, no more than 7000, no more than 6000, no more than 5000, no more than 4000, no more than 3000, no more than 2000, no more than 1000, no more than 900, no more than 800, no more than 700, no more than 600, no more than 500, no more than 400, no more than 300, no more than 200, no more than 100, or no more than 50 neurons. In some embodiments, the corresponding plurality of hidden neurons is pseudo-randomly assigned by the using the random seed to be between 2 and 20, between 2 and 200, between 2 and 1000, between 10 and 50, between 10 and 200, between 20 and 500, between 100 and 800, between 50 and 1000, between 500 and 2000, between 1000 and 5000, between 5000 and 10,000, between 10,000 and 15,000, between 15,000 and 20,000, or between 20,000 and 30,000 neurons. In some embodiments, the corresponding plurality of hidden neurons is pseudo-randomly assigned by the using the random seed to fall within another range starting no lower than 2 neurons and ending no higher than 30,000 neurons.

In some embodiments, each classifier in the plurality of classifiers has the same number of neurons (*e.g.,* for classifiers having the same number of hidden layers). In some embodiments, a first classifier has a different number of neurons than a second classifier (*e.g.,* different neural networks can be different sizes). In some embodiments, the number of hidden neurons in each classifier in a plurality of classifiers is independently determined. In some embodiments, the number of hidden neurons is experimentally determined and/or optimized based on the performance of the corresponding classifier.

In some embodiments, a first classifier has a different number of layers than a second classifier in the plurality of classifiers (*e.g.,* different neural networks can have different numbers of layers). In some embodiments, the number of hidden layers in a corresponding classifier is independently determined. In some embodiments, the number of hidden layers is experimentally determined and/or optimized based on the performance of the corresponding classifier. For example, in some embodiments, the performance of each corresponding neural network depends on the size of the neural network (*e.g.,* the number of hidden units and/or layers) relative to the amount of available data in a training or test dataset. For example, in some embodiments, a smaller number of hidden units and/or hidden layers can improve the performance of a corresponding neural network where limited input data is available.

In some embodiments, each respective classifier in the plurality of classifiers is pseudo-randomly assigned by the using the random seed to be between 1 and 50 hidden layers. In some embodiments, each respective classifier in the plurality of classifiers is pseudo-randomly assigned by the using the random seed to be between 1 and 20 hidden layers. In some embodiments, the corresponding plurality of hidden layers is pseudo-randomly assigned by the using the random seed to be at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 30, at least 40, at least 50, at least 60, at least 70, at least 80, at least 90, or at least 100 hidden layers. In some embodiments, the corresponding plurality of hidden layers is pseudo-randomly assigned by the using the random seed to be no more than 100, no more than 90, no more than 80, no more than 70, no more than 60, no more than 50, no more than 40, no more than 30, no more than 20, no more than 10, no more than 9, no more than 8, no more than 7, no more than 6, or no more than 5 hidden layers. In some embodiments, the corresponding plurality of hidden layers is pseudo-randomly assigned by the using the random seed to be between 1 and 5, between 1 and 10, between 1 and 20, between 10 and 50, between 2 and 80, between 5 and 100, between 10 and 100, between 50 and 100, or between 3 and 30 hidden layers. In some embodiments, the corresponding plurality of hidden layers is pseudo-randomly assigned by the using the random seed to fall within another range starting no lower than 1 layer and ending no higher than 100 layers.

In some embodiments, a classifier is a shallow neural network. A shallow neural network refers to a neural network with a small number of hidden layers. In some embodiments, such neural network architectures improve the efficiency of neural network training and conserve computational power due to the reduced number of layers involved in the training. In some embodiments, a classifier has only one hidden layer.

In some embodiments, a classifier in a plurality of classifiers (*e.g.,* in the plurality of initial classifiers and/or in an ensemble classifier) comprises a plurality of hidden layers, and each hidden layer comprises the same number of hidden units. In some alternative embodiments, a classifier in a plurality of classifiers (*e.g.,* in the plurality of initial classifiers and/or in an ensemble classifier) comprises a plurality of hidden layers, and the plurality of hidden layers comprises two or more hidden layers having different numbers of hidden units.

For instance, in some embodiments, the ensemble classifier (e.g., obtained as described in the section entitled "Selection of Configurations," above) comprises at least 2, at least 3, at least 4, at least 5, at least 6, at least 7, at least 8, at least 9, at least 10, at least 20, at least 30, at least 40, at least 50, at least 60, at least 70, at least 80, at least 90, at least 100, at least 200, at least 300, at least 400, or at least 500 classifiers. In some such embodiments, the ensemble classifier comprises at least 10, at least 11, at least 12, at least 13, at least 14, at least 15, at least 16, at least 17, at least 18, at least 19, at least 20, at least 30, at least 40, at least 50, at least 60, at least 70, at least 80, at least 90, at least 100, at least 200, at least 300, at least 400, at least 500, at least 600, at least 700, at least 800, at least 900, at least 1000, at least 2000, at least 3000, at least 4000, at least 5000, at least 6000, at least 7000, at least 8000, at least 9000, at least 10,000, at least 15,000, at least 20,000, at least 30,000, at least 40,000, at least 50,000, at least 60,000, at least 70,000, at least 80,000, at least 90,000, at least 100,000, or at least 200,000 neurons across the plurality of classifiers in the ensemble classifier. In some embodiments, the ensemble classifier comprises no more than 500, no more than 400, no more than 300, no more than 200, no more than 100, no more than 50, no more than 40, no more than 30, or no more than 20 classifiers. In some such embodiments, the ensemble classifier comprises no more than 200,000, no more than 100,000, no more than 50,000, no more than 30,000, no more than 20,000, no more than 15,000, no more than 10,000, no more than 9000, no more than 8000, no more than 7000, no more than 6000, no more than 5000, no more than 4000, no more than 3000, no more than 2000, no more than 1000, no more than 900, no more than 800, no more than 700, no more than 600, no more than 500, no more than 400, no more than 300, no more than 200, no more than 100, or no more than 50 neurons across the plurality of classifiers in the ensemble classifier. In some embodiments, the ensemble classifier comprises a plurality of selected classifiers that falls within a range starting no lower than 1 and ending no higher than 500, and a plurality of neurons that falls within a range starting no lower than 10 and ending no higher than 200,000 neurons, across the plurality of classifiers in the ensemble classifier.

In some embodiments, the plurality of hyperparameters comprises a regularization hyperparameter that penalizes one or more parameters in the corresponding plurality of parameters, for each respective initial classifier in the plurality of initial classifiers. In some embodiments, the regularization hyperparameter is pseudo-randomly assigned by the using the random seed to be an L1 or L2 penalty. In some embodiments, the regularization hyperparameter is an L1 regularization penalty, and the L1 regularization penalty is pseudo-randomly assigned by the using the random seed to be at least $\exp(-100)$, at least $\exp(-90)$, at least $\exp(-80)$, at least $\exp(-70)$, at least $\exp(-60)$, at least $\exp(-50)$, at least $\exp(-40)$, at least $\exp(-30)$, at least $\exp(-20)$, at least $\exp(-10)$, at least $\exp(-5)$, at least $\exp(4)$, at least $\exp(-3)$, at least $\exp(-2)$, at least $\exp(-1)$, or at least $\exp(0)$. In some embodiments, the L1 regularization penalty is pseudo-randomly assigned by the using the random seed to be between $\exp(0)$ and $\exp(-100)$, between $\exp(0)$ and $\exp(-80)$, between $\exp(0)$ and $\exp(-50)$, or between $\exp(0)$ and $\exp(-10)$. In some embodiments, the L1 regularization penalty is pseudo-randomly assigned by the using the random seed to fall within another range starting no lower than $\exp(-100)$ and ending no higher than $\exp(0)$. In some embodiments, the regularization hyperparameter is an L2 regularization penalty, and the L2 regularization penalty is pseudo-randomly assigned by the using the random seed to be at least $\exp(-100)$, at least exp(-90), at least exp(-80), at least exp(-70), at least exp(-60), at least exp(-50), at least exp(-40), at least exp(-30), at least exp(-20), at least exp(-10), at least exp(-5), at least exp(4), at least exp(-3), at least exp(-2), at least exp(-1), or at least exp(0). In some embodiments, the L2 regularization penalty is pseudo-randomly assigned by the using the random seed to be between exp(0) and exp(-100), between exp(0) and exp(-80), between exp(0) and exp(-50), between exp(0) and exp(-12), or between exp(0) and exp(-10). In some embodiments, the L2 regularization penalty is pseudo-randomly assigned by the using the random seed to fall within another range starting no lower than exp(-100) and ending no higher than exp(0).

In some embodiments, the plurality of hyperparameters comprises a learning rate. For example, in some embodiments, the learning rate is used to update parameters (e.g., weights) during classifier training, such that the parameters are updated by adjusting the value based on a calculated loss metered by a predetermined learning rate hyperparameter that dictates the degree or severity to which parameters are updated (e.g., small adjustments versus large adjustments), thereby training the classifier.

In some embodiments, the learning rate is pseudo-randomly assigned by the using the random seed to be at least exp(-100), at least exp(-90), at least exp(-80), at least exp(-70), at least exp(-60), at least exp(-50), at least exp(-40), at least exp(-30), at least exp(-20), at least exp(-10), at least exp(-9), at least exp(-8), at least exp(-7), at least exp(-6), at least exp(-5), at least exp(4), at least exp(-3), at least exp(-2), at least exp(-1), or at least exp(0). In some embodiments, the learning rate is pseudo-randomly assigned by the using the random seed to be between exp(-1) and exp(-100), between exp(-20) and exp(-80), between exp(-10) and exp(-50), between exp(-1) and exp(-12), or between exp(-2) and exp(-20). In some embodiments, the L2 regularization penalty is pseudo-randomly assigned by the using the random seed to fall within another range starting no lower than exp(-100) and ending no higher than exp(0).

In some embodiments, each respective initial classifier in the plurality of initial classifiers is assigned a different plurality of values for the respective plurality of hyperparameters (e.g., where each initial classifier has a different, pseudo-randomly assigned hyperparameter configuration).

*Training Classifiers*

As used herein the term "untrained model" *(e.g.,* "untrained classifier" and/or "untrained ensemble classifier") refers to a machine learning model or algorithm such as a classifier or a neural network that has not been trained on a training dataset. In some embodiments, "training a model" refers to the process of training an untrained or partially untrained model. Moreover, it will be appreciated that the term "untrained model" does not exclude the possibility that transfer learning techniques are used in such training of the untrained model. For instance, Fernandes et al., 2017, "Transfer Learning with Partial Observability Applied to Cervical Cancer Screening," Pattern Recognition and Image Analysis: 8th Iberian Conference Proceedings, 243-250, which is hereby incorporated by reference, provides non-limiting examples of such transfer learning. In instances where transfer learning is used, the untrained classifier described above is provided with additional data over and beyond that of the primary training dataset.

Generally, training a classifier *(e.g.,* a neural network and/or an ensemble model) comprises updating the plurality of parameters (e.g., the plurality of weights) for the respective classifier through backpropagation *(e.g.,* gradient descent). First, a forward propagation is performed, in which input data is accepted into the neural network, and an output is calculated based on the selected activation function and an initial set of parameters *(e.g.,* including any hyperparameters selected through the configuration selection process described herein). A backward pass is then performed by calculating an error gradient for each respective parameter *(e.g.,* weight) corresponding to each respective unit in each layer, where the error for each parameter is determined by calculating a loss *(e.g.,* error) based on the network output *(e.g.,* the predicted value) and the input data *(e.g.,* the expected value or true labels).

Parameters are then updated by adjusting the value based on the calculated loss metered by a predetermined learning rate hyperparameter that dictates the degree or severity to which parameters are updated *(e.g.,* small adjustments versus large adjustments), thereby training the neural network.

For example, in some general embodiments of machine learning, backpropagation is a method of training a network with hidden layers comprising a plurality of weights *(e.g.,* embeddings). The output of an untrained model *(e.g.,* the prediction value for an infectious disease state generated by a neural network) is generated using a set of arbitrarily selected initial weights. The output is then compared with the original input *(e.g.,* the corresponding label for the infectious disease state of the respective training subject from which the biological sample is obtained) by evaluating an error function to compute an error *(e.g.,* using a loss function). The weights are then updated such that the error is minimized *(e.g.,* according to the loss function). In some embodiments, any one of a variety of backpropagation algorithms and/or methods are used to update the first and second plurality of weights, as will be apparent to one skilled in the art.

In some embodiments, the error is computed using an error function *(e.g.,* a loss function). In some embodiments, the loss function is mean square error, quadratic loss, mean absolute error, mean bias error, hinge, multi-class support vector machine, and/or cross-entropy. In some embodiments, training the untrained neural network comprises computing an error in accordance with a gradient descent algorithm and/or a minimization function.

In some embodiments, the error function is used to update one or more parameters *(e.g.,* weights) in a neural network by adjusting the value of the one or more parameters *(e.g.,* weights) by an amount proportional to the calculated loss, thereby training the neural network. In some embodiments, the amount by which the parameters are adjusted is metered by a predetermined learning rate that dictates the degree or severity to which parameters are updated *(e.g.,* smaller or larger adjustments). In some embodiments, the learning rate is a hyperparameter that can be selected by a practitioner.

In some embodiments, the training further uses a regularization on the corresponding parameter *(e.g.,* weight) of each hidden neuron in the corresponding plurality of hidden neurons. For example, in some embodiments, a regularization is performed by adding a penalty to the loss function, where the penalty is proportional to the values of the parameters in the trained or untrained neural network.

Generally, regularization reduces the complexity of the model by adding a penalty to one or more parameters to decrease the importance of the respective hidden neurons associated with those parameters. Such practice can result in a more generalized model and reduce overfitting of the data.

In some embodiments, the regularization includes an L1 or L2 penalty. For example, in some preferred embodiments, the regularization includes an L2 penalty on lower and upper weights. In some embodiments, the regularization comprises spatial regularization (e.g., determined based on *a priori* and/or experimental knowledge of biomarker patterns in one or more infectious disease states) or dropout regularization. In some embodiments, the regularization comprises penalties that are independently optimized.

In some embodiments, any of the parameters *(e.g.,* hyperparameters and/or weights) used for initializing and/or training the ensemble classifier are pseudo-randomly assigned *(e.g.,* as described above). In some embodiments, any of the parameters *(e.g.,* hyperparameters and/or weights) used for initializing and/or training the ensemble classifier are selected using a configuration selection process *(e.g.,* as described above).

In some embodiments, training the untrained ensemble classifier forms a trained ensemble classifier following a first evaluation of an error function. In some such embodiments, training the untrained ensemble classifier forms a trained ensemble classifier following a first updating of one or more parameters *(e.g.,* weights) based on a first evaluation of an error function. In some alternative embodiments, training the untrained ensemble classifier forms a trained ensemble classifier following at least 1, at least 2, at least 3, at least 4, at least 5, at least 6, at least 7, at least 8, at least 9, at least 10, at least 20, at least 30, at least 40, at least 50, at least 100, at least 500, at least 1000, at least 10,000, at least 50,000, at least 100,000, at least 200,000, at least 500,000, or at least 1 million evaluations of an error function. In some such embodiments, training the untrained ensemble classifier forms a trained ensemble classifier following at least 1, at least 2, at least 3, at least 4, at least 5, at least 6, at least 7, at least 8, at least 9, at least 10, at least 20, at least 30, at least 40, at least 50, at least 100, at least 500, at least 1000, at least 10,000, at least 50,000, at least 100,000, at least 200,000, at least 500,000, or at least 1 million updatings of one or more parameters (e.g., weights) based on the at least 1, at least 2, at least 3, at least 4, at least 5, at least 6, at least 7, at least 8, at least 9, at least 10, at least 20, at least 30, at least 40, at least 50, at least 100, at least 500, at least 1000, at least 10,000, at least 50,000, at least 100,000, at least 200,000, at least 500,000, or at least 1 million evaluations of an error function.

In some embodiments, training the untrained ensemble classifier forms a trained ensemble classifier when the neural network satisfies a minimum performance requirement. For example, in some embodiments, training the untrained ensemble classifier forms a trained ensemble classifier when the error calculated for the trained ensemble classifier, following an evaluation of an error function across one or more training datasets for a respective one or more training subjects, satisfies an error threshold. In some embodiments, the error calculated by the error function across one or more training datasets for a respective one or more training subjects satisfies an error threshold when the error is less than 20 percent, less than 18 percent, less than 15 percent, less than 10 percent, less than 5 percent, or less than 3 percent.

In some embodiments, training the untrained ensemble classifier forms a trained ensemble classifier when the ensemble classifier satisfies a minimum performance requirement based on a validation training. In some embodiments, validation training is performed through K-fold cross-validation.

In some embodiments, training is performed on a plurality of machines (e.g., computers and/or systems).

In some embodiments, training an untrained ensemble classifier further comprises fixing one or more parameters in the plurality of parameters *(e.g.,* weights), thereby obtaining a corresponding trained ensemble classifier that can be used to perform determination and/or classification (e.g., of infectious disease states).

Any other parameters and architectures suitable for training are contemplated, as will be apparent to one skilled in the art.

In some embodiments, the method comprises training the ensemble classifier *(e.g.,* obtained using any of the methods described herein) using a training dataset.

In some embodiments, the ensemble model training dataset comprises, in electronic form, for each respective training subject in a plurality of training subjects *(e.g.,* 100 training subjects or more), (i) a corresponding label for the infectious disease state of the respective training subject and (ii) a respective attribute value for each corresponding gene in a plurality of genes obtained from a biological sample of the respective training subject. In some embodiments, training the ensemble classifier uses the same training dataset used for selecting hyperparameters and obtaining the ensemble classifier.

In some embodiments, the ensemble classifier is trained using a corresponding label for the infectious disease state of each respective training subject in the plurality of training subjects. In some embodiments, the ensemble classifier is trained using a plurality of corresponding labels for the infectious disease states of the plurality of training subjects. In some embodiments, the infectious state is any of the infectious disease states described above (*see,* Subjects).

As described above, the output layer of a neural network generates, in some embodiments, a prediction value. In some embodiments, the output is a score *(e.g.,* an indication and/or a probability) that an input *(e.g.,* an attribute value for a gene in the plurality of genes) belongs to one or more predetermined classes *(e.g.,* infectious disease states).

In some embodiments, the ensemble classifier provides only a single-class output *(e.g.,* infected or not infected, bacterial infection or not bacterial infection, *etc.).* In some embodiments, the ensemble classifier provides a multi-class output *(e.g.,* infected with a bacteria, infected with a virus, not infected, sepsis, no sepsis, severe, not severe, inflammation, no inflammation, *etc.).* In some embodiments, the ensemble classifier provides a probability that a respective subject has a respective infectious disease state *(e.g.,* a value from 0-1, a value from 0 to 100, and/or a percentage from 0-100%, *etc.).* In some embodiments, the ensemble classifier provides a binary indication that a respective subject has a respective infectious disease state *(e.g.,* an indication of presence or absence, a positive or negative result, a yes/no result, *etc.).* In some embodiments, additional outputs are possible where probabilities and/or indications cannot be accurately determined *(e.g.,* ambiguous, inconclusive, indeterminate, *etc.).*

In some embodiments, a separate determination can be calculated for any one of the plurality of possible infectious disease states. In some embodiments, a separate determination is calculated for at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, or at least 20 possible infectious disease states.

*Determining Infectious Disease States*

Referring to Block 226, in some embodiments, the method further comprises obtaining a test dataset *(e.g.,* a test dataset 130, as illustrated in Figure 1) comprising, in electronic form, a respective attribute value (e.g., attribute values 134) for each corresponding gene in the plurality of genes obtained from a biological sample of a test subject *(e.g.,* test subject 132), and using the ensemble classifier to determine the infectious disease state of the test subject (e.g., using a classification module 146), based on at least the plurality of attribute values for the plurality of genes.

In some embodiments, the test subject is a subject that is applied to a trained model *(e.g.,* a machine learning algorithm, a neural network, and/or an ensemble classifier). In some embodiments, a test subject is a subject for which the corresponding label *(e.g.,* an infectious disease state and/or a disease condition) is unknown. In some embodiments, the trained model is used to generate an output *(e.g.,* a score, a classification, and/or a determination) based at least in part on a plurality of mRNA abundance values for a plurality of biomarkers obtained from a biological sample of test subject. For example, in some embodiments, the trained model is used to generate a determination of an infectious disease state in the test subject. In some such embodiments, the trained model accepts as input one or more datasets (e.g., test datasets) for each respective test subject.

As disclosed herein, any test subject, biological sample obtained from a test subject, test dataset, infectious disease state, plurality of genes, test subject attribute values and methods of measurement thereof, trained and untrained ensemble classifier including methods of classifier selection, training, and use thereof, and classifier architecture including inputs, outputs, parameters, hyperparameters, and functions, shall be considered to include any of the embodiments as for the plurality of training subjects, biological samples obtained from the plurality of training subjects, training dataset, infectious disease states, plurality of genes, training subject attribute values and methods of measurement thereof, trained and untrained ensemble classifier including methods of classifier selection, training, and use thereof, and/or classifier architecture including inputs, outputs, parameters, hyperparameters, and functions, as described in the preceding sections, and/or any substitutions, modifications, additions, deletions, and/or combinations thereof, as will be apparent to one skilled in the art.

For example, in some embodiments, the biological sample is a blood sample of the test subject. In some embodiments, the biological sample comprises blood, whole blood, plasma, serum, urine, cerebrospinal fluid, fecal, saliva, sweat, tears, pleural fluid, pericardial fluid, peritoneal fluid, nasal swabs, nasopharyngeal swabs, or oropharyngeal swabs of the test subject.

In some embodiments, the plurality of genes used for the determining of the infectious disease state is the same plurality of genes used for the obtaining the classifier and the training the classifier, as described in the preceding sections. For example, in some embodiments, each gene in the plurality of genes is selected for use in a biomarker panel *(e.g.,* via detection of an mRNA transcript for the gene). In some embodiments, the plurality of genes comprises at least 20 genes selected from Table 1. In some embodiments, the plurality of genes comprises at least 20 genes selected from Table 2. In some embodiments, the plurality of genes comprises at least 20 genes selected from Table 9. In some embodiments, the plurality of genes comprises at least 29 genes selected from Table 1. In some embodiments, the plurality of genes comprises at least 29 genes selected from Table 2. In some embodiments, the plurality of genes comprises at least 29 genes selected from Table 9. In some embodiments, the attribute value for each corresponding gene in the plurality of genes is obtained using real-time quantitative isothermal amplification on one or more nucleic acid molecules in the biological sample of the test subject. In some embodiments, the real-time quantitative isothermal amplification is real-time quantitative loop-mediated isothermal amplification (LAMP). In some embodiments, the attribute value for each corresponding gene in the plurality of genes is mRNA abundance data. In some embodiments, the plurality of genes is a panel of genes selected for use in a biomarker panel *(e.g.,* comprising at least 20 genes selected from one or more of Table 1, Table 2, and Table 9), and the panel of genes is also used for selection of hyperparameters and training the ensemble classifier.

In some embodiments, the ensemble classifier is a trained ensemble classifier *(e.g.,* as described above). In some embodiments, the infectious disease state determined for the test subject is one or more of: infected with a bacteria, infected with a virus, not-infected, sepsis, and severity. In some embodiments, the infectious disease state determined for the test subject further comprises an indication *(e.g.,* a probability for one or more labels, a binary indication, and/or a classification label) of whether or not the test subject has the infectious disease state.

For instance, in some embodiments, the ensemble classifier *(e.g.,* obtained as described in the section entitled "Selection of Configurations," above) comprises at least 2, at least 3, at least 4, at least 5, at least 6, at least 7, at least 8, at least 9, at least 10, at least 20, at least 30, at least 40, at least 50, at least 60, at least 70, at least 80, at least 90, at least 100, at least 200, at least 300, at least 400, or at least 500 classifiers. In some embodiments, the ensemble classifier comprises no more than 500, no more than 400, no more than 300, no more than 200, no more than 100, no more than 50, no more than 40, no more than 30, or no more than 20 classifiers. In some embodiments, the ensemble classifier comprises between 1 and 50, between 2 and 20, between 5 and 50, between 10 and 80, between 5 and 15, between 3 and 30, between 10 and 500, between 2 and 100, or between 50 and 100 classifiers. In some embodiments, the plurality of selected classifiers that forms the ensemble classifier falls within another range starting no lower than 1 and ending no higher than 500.

In some embodiments, the ensemble classifier comprises at least 10, at least 11, at least 12, at least 13, at least 14, at least 15, at least 16, at least 17, at least 18, at least 19, at least 20, at least 30, at least 40, at least 50, at least 60, at least 70, at least 80, at least 90, at least 100, at least 200, at least 300, at least 400, at least 500, at least 600, at least 700, at least 800, at least 900, at least 1000, at least 2000, at least 3000, at least 4000, at least 5000, at least 6000, at least 7000, at least 8000, at least 9000, at least 10,000, at least 15,000, at least 20,000, at least 30,000, at least 40,000, at least 50,000, at least 60,000, at least 70,000, at least 80,000, at least 90,000, at least 100,000, or at least 200,000 neurons across the plurality of classifiers in the ensemble classifier. In some embodiments, the ensemble classifier comprises no more than 200,000, no more than 100,000, no more than 50,000, no more than 30,000, no more than 20,000, no more than 15,000, no more than 10,000, no more than 9000, no more than 8000, no more than 7000, no more than 6000, no more than 5000, no more than 4000, no more than 3000, no more than 2000, no more than 1000, no more than 900, no more than 800, no more than 700, no more than 600, no more than 500, no more than 400, no more than 300, no more than 200, no more than 100, or no more than 50 neurons across the plurality of classifiers in the ensemble classifier. In some embodiments, the ensemble classifier comprises between 10 and 200, between 20 and 500, between 100 and 800, between 500 and 2000, between 1000 and 5000 neurons, between 5000 and 10,000, between 10,000 and 15,000, between 15,000 and 20,000, or between 20,000 and 30,000 neurons. In some embodiments, the ensemble classifier comprises a plurality of neurons that falls within a range starting no lower than 10 and ending no higher than 200,000 neurons, across the plurality of classifiers in the ensemble classifier.

In some embodiments, the determination of the infectious disease state of the test subject comprises at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, or at least 20 separate indications for a corresponding one or more infectious disease states. In some embodiments, the determination of the infectious disease state of the test subject comprises between 2 and 8 separate indications for a corresponding one or more infectious disease states.

For example, in some embodiments, the determination of the infectious disease state of the test subject comprises an indication for whether or not a subject has a bacterial infection, whether or not a subject has a viral infection, whether or not a subject has sepsis, and/or a severity of a disease *(e.g.,* infectious or noninfectious) in the subject. Thus, in some implementations, a subject can be determined to have, *e.g.,* a bacterial infection with low severity, a bacterial infection with high severity, a viral infection with low severity, and/or a viral infection with high severity, each of which can provide differential conclusions that indicate the appropriate course of action and thus are highly clinically actionable *(e.g.,* administration of antibiotics, administration of broad-spectrum antibiotics, admission and/or discharge from intensive care unit, and/or other diagnoses).

In some embodiments, the determination of the infectious disease state of the test subject comprises one or more scores for the plurality of indicators that are determined based on a sensitivity and/or specificity of detection of the biomarker. For example, a determination of an infectious disease state with varying measures of sensitivity and/or specificity can be stratified according to one or more thresholds or ranges of acceptable values. Thus, in some implementations, a determination with a high sensitivity (*e.g.,* 95-99%; "LR" ~0.05) is classified as "very unlikely"; a determination with a moderate sensitivity (*e.g.,* 71-91%; "LR" ~0.3) is classified as "unlikely"; a determination with a moderate specificity (*e.g.,* 83-96%; "LR" ~1.0) is classified as "possible"; and a determination with a high specificity (*e.g.,* 96-99%; "LR" ~10) is classified as "very likely". Other suitable types of stratified indications include thresholds for predicted probabilities of various degrees of severity, inflammation, and/or sepsis, such that high output probabilities (*e.g.,* 80-100%) are accompanied by a first annotation (*e.g.,* "likely high"), moderate output probabilities (*e.g.,* 50-80%) are accompanied by a second annotation (*e.g.,* "moderate"), low output probabilities (*e.g.,* 0-50%) are accompanied by a third annotation (*e.g.,* "likely low"), and so on. In some embodiments, an indication for whether or not a subject has a bacterial infection, whether or not a subject has a viral infection, whether or not a subject has sepsis, and/or a severity of a disease is determined based upon one or more risk scores (*e.g.,* a stratified scale between 0-40). For example, as illustrated in Figure 11, in some embodiments, a bacterial and/or a viral infection is determined as "very unlikely" based upon a risk score of 0 to 10, "unlikely" based upon a risk score of 10 to 20, "possible" based upon a risk score of 20 to 30, and "very likely" based upon a risk score of 30 to 40. Additionally, as illustrated in Figure 11, in some embodiments, a severity is determined as "likely low" based upon a risk score of 0 to 10, "moderate" based upon a risk score of 10 to 30, and "likely high" based upon a risk score of 30 to 40.

Other possible indications for infectious disease states can include an indication for whether an infectious disease agent (*e.g.,* a bacterial and/or a virus) is "alive" or "dead." In some embodiments, an indication of an infectious disease state includes a notation indicating one or more classes (*e.g.,* 0 = bacterial, 1= viral, 2 = noninfected; and/or 0 = alive, 1 = dead; *etc.*). Various embodiments for indications of infectious disease states provided by an ensemble classifier are possible in addition to those provided here, as will be apparent to one skilled in the art.

In some embodiments, the attribute values (*e.g.,* mRNA abundance levels) of the plurality of genes (*e.g.,* biomarkers) for a respective test subject are compared to time-matched reference values ranges for one or more reference subjects (*e.g.,* non-infected or infected subjects).

For example, in some embodiments, the method further comprises obtaining a reference dataset comprising, in electronic form, a respective attribute value for each corresponding gene in a plurality of genes obtained from a biological sample of a reference subject (*e.g.,* a time-matched reference subject), wherein the reference subject is matched to the test subject based on a corresponding clinical event time (*e.g.,* time-matched on sample collection, study start/time points, clinical trial onset, *etc.*), using the ensemble classifier to determine the infectious disease state of the reference subject, based on at least the plurality of attribute values for the plurality of genes in the reference subject, and comparing the infectious disease state determined for the respective reference subject with the infectious disease state determined for the matched test subject.

*Clinical Applications*

In some embodiments, the methods described herein further include, when the infectious disease state determined for the test subject indicates the presence of an infection (*e.g.,* a bacterial infection and/or a viral infection), administering a first therapeutic regimen tailored for treatment of the subject in the presence of the infection; and when the infectious disease state determined for the test subject indicates the absence of an infection (*e.g.,* no infection), administering a second therapeutic regimen tailored for treatment of the subject in the absence of the infection.

Thus, for example, in some embodiments, a therapeutic regimen is tailored depending on any one or more characteristics related to an infectious disease, including bacterial, viral, noninfectious, sepsis, and/or severity.

In some embodiments, the method comprises treating a subject determined to have (*e.g.,* diagnosed with) an infection, the method comprising: a) receiving information regarding the infectious disease state of the subject according to a method described herein; and b) administering a therapeutically effective amount of an anti-viral agent if the patient is diagnosed with a viral infection or administering an effective amount of an antibiotic if the patient is diagnosed with a bacterial infection.

In certain embodiments, a subject diagnosed with a viral infection by a method described herein is administered a therapeutically effective dose of an antiviral agent, such as a broad-spectrum antiviral agent, an antiviral vaccine, a neuraminidase inhibitor (*e.g.,* zanamivir (Relenza) and oseltamivir (Tamiflu)), a nucleoside analogue (*e.g.,* acyclovir, zidovudine (AZT), and lamivudine), an antisense antiviral agent (*e.g.,* phosphorothioate antisense antiviral agents (*e.g.,* Fomivirsen (Vitravene) for cytomegalovirus retinitis), morpholino antisense antiviral agents), an inhibitor of viral uncoating (*e.g.,* Amantadine and rimantadine for influenza, Pleconaril for rhinoviruses), an inhibitor of viral entry (*e.g.,* Fuzeon for HIV), an inhibitor of viral assembly (*e.g.,* Rifampicin), or an antiviral agent that stimulates the immune system (*e.g.,* interferons). Exemplary antiviral agents include Abacavir, Aciclovir, Acyclovir, Adefovir, Amantadine, Amprenavir, Ampligen, Arbidol, Atazanavir, Atripla (fixed dose drug), Balavir, Cidofovir, Combivir (fixed dose drug), Dolutegravir, Darunavir, Delavirdine, Didanosine, Docosanol, Edoxudine, Efavirenz, Emtricitabine, Enfuvirtide, Entecavir, Ecoliever, Famciclovir, Fixed dose combination (antiretroviral), Fomivirsen, Fosamprenavir, Foscarnet, Fosfonet, Fusion inhibitor, Ganciclovir, Ibacitabine, Imunovir, Idoxuridine, Imiquimod, Indinavir, Inosine, Integrase inhibitor, Interferon type III, Interferon type II, Interferon type I, Interferon, Lamivudine, Lopinavir, Loviride, Maraviroc, Moroxydine, Methisazone, Nelfinavir, Nevirapine, Nexavir, Nitazoxanide, Nucleoside analogues, Novir, Oseltamivir (Tamiflu), Peginterferon alfa-2a, Penciclovir, Peramivir, Pleconaril, Podophyllotoxin, Protease inhibitor, Raltegravir, Reverse transcriptase inhibitor, Ribavirin, Rimantadine, Ritonavir, Pyramidine, Saquinavir, Sofosbuvir, Stavudine, Synergistic enhancer (antiretroviral), Telaprevir, Tenofovir, Tenofovir disoproxil, Tipranavir, Trifluridine, Trizivir, Tromantadine, Truvada, Valaciclovir (Valtrex), Valganciclovir, Vicriviroc, Vidarabine, Viramidine, Zalcitabine, Zanamivir (Relenza), and Zidovudine.

In certain embodiments, a subject diagnosed with a bacterial infection by a method described herein is administered a therapeutically effective dose of an antibiotic. Antibiotics may include broad spectrum, bactericidal, or bacteriostatic antibiotics. Exemplary antibiotics include aminoglycosides such as Amikacin, Amikin, Gentamicin, Garamycin, Kanamycin, Kantrex, Neomycin, Neo-Fradin, Netilmicin, Netromycin, Tobramycin, Nebcin, Paromomycin, Humatin, Streptomycin, Spectinomycin(Bs), and Trobicin; ansamycins such as Geldanamycin, Herbimycin, Rifaximin, and Xifaxan; carbacephems such as Loracarbef and Lorabid; carbapenems such as Ertapenem, Invanz, Doripenem, Doribax, Imipenem/Cilastatin, Primaxin, Meropenem, and Merrem; cephalosporins such as Cefadroxil, Duricef, Cefazolin, Ancef, Cefalotin or Cefalothin, Keflin, Cefalexin, Keflex, Cefaclor, Distaclor, Cefamandole, Mandol, Cefoxitin, Mefoxin, Cefprozil, Cefzil, Cefuroxime, Ceftin, Zinnat, Cefixime, Cefdinir, Cefditoren, Cefoperazone, Cefotaxime, Cefpodoxime, Ceftazidime, Ceftibuten, Ceftizoxime, Ceftriaxone, Cefepime, Maxipime, Ceftaroline fosamil, Teflaro, Ceftobiprole, and Zeftera; glycopeptides such as Teicoplanin, Targocid, Vancomycin, Vancocin, Telavancin, Vibativ, Dalbavancin, Dalvance, Oritavancin, and Orbactiv; lincosamides such as Clindamycin, Cleocin, Lincomycin, and Lincocin; lipopeptides such as Daptomycin and Cubicin; macrolides such as Azithromycin, Zithromax, Surnamed, Xithrone, Clarithromycin, Biaxin, Dirithromycin, Dynabac, Erythromycin, Erythocin, Erythroped, Roxithromycin, Troleandomycin, Tao, Telithromycin, Ketek, Spiramycin, and Rovamycine; monobactams such as Aztreonam and Azactam; nitrofurans such as Furazolidone, Furoxone, Nitrofurantoin, Macrodantin, and Macrobid; oxazolidinones such as Linezolid, Zyvox, VRSA, Posizolid, Radezolid, and Torezolid; penicillins such as Penicillin V, Veetids (Pen-Vee-K), Piperacillin, Pipracil, Penicillin G, Pfizerpen, Temocillin, Negaban, Ticarcillin, and Ticar; penicillin combinations such as Amoxicillin/clavulanate, Augmentin, Ampicillin/sulbactam, Unasyn, Piperacillin/tazobactam, Zosyn, Ticarcillin/clavulanate, and Timentin; polypeptides such as Bacitracin, Colistin, Coly-Mycin-S, and Polymyxin B; quinolones/fluoroquinolones such as Ciprofloxacin, Cipro, Ciproxin, Ciprobay, Enoxacin, Penetrex, Gatifloxacin, Tequin, Gemifloxacin, Factive, Levofloxacin, Levaquin, Lomefloxacin, Maxaquin, Moxifloxacin, Avelox, Nalidixic acid, NegGram, Norfloxacin, Noroxin, Ofloxacin, Floxin, Ocuflox Trovafloxacin, Trovan, Grepafloxacin, Raxar, Sparfloxacin, Zagam, Temafloxacin, and Omniflox; sulfonamides such as Amoxicillin, Novamox, Amoxil, Ampicillin, Principen, Azlocillin, Carbenicillin, Geocillin, Cloxacillin, Tegopen, Dicloxacillin, Dynapen, Flucloxacillin, Floxapen, Mezlocillin, Mezlin, Methicillin, Staphcillin, Nafcillin, Unipen, Oxacillin, Prostaphlin, Penicillin G, Pentids, Mafenide, Sulfamylon, Sulfacetamide, Sulamyd, Bleph-10, Sulfadiazine, Micro-Sulfon, Silver sulfadiazine, Silvadene, Sulfadimethoxine Di-Methox, Albon, Sulfamethizole, Thiosulfil Forte, Sulfamethoxazole, Gantanol, Sulfanilimide, Sulfasalazine, Azulfidine, Sulfisoxazole, Gantrisin, Trimethoprim-Sulfamethoxazole (Co-trimoxazole) (TMP-SMX), Bactrim, Septra, Sulfonamidochrysoidine, and Prontosil; tetracyclines such as Demeclocycline, Declomycin, Doxycycline, Vibramycin, Minocycline, Minocin, Oxytetracycline, Terramycin, Tetracycline and Sumycin, Achromycin V, and Steclin; drugs against mycobacteria such as Clofazimine, Lamprene, Dapsone, Avlosulfon, Capreomycin, Capastat, Cycloserine, Seromycin, Ethambutol, Myambutol, Ethionamide, Trecator, Isoniazid, I.N.H., Pyrazinamide, Aldinamide, Rifampicin, Rifadin, Rimactane, Rifabutin, Mycobutin, Rifapentine, Priftin, and Streptomycin; others antibiotics such as Arsphenamine, Salvarsan, Chloramphenicol, Chloromycetin, Fosfomycin, Monurol, Monuril, Fusidic acid, Fucidin, Metronidazole, Flagyl, Mupirocin, Bactroban, Platensimycin, Quinupristin/Dalfopristin, Synercid, Thiamphenicol, Tigecycline, Tigacyl, Tinidazole, Tindamax Fasigyn, Trimethoprim, Proloprim, and Trimpex.

*Additional Embodiments*

Another aspect of the present disclosure provides a method 300, with reference to Figure 3.

Referring to Block 302, the present disclosure provides a method for determining an infectious disease state of a test subject, at a computer system comprising at least one processor and a memory storing at least one program for execution by the at least one processor.

Referring to Block 304, the method comprises obtaining, in electronic form, a dataset *(e.g.,* a test dataset 130, as illustrated in Figure 1) comprising a respective attribute value *(e.g.,* attribute values 134) for each corresponding gene in a plurality of genes obtained from a biological sample of the test subject *(e.g.,* test subject 132), thereby obtaining a plurality of attribute values, where the plurality of genes comprises at least 20 genes selected from Table 1, at least 20 genes selected from Table 2, and/or at least 20 genes from Table 9.

Referring to Block 306, responsive to inputting the plurality of attribute values to a trained classifier, a determination is obtained, as output from the trained classifier, as to whether the test subject has an infectious disease state selected from: infected with a bacteria, infected with a virus, and not-infected *(e.g.,* where the determination is obtained using a classification module 146, based at least in part on attribute values 134 for test subject 132 in test dataset 130).

As disclosed herein, any test subject, biological sample obtained from a test subject, test dataset, infectious disease state, plurality of genes, test subject attribute values and methods of measurement thereof, trained and untrained ensemble classifier including methods of classifier selection, training, and use thereof, and classifier architecture including inputs, outputs, parameters, hyperparameters, and functions, in the following sections, shall be considered to include any of the embodiments as for the plurality of training subjects, biological samples obtained from the plurality of training subjects, training dataset, infectious disease states, plurality of genes, training subject attribute values and methods of measurement thereof, trained and untrained ensemble classifier including methods of classifier selection, training, and use thereof, and/or classifier architecture including inputs, outputs, parameters, hyperparameters, and functions, as described in the preceding sections, and/or any substitutions, modifications, additions, deletions, and/or combinations thereof, as will be apparent to one skilled in the art.

For example, in some embodiments, each gene in the plurality of genes is selected for use in a biomarker panel (*e.g.,* via detection of an mRNA transcript for the gene). In some embodiments, the plurality of genes comprises at least 29 genes selected from Table 1. In some embodiments, the plurality of genes comprises at least 29 genes selected from Table 2. In some embodiments, the plurality of genes comprises at least 29 genes selected from Table 9.

In some embodiments, the plurality of genes comprises at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, at least 45, at least 46, at least 47, or at least 48 genes selected from Table 1. In some embodiments, the plurality of genes comprises at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, or at least 29 genes selected from Table 2. In some embodiments, the plurality of genes comprises at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, at least 45, at least 46, at least 47, at least 48, at least 49, at least 50, at least 51, at least 52, at least 53, at least 54, at least 55, at least 56, at least 57, at least 58, at least 59, at least 60, at least 61, at least 62, at least 63, and at least 64 genes selected from Table 9.

In some embodiments, all of the genes are selected from Table 1. That is, in some embodiments, the plurality of genes consists of 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, or 48 genes selected from Table 1. In some embodiments, the plurality of genes consists of from 5 to 20, from 10 to 30, from 20 to 40, from 15 to 48, or from 10 to 48 genes selected from Table 1. In some embodiments, the plurality of genes falls within another range starting no lower than 5 genes and ending no higher than 48 genes from Table 1.

In some embodiments, all of the genes are selected from Table 2. That is, in some embodiments, the plurality of genes consists of 2, 3, 4, 5, 6, 7, 8, 9, 10, 11, 12, 13, 14, 15, 16, 17, 18, 19, 20, 21, 22, 23, 24, 25, 26, 27, 28, or 29 genes selected from Table 2. In some embodiments, the plurality of genes consists of from 10 to 15, from 10 to 25, from 5 to 20, from 10 to 29, or from 15 to 29 genes selected from Table 2. In some embodiments, the plurality of genes falls within another range starting no lower than 5 genes and ending no higher than 29 genes from Table 2.

In some embodiments, all of the genes are selected from Table 9. That is, in some embodiments, the plurality of genes consists of 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, or 64 genes selected from Table 9. In some embodiments, the plurality of genes consists of from 5 to 20, from 10 to 30, from 20 to 40, from 30 to 50, or from 40 to 60 genes selected from Table 9. In some embodiments, the plurality of genes falls within another range starting no lower than 5 genes and ending no higher than 64 genes from Table 9.

In some embodiments, the plurality of genes comprises at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, at least 45, at least 46, at least 47, or at least 48 genes. In some embodiments, the plurality of genes comprises at least 10, at least 20, at least 30, at least 40, at least 50, at least 60, at least 70, at least 80, at least 90, or at least 100 genes. In some embodiments, the plurality of genes comprises at least 100, at least 200, at least 300, at least 400, at least 500, at least 600, at least 700, at least 800, at least 900, or at least 1000 genes.

In some embodiments, the plurality of genes comprises no more than 2000, no more than 1000, no more than 900, no more than 800, no more than 700, no more than 600, no more than 500, no more than 400, no more than 300, no more than 200, no more than 100, no more than 90, no more than 80, no more than 70, no more than 60, no more than 50, no more than 40, or no more than 30 genes. In some embodiments, the plurality of genes comprises between 5 and 10, between 2 and 50, between 10 and 200, between 20 and 500, between 10 and 80, between 30 and 100, between 100 and 1000, between 300 and 2000, or between 1000 and 2000 genes. In some embodiments, the plurality of genes includes between 15 genes and 50 genes. In some embodiments, the plurality of genes includes between 15 genes and 40 genes. In some embodiments, the plurality of genes includes between 15 genes and 30 genes. In some embodiments, the plurality of genes includes between 20 genes and 50 genes. In some embodiments, the plurality of genes includes between 20 genes and 40 genes. In some embodiments, the plurality of genes includes between 20 genes and 30 genes. In some embodiments, the plurality of genes includes between 25 genes and 50 genes. In some embodiments, the plurality of genes includes between 25 genes and 40 genes. In some embodiments, the plurality of genes includes between 25 genes and 35 genes. In some embodiments, the plurality of genes includes between 25 genes and 30 genes. In some embodiments, the plurality of genes falls within another range starting no lower than 10 genes and ending no higher than 2000 genes.

In some embodiments, the biological sample is a blood sample of the test subject. In some embodiments, the biological sample comprises blood, whole blood, plasma, serum, urine, cerebrospinal fluid, fecal, saliva, sweat, tears, pleural fluid, pericardial fluid, peritoneal fluid, nasal swabs, nasopharyngeal swabs, or oropharyngeal swabs of the test subject. In some embodiments, the attribute value for each corresponding gene in the plurality of genes is obtained using real-time quantitative isothermal amplification on one or more nucleic acid molecules in the biological sample of the test subject. In some embodiments, the real-time quantitative isothermal amplification is real-time quantitative loop-mediated isothermal amplification (LAMP). In some embodiments, the attribute value for each corresponding gene in the plurality of genes is mRNA abundance data.

In some embodiments, the infectious disease state determined for the test subject further comprises one or more of: infected with a bacteria, infected with a virus, not-infected, sepsis, and severity. In some embodiments, the infectious disease state determined for the test subject further comprises an indication of whether or not the test subject has the infectious disease state.

In some embodiments, the method further comprises obtaining a reference dataset comprising, in electronic form, a respective attribute value for each corresponding gene in a plurality of genes obtained from a biological sample of a reference subject *(e.g.,* a time-matched reference subject), where the reference subject is matched to the test subject based on a corresponding clinical event time, using the trained classifier to determine the infectious disease state of the reference subject, based on at least the plurality of attribute values for the plurality of genes in the reference subject, and comparing the infectious disease state determined for the respective reference subject with the infectious disease state determined for the matched test subject.

Referring to Block 310, in some embodiments, the method further comprises, when the infectious disease state determined for the test subject indicates the presence of an infection *(e.g.,* a bacterial infection and/or a viral infection), administering a first therapeutic regimen tailored for treatment of the subject in the presence of the infection; and when the infectious disease state determined for the test subject indicates the absence of an infection *(e.g.,* no infection), administering a second therapeutic regimen tailored for treatment of the subject in the absence of the infection.

In some embodiments, a therapeutic regimen is tailored depending on any one or more characteristics related to an infectious disease, including bacterial, viral, noninfectious, sepsis, and/or severity.

In some embodiments, the method comprises treating a subject determined to have *(e.g.,* diagnosed with) an infection, the method comprising: a) receiving information regarding the infectious disease state of the subject according to a method described herein; and b) administering a therapeutically effective amount of an anti-viral agent if the patient is diagnosed with a viral infection or administering an effective amount of an antibiotic if the patient is diagnosed with a bacterial infection.

In certain embodiments, a subject diagnosed with a viral infection by a method described herein is administered a therapeutically effective dose of an antiviral agent, such as a broad-spectrum antiviral agent, an antiviral vaccine, a neuraminidase inhibitor (e.g., zanamivir (Relenza) and oseltamivir (Tamiflu)), a nucleoside analogue *(e.g.,* acyclovir, zidovudine (AZT), and lamivudine), an antisense antiviral agent *(e.g.,* phosphorothioate antisense antiviral agents *(e.g.,* Fomivirsen (Vitravene) for cytomegalovirus retinitis), morpholino antisense antiviral agents), an inhibitor of viral uncoating *(e.g.,* Amantadine and rimantadine for influenza, Pleconaril for rhinoviruses), an inhibitor of viral entry *(e.g.,* Fuzeon for HIV), an inhibitor of viral assembly *(e.g.,* Rifampicin), or an antiviral agent that stimulates the immune system *(e.g.,* interferons). Exemplary antiviral agents include Abacavir, Aciclovir, Acyclovir, Adefovir, Amantadine, Amprenavir, Ampligen, Arbidol, Atazanavir, Atripla (fixed dose drug), Balavir, Cidofovir, Combivir (fixed dose drug), Dolutegravir, Darunavir, Delavirdine, Didanosine, Docosanol, Edoxudine, Efavirenz, Emtricitabine, Enfuvirtide, Entecavir, Ecoliever, Famciclovir, Fixed dose combination (antiretroviral), Fomivirsen, Fosamprenavir, Foscarnet, Fosfonet, Fusion inhibitor, Ganciclovir, Ibacitabine, Imunovir, Idoxuridine, Imiquimod, Indinavir, Inosine, Integrase inhibitor, Interferon type III, Interferon type II, Interferon type I, Interferon, Lamivudine, Lopinavir, Loviride, Maraviroc, Moroxydine, Methisazone, Nelfinavir, Nevirapine, Nexavir, Nitazoxanide, Nucleoside analogues, Novir, Oseltamivir (Tamiflu), Peginterferon alfa-2a, Penciclovir, Peramivir, Pleconaril, Podophyllotoxin, Protease inhibitor, Raltegravir, Reverse transcriptase inhibitor, Ribavirin, Rimantadine, Ritonavir, Pyramidine, Saquinavir, Sofosbuvir, Stavudine, Synergistic enhancer (antiretroviral), Telaprevir, Tenofovir, Tenofovir disoproxil, Tipranavir, Trifluridine, Trizivir, Tromantadine, Truvada, Valaciclovir (Valtrex), Valganciclovir, Vicriviroc, Vidarabine, Viramidine, Zalcitabine, Zanamivir (Relenza), and Zidovudine.

In certain embodiments, a subject diagnosed with a bacterial infection by a method described herein is administered a therapeutically effective dose of an antibiotic. Antibiotics may include broad spectrum, bactericidal, or bacteriostatic antibiotics. Exemplary antibiotics include aminoglycosides such as Amikacin, Amikin, Gentamicin, Garamycin, Kanamycin, Kantrex, Neomycin, Neo-Fradin, Netilmicin, Netromycin, Tobramycin, Nebcin, Paromomycin, Humatin, Streptomycin, Spectinomycin(Bs), and Trobicin; ansamycins such as Geldanamycin, Herbimycin, Rifaximin, and Xifaxan; carbacephems such as Loracarbef and Lorabid; carbapenems such as Ertapenem, Invanz, Doripenem, Doribax, Imipenem/Cilastatin, Primaxin, Meropenem, and Merrem; cephalosporins such as Cefadroxil, Duricef, Cefazolin, Ancef, Cefalotin or Cefalothin, Keflin, Cefalexin, Keflex, Cefaclor, Distaclor, Cefamandole, Mandol, Cefoxitin, Mefoxin, Cefprozil, Cefzil, Cefuroxime, Ceftin, Zinnat, Cefixime, Cefdinir, Cefditoren, Cefoperazone, Cefotaxime, Cefpodoxime, Ceftazidime, Ceftibuten, Ceftizoxime, Ceftriaxone, Cefepime, Maxipime, Ceftaroline fosamil, Teflaro, Ceftobiprole, and Zeftera; glycopeptides such as Teicoplanin, Targocid, Vancomycin, Vancocin, Telavancin, Vibativ, Dalbavancin, Dalvance, Oritavancin, and Orbactiv; lincosamides such as Clindamycin, Cleocin, Lincomycin, and Lincocin; lipopeptides such as Daptomycin and Cubicin; macrolides such as Azithromycin, Zithromax, Surnamed, Xithrone, Clarithromycin, Biaxin, Dirithromycin, Dynabac, Erythromycin, Erythocin, Erythroped, Roxithromycin, Troleandomycin, Tao, Telithromycin, Ketek, Spiramycin, and Rovamycine; monobactams such as Aztreonam and Azactam; nitrofurans such as Furazolidone, Furoxone, Nitrofurantoin, Macrodantin, and Macrobid; oxazolidinones such as Linezolid, Zyvox, VRSA, Posizolid, Radezolid, and Torezolid; penicillins such as Penicillin V, Veetids (Pen-Vee-K), Piperacillin, Pipracil, Penicillin G, Pfizerpen, Temocillin, Negaban, Ticarcillin, and Ticar; penicillin combinations such as Amoxicillin/clavulanate, Augmentin, Ampicillin/sulbactam, Unasyn, Piperacillin/tazobactam, Zosyn, Ticarcillin/clavulanate, and Timentin; polypeptides such as Bacitracin, Colistin, Coly-Mycin-S, and Polymyxin B; quinolones/fluoroquinolones such as Ciprofloxacin, Cipro, Ciproxin, Ciprobay, Enoxacin, Penetrex, Gatifloxacin, Tequin, Gemifloxacin, Factive, Levofloxacin, Levaquin, Lomefloxacin, Maxaquin, Moxifloxacin, Avelox, Nalidixic acid, NegGram, Norfloxacin, Noroxin, Ofloxacin, Floxin, Ocuflox Trovafloxacin, Trovan, Grepafloxacin, Raxar, Sparfloxacin, Zagam, Temafloxacin, and Omniflox; sulfonamides such as Amoxicillin, Novamox, Amoxil, Ampicillin, Principen, Azlocillin, Carbenicillin, Geocillin, Cloxacillin, Tegopen, Dicloxacillin, Dynapen, Flucloxacillin, Floxapen, Mezlocillin, Mezlin, Methicillin, Staphcillin, Nafcillin, Unipen, Oxacillin, Prostaphlin, Penicillin G, Pentids, Mafenide, Sulfamylon, Sulfacetamide, Sulamyd, Bleph-10, Sulfadiazine, Micro-Sulfon, Silver sulfadiazine, Silvadene, Sulfadimethoxine Di-Methox, Albon, Sulfamethizole, Thiosulfil Forte, Sulfamethoxazole, Gantanol, Sulfanilimide, Sulfasalazine, Azulfidine, Sulfisoxazole, Gantrisin, Trimethoprim-Sulfamethoxazole (Co-trimoxazole) (TMP-SMX), Bactrim, Septra, Sulfonamidochrysoidine, and Prontosil; tetracyclines such as Demeclocycline, Declomycin, Doxycycline, Vibramycin, Minocycline, Minocin, Oxytetracycline, Terramycin, Tetracycline and Sumycin, Achromycin V, and Steclin; drugs against mycobacteria such as Clofazimine, Lamprene, Dapsone, Avlosulfon, Capreomycin, Capastat, Cycloserine, Seromycin, Ethambutol, Myambutol, Ethionamide, Trecator, Isoniazid, I.N.H., Pyrazinamide, Aldinamide, Rifampicin, Rifadin, Rimactane, Rifabutin, Mycobutin, Rifapentine, Priftin, and Streptomycin; others antibiotics such as Arsphenamine, Salvarsan, Chloramphenicol, Chloromycetin, Fosfomycin, Monurol, Monuril, Fusidic acid, Fucidin, Metronidazole, Flagyl, Mupirocin, Bactroban, Platensimycin, Quinupristin/Dalfopristin, Synercid, Thiamphenicol, Tigecycline, Tigacyl, Tinidazole, Tindamax Fasigyn, Trimethoprim, Proloprim, and Trimpex. See, for example, the section entitled "Clinical Applications," above.

In some embodiments, the trained classifier is a neural network algorithm (e.g., a multi-layer perceptron, fully connected neural network, and/or partially connected neural network), a support vector machine algorithm, a Naive Bayes algorithm, a nearest neighbor algorithm, a boosted trees algorithm (e.g., XGBoost), a random forest algorithm, a decision tree algorithm, a multinomial logistic regression algorithm, a linear model, or a linear regression algorithm.

Referring to Block 308, in some embodiments, the trained classifier is an ensemble classifier (e.g., where the ensemble classifier is obtained using classifier construction model 136).

For instance, in some embodiments, the ensemble classifier (e.g., obtained as described in the section entitled "Selection of Configurations," above) comprises at least 2, at least 3, at least 4, at least 5, at least 6, at least 7, at least 8, at least 9, at least 10, at least 20, at least 30, at least 40, at least 50, at least 60, at least 70, at least 80, at least 90, at least 100, at least 200, at least 300, at least 400, or at least 500 classifiers. In some embodiments, the ensemble classifier comprises no more than 500, no more than 400, no more than 300, no more than 200, no more than 100, no more than 50, no more than 40, no more than 30, or no more than 20 classifiers. In some embodiments, the ensemble classifier comprises between 1 and 50, between 2 and 20, between 5 and 50, between 10 and 80, between 5 and 15, between 3 and 30, between 10 and 500, between 2 and 100, or between 50 and 100 classifiers. In some embodiments, the plurality of selected classifiers that forms the ensemble classifier falls within another range starting no lower than 1 and ending no higher than 500.

In some embodiments, the ensemble classifier comprises at least 10, at least 11, at least 12, at least 13, at least 14, at least 15, at least 16, at least 17, at least 18, at least 19, at least 20, at least 30, at least 40, at least 50, at least 60, at least 70, at least 80, at least 90, at least 100, at least 200, at least 300, at least 400, at least 500, at least 600, at least 700, at least 800, at least 900, at least 1000, at least 2000, at least 3000, at least 4000, at least 5000, at least 6000, at least 7000, at least 8000, at least 9000, at least 10,000, at least 15,000, at least 20,000, at least 30,000, at least 40,000, at least 50,000, at least 60,000, at least 70,000, at least 80,000, at least 90,000, at least 100,000, or at least 200,000 neurons across the plurality of classifiers in the ensemble classifier. In some embodiments, the ensemble classifier comprises no more than 200,000, no more than 100,000, no more than 50,000, no more than 30,000, no more than 20,000, no more than 15,000, no more than 10,000, no more than 9000, no more than 8000, no more than 7000, no more than 6000, no more than 5000, no more than 4000, no more than 3000, no more than 2000, no more than 1000, no more than 900, no more than 800, no more than 700, no more than 600, no more than 500, no more than 400, no more than 300, no more than 200, no more than 100, or no more than 50 neurons across the plurality of classifiers in the ensemble classifier. In some embodiments, the ensemble classifier comprises between 10 and 200, between 20 and 500, between 100 and 800, between 500 and 2000, between 1000 and 5000 neurons, between 5000 and 10,000, between 10,000 and 15,000, between 15,000 and 20,000, or between 20,000 and 30,000 neurons. In some embodiments, the ensemble classifier comprises a plurality of neurons that falls within a range starting no lower than 10 and ending no higher than 200,000 neurons, across the plurality of classifiers in the ensemble classifier. See, for example, the sections entitled "Selection of Configurations," "Classifiers and Hyperparameters," "Training Classifiers," and "Determining Infectious Disease States," above.

In some embodiments, the trained ensemble classifier is obtained by a method comprising obtaining a training dataset (e.g., a training dataset 122), where the training dataset comprises, in electronic form, for each respective training subject (e.g., training subjects 124 in training dataset 122) in a plurality of training subjects (e.g., 100 training subjects or more), (i) a corresponding label for the infectious disease state of the respective training subject (e.g., labels 126) and (ii) a respective attribute value for each corresponding gene in the plurality of genes (e.g., attribute values 128) obtained from a biological sample of the respective training subject. The method includes, for each respective random seed in a plurality of random seeds (e.g., random seed set 138), performing a corresponding instance of an outer loop, where each corresponding instance of the outer loop is characterized by a respective downsampling rate and a respective maximum iteration rate. The corresponding instance of the outer loop comprises, A) for each respective initial classifier in a plurality of initial classifiers, using the random seed to pseudo-randomly assign values for each respective hyperparameter in a plurality of hyperparameters for the respective initial classifier (e.g., where pseudo-random assignment of values is performed using a hyperparameter assignment construct 140 in classifier construction module 136). Each respective hyperparameter in the plurality of hyperparameters has a respective value selected from a respective plurality of candidate values for the respective hyperparameter, and each respective initial classifier in the plurality of initial classifiers has a corresponding plurality of parameters (e.g., more than 500 parameters).

The corresponding instance of the outer loop further comprises B) binning the plurality of initial classifiers into a plurality of bins, where each bin in the plurality of bins is characterized by a respective initial number of initial classifiers in the plurality of initial classifiers, a respective initial number of iterations, and the downsampling rate. For each respective bin in the plurality of bins, a corresponding inner loop is performed, in which an iteration count is initially set to the respective initial number of iterations.

The corresponding inner loop comprises, i) for a number of iterations equal to the iteration count, training each initial classifier in the respective bin in a K-fold cross-validation context, where the K-fold cross-validation comprises refining each initial classifier in the respective bin against the training dataset using the values assigned for each respective hyperparameter in the plurality of hyperparameters for the respective initial classifier (e.g., using validation construct 142 in the classifier construction module 136), ii) determining, based on the K-fold cross-validation, a corresponding evaluation score for each initial classifier in the respective bin (e.g., using evaluation construct 144 in classifier construction module 136), iii) removing, from the respective bin, a subset of initial classifiers in accordance with the downsampling rate and the corresponding evaluation score for each initial classifier in the respective bin, iv) increasing the iteration count as a function of an inverse of the downsampling rate; and v) repeating the performing i), determining ii), removing iii) and increasing iv) for a number of repetitions that is determined based on a corresponding identity for the respective bin.

The corresponding instance of the outer loop further includes C) selecting, from among all initial classifiers in the plurality of initial classifiers, a corresponding classifier that has the best corresponding evaluation score as representative of the respective random seed in the plurality of random seeds. The ensemble classifier is formed from the corresponding classifier selected by the selecting C) for each respective random seed in the plurality of random seeds.

In some embodiments, the K-fold cross-validation is performed with a value for K that is between 2 and 20 or between 3 and 8. In some embodiments, the performing K-fold cross-validation further comprises, for each initial classifier in the respective bin, obtaining one or more cross-validation scores based on a performance measure of the respective initial classifier, and the determining a corresponding evaluation score for the respective initial classifier is determined from the one or more cross-validation scores obtained from the K-fold cross-validation.

In some embodiments, each respective initial classifier in a plurality of initial classifiers is a neural network algorithm, a support vector machine algorithm, a Naive Bayes algorithm, a nearest neighbor algorithm, a boosted trees algorithm, a random forest algorithm, a decision tree algorithm, a multinomial logistic regression algorithm, a linear model, or a linear regression algorithm. In some embodiments, each respective initial classifier in the plurality of initial classifiers is assigned a different plurality of values for the respective plurality of hyperparameters.

In some embodiments, the ensemble classifier is formed by combining a plurality of outputs obtained from the plurality of classifiers selected by the selecting C). In some embodiments, the plurality of random seeds comprises between 2 and 100 random seeds.

In some embodiments, the method comprises obtaining a single ensemble model.

In some embodiments, the ensemble model provides, as output, a plurality of scores *(e.g.,* probability, label, and/or other indication) for a plurality of different infectious disease states. For example, in some embodiments, the ensemble model provides a first score indicating a first infectious disease state *(e.g.,* infected with a bacteria or not infected with a bacteria), a second score indicating a second infectious disease state other than the first infectious disease state *(e.g.,* infected with a virus or not infected with a virus), and a third score indicating a third infectious disease state *(e.g.,* a severity of disease).

In some embodiments, the ensemble model comprises a plurality of sets of single-label component classifiers, each respective set of classifiers corresponding to a respective different infectious disease state *(e.g.,* a first set of single-label component classifiers corresponding to outputs for bacterial infection, a second set of single-label component classifiers corresponding to outputs for viral infection, and a third set of single-label component classifiers corresponding to outputs for severity). In some such embodiments, each single-label classifier in a respective set of single-label component classifiers provides a score for the respective infectious disease state. Thus, for example, in some such embodiments, the ensemble model is formed by combining a first set of scores from a first set of component classifiers, a second set of scores from a second set of component classifiers, and a third set of scores from a third set of component classifiers, where each respective set of scores indicates a respective different infectious disease state.

For example, referring to Figure 11, in an example embodiment of a determination of an infectious disease state, an output is provided that includes three scores for a respective subject: (i) a probability score for a bacterial etiology, (ii) a probability score for a viral etiology, and (iii) a score for the severity of the subject's condition. An example system for determining three scores for the respective subject is further described in Example 1 and illustrated in Figure 5. Thus, in some embodiments, the single ensemble model provides three scores by combining (i) a first set of bacterial etiology scores provided by a first set of bacterial etiology classifiers, (ii) a second set of viral etiology scores provided by a second set of viral etiology classifiers, and (iii) a third set of severity scores provided by a third set of severity classifiers.

In some embodiments, the ensemble model provides at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 25, at least 30, at least 35, at least 40, at least 45, or at least 50 outputs. In some embodiments, the ensemble model provides no more than 50, no more than 40, no more than 30, no more than 20, no more than 15, or no more than 10 outputs. In some embodiments, the ensemble model provides between 2 and 10, between 5 and 15, between 5 and 20, between 2 and 8, or between 10 and 50 outputs. In some embodiments, the ensemble model comprises at least as many component classifiers as desired outputs *(e.g.,* for different infectious disease states). In some embodiments, the ensemble model comprises the same number of component classifiers as desired outputs.

In some embodiments, the ensemble model comprises a plurality of multi-label component classifiers, each respective multi-label component classifier providing, as output, a plurality of scores (e.g., probability, label, and/or other indication) for a plurality of different infectious disease states. For example, in some embodiments, each component classifier in the ensemble model provides a first score indicating a first infectious disease state (e.g., infected with a bacteria or not infected with a bacteria), a second score indicating a second infectious disease state other than the first infectious disease state *(e.g.,* infected with a virus or not infected with a virus), and a third score indicating a third infectious disease state *(e.g.,* a severity of disease).

Thus, for example, in some such embodiments, the ensemble model is formed by combining, for each respective multi-class classifier in the plurality of multi-class classifiers, a plurality of scores for a respective plurality of different infectious disease states, thus obtaining a final plurality of scores from the ensemble model.

In some embodiments, each multi-class component classifier in the ensemble model provides at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 25, at least 30, at least 35, at least 40, at least 45, or at least 50 outputs. In some embodiments, each multi-class component classifier in the ensemble model provides no more than 50, no more than 40, no more than 30, no more than 20, no more than 15, or no more than 10 outputs. In some embodiments, each multi-class component classifier in the ensemble model provides between 2 and 10, between 5 and 15, between 5 and 20, between 2 and 8, or between 10 and 50 outputs.

Thus, referring again to Figure 11, in some embodiments, the single ensemble model provides three scores by combining (i) a plurality of bacterial etiology scores, (ii) a plurality of viral etiology scores, and (iii) a plurality of severity scores, where the bacterial, viral, and severity scores are obtained from each respective component classifier in the ensemble model.

In some embodiments, the method comprises obtaining a plurality of ensemble models. For example, in some embodiments, the plurality of ensemble models comprises at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 25, at least 30, at least 35, at least 40, at least 45, or at least 50 ensemble models. In some embodiments, the plurality of ensemble models comprises no more than 50, no more than 40, no more than 30, no more than 20, no more than 15, or no more than 10 ensemble models. In some embodiments, the plurality of ensemble models comprises between 2 and 10, between 5 and 15, between 5 and 20, between 2 and 8, or between 10 and 50 ensemble models. In some embodiments, the plurality of ensemble models falls within another range starting no lower than 2 ensemble models and ending no higher than 50 ensemble models. In some embodiments, the plurality of ensemble models comprises at least as many ensemble models as desired outputs (*e.g.*, for different infectious disease states). In some embodiments, the plurality of ensemble models comprises the same number of ensemble models as desired outputs.

In some embodiments, each ensemble model in the plurality of ensemble models provides, as output, an indication of a different infectious disease state. For example, in some embodiments, a first ensemble model provides an output indicating a first infectious disease state (*e.g.*, infected with a bacteria or not infected with a bacteria), and a second ensemble model provides an output indicating a second infectious disease state other than the first infectious disease state (*e.g.*, infected with a virus or not infected with a virus). In some such embodiments, a third ensemble model provides an output indicating a third infectious disease state *(e.g.,* a severity of disease). In some embodiments, each ensemble model in the plurality of ensemble models comprises a respective plurality of selected (*e.g.*, component) classifiers, where each classifier in the plurality of component classifiers in the respective ensemble model similarly provides an output indicating the respective infectious disease state. Thus, for example, in some such embodiments, a respective first ensemble model is formed by combining a plurality of outputs from a plurality of component classifiers, where each output from each respective component classifier is for a respective first infectious disease state, and the combined output from the first ensemble model is for the respective first infectious disease state.

Thus, referring again to Figure 11, in some embodiments, (i) the bacterial etiology score is provided by a first ensemble classifier comprising a plurality of component classifiers, each component classifier providing a component bacterial etiology score, (ii) the viral etiology score is provided by a second ensemble classifier comprising a plurality of component classifiers, each component classifier providing a component viral etiology score, and (iii) the severity score is provided by a third ensemble classifier comprising a plurality of component classifiers, each component classifier providing a component severity score.

Another aspect of the present disclosure provides a computer system for determining an infectious disease state of a subject, the infectious disease state being one or more of infected with a bacteria, infected with a virus, and not-infected, the computer system comprising at least one processor; and a memory storing at least one program for execution by the at least one processor, the at least one program comprising instructions for performing any of the methods and embodiments disclosed herein, and/or any combinations thereof as will be apparent to one skilled in the art.

Another aspect of the present disclosure provides a non-transitory computer-readable storage medium having stored thereon program code instructions that, when executed by a processor, cause the processor to perform a method of determining an infectious disease state of a subject, the infectious disease state being one or more of infected with a bacteria, infected with a virus, and not-infected, the method comprising, at a computer system comprising at least one processor and a memory storing at least one program for execution by the at least one processor, the at least one program comprising instructions for performing any of the methods and embodiments disclosed herein, and/or any combinations thereof as will be apparent to one skilled in the art.

*Compositions*

Another aspect of the present disclosure provides a composition comprising a plurality of amplification primers for determining an infectious disease state of a subject, the plurality of amplification primers comprising, for each respective gene in a plurality of genes comprising at least 20 genes selected from Table 1, at least 20 genes selected from Table 2, and/or at least 20 genes selected from Table 9, a respective forward amplification primer and a respective reverse amplification primer. The respective forward amplification primer comprises a 3' binding region and a 5' auxiliary region, where the 3' binding region consists of from 10 to 50 nucleotides and has a sequence that is complementary to a first target sequence in a first strand of the respective gene or a transcript thereof, and the 5' auxiliary region has a sequence that is not complementary to the sequence of the first strand of the respective gene or a transcript thereof. The respective reverse amplification primer comprises a binding region, wherein the binding region consists of from 10 to 50 nucleotides and has a sequence that is complementary to a second target sequence in the second strand of the respective gene or a transcript thereof.

In some embodiments, the plurality of genes comprises at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, least 39, at least 40, at least 41, at least 42, at least 43, at least 44, at least 45, at least 46, at least 47, or at least 48 genes selected from Table 1. In some embodiments, the plurality of genes comprises at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, or at least 29 genes selected from Table 2. In some embodiments, the plurality of genes comprises at least 1, at least 2, at least 3, at least 4, at least 5, at least 6, at least 7, at least 8, at least 9, at least 10, at least 11, at least 12, least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, least 45, at least 46, at least 47, at least 48, at least 49, at least 50, at least 51, at least 52, at least 53, at least 54, at least 55, at least 56, at least 57, at least 58, at least 59, at least 60, at least 61, at least 62, at least 63, or at least 64 genes selected from Table 9.

In some embodiments, all of the genes are selected from Table 1. That is, in some embodiments, the plurality of genes consists of 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, or 48 genes selected from Table 1. In some embodiments, the plurality of genes consists of from 5 to 20, from 10 to 30, from 20 to 40, from 15 to 48, or from 10 to 48 genes selected from Table 1. In some embodiments, the plurality of genes falls within another range starting no lower than 5 genes and ending no higher than 48 genes from Table 1.

In some embodiments, all of the genes are selected from Table 2. That is, in some embodiments, the plurality of genes consists of 2, 3, 4, 5, 6, 7, 8, 9, 10, 11, 12, 13, 14, 15, 16, 17, 18, 19, 20, 21, 22, 23, 24, 25, 26, 27, 28, or 29 genes selected from Table 2. In some embodiments, the plurality of genes consists of from 10 to 15, from 10 to 25, from 5 to 20, from 10 to 29, or from 15 to 29 genes selected from Table 2. In some embodiments, the plurality of genes falls within another range starting no lower than 5 genes and ending no higher than 29 genes from Table 2.

In some embodiments, all of the genes are selected from Table 9. That is, in some embodiments, the plurality of genes consists of 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, or 64 genes selected from Table 9. In some embodiments, the plurality of genes consists of from 5 to 20, from 10 to 30, from 20 to 40, from 30 to 50, or from 40 to 60 genes selected from Table 9. In some embodiments, the plurality of genes falls within another range starting no lower than 5 genes and ending no higher than 64 genes from Table 9.

In some embodiments, the plurality of genes comprises at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, at least 45, at least 46, at least 47, or at least 48 genes. In some embodiments, the plurality of genes comprises at least 10, at least 20, at least 30, at least 40, at least 50, at least 60, at least 70, at least 80, at least 90, or at least 100 genes. In some embodiments, the plurality of genes comprises at least 100, at least 200, at least 300, at least 400, at least 500, at least 600, at least 700, at least 800, at least 900, or at least 1000 genes.

In some embodiments, the plurality of genes comprises no more than 2000, no more than 1000, no more than 900, no more than 800, no more than 700, no more than 600, no more than 500, no more than 400, no more than 300, no more than 200, no more than 100, no more than 90, no more than 80, no more than 70, no more than 60, no more than 50, no more than 40, or no more than 30 genes. In some embodiments, the plurality of genes comprises between 5 and 10, between 2 and 50, between 10 and 200, between 20 and 500, between 10 and 80, between 30 and 100, between 100 and 1000, between 300 and 2000, or between 1000 and 2000 genes. In some embodiments, the plurality of genes includes between 15 genes and 50 genes. In some embodiments, the plurality of genes includes between 15 genes and 40 genes. In some embodiments, the plurality of genes includes between 15 genes and 30 genes. In some embodiments, the plurality of genes includes between 20 genes and 50 genes. In some embodiments, the plurality of genes includes between 20 genes and 40 genes. In some embodiments, the plurality of genes includes between 20 genes and 30 genes. In some embodiments, the plurality of genes includes between 25 genes and 50 genes. In some embodiments, the plurality of genes includes between 25 genes and 40 genes. In some embodiments, the plurality of genes includes between 25 genes and 35 genes. In some embodiments, the plurality of genes includes between 25 genes and 30 genes. In some embodiments, the plurality of genes falls within another range starting no lower than 10 genes and ending no higher than 2000 genes.

In some embodiments, each respective amplification primer in the plurality of amplification primers is between 10 and 100 base pairs. In some embodiments, each respective amplification primer in the plurality of amplification primers is between 10 and 70 base pairs. In some embodiments, each respective amplification primer comprises at least 5, at least 10, at least 20, at least 30, at least 40, at least 50, at least 60, at least 70, at least 80, at least 90, or at least 100 base pairs. In some embodiments, each respective amplification primer comprises no more than 100, no more than 90, no more than 80, no more than 70, no more than 60, no more than 50, no more than 40, no more than 30, or no more than 20 base pairs. In some embodiments, each respective amplification primer comprises between 10 and 50, between 5 and 40, between 20 and 100, or between 10 and 30 base pairs.

In some embodiments, for each respective forward amplification primer in the plurality of amplification primers, the 5' auxiliary region comprises a binding region consisting of from 10 to 50 nucleotides and having a sequencing that is complementary to a third target sequence in the second strand of the respective gene or a transcript thereof.

For example, in some embodiments, the plurality of amplification primers is optimized for real-time quantitative loop-mediated isothermal amplification (LAMP). In some embodiments, the plurality of amplification primers comprises, for each respective gene in a plurality of genes, at least 4 amplification primers including the respective forward amplification primer and the respective reverse amplification primer.

In some embodiments, each respective amplification primer in the plurality of amplification primers further comprises an identifier sequence (*e.g.,* a unique molecular index UMI and/or a barcode) that is common to all or a subset of the amplification primers in the plurality of amplification primers (*e.g.,* a UMI common to all or a subset of amplification primers in the plurality of amplification primers).

In some embodiments, each respective amplification primer in the plurality of amplification primers is further conjugated to a respective affinity moiety (*e.g.,* a detection moiety).

In some embodiments, each gene in the plurality of genes is selected for use in a biomarker panel (*e.g.,* via detection of an mRNA transcript for the gene). For example, in some embodiments, the plurality of genes includes any of the embodiments described herein under the sections entitled "Biomarkers" and "Measurement of Biomarkers," above. In some embodiments, the plurality of genes comprises at least 29 genes selected from Table 1. In some embodiments, the plurality of genes comprises at least 29 genes selected from Table 2. In some embodiments, the plurality of genes comprises at least 29 genes from Table 9. In some embodiments, the plurality of genes comprises no more than 1000 genes. In some embodiments, the plurality of genes comprises no more than 200 genes.

In some embodiments, each gene in the plurality of genes satisfies an abundance threshold based on a measure of abundance for the respective gene in a reference dataset. In some embodiments, the abundance threshold is between 10 and 500 copies per 150 ng total RNA load. In some embodiments, each gene in the plurality of genes satisfies a dynamic range threshold based on a measure of dynamic range for the respective gene in a reference dataset. In some embodiments, the dynamic range threshold is between 2-fold and 40-fold.

Another aspect of the present disclosure provides a plurality of conjugated nucleic acid probes for determining an infectious disease state of a subject, the plurality of conjugated nucleic acid probes comprising, for each respective gene in a plurality of genes comprising at least 20 genes selected from Table 1, at least 20 genes selected from Table 2, and/or at least 20 genes from Table 9, a respective nucleic acid probe comprising a respective nucleic acid conjugated to a non-nucleic acid detection moiety, wherein the respective nucleic acid is complementary to the respective gene.

In some embodiments, the plurality of genes comprises at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, at least 45, at least 46, at least 47, or at least 48 genes selected from Table 1. In some embodiments, the plurality of genes comprises at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, or at least 29 genes selected from Table 2. In some embodiments, the plurality of genes comprises at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, at least 45, at least 46, at least 47, at least 48, at least 49, at least 50, at least 51, at least 52, at least 53, at least 54, at least 55, at least 56, at least 57, at least 58, at least 59, at least 60, at least 61, at least 62, at least 63, or at least 64 genes selected from Table 9.

In some embodiments, all of the genes are selected from Table 1. That is, in some embodiments, the plurality of genes consists of 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, or 48 genes selected from Table 1. In some embodiments, the plurality of genes consists of from 5 to 20, from 10 to 30, from 20 to 40, from 15 to 48, or from 10 to 48 genes selected from Table 1. In some embodiments, the plurality of genes falls within another range starting no lower than 5 genes and ending no higher than 48 genes from Table 1.

In some embodiments, all of the genes are selected from Table 2. That is, in some embodiments, the plurality of genes consists of 2, 3, 4, 5, 6, 7, 8, 9, 10, 11, 12, 13, 14, 15, 16, 17, 18, 19, 20, 21, 22, 23, 24, 25, 26, 27, 28, or 29 genes selected from Table 2. In some embodiments, the plurality of genes consists of from 10 to 15, from 10 to 25, from 5 to 20, from 10 to 29, or from 15 to 29 genes selected from Table 2. In some embodiments, the plurality of genes falls within another range starting no lower than 5 genes and ending no higher than 29 genes from Table 2.

In some embodiments, all of the genes are selected from Table 9. That is, in some embodiments, the plurality of genes consists of 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, or 64 genes selected from Table 9. In some embodiments, the plurality of genes consists of from 5 to 20, from 10 to 30, from 20 to 40, from 30 to 50, or from 40 to 60 genes selected from Table 9. In some embodiments, the plurality of genes falls within another range starting no lower than 5 genes and ending no higher than 64 genes from Table 9.

In some embodiments, the plurality of genes comprises at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, at least 45, at least 46, at least 47, or at least 48 genes. In some embodiments, the plurality of genes comprises at least 10, at least 20, at least 30, at least 40, at least 50, at least 60, at least 70, at least 80, at least 90, or at least 100 genes. In some embodiments, the plurality of genes comprises at least 100, at least 200, at least 300, at least 400, at least 500, at least 600, at least 700, at least 800, at least 900, or at least 1000 genes.

In some embodiments, the plurality of genes comprises no more than 2000, no more than 1000, no more than 900, no more than 800, no more than 700, no more than 600, no more than 500, no more than 400, no more than 300, no more than 200, no more than 100, no more than 90, no more than 80, no more than 70, no more than 60, no more than 50, no more than 40, or no more than 30 genes. In some embodiments, the plurality of genes comprises between 5 and 10, between 2 and 50, between 10 and 200, between 20 and 500, between 10 and 80, between 30 and 100, between 100 and 1000, between 300 and 2000, or between 1000 and 2000 genes. In some embodiments, the plurality of genes includes between 15 genes and 50 genes. In some embodiments, the plurality of genes includes between 15 genes and 40 genes. In some embodiments, the plurality of genes includes between 15 genes and 30 genes. In some embodiments, the plurality of genes includes between 20 genes and 50 genes. In some embodiments, the plurality of genes includes between 20 genes and 40 genes. In some embodiments, the plurality of genes includes between 20 genes and 30 genes. In some embodiments, the plurality of genes includes between 25 genes and 50 genes. In some embodiments, the plurality of genes includes between 25 genes and 40 genes. In some embodiments, the plurality of genes includes between 25 genes and 35 genes. In some embodiments, the plurality of genes includes between 25 genes and 30 genes. In some embodiments, the plurality of genes falls within another range starting no lower than 10 genes and ending no higher than 2000 genes.

In some embodiments, the plurality of genes includes any of the embodiments described herein under the sections entitled "Biomarkers" and "Measurement of Biomarkers," above.

For example, in some embodiments, each gene in the plurality of genes is selected for use in a biomarker panel (*e.g.,* via detection of an mRNA transcript for the gene). In some embodiments, the plurality of genes comprises at least 29 genes selected from Table 1. In some embodiments, the plurality of genes comprises at least 29 genes selected from Table 2. In some embodiments, the plurality of genes comprises at least 29 genes selected from Table 9. In some embodiments, the plurality of genes comprises no more than 1000 genes. In some embodiments, the plurality of genes comprises no more than 200 genes. In some embodiments, each gene in the plurality of genes satisfies an abundance threshold based on a measure of abundance for the respective gene in a reference dataset. In some embodiments, the abundance threshold is between 10 and 500 copies per 150 ng total RNA load. In some embodiments, each gene in the plurality of genes satisfies a dynamic range threshold based on a measure of dynamic range for the respective gene in a reference dataset. In some embodiments, the dynamic range threshold is between 2-fold and 40-fold.

*Kits*

In another aspect of the present disclosure, the invention provides kits for determining an infectious disease state (*e.g.,* diagnosing an infection) in a subject, where the kits can be used to detect the plurality of genes (*e.g.,* biomarkers) described herein. For example, the kits can be used to detect any one or more of the biomarkers described herein, which are differentially expressed in samples of a subject having a viral or bacterial infection and/or in healthy or non-infected subjects.

Accordingly, the present disclosure provides a kit comprising agents for determining an infectious disease state of a subject, comprising a plurality of amplification primers comprising, for each respective gene in a plurality of genes comprising at least 20 genes selected from Table 1, at least 20 genes selected from Table 2, and/or at least 20 genes selected from Table 9, a respective forward amplification primer and a respective reverse amplification primer. The respective forward amplification primer comprises a 3' binding region and a 5' auxiliary region, where the 3' binding region consists of from 10 to 50 nucleotides and has a sequence that is complementary to a first target sequence in a first strand of the respective gene or a transcript thereof, and the 5' auxiliary region has a sequence that is not complementary to the sequence of the first strand of the respective gene or a transcript thereof. The respective reverse amplification primer comprises a binding region, where the binding region consists of from 10 to 50 nucleotides and has a sequence that is complementary to a second target sequence in the second strand of the respective gene or a transcript thereof.

In some embodiments, the kit comprises a plurality of probes for detection of gene expression of a set of viral response genes and a set of bacterial response genes and/or a set of sepsis response genes.

In some embodiments, the kit comprises a plurality of conjugated nucleic acid probes for determining an infectious disease state of a subject, the plurality of conjugated nucleic acid probes comprising, for each respective gene in a plurality of genes comprising at least 20 genes selected from Table 1, at least 20 genes selected from Table 2, and/or at least 20 genes selected from Table 9, a respective nucleic acid probe comprising a respective nucleic acid conjugated to a non-nucleic acid detection moiety, wherein the respective nucleic acid is complementary to the respective gene.

In some embodiments, the plurality of genes comprises at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, at least 45, at least 46, at least 47, or at least 48 genes selected from Table 1. In some embodiments, the plurality of genes comprises at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, or at least 29 genes selected from Table 2. In some embodiments, the plurality of genes comprises at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, at least 45, at least 46, at least 47, at least 48, at least 49, at least 50, at least 51, at least 52, at least 53, at least 54, at least 55, at least 56, at least 57, at least 58, at least 59, at least 60, at least 61, at least 62, at least 63, or at least 64 genes selected from Table 9.

In some embodiments, all of the genes are selected from Table 1. That is, in some embodiments, the plurality of genes consists of 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, or 48 genes selected from Table 1. In some embodiments, the plurality of genes consists of from 5 to 20, from 10 to 30, from 20 to 40, from 15 to 48, or from 10 to 48 genes selected from Table 1. In some embodiments, the plurality of genes falls within another range starting no lower than 5 genes and ending no higher than 48 genes from Table 1.

In some embodiments, all of the genes are selected from Table 2. That is, in some embodiments, the plurality of genes consists of 2, 3, 4, 5, 6, 7, 8, 9, 10, 11, 12, 13, 14, 15, 16, 17, 18, 19, 20, 21, 22, 23, 24, 25, 26, 27, 28, or 29 genes selected from Table 2. In some embodiments, the plurality of genes consists of from 10 to 15, from 10 to 25, from 5 to 20, from 10 to 29, or from 15 to 29 genes selected from Table 2. In some embodiments, the plurality of genes falls within another range starting no lower than 5 genes and ending no higher than 29 genes from Table 2.

In some embodiments, all of the genes are selected from Table 9. That is, in some embodiments, the plurality of genes consists of 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, or 64 genes selected from Table 9. In some embodiments, the plurality of genes consists of from 5 to 20, from 10 to 30, from 20 to 40, from 30 to 50, or from 40 to 60 genes selected from Table 9. In some embodiments, the plurality of genes falls within another range starting no lower than 5 genes and ending no higher than 64 genes from Table 9.

In some embodiments, the plurality of genes comprises at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, at least 45, at least 46, at least 47, or at least 48 genes. In some embodiments, the plurality of genes comprises at least 10, at least 20, at least 30, at least 40, at least 50, at least 60, at least 70, at least 80, at least 90, or at least 100 genes. In some embodiments, the plurality of genes comprises at least 100, at least 200, at least 300, at least 400, at least 500, at least 600, at least 700, at least 800, at least 900, or at least 1000 genes.

In some embodiments, the plurality of genes comprises no more than 2000, no more than 1000, no more than 900, no more than 800, no more than 700, no more than 600, no more than 500, no more than 400, no more than 300, no more than 200, no more than 100, no more than 90, no more than 80, no more than 70, no more than 60, no more than 50, no more than 40, or no more than 30 genes. In some embodiments, the plurality of genes comprises between 5 and 10, between 2 and 50, between 10 and 200, between 20 and 500, between 10 and 80, between 30 and 100, between 100 and 1000, between 300 and 2000, or between 1000 and 2000 genes. In some embodiments, the plurality of genes includes between 15 genes and 50 genes. In some embodiments, the plurality of genes includes between 15 genes and 40 genes. In some embodiments, the plurality of genes includes between 15 genes and 30 genes. In some embodiments, the plurality of genes includes between 20 genes and 50 genes. In some embodiments, the plurality of genes includes between 20 genes and 40 genes. In some embodiments, the plurality of genes includes between 20 genes and 30 genes. In some embodiments, the plurality of genes includes between 25 genes and 50 genes. In some embodiments, the plurality of genes includes between 25 genes and 40 genes. In some embodiments, the plurality of genes includes between 25 genes and 35 genes. In some embodiments, the plurality of genes includes between 25 genes and 30 genes. In some embodiments, the plurality of genes falls within another range starting no lower than 10 genes and ending no higher than 2000 genes.

In some embodiments, the kit comprises a composition as described herein under the section entitled "Compositions," above.

In some embodiments, the kit further comprises information, in electronic or paper form, comprising instructions for measuring attributes (*e.g.*, mRNA abundance levels) of the plurality of genes in a biological sample of the subject, thereby obtaining a plurality of attribute values for the plurality of genes. In some embodiments, the kit further comprises information, in electronic or paper form, comprising instructions for using the plurality of attribute values with a trained classifier to determine an infectious disease state of the subject, the infectious disease state being one or more of infected with a bacteria, infected with a virus, and not-infected.

For example, in some embodiments, the kit includes one or more agents for measuring the levels of expression of a set of viral response genes and a set of bacterial response genes, a container for holding a biological sample isolated from a subject suspected of having an infection, and printed instructions for reacting agents with the biological sample or a portion of the biological sample for measuring the levels of expression of a set of viral response genes and a set of bacterial response genes in the biological sample. In some embodiments, the agents are packaged in separate containers. In some embodiments, the kit further comprises one or more control reference samples and reagents for performing an immunoassay, PCR, or microarray analysis.

In some embodiments, the plurality of genes includes any of the embodiments described herein under the sections entitled "Biomarkers" and "Measurement of Biomarkers," above.

For example, in some embodiments, each gene in the plurality of genes is selected for use in a biomarker panel (*e.g.,* via detection of an mRNA transcript for the gene). In some embodiments, the plurality of genes comprises at least 29 genes selected from Table 1. In some embodiments, the plurality of genes comprises at least 29 genes selected from Table 2. In some embodiments, the plurality of genes comprises at least 29 genes selected from Table 9. In some embodiments, the plurality of genes comprises no more than 1000 genes. In some embodiments, the plurality of genes comprises no more than 200 genes. In some embodiments, each gene in the plurality of genes satisfies an abundance threshold based on a measure of abundance for the respective gene in a reference dataset. In some embodiments, the abundance threshold is between 10 and 500 copies per 150 ng total RNA load. In some embodiments, each gene in the plurality of genes satisfies a dynamic range threshold based on a measure of dynamic range for the respective gene in a reference dataset. In some embodiments, the dynamic range threshold is between 2-fold and 40-fold.

The kit can comprise one or more containers for compositions contained in the kit. Compositions can be in liquid form or can be lyophilized. Suitable containers for the compositions include, for example, bottles, vials, syringes, and test tubes. Containers can be formed from a variety of materials, including glass or plastic. The kit can also comprise a package insert containing written instructions for methods of diagnosing infections.

In some embodiments, the kit comprises an instrument for measuring attribute values (*e.g.,* mRNA abundance values) for one or more genes in the plurality of genes. In some embodiments, the kit comprises a cartridge comprising, *e.g.*, a receptacle for a biological sample and reagents for measuring attribute values (*e.g.*, mRNA abundance values) for one or more genes in the plurality of genes. In some embodiments, the kit comprises system comprising an instrument and one or more cartridges for measuring attribute values (*e.g.*, mRNA abundance values) for one or more genes in the plurality of genes. An example of a system in accordance with some embodiments of the present disclosure is described with reference to Figure 5 in Example 1, below.

The kits of the invention have a number of applications. For example, the kits can be used to determine if a subject has an infection or some other inflammatory condition arising from a noninfectious source, such as traumatic injury, surgery, autoimmune disease, thrombosis, or systemic inflammatory response syndrome (SIRS). If a patient is diagnosed with an infection, the kits can be used to further determine the type of infection (*e.g.*, viral or bacterial infection). In another example, the kits can be used to determine if a patient having acute inflammation should be treated, for example, with broad spectrum antibiotics or antiviral agents. In another example, kits can be used to monitor the effectiveness of treatment of a patient having an infection. In a further example, the kits can be used to identify compounds that modulate expression of one or more of the biomarkers in in vitro or in vivo animal models to determine the effects of treatment.

*Embodiments Integrating Multiple Improvements*

In some embodiments, a method for determining an infectious disease state in a subject is provided that integrates at least an improvement in a method for using a classifier, as described above in the sections entitled "Selection of Configurations" and "Classifiers and Hyperparameters," and an improvement in a plurality of genes (*e.g.*, biomarkers) for detection of attribute values, as described above in the sections entitled "Biomarkers" and "Measurement of Biomarkers."

Accordingly, a method is provided for determining an infectious disease state of a test subject, the method comprising obtaining a dataset having attribute values for a plurality of genes from a biological sample of the test subject, and, responsive to inputting the plurality of attribute values to a classifier, obtaining a determination as to whether the test subject has an infectious disease state selected from infected with a bacteria, infected with a virus, and not-infected, where the classifier is obtained by performing a method comprising obtaining a training dataset including labels for infectious disease states and respective attribute values for the plurality of genes obtained from biological samples of a plurality of training subjects and performing a classifier selection process as described above in the section entitled "Selection of Configurations."

In some embodiments, the classifier is any classifier, as described above in the section entitled "Classifiers and Hyperparameters," and the plurality of genes comprises any one or more biomarkers for determining an infectious disease state.

In some embodiments, the classifier is any classifier, as described above in the section entitled "Classifiers and Hyperparameters," and the plurality of genes comprises at least 10 biomarkers from Table 1, as described in the above section entitled "Biomarkers."

In some embodiments, the classifier is any classifier, as described above in the section entitled "Classifiers and Hyperparameters," and the plurality of genes comprises at least 10 biomarkers from Table 2, as described in the above section entitled "Biomarkers."

In some embodiments, the classifier is any classifier, as described above in the section entitled "Classifiers and Hyperparameters," and the plurality of genes comprises at least 10 biomarkers from Table 9, as described in the above section entitled "Biomarkers."

In some embodiments, the classifier is any classifier, as described above in the section entitled "Classifiers and Hyperparameters," and the plurality of genes comprises at least 20 biomarkers from Table 1, as described in the above section entitled "Biomarkers."

In some embodiments, the classifier is any classifier, as described above in the section entitled "Classifiers and Hyperparameters," and the plurality of genes comprises at least 20 biomarkers from Table 2, as described in the above section entitled "Biomarkers."

In some embodiments, the classifier is any classifier, as described above in the section entitled "Classifiers and Hyperparameters," and the plurality of genes comprises at least 20 biomarkers from Table 9, as described in the above section entitled "Biomarkers."

In some embodiments, the classifier is any classifier, as described above in the section entitled "Classifiers and Hyperparameters," the plurality of genes comprises 20 biomarkers from Table 1, and the plurality of attribute values is measured, for the plurality of genes, using isothermal amplification from a biological sample comprising blood, as described in the above sections entitled "Biomarkers" and "Measurement of Biomarkers."

In some embodiments, the classifier is any classifier, as described above in the section entitled "Classifiers and Hyperparameters," the plurality of genes comprises 20 biomarkers from Table 2, and the plurality of attribute values is measured, for the plurality of genes, using isothermal amplification from a biological sample comprising blood, as described in the above sections entitled "Biomarkers" and "Measurement of Biomarkers."

In some embodiments, the classifier is any classifier, as described above in the section entitled "Classifiers and Hyperparameters," the plurality of genes comprises 20 biomarkers from Table 9, and the plurality of attribute values is measured, for the plurality of genes, using isothermal amplification from a biological sample comprising blood, as described in the above sections entitled "Biomarkers" and "Measurement of Biomarkers."

In some embodiments, the classifier is any classifier, as described above in the section entitled "Classifiers and Hyperparameters," and the plurality of genes comprises 29 biomarkers from Table 2, as described in the above section entitled "Biomarkers."

In some embodiments, the classifier is any classifier, as described above in the section entitled "Classifiers and Hyperparameters," the plurality of genes comprises 29 biomarkers from Table 2, and the plurality of attribute values is measured, for the plurality of genes, using isothermal amplification from a biological sample comprising blood, as described in the above sections entitled "Biomarkers" and "Measurement of Biomarkers."

In some embodiments, the classifier is any classifier, as described above in the section entitled "Classifiers and Hyperparameters," and the plurality of genes comprises 29 biomarkers from Table 9, as described in the above section entitled "Biomarkers."

In some embodiments, the classifier is any classifier, as described above in the section entitled "Classifiers and Hyperparameters," the plurality of genes comprises 29 biomarkers from Table 9, and the plurality of attribute values is measured, for the plurality of genes, using isothermal amplification from a biological sample comprising blood, as described in the above sections entitled "Biomarkers" and "Measurement of Biomarkers."

In some embodiments, the classifier is a neural network, as described above in the section entitled "Classifiers and Hyperparameters," and the plurality of genes comprises any one or more biomarkers for determining an infectious disease state, as described in the above section entitled "Biomarkers."

In some embodiments, the classifier is a neural network, as described above in the section entitled "Classifiers and Hyperparameters," and the plurality of genes comprises at least 10 biomarkers from Table 1, as described in the above section entitled "Biomarkers."

In some embodiments, the classifier is a neural network, as described above in the section entitled "Classifiers and Hyperparameters," and the plurality of genes comprises at least 10 biomarkers from Table 2, as described in the above section entitled "Biomarkers."

In some embodiments, the classifier is a neural network, as described above in the section entitled "Classifiers and Hyperparameters," and the plurality of genes comprises at least 10 biomarkers from Table 9, as described in the above section entitled "Biomarkers."

In some embodiments, the classifier is a neural network, as described above in the section entitled "Classifiers and Hyperparameters," and the plurality of genes comprises at least 20 biomarkers from Table 1, as described in the above section entitled "Biomarkers."

In some embodiments, the classifier is a neural network, as described above in the section entitled "Classifiers and Hyperparameters," and the plurality of genes comprises at least 20 biomarkers from Table 2, as described in the above section entitled "Biomarkers."

In some embodiments, the classifier is a neural network, as described above in the section entitled "Classifiers and Hyperparameters," and the plurality of genes comprises at least 20 biomarkers from Table 9, as described in the above section entitled "Biomarkers."

In some embodiments, the classifier is a neural network, as described above in the section entitled "Classifiers and Hyperparameters," the plurality of genes comprises 20 biomarkers from Table 1, and the plurality of attribute values is measured, for the plurality of genes, using isothermal amplification from a biological sample comprising blood, as described in the above sections entitled "Biomarkers" and "Measurement of Biomarkers."

In some embodiments, the classifier is a neural network, as described above in the section entitled "Classifiers and Hyperparameters," the plurality of genes comprises 20 biomarkers from Table 2, and the plurality of attribute values is measured, for the plurality of genes, using isothermal amplification from a biological sample comprising blood, as described in the above sections entitled "Biomarkers" and "Measurement of Biomarkers."

In some embodiments, the classifier is a neural network, as described above in the section entitled "Classifiers and Hyperparameters," the plurality of genes comprises 20 biomarkers from Table 9, and the plurality of attribute values is measured, for the plurality of genes, using isothermal amplification from a biological sample comprising blood, as described in the above sections entitled "Biomarkers" and "Measurement of Biomarkers."

In some embodiments, the classifier is a neural network, as described above in the section entitled "Classifiers and Hyperparameters," and the plurality of genes comprises 29 biomarkers from Table 2, as described in the above section entitled "Biomarkers."

In some embodiments, the classifier is a neural network, as described above in the section entitled "Classifiers and Hyperparameters," the plurality of genes comprises 29 biomarkers from Table 2, and the plurality of attribute values is measured, for the plurality of genes, using isothermal amplification from a biological sample comprising blood, as described in the above sections entitled "Biomarkers" and "Measurement of Biomarkers."

In some embodiments, the classifier is a neural network, as described above in the section entitled "Classifiers and Hyperparameters," and the plurality of genes comprises 29 biomarkers from Table 9, as described in the above section entitled "Biomarkers."

In some embodiments, the classifier is a neural network, as described above in the section entitled "Classifiers and Hyperparameters," the plurality of genes comprises 29 biomarkers from Table 9, and the plurality of attribute values is measured, for the plurality of genes, using isothermal amplification from a biological sample comprising blood, as described in the above sections entitled "Biomarkers" and "Measurement of Biomarkers."

In some embodiments, the classifier is a neural network comprising a plurality of hyperparameters selected using a configuration selection process (*e.g.,* a hyperband method), as described above in the section entitled "Selection of Configurations," and the plurality of genes comprises any one or more biomarkers for determining an infectious disease state, as described in the above section entitled "Biomarkers."

In some embodiments, the classifier is a neural network comprising a plurality of hyperparameters selected using a configuration selection process (*e.g.,* a hyperband method), as described above in the section entitled "Selection of Configurations," and the plurality of genes comprises at least 10 biomarkers from Table 1, as described in the above section entitled "Biomarkers."

In some embodiments, the classifier is a neural network comprising a plurality of hyperparameters selected using a configuration selection process *(e.g.,* a hyperband method), as described above in the section entitled "Selection of Configurations," and the plurality of genes comprises at least 10 biomarkers from Table 2, as described in the above section entitled "Biomarkers."

In some embodiments, the classifier is a neural network comprising a plurality of hyperparameters selected using a configuration selection process (*e.g.*, a hyperband method), as described above in the section entitled "Selection of Configurations," and the plurality of genes comprises at least 10 biomarkers from Table 9, as described in the above section entitled "Biomarkers."

In some embodiments, the classifier is a neural network comprising a plurality of hyperparameters selected using a configuration selection process (*e.g.*, a hyperband method), as described above in the section entitled "Selection of Configurations," and the plurality of genes comprises at least 20 biomarkers from Table 1, as described in the above section entitled "Biomarkers."

In some embodiments, the classifier is a neural network comprising a plurality of hyperparameters selected using a configuration selection process (*e.g.,* a hyperband method), as described above in the section entitled "Selection of Configurations," and the plurality of genes comprises at least 20 biomarkers from Table 2, as described in the above section entitled "Biomarkers."

In some embodiments, the classifier is a neural network comprising a plurality of hyperparameters selected using a configuration selection process (*e.g.,* a hyperband method), as described above in the section entitled "Selection of Configurations," and the plurality of genes comprises at least 20 biomarkers from Table 9, as described in the above section entitled "Biomarkers."

In some embodiments, the classifier is a neural network comprising a plurality of hyperparameters selected using a configuration selection process (*e.g.,* a hyperband method), as described above in the section entitled "Selection of Configurations," the plurality of genes comprises 20 biomarkers from Table 1, and the plurality of attribute values is measured, for the plurality of genes, using isothermal amplification from a biological sample comprising blood, as described in the above sections entitled "Biomarkers" and "Measurement of Biomarkers."

In some embodiments, the classifier is a neural network comprising a plurality of hyperparameters selected using a configuration selection process (*e.g.,* a hyperband method), as described above in the section entitled "Selection of Configurations," the plurality of genes comprises 20 biomarkers from Table 2, and the plurality of attribute values is measured, for the plurality of genes, using isothermal amplification from a biological sample comprising blood, as described in the above sections entitled "Biomarkers" and "Measurement of Biomarkers."

In some embodiments, the classifier is a neural network comprising a plurality of hyperparameters selected using a configuration selection process (*e.g.*, a hyperband method), as described above in the section entitled "Selection of Configurations," the plurality of genes comprises 20 biomarkers from Table 9, and the plurality of attribute values is measured, for the plurality of genes, using isothermal amplification from a biological sample comprising blood, as described in the above sections entitled "Biomarkers" and "Measurement of Biomarkers."

In some embodiments, the classifier is a neural network comprising a plurality of hyperparameters selected using a configuration selection process (*e.g.*, a hyperband method), as described above in the section entitled "Selection of Configurations," and the plurality of genes comprises 29 biomarkers from Table 2, as described in the above section entitled "Biomarkers."

In some embodiments, the classifier is a neural network comprising a plurality of hyperparameters selected using a configuration selection process (*e.g.*, a hyperband method), as described above in the section entitled "Selection of Configurations," the plurality of genes comprises 29 biomarkers from Table 2, and the plurality of attribute values is measured, for the plurality of genes, using isothermal amplification from a biological sample comprising blood, as described in the above sections entitled "Biomarkers" and "Measurement of Biomarkers."

In some embodiments, the classifier is a neural network comprising a plurality of hyperparameters selected using a configuration selection process (*e.g.,* a hyperband method), as described above in the section entitled "Selection of Configurations," and the plurality of genes comprises 29 biomarkers from Table 9, as described in the above section entitled "Biomarkers."

In some embodiments, the classifier is a neural network comprising a plurality of hyperparameters selected using a configuration selection process (*e.g.,* a hyperband method), as described above in the section entitled "Selection of Configurations," the plurality of genes comprises 29 biomarkers from Table 9, and the plurality of attribute values is measured, for the plurality of genes, using isothermal amplification from a biological sample comprising blood, as described in the above sections entitled "Biomarkers" and "Measurement of Biomarkers."

In some embodiments, the classifier is an ensemble classifier, as described above in the section entitled "Selection of Configurations," and the plurality of genes comprises any one or more biomarkers for determining an infectious disease state, as described in the above section entitled "Biomarkers."

In some embodiments, the classifier is an ensemble classifier, as described above in the section entitled "Selection of Configurations," and the plurality of genes comprises at least 10 biomarkers from Table 1, as described in the above section entitled "Biomarkers."

In some embodiments, the classifier is an ensemble classifier, as described above in the section entitled "Selection of Configurations," and the plurality of genes comprises at least 10 biomarkers from Table 2, as described in the above section entitled "Biomarkers."

In some embodiments, the classifier is an ensemble classifier, as described above in the section entitled "Selection of Configurations," and the plurality of genes comprises at least 10 biomarkers from Table 9, as described in the above section entitled "Biomarkers."

In some embodiments, the classifier is an ensemble classifier, as described above in the section entitled "Selection of Configurations," and the plurality of genes comprises at least 20 biomarkers from Table 1, as described in the above section entitled "Biomarkers."

In some embodiments, the classifier is an ensemble classifier, as described above in the section entitled "Selection of Configurations," and the plurality of genes comprises at least 20 biomarkers from Table 2, as described in the above section entitled "Biomarkers."

In some embodiments, the classifier is an ensemble classifier, as described above in the section entitled "Selection of Configurations," and the plurality of genes comprises at least 20 biomarkers from Table 9, as described in the above section entitled "Biomarkers."

In some embodiments, the classifier is an ensemble classifier, as described above in the section entitled "Selection of Configurations," the plurality of genes comprises 20 biomarkers from Table 1, and the plurality of attribute values is measured, for the plurality of genes, using isothermal amplification from a biological sample comprising blood, as described in the above sections entitled "Biomarkers" and "Measurement of Biomarkers."

In some embodiments, the classifier is an ensemble classifier, as described above in the section entitled "Selection of Configurations," the plurality of genes comprises 20 biomarkers from Table 2, and the plurality of attribute values is measured, for the plurality of genes, using isothermal amplification from a biological sample comprising blood, as described in the above sections entitled "Biomarkers" and "Measurement of Biomarkers."

In some embodiments, the classifier is an ensemble classifier, as described above in the section entitled "Selection of Configurations," the plurality of genes comprises 20 biomarkers from Table 9, and the plurality of attribute values is measured, for the plurality of genes, using isothermal amplification from a biological sample comprising blood, as described in the above sections entitled "Biomarkers" and "Measurement of Biomarkers."

In some embodiments, the classifier is an ensemble classifier, as described above in the section entitled "Selection of Configurations," and the plurality of genes comprises at least 29 biomarkers from Table 2, as described in the above section entitled "Biomarkers."

In some embodiments, the classifier is an ensemble classifier, as described above in the section entitled "Selection of Configurations," the plurality of genes comprises 29 biomarkers from Table 2, and the plurality of attribute values is measured, for the plurality of genes, using isothermal amplification from a biological sample comprising blood, as described in the above sections entitled "Biomarkers" and "Measurement of Biomarkers."

In some embodiments, the classifier is an ensemble classifier, as described above in the section entitled "Selection of Configurations," and the plurality of genes comprises at least 29 biomarkers from Table 9, as described in the above section entitled "Biomarkers."

In some embodiments, the classifier is an ensemble classifier, as described above in the section entitled "Selection of Configurations," the plurality of genes comprises 29 biomarkers from Table 9, and the plurality of attribute values is measured, for the plurality of genes, using isothermal amplification from a biological sample comprising blood, as described in the above sections entitled "Biomarkers" and "Measurement of Biomarkers."

In some embodiments, the classifier is an ensemble classifier comprising a plurality of neural networks, as described above in the section entitled "Selection of Configurations," and the plurality of genes comprises any one or more biomarkers for determining an infectious disease state, as described in the above section entitled "Biomarkers."

In some embodiments, the classifier is an ensemble classifier comprising a plurality of neural networks, as described above in the section entitled "Selection of Configurations," and the plurality of genes comprises at least 10 biomarkers from Table 1, as described in the above section entitled "Biomarkers."

In some embodiments, the classifier is an ensemble classifier comprising a plurality of neural networks, as described above in the section entitled "Selection of Configurations," and the plurality of genes comprises at least 10 biomarkers from Table 2, as described in the above section entitled "Biomarkers."

In some embodiments, the classifier is an ensemble classifier comprising a plurality of neural networks, as described above in the section entitled "Selection of Configurations," and the plurality of genes comprises at least 10 biomarkers from Table 9, as described in the above section entitled "Biomarkers."

In some embodiments, the classifier is an ensemble classifier comprising a plurality of neural networks, as described above in the section entitled "Selection of Configurations," and the plurality of genes comprises at least 20 biomarkers from Table 1, as described in the above section entitled "Biomarkers."

In some embodiments, the classifier is an ensemble classifier comprising a plurality of neural networks, as described above in the section entitled "Selection of Configurations," and the plurality of genes comprises at least 20 biomarkers from Table 2, as described in the above section entitled "Biomarkers."

In some embodiments, the classifier is an ensemble classifier comprising a plurality of neural networks, as described above in the section entitled "Selection of Configurations," and the plurality of genes comprises at least 20 biomarkers from Table 9, as described in the above section entitled "Biomarkers."

In some embodiments, the classifier is an ensemble classifier comprising a plurality of neural networks, as described above in the section entitled "Selection of Configurations," the plurality of genes comprises 20 biomarkers from Table 1, and the plurality of attribute values is measured, for the plurality of genes, using isothermal amplification from a biological sample comprising blood, as described in the above sections entitled "Biomarkers" and "Measurement of Biomarkers."

In some embodiments, the classifier is an ensemble classifier comprising a plurality of neural networks, as described above in the section entitled "Selection of Configurations," the plurality of genes comprises 20 biomarkers from Table 2, and the plurality of attribute values is measured, for the plurality of genes, using isothermal amplification from a biological sample comprising blood, as described in the above sections entitled "Biomarkers" and "Measurement of Biomarkers."

In some embodiments, the classifier is an ensemble classifier comprising a plurality of neural networks, as described above in the section entitled "Selection of Configurations," the plurality of genes comprises 20 biomarkers from Table 9, and the plurality of attribute values is measured, for the plurality of genes, using isothermal amplification from a biological sample comprising blood, as described in the above sections entitled "Biomarkers" and "Measurement of Biomarkers."

In some embodiments, the classifier is an ensemble classifier comprising a plurality of neural networks, as described above in the section entitled "Selection of Configurations," and the plurality of genes comprises at least 29 biomarkers from Table 2, as described in the above section entitled "Biomarkers."

In some embodiments, the classifier is an ensemble classifier comprising a plurality of neural networks, as described above in the section entitled "Selection of Configurations," the plurality of genes comprises 29 biomarkers from Table 2, and the plurality of attribute values is measured, for the plurality of genes, using isothermal amplification from a biological sample comprising blood, as described in the above sections entitled "Biomarkers" and "Measurement of Biomarkers."

In some embodiments, the classifier is an ensemble classifier comprising a plurality of neural networks, as described above in the section entitled "Selection of Configurations," and the plurality of genes comprises at least 29 biomarkers from Table 9, as described in the above section entitled "Biomarkers."

In some embodiments, the classifier is an ensemble classifier comprising a plurality of neural networks, as described above in the section entitled "Selection of Configurations," the plurality of genes comprises 29 biomarkers from Table 9, and the plurality of attribute values is measured, for the plurality of genes, using isothermal amplification from a biological sample comprising blood, as described in the above sections entitled "Biomarkers" and "Measurement of Biomarkers."

In some embodiments, the classifier is an ensemble classifier comprising a plurality of hyperparameters selected using a configuration selection process, as described above in the section entitled "Selection of Configurations," and the plurality of genes comprises any one or more biomarkers for determining an infectious disease state, as described in the above section entitled "Biomarkers."

In some embodiments, the classifier is an ensemble classifier comprising a plurality of hyperparameters selected using a configuration selection process, as described above in the section entitled "Selection of Configurations," and the plurality of genes comprises at least 10 biomarkers from Table 1, as described in the above section entitled "Biomarkers."

In some embodiments, the classifier is an ensemble classifier comprising a plurality of hyperparameters selected using a configuration selection process, as described above in the section entitled "Selection of Configurations," and the plurality of genes comprises at least 10 biomarkers from Table 2, as described in the above section entitled "Biomarkers."

In some embodiments, the classifier is an ensemble classifier comprising a plurality of hyperparameters selected using a configuration selection process, as described above in the section entitled "Selection of Configurations," and the plurality of genes comprises at least 10 biomarkers from Table 9, as described in the above section entitled "Biomarkers."

In some embodiments, the classifier is an ensemble classifier comprising a plurality of hyperparameters selected using a configuration selection process, as described above in the section entitled "Selection of Configurations," and the plurality of genes comprises at least 20 biomarkers from Table 1, as described in the above section entitled "Biomarkers."

In some embodiments, the classifier is an ensemble classifier comprising a plurality of hyperparameters selected using a configuration selection process, as described above in the section entitled "Selection of Configurations," and the plurality of genes comprises at least 20 biomarkers from Table 2, as described in the above section entitled "Biomarkers."

In some embodiments, the classifier is an ensemble classifier comprising a plurality of hyperparameters selected using a configuration selection process, as described above in the section entitled "Selection of Configurations," and the plurality of genes comprises at least 20 biomarkers from Table 9, as described in the above section entitled "Biomarkers."

In some embodiments, the classifier is an ensemble classifier comprising a plurality of hyperparameters selected using a configuration selection process, as described above in the section entitled "Selection of Configurations," the plurality of genes comprises 20 biomarkers from Table 1, and the plurality of attribute values is measured, for the plurality of genes, using isothermal amplification from a biological sample comprising blood, as described in the above sections entitled "Biomarkers" and "Measurement of Biomarkers."

In some embodiments, the classifier is an ensemble classifier comprising a plurality of hyperparameters selected using a configuration selection process, as described above in the section entitled "Selection of Configurations," the plurality of genes comprises 20 biomarkers from Table 2, and the plurality of attribute values is measured, for the plurality of genes, using isothermal amplification from a biological sample comprising blood, as described in the above sections entitled "Biomarkers" and "Measurement of Biomarkers."

In some embodiments, the classifier is an ensemble classifier comprising a plurality of hyperparameters selected using a configuration selection process, as described above in the section entitled "Selection of Configurations," the plurality of genes comprises 20 biomarkers from Table 9, and the plurality of attribute values is measured, for the plurality of genes, using isothermal amplification from a biological sample comprising blood, as described in the above sections entitled "Biomarkers" and "Measurement of Biomarkers."

In some embodiments, the classifier is an ensemble classifier comprising a plurality of hyperparameters selected using a configuration selection process, as described above in the section entitled "Selection of Configurations," and the plurality of genes comprises at least 29 biomarkers from Table 2, as described in the above section entitled "Biomarkers."

In some embodiments, the classifier is an ensemble classifier comprising a plurality of hyperparameters selected using a configuration selection process, as described above in the section entitled "Selection of Configurations," the plurality of genes comprises 29 biomarkers from Table 2, and the plurality of attribute values is measured, for the plurality of genes, using isothermal amplification from a biological sample comprising blood, as described in the above sections entitled "Biomarkers" and "Measurement of Biomarkers."

In some embodiments, the classifier is an ensemble classifier comprising a plurality of hyperparameters selected using a configuration selection process, as described above in the section entitled "Selection of Configurations," and the plurality of genes comprises at least 29 biomarkers from Table 9, as described in the above section entitled "Biomarkers."

In some embodiments, the classifier is an ensemble classifier comprising a plurality of hyperparameters selected using a configuration selection process, as described above in the section entitled "Selection of Configurations," the plurality of genes comprises 29 biomarkers from Table 9, and the plurality of attribute values is measured, for the plurality of genes, using isothermal amplification from a biological sample comprising blood, as described in the above sections entitled "Biomarkers" and "Measurement of Biomarkers."

In some embodiments, the classifier is an ensemble classifier comprising a plurality of neural networks, each neural network comprising a plurality of hyperparameters selected using a configuration selection process, as described above in the section entitled "Selection of Configurations," and the plurality of genes comprises any one or more biomarkers for determining an infectious disease state, as described in the above section entitled "Biomarkers."

In some embodiments, the classifier is an ensemble classifier comprising a plurality of neural networks, each neural network comprising a plurality of hyperparameters selected using a configuration selection process, as described above in the section entitled "Selection of Configurations," and the plurality of genes comprises at least 10 biomarkers from Table 1, as described in the above section entitled "Biomarkers."

In some embodiments, the classifier is an ensemble classifier comprising a plurality of neural networks, each neural network comprising a plurality of hyperparameters selected using a configuration selection process, as described above in the section entitled "Selection of Configurations," and the plurality of genes comprises at least 10 biomarkers from Table 2, as described in the above section entitled "Biomarkers."

In some embodiments, the classifier is an ensemble classifier comprising a plurality of neural networks, each neural network comprising a plurality of hyperparameters selected using a configuration selection process, as described above in the section entitled "Selection of Configurations," and the plurality of genes comprises at least 10 biomarkers from Table 9, as described in the above section entitled "Biomarkers."

In some embodiments, the classifier is an ensemble classifier comprising a plurality of neural networks, each neural network comprising a plurality of hyperparameters selected using a configuration selection process, as described above in the section entitled "Selection of Configurations," and the plurality of genes comprises at least 20 biomarkers from Table 1, as described in the above section entitled "Biomarkers."

In some embodiments, the classifier is an ensemble classifier comprising a plurality of neural networks, each neural network comprising a plurality of hyperparameters selected using a configuration selection process, as described above in the section entitled "Selection of Configurations," and the plurality of genes comprises at least 20 biomarkers from Table 2, as described in the above section entitled "Biomarkers."

In some embodiments, the classifier is an ensemble classifier comprising a plurality of neural networks, each neural network comprising a plurality of hyperparameters selected using a configuration selection process, as described above in the section entitled "Selection of Configurations," and the plurality of genes comprises at least 20 biomarkers from Table 9, as described in the above section entitled "Biomarkers."

In some embodiments, the classifier is an ensemble classifier comprising a plurality of neural networks, each neural network comprising a plurality of hyperparameters selected using a configuration selection process, as described above in the section entitled "Selection of Configurations," the plurality of genes comprises 20 biomarkers from Table 1, and the plurality of attribute values is measured, for the plurality of genes, using isothermal amplification from a biological sample comprising blood, as described in the above sections entitled "Biomarkers" and "Measurement of Biomarkers."

In some embodiments, the classifier is an ensemble classifier comprising a plurality of neural networks, each neural network comprising a plurality of hyperparameters selected using a configuration selection process, as described above in the section entitled "Selection of Configurations," the plurality of genes comprises 20 biomarkers from Table 2, and the plurality of attribute values is measured, for the plurality of genes, using isothermal amplification from a biological sample comprising blood, as described in the above sections entitled "Biomarkers" and "Measurement of Biomarkers."

In some embodiments, the classifier is an ensemble classifier comprising a plurality of neural networks, each neural network comprising a plurality of hyperparameters selected using a configuration selection process, as described above in the section entitled "Selection of Configurations," the plurality of genes comprises 20 biomarkers from Table 9, and the plurality of attribute values is measured, for the plurality of genes, using isothermal amplification from a biological sample comprising blood, as described in the above sections entitled "Biomarkers" and "Measurement of Biomarkers."

In some embodiments, the classifier is an ensemble classifier comprising a plurality of neural networks, each neural network comprising a plurality of hyperparameters selected using a configuration selection process, as described above in the section entitled "Selection of Configurations," and the plurality of genes comprises at least 29 biomarkers from Table 2, as described in the above section entitled "Biomarkers."

In some embodiments, the classifier is an ensemble classifier comprising a plurality of neural networks, each neural network comprising a plurality of hyperparameters selected using a configuration selection process, as described above in the section entitled "Selection of Configurations," the plurality of genes comprises 29 biomarkers from Table 2, and the plurality of attribute values is measured, for the plurality of genes, using isothermal amplification from a biological sample comprising blood, as described in the above sections entitled "Biomarkers" and "Measurement of Biomarkers."

In some embodiments, the classifier is an ensemble classifier comprising a plurality of neural networks, each neural network comprising a plurality of hyperparameters selected using a configuration selection process, as described above in the section entitled "Selection of Configurations," and the plurality of genes comprises at least 29 biomarkers from Table 9, as described in the above section entitled "Biomarkers."

In some embodiments, the classifier is an ensemble classifier comprising a plurality of neural networks, each neural network comprising a plurality of hyperparameters selected using a configuration selection process, as described above in the section entitled "Selection of Configurations," the plurality of genes comprises 29 biomarkers from Table 9, and the plurality of attribute values is measured, for the plurality of genes, using isothermal amplification from a biological sample comprising blood, as described in the above sections entitled "Biomarkers" and "Measurement of Biomarkers."

*Additional Biomarkers*

In some embodiments, the systems and methods for determining an infectious disease state in a subject disclosed herein comprise obtaining attribute values from a biological sample of the respective subject for a plurality of genes, where the plurality of genes comprises one or more genes selected from Table 8.

Table 8: Genes for Determining Infectious Disease States

| Gene | Gene | Gene | Gene | Gene |
|---|---|---|---|---|
| AANAT | CREBBP | ICK | NDUFS8 | SH3TC1 |
| ABAT | CREBZF | ICOS | NDUFV2 | SHARPIN |
| ABCA1 | CRELD1 | ICOSLG | NECAP2 | SHISA4 |
| ABCA13 | CRELD2 | ID3 | NEK1 | SHISA5 |
| ABCA2 | CRH | IDE | NEK6 | SIAE |
| ABCB4 | CRIP1 | IDH3A | NEK7 | SIAH2 |
| ABCC4 | CRISP3 | IDO1 | NELL2 | SIDT1 |
| ABCD4 | CRK | IDUA | NEO1 | SIDT2 |
| ABCE1 | CRKL | IER5 | NEU1 | SIGIRR |

(continued)

| Gene | Gene | Gene | Gene | Gene |
|---|---|---|---|---|
| ABCG1 | CRLF3 | IFI16 | NEURL | SIGLEC1 |
| ABHD15 | CROC4 | IFI27 | NFAT5 | SIGLEC15 |
| ABHD16A | CROCC | IFI30 | NFATC1 | SIGLEC5 |
| ABHD16B | CRP | IFI35 | NFATC2 | SIGLEC9 |
| ABHD2 | CRTAP | IFI44 | NFATC3 | SIPA1 |
| ABI1 | CRTC1 | IFI44L | NFATC4 | SIRPA |
| ABLIM1 | CRTC3 | IFI6 | NFE2L2 | SIRPB1 |
| ABP1 | CRYL1 | IFIH1 | NFIC | SIRT6 |
| ABT1 | CRYZ | IFIT 1 | NFIL3 | SIVA1 |
| ABTB2 | CSAD | IFIT1B | NFkB | SIX5 |
| ACAA1 | CSDA | IFIT1L | NFKB1 | SKAP1 |
| ACAP2 | CSF1 | IFIT2 | NFKB2 | SLAMF7 |
| ACAP3 | CSF1R | IFIT3 | NFKBIA | SLAMF8 |
| ACE | CSF2 | IFIT5 | NFKBIB | SLC11A1 |
| ACER3 | CSF2RA | IFITM1 | NFKBIE | SLC12A7 |
| ACKR2 | CSF2RB | IFITM2 | NFX1 | SLC12A9 |
| ACKR3 | CSF3 | IFITM3 | NFXL1 | SLC14A1 |
| ACKR4 | CSF3R | IFNA1/13 | NFYA | SLC15A2 |
| ACN9 | CSK | IFNA14/16 | NGDN | SLC15A3 |
| ACOX1 | CSNK1D | IFNA2 | NGFR | SLC16A13 |
| ACP5 | CSNK1G2 | IFNA4/7/10/17/21 | NGLY1 | SLC19A1 |
| ACPL2 | CSRNP1 | IFNA5 | NINJ2 | SLC1A3 |
| ACPP | CST3 | IFNA6 | NIP7 | SLC1A5 |
| ACSL1 | CSTB | IFNA8 | NIT1 | SLC22A18 |
| ACSL3 | CSTF2T | IFNAR1 | NKG7 | SLC25A11 |
| ACSL4 | CTBP1 | IFNAR2 | NKIRAS2 | SLC25A22 |
| ACTA2 | CTBP2 | IFNB1 | NLE1 | SLC25A28 |
| ACTG1 | CTDSP2 | IFNg | NLRC4 | SLC25A32 |
| ACTL10 | CTLA4 | IFNGR2 | NLRC5 | SLC25A5-AS1 |
| ACVR1 | CTSA | IFNK | NLRP 1 | SLC26A1 |
| ACVR1B | CTSB | IFNL1 | NLRP3 | SLC26A8 |
| ADA | CTSG | IFNL2/3 | NME4 | SLC27A3 |
| ADAM19 | CTSL | IFNL4 | NMES1 | SLC2A3 |
| ADAM8 | CTSL1 | IFNLR1 | NMUR1 | SLC2A4RG |
| ADAMTS3 | CTSO | IFNW1 | NNAT | SLC2A6 |
| ADAP1 | CTSS | IFP38 | NOA1 | SLC30A1 |
| ADAR | CTSW | IFTI1 | NOC3L | SLC35A3 |
| ADCK2 | CTSZ | IGF2BP2 | NOD1 | SLC35A4 |
| ADCK4 | CTU1 | IGF2R | NOD2 | SLC35C1 |

(continued)

| Gene | Gene | Gene | Gene | Gene |
|---|---|---|---|---|
| ADCK5 | CTU2 | IGFBP7 | NOL10 | SLC35D1 |
| ADCY3 | CUEDC2 | IGHMBP2 | NOL8 | SLC37A1 |
| ADCY7 | CUL1 | IGJ | NOS1AP | SLC37A3 |
| ADGRE3 | CX3CL1 | IGLV6 | NOS2 | SLC38A1 |
| ADGRE5 | CX3CR1 | IGSF6 | NOSIP | SLC38A10 |
| ADGRE5(CD97) | CXCL1 | IK | NOTCH1 | SLC39A1 |
| ADGRG3 | CXCL10 | IKBKAP | NOTCH2 | SLC39A13 |
| ADIPOR1 | CXCL11 | IKBKB | NOV | SLC39A7 |
| ADK | CXCL12 | IKBKE | NOX1 | SLC39A8 |
| ADM | CXCL13 | IKBKG | NP | SLC39A9 |
| ADO | CXCL14 | IKZF2 | NPAT | SLC4A1 |
| ADORA2A | CXCL16 | IKZF5 | NPC2 | SLC6A12 |
| ADORA3 | CXCL17 | IL10 | NPCDR1 | SLC7A5 |
| ADPRHL2 | CXCL2 | IL10RA | NPL | SLC7A7 |
| ADRBK2 | CXCL3 | IL10RB | NPW | SLC8A1 |
| ADRM1 | CXCL5 | IL11 | NQO2 | SLC9A3R2 |
| ADSL | CXCL6 | IL11RA | NR1H2 | SLCO3A1 |
| AGA | CXCL8 | IL12A | NR2C1 | SLCO4C1 |
| AGPAT2 | CXCL9 | IL12B | NR2F6 | SLPI |
| AGPAT3 | CXCR1 | IL12RB1 | NR3C1 | SMAD3 |
| AGPAT5 | CXCR2 | IL12RB2 | NR4A1 | SMAD4 |
| AGT | CXCR3 | IL13 | NRAS | SMAD5 |
| AGTRAP | CXCR4 | IL13RA1 | NRBF2 | SMAD7 |
| AHCTF1 | CXCR5 | IL13RA2 | NRD1 | SMARCD3 |
| AHNAK | CXCR6 | IL15 | NRN1L | SMC3 |
| AHR | CYB561D1 | IL15RA | NSUN3 | SMC6 |
| AIF1 | CYB5R3 | IL16 | NSUN5 | SMIM16 |
| AIG1 | CYBA | IL17A | NT5E | SMOX |
| AIM1L | CYBASC3 | IL17B | NTNG2 | SMPD4 |
| AIM2 | CYBRD1 | IL17C | NUB1 | SMPDL3A |
| AIRE | CYHR1 | IL17D | NUBP2 | SNAPC2 |
| AK1 | CYLD | IL17F | NUCB1 | SNAPC4 |
| AKIRIN2 | CYP1B1 | IL17RA | NUDT16L1 | SNAPIN |
| AKR1B1 | CYP27A1 | IL17RB | NUMB | SNCA |
| AKT1 | CYP2E1 | IL17RC | NUP160 | SNN |
| AKT1S1 | CYP4F3 | IL17RD | NUP205 | SNORD23 |
| AKT2 | CYSLTR1 | IL 17RE | OAF | SNRK |
| AKT3 | CYSTM1 | IL18 | OAS1 | SNTA1 |
| ALAS2 | CYTH1 | IL18BP | OAS2 | SNTB1 |

(continued)

| Gene | Gene | Gene | Gene | Gene |
|---|---|---|---|---|
| ALB | CYTH4 | IL18R1 | OAS3 | SNX1 |
| ALCAM | DAAM2 | IL18RAP | OASL | SNX15 |
| ALDH16A1 | DACH1 | IL19 | OBFC1 | SNX20 |
| ALDH1A1 | DALRD3 | IL1A | OCEL1 | SNX27 |
| ALDH2 | DAPK2 | IL1B | OCLN | SOAT1 |
| ALDH3A2 | DAPP1 | IL1F10 | OCR1 | SOATI |
| ALDH5A1 | DAZAP1 | IL1R1 | ODC1 | SOCS1 |
| ALG13 | DBNDD1 | IL1R2 | ODF3B | SOCS3 |
| ALKBH5 | DBNDD2 | IL1RAP | ODZ1 | SOD1 |
| ALKBH7 | DBP | IL1RAPL1 | OGFR | SOD2 |
| ALOX12 | DCP2 | IL1RAPL2 | OGFRL1 | SOLH |
| ALOX15 | DCTN5 | IL1RL1 | OGG1 | SON |
| ALOX5 | DDAH2 | IL1RL2 | OLAH | SORL1 |
| ALOX5AP | DDIT3 | IL1RN | OLFM1 | SORT1 |
| ALPK1 | DDIT4 | IL2 | OLFM4 | SOS2 |
| ALPL | DDOST | IL20 | OLIG1 | SOWAHD |
| ALX3 | DDX23 | IL20RA | OMG | SOX4 |
| AMFR | DDX31 | IL20RB | OPLAH | SP1 |
| AMICA1 | DDX3Y | IL21 | OPN3 | SP100 |
| ANAPC11 | DDX5 | IL21R | OPRL1 | SP3 |
| ANG | DDX58 | IL22 | OPTN | SPARC |
| ANK3 | DDX60 | IL22RA1 | OR52R1 | SPATA2 |
| ANKRD22 | DEAF1 | IL22RA2 | OR9A2 | SPATA2L |
| ANKRD28 | DEFA4 | IL23A | ORAI3 | SPATA5L1 |
| ANKRD34B | DEFB103A/B | IL23R | OS9 | SPATA6 |
| ANKRD49 | DENND1A | IL24 | OSBP2 | SPC25 |
| ANP32A | DENND3 | IL25 | OSBPL11 | SPHK2 |
| ANPEP | DENND4B | IL26 | OSBPL2 | SPI1 |
| ANXA2R | DERL1 | IL27 | OSCAR | SPIB |
| ANXA3 | DEXI | IL27RA | OSGIN1 | SPIN1 |
| AOAH | DGCR2 | IL2RA | OSM | SPINT2 |
| AP1G1 | DGKA | IL2RB | OSTalpha | SPNS 1 |
| AP1M1 | DHCR7 | IL2RG | OTOF | SPON2 |
| AP1S2 | DHRS7B | IL3 | OVCA2 | SPPL2A |
| AP2A1 | DHRS9 | IL31 | P2RX1 | SPSB2 |
| AP3B2 | DHX58 | IL31RA | P2RX7 | SPSB3 |
| AP5B1 | DIABLO | IL32 | P2RY10 | SQRDL |
| AP5Z1 | DIAPH2 | IL33 | P2RY14 | SRC |
| APBA3 | DIDO1 | IL34 | P2RY2 | SRF |

(continued)

| Gene | Gene | Gene | Gene | Gene |
|------|------|------|------|------|
| APBB1IP | DLEU2 | IL36A | P2RY6 | SRPK2 |
| APEX1 | DLGAP4 | IL36B | P4HA1 | SRXN1 |
| APEX2 | DMWD | IL36G | PACSIN2 | SSBP2 |
| APH1A | DNAAF2 | IL36RN | PADI2 | SSBP4 |
| APLP2 | DNAJA2 | IL37 | PADI4 | SSFA2 |
| APOBEC3B | DNAJA4 | IL3RA | PAFAH1B1 | SSNA1 |
| APOBEC3G | DNAJB1 | IL4 | PAK1 | SSPO |
| APOL1 | DNAJC10 | IL4R | PAK4 | SSR1 |
| APOL2 | DNAJC13 | IL5 | PAM | ST13 |
| APOL6 | DNAJC3 | IL5RA | PANK2 | ST3GAL1 |
| APOLD1 | DNAJC30 | IL6 | PANX1 | ST3GAL2 |
| APP | DNAJC9 | IL6R | PARP1 | ST3GAL5 |
| AQP7P1 | DNAL4 | IL6ST | PARP10 | ST6GALNAC4 |
| ARAP1 | DNMT1 | IL7 | PARP12 | STAB1 |
| AREG | DOCK10 | IL7R | PARP3 | STAM2 |
| ARF1 | DOCK2 | IL8 | PARP8 | STARD3NL |
| ARF5 | DOCK5 | IL9 | PARP9 | STAT1 |
| ARF6 | DOCK9 | IL9R | PATZ1 | STAT2 |
| ARFRP1 | DOK3 | IMP3 | PBX1 | STAT3 |
| ARG1 | DOK7 | IMPA2 | PBX3 | STAT4 |
| ARHGAP15 | DPAGT1 | IMPDH1 | PCBP1 | STAT5A |
| ARHGAP17 | DPEP2 | INHBA | PCBP2 | STAT5B |
| ARHGAP22 | DPF2 | INPP5D | PCF11 | STAT6 |
| ARHGAP25 | DPH3 | INPP5E | PCGF5 | STEAP4 |
| ARHGAP26 | DPM2 | INSIG1 | PCID2 | STING1 |
| ARHGAP27 | DPYSL2 | INSIG2 | PCMT1 | STK11IP |
| ARHGAP39 | DR1 | INTS1 | PCNX | STK17B |
| ARHGAP5 | DRAP1 | IPO7 | PCOLCE2 | STK19 |
| ARHGEF10L | DSC2 | IQCB1 | PCYT1A | STK25 |
| ARHGEF12 | DSE | IQCE | PD1 | STK3 |
| ARHGEF18 | DTNBP1 | IQSEC1 | PDCD1 | STK38L |
| ARHGEF19 | DTX3L | IRAK1 | PDCD10 | STMN3 |
| ARHGEF2 | DUSP16 | IRAK3 | PDCD1LG2 | STOM |
| ARHGEF6 | DUSP22 | IRAK4 | PDCD6IP | STOML1 |
| ARID1A | DUSP3 | IRF1 | PDCL3 | STRAP |
| ARIH2 | DUSP6 | IRF2 | PDE3B | STT3B |
| ARL14EP | DVL1 | IRF2BP1 | PDE4D | STUB1 |
| ARL17P1 | DYNLL1 | IRF3 | PDE6D | STX10 |
| ARL2BP | DYRK1B | IRF4 | PDE6H | STX11 |

(continued)

| Gene | Gene | Gene | Gene | Gene |
|------|------|------|------|------|
| ARL4C | DYRK2 | IRF5 | PDGFC | STX3 |
| ARL6IP5 | DYSF | IRF 7 | PDHB | STX6 |
| ARL8A | E2F6 | IRF8 | PDIA3 | STYXL1 |
| ARMC5 | EAF2 | IRF9 | PDK3 | SUCLG2 |
| ARRB1 | EBI3 | IRS2 | PDK4 | SUCNR1 |
| ARRB2 | ECHDC3 | ISCA2 | PDLIM1 | SUGT1 |
| ARRDC1 | EDEM2 | ISG15 | PDLIM2 | SULF2 |
| ASAP1 | EFCAB2 | ISG20 | PDPK1 | SULT1B1 |
| ASCC2 | EFHD2 | ISOC2 | PDS5B | SUOX |
| ASCC3 | EFTUD1 | ISY1-RAB43 | PDZK1IP1 | SUPT7L |
| ASCL2 | EGLN1 | ITGA1 | PEBP1 | SURF6 |
| ASGR1 | EGR1 | ITGA2B | PECAM1 | SYCE1L |
| ASGR2 | EHBP1L1 | ITGA4 | PECR | SYK |
| ASH2L | EHD1 | ITGA7 | PELI1 | SYNE2 |
| ASIC3 | EIF1AX | ITGAE | PELI2 | SYNGR2 |
| ASPH | EIF1AY | ITGAL | PEMT | SYPL1 |
| ASPHD2 | EIF2AK1 | ITGAM | PEN2 | SYT11 |
| ASPSCR1 | EIF2AK2 | ITGAX | PER1 | SYTL2 |
| ASXL2 | EIF2AK3 | ITGB2 | PEX1 | SZRD 1 |
| ATAD2B | EIF3F | ITGB7 | PEX10 | TAAR1 |
| ATF2 | EIF3H | ITIH4 | PEX6 | TAB1 |
| ATF3 | EIF4A2 | ITK | PF4 | TAB2 |
| ATF4 | EIF5A | ITLN1 | PF4V1 | TAC4 |
| ATF6 | EIF5B | ITM2A | PFDN5 | TADA2B |
| ATF7 | ELANE | ITM2C | PFKFB2 | TAF10 |
| ATF7IP2 | ELF4 | ITPKB | PFKFB3 | TAF12 |
| ATG10 | ELK1 | ITPKC | PGD | TAF13 |
| ATG12 | ELMO3 | ITPR3 | PGLS | TAF1C |
| ATG13 | ELOF1 | ITSN2 | PGLYRP1 | TAGLN2 |
| ATG3 | EMC10 | JAGN1 | PGP | TALDO1 |
| ATG4A | EMC6 | JAK1 | PGRMC1 | TANK |
| ATG7 | EMC8 | JAK2 | PGS1 | TAOK2 |
| ATHL1 | EMC9 | JAK3 | PHC2 | TAP1 |
| ATIC | EME2 | JAM3 | PHC3 | TAP2 |
| ATM | EMILIN2 | JAML | PHF11 | TAPI |
| ATOX1 | EMP1 | JARID2 | PHF2 | TAPT1-AS1 |
| ATP11B | EMR1 | JKAMP | PHF20 | TARBP1 |
| ATP13A3 | EMR2 | JTB | PHF20L1 | TAS2R31 |
| ATP2A2 | EMR3 | JUN | PHF3 | TBC1D10A |

(continued)

| Gene | Gene | Gene | Gene | Gene |
|------|------|------|------|------|
| ATP2B1 | ENDOG | JUNB | PHLDA2 | TBC1D20 |
| ATP5L | ENDOU | JUND | PHOSPHO1 | TBC1D22A |
| ATP6AP2 | ENGASE | JUP | PHTF1 | TBC1D2B |
| ATP6V0B | ENO1 | KBTBD2 | PI3 | TBC1D4 |
| ATP6V0C | ENOSF1 | KCMF1 | PIAS1 | TBC1D8 |
| ATP6VOD1 | ENPP2 | KCNC3 | PICALM | TBCB |
| ATP6V1B2 | ENTHD2 | KCNC4 | PIEZO1 | TBCE |
| ATP6V1C1 | ENTPD1 | KCND1 | PIGQ | TBK1 |
| ATP8A1 | ENTPD7 | KCNE1 | PIK3C2A | TBP |
| ATP8B4 | EOMES | KCNG1 | PIK3C3 | TBX21 |
| ATP9A | EPB41L3 | KCNJ2 | PIK3CA | TBXAS1 |
| ATXN3 | EPB42 | KCNJ2-AS1 | PIK3CB | TCAP |
| AUP1 | EPB49 | KCNMA1 | PIK3CD | TCF12 |
| AURKA | EPHB1 | KCTD13 | PIK3CG | TCF4 |
| AURKAIP1 | EPHB4 | KCTD14 | PIK3IP1 | TCF7 |
| AVEN | EPHX2 | KCTD15 | PIK3R1 | TCF7L2 |
| AZU1 | EPN1 | KCTD17 | PIK3R2 | TCFL5 |
| B3GALT4 | EPS8L1 | KCTD18 | PIK3R3 | TCIRG1 |
| B3GALT6 | EPSTI1 | KCTD5 | PIK3R4 | TCL1A |
| B3GAT3 | ERBB2 | KDM6B | PIK3R5 | TCN1 |
| B3GNT5 | ERBB2IP | KIAA0101 | PIK3R6 | TCN2 |
| B3GNT8 | ERCC4 | KIAA0232 | PIM2 | TCTN1 |
| B4GALT3 | ERGIC1 | KIAA0247 | PIM3 | TDRD9 |
| B4GALT4 | ERLIN1 | KIAA0319L | PINK1 | TECPR1 |
| B9D2 | ERN1 | KIAA0355 | PISD | TELO2 |
| BACH1 | ESF1 | KIAA0391 | PITPNA | TEP1 |
| BANK1 | ESRRA | KIAA0513 | PITPNM1 | TERF1 |
| BANP | ETS1 | KIAA0746 | PKD1 | TESC |
| BATF | ETS2 | KIAA0882 | PKD1P1 | TESK1 |
| BATF2 | ETV6 | KIAA0907 | PKN1 | TEX261 |
| BATF3 | ETV7 | KIAA1257 | PLA2G15 | TF |
| BAZ2B | EVI2A | KIAA1324 | PLA2G7 | TFDP2 |
| BBS10 | EVI5L | KIAA1370 | PLAC8 | TFE3 |
| BCAT1 | EVL | KIAA1598 | PLAT | TFEB |
| BCKDHB | EXOC3L1 | KIF11 | PLAU | TFIP11 |
| BCL11A | EXOC7 | KIF1B | PLAUR | TFRC |
| BCL11B | EXOSC10 | KIF1C | PLB1 | TGFA |
| BCL2 | EXOSC1O | KIF2C | PLCG1 | TGFB1 |
| BCL2L1 | EXOSC2 | KIFC2 | PLCG2 | TGFB2 |

(continued)

| Gene | Gene | Gene | Gene | Gene |
|---|---|---|---|---|
| BCL3 | EXOSC4 | KIR2DL1 | PLCL2 | TGFB3 |
| BCL6 | EXOSC8 | KIR2DL3 | PLEK | TGFBI |
| BCL7B | EXOSC9 | KIR3DL1 | PLEK2 | TGFBR1 |
| BCL7C | EXTL3 | KIR3DL1/2 | PLEKHA1 | TGFBR2 |
| BCR | F13A1 | KL | PLEKHA3 | TGFBR3 |
| BDKRB1 | F2RL1 | KLF1 | PLEKHF2 | TGFBR3L |
| BDKRB2 | F5 | KLF16 | PLEKHO1 | TGM1 |
| BECN1 | FABP2 | KLF2 | PLEKHO2 | TGOLN2 |
| BEX1 | FAIM3 | KLF3 | PLG | TGS1 |
| BEX4 | FAM108A1 | KLF4 | PLIN2 | THAP4 |
| BIRC3 | FAM109A | KLF6 | PLIN4 | THAP8 |
| BIRC5 | FAM110A | KLF7 | PLK1 | THBD |
| BLCAP | FAM118A | KLHDC2 | PLP2 | THBS1 |
| BLK | FAM118B | KLHDC8A | PLSCR1 | THOC2 |
| BLM | FAM122A | KLHL17 | PLXNC1 | THOP1 |
| BLNK | FAM127A | KLHL2 | PMAIP1 | TIA1 |
| BLOC1S4 | FAM127B | KLHL20 | PML | TIAM1 |
| BLVRA | FAM129A | KLHL24 | PMS2CL | TICAM 1 |
| BLVRB | FAM131A | KLHL26 | PNMA1 | TIFA |
| BMP2K | FAM134A | KLHL5 | PNOC | TIGD5 |
| BMX | FAM173A | KLHL6 | PNPLA1 | TIGIT |
| BNIP3 | FAM195A | KLRB1 | PNPLA6 | TIMM10 |
| BNIP3L | FAM195B | KLRC1 | PNRC1 | TIMMDC1 |
| BOP1 | FAM20C | KLRC3 | POGZ | TIMP2 |
| BPGM | FAM21B | KLRD1 | POLB | TIPARP |
| BPI | FAM229A | KLRF1 | POLD3 | TJAP1 |
| BRCC3 | FAM26F | KLRK1 | POLD4 | TKT |
| BRD1 | FAM30A | KPNA1 | POLDIP3 | TLE3 |
| BRD4 | FAM43A | KPNA5 | POLE2 | TLE4 |
| BSG | FAM46C | KPNB1 | POLL | TLK1 |
| BST1 | FAM50B | KPTN | POLR1D | TLN1 |
| BST2 | FAM65B | KRAS | POLR2A | TLR1 |
| BTBD2 | FAM83A | KREMEN1 | POLR2J | TLR10 |
| BTG1 | FAM89A | KRIT1 | POLRMT | TLR2 |
| BTG2 | FAM96B | KRT10 | POMP | TLR3 |
| BTN2A1 | FAM98C | KRT23 | POP7 | TLR4 |
| BTN3A1 | FAR2 | KRTAP15 | POR | TLR5 |
| BTN3A2 | FARP1 | KSR1 | POU2AF1 | TLR6 |
| BZRAP1 | FAS | LAG3 | PPARD | TLR7 |

(continued)

| Gene | Gene | Gene | Gene | Gene |
|------|------|------|------|------|
| BZW2 | FASLG | LAGE3 | PPBP | TLR8 |
| C11orf35 | FASTK | LAIR1 | PPCS | TLR9 |
| C11orf68 | FASTKD2 | LAMP1 | PPDPF | TM2D3 |
| C11orf74 | FBL | LAMP2 | PPIA | TMBIM1 |
| C11orf82 | FBP1 | LAMP3 | PPIF | TMCC2 |
| C12orf35 | FBRSL1 | LANCL1 | PPMIF | TMCO4 |
| C13orf18 | FBXL12 | LAP3 | PPM1M | TMEM101 |
| C14orf1 | FBXL13 | LAPTM4B | PPP1R10 | TMEM102 |
| C14orf101 | FBXL14 | LAPTM5 | PPP1R11 | TMEM106B |
| C14orf159 | FBXL15 | LARP1 | PPP1R12C | TMEM119 |
| C14orf169 | FBXL16 | LARP4 | PPP1R16A | TMEM123 |
| C14orf45 | FBXL6 | LASS4 | PPP1R18 | TMEM127 |
| C15orf39 | FBXO11 | LAT | PPP1R2 | TMEM129 |
| C15orf54 | FBXO28 | LAT2 | PPP1R35 | TMEM140 |
| C16orf72 | FBXO6 | LAX1 | PPP1R3D | TMEM144 |
| C16orf86 | FBXO7 | LBH | PPP1R3F | TMEM150A |
| C16orf95 | FBXO9 | LCK | PPP2R5A | TMEM164 |
| C17orf59 | FCAR | LCMT2 | PPP3R1 | TMEM165 |
| C17orf62 | FCER1A | LCN10 | PPP4R1 | TMEM179B |
| C17orf67 | FCER1G | LCN2 | PPP6R2 | TMEM187 |
| C17orf70 | FCER2 | LCP1 | PQLC3 | TMEM203 |
| C18orf10 | FCF1 | LCP2 | PRCC | TMEM204 |
| C19orf12 | FCGR1A | LDHA | PRCP | TMEM223 |
| C 19orf24 | FCGR1A/B | LDHB | PRDM1 | TMEM229B |
| C19orf25 | FCGR1B | LDLR | PRDM11 | TMEM230 |
| C19orf52 | FCGR1C | LDLRAP1 | PRDM8 | TMEM259 |
| C19orf66 | FCGR2A | LEF1 | PRELID1 | TMEM40 |
| C19orf71 | FCGR2B | LEMD2 | PREPL | TMEM50B |
| C1orf122 | FCGR3A/B | LENG1 | PRF1 | TMEM62 |
| C1orf128 | FCGRT | LENG9 | PRKAA1 | TMEM70 |
| C1orf159 | FCRL2 | LEPROTL1 | PRKAB1 | TMEM71 |
| C1orf161 | FCRL4 | LGALS1 | PRKAB2 | TMEM79 |
| C1orf162 | FDX1L | LGALS2 | PRKAG2 | TMEM87A |
| C1orf233 | FECH | LGALS3 | PRKAR2A | TMEM8A |
| C1QA | FEM1A | LGALS9 | PRKAR2B | TMEM8B |
| C1QB | FER1L3 | LHFP | PRKCA | TMOD1 |
| C1QBP | FES | LHFPL2 | PRKCD | TMPRSS2 |
| C1QR1 | FFAR2 | LIF | PRKCH | TMTC1 |
| C2 | FFAR3 | LILRA2 | PRKCQ | TMUB1 |

(continued)

| Gene | Gene | Gene | Gene | Gene |
|------|------|------|------|------|
| C20orf201 | FGD2 | LILRA3 | PRKCSH | TNF |
| C20orf24 | FGD3 | LILRA5 | PRKD2 | TNFa |
| C21orf7 | FGF11 | LILRA6 | PRKDC | TNFAIP2 |
| C22orf34 | FGF13 | LILRB2 | PRKRA | TNFAIP3 |
| C22orf37 | FGFBP2 | LILRB3 | PRKRIR | TNFAIP6 |
| C2CD2L | FGG | LIME1 | PRMT2 | TNFRSF10B |
| C2orf42 | FGL2 | LIMK1 | PROS1 | TNFRSF10D |
| C2orf47 | FGR | LIMK2 | PRPF38B | TNFRSF14 |
| C2orf68 | FIG4 | LIN7A | PRPF39 | TNFRSF17 |
| C2orf88 | FIS1 | LINC00174 | PRR13 | TNFRSF18 |
| C3 | FIZ1 | LINC00202-2 | PRR14 | TNFRSF1A |
| C3AR1 | FKBP11 | LIPT2 | PRR24 | TNFRSF25 |
| C3orf18 | FKBP4 | LITAF | PRR5L | TNFRSF4 |
| C3orf38 | FKBP5 | LMAN2L | PRR7 | TNFRSF6B |
| C4orf3 | FKBP8 | LMF2 | PRRG4 | TNFRSF9 |
| C4orf32 | FLII | LMNB1 | PRSS23 | TNFSF10 |
| C5 | FLJ10357 | LMO2 | PRSS30P | TNFSF12-TNFSF13 |
| C5AR1 | FLJ14186 | LOC100128751 | PRSS36 | TNFSF13 |
| C5orf4 | FLJ45445 | LOC100128822 | PRTN3 | TNFSF13B |
| C5orf56 | FLOT1 | LOC100128881 | PRUNE | TNFSF18 |
| C6orf1 | FLOT2 | LOC100129726 | PSAP | TNFSF4 |
| C6orf155 | FLT3 | LOC100130992 | PSEN1 | TNFSF8 |
| C7orf29 | FLT4 | LOC100131655 | PSENEN | TNFSF9 |
| C7orf50 | FNBP1 | LOC100132273 | PSKH1 | TNIP1 |
| C7orf53 | FNDC9 | LOC100133161 | PSMA5 | TNIP2 |
| C7orf58 | FNTA | LOC100133445 | PSMA6 | TNK2 |
| C8orf58 | FOLR3 | LOC100499489 | PSMB10 | TNRC6B |
| C9orf103 | FOS | LOC100506229 | PSMB3 | TNS1 |
| C9orf142 | FOSB | LOC100507463 | PSMB4 | TOLLIP |
| C9orf173 | FOXD4L3 | LOC115110 | PSMB8 | TOMM20 |
| C9orf69 | FOXJ2 | LOC136143 | PSMB9 | TOP2A |
| C9orf72 | FOXJ3 | LOC200230 | PSMD5 | TOP2B |
| C9orf78 | FOXO1 | LOC200772 | PSME1 | TOP3B |
| C9orf95 | FOXO3 | LOC284757 | PSME2 | TOPORS |
| CA1 | FOXP3 | LOC389734 | PSTPIP1 | TOR4A |
| CA4 | FPR1 | LOC401074 | PSTPIP2 | TP53I13 |
| CA5BP1 | FPR2 | LOC55924 | PTAFR | TP53I3 |
| CACFD1 | FRAT1 | LOC649143 | PTCHD3P1 | TP53RK |
| CACNA2D3 | FRAT2 | LOC729683 | PTEN | TP53TG1 |

(continued)

| Gene | Gene | Gene | Gene | Gene |
|---|---|---|---|---|
| CACTIN | FRG1B | LOC729852 | PTGDR | TPGS1 |
| CACTIN-AS1 | FRMD3 | LOC91561 | PTGER2 | TPK1 |
| CACYBP | FRMD8 | LONRF1 | PTGER4 | TPP1 |
| CAHM | FRS3 | LPA | PTGES3 | TPPP3 |
| CALM1 | FRY | LPAR2 | PTGS1 | TPSAB1B2 |
| CALM2 | FSD1L | LPAR5 | PTGS2 | TPST1 |
| CAMK1D | FTSJ1 | LPAR6 | PTK2B | TPST2 |
| CAMK2G | FTSJD2 | LPCAT2 | PTPN1 | TPX2 |
| CAMK4 | FUK | LPIN2 | PTPN20 | TRAF2 |
| CAMP | FURIN | LRCH4 | PTPN4 | TRAF3 |
| CANT1 | FUT7 | LRFN4 | PTPN6 | TRAF3IP2 |
| CAP1 | FUT8 | LRG1 | PTPRC | TRAF3IP3 |
| CAPN10 | FYB | LRMP | PTPRCAP | TRAF5 |
| CAPN2 | FYN | LRP10 | PTPRE | TRAF6 |
| CARD11 | G0S2 | LRRC41 | PTPRO | TRAFD1 |
| CARD16 | G3BP1 | LRRC47 | PTPRU | TRAK1 |
| CARD17 | G6PC3 | LRRC6 | PUM2 | TRAK2 |
| CARD9 | G6PD | LRRC61 | PUSL1 | TRAM1 |
| CARS2 | GAA | LRRC70 | PVRIG | TRAPPC12 |
| CASC3 | GAB2 | LRRC8C | PWP1 | TRAPPC2 |
| CASP1 | GABARAP | LRRC8D | PWWP2B | TRAT1 |
| CASP10 | GADD45A | LRRFIP1 | PXN | TRDD3 |
| CASP3 | GADD45B | LRRK2 | PYCARD | TREM1 |
| CASP4 | GALNT2 | LRRN3 | PYGL | TREML1 |
| CASP5 | GALNT3 | LSP1 | PYHIN1 | TRIB1 |
| CASP8 | GAS6 | LST1 | QDPR | TRIB2 |
| CASS4 | GAS7 | LTA | QRICH1 | TRIF |
| CASZ1 | GAS8 | LTA4H | R3HDM2 | TRIM 11 |
| CAT | GATA1 | LTB | R3HDM4 | TRIM14 |
| CBFA2T3 | GATA2 | LTBR | RAB10 | TRIM21 |
| CBFB | GATA3 | LTC4S | RAB11B | TRIM22 |
| CBL | GATAD2A | LTF | RAB11FIP1 | TRIM25 |
| CBLB | GBA | LY6E | RAB11FIP2 | TRIM27 |
| CBLL1 | GBGT1 | LY6G5B | RAB11FIP3 | TRIM3 |
| CBLN3 | GBP1 | LY86 | RAB 14 | TRIM33 |
| CBR1 | GBP2 | LY9 | RAB20 | TRIM5 |
| CBX7 | GBP3 | LY96 | RAB27A | TRIM56 |
| CBX8 | GBP4 | LYL1 | RAB31 | TRIM58 |
| CCDC101 | GBP5 | LYN | RAB32 | TRIM6 |

(continued)

| Gene | Gene | Gene | Gene | Gene |
|---|---|---|---|---|
| CCDC107 | GCA | LYNX1 | RAB35 | TRIM8 |
| CCDC115 | GCC1 | LYPLA2 | RAB40B | TRIOBP |
| CCDC 125 | GCC2 | LYSMD2 | RAB4B | TRIP11 |
| CCDC135 | GCH1 | LYST | RABSC | TRIP6 |
| CCDC154 | GCLM | MACF1 | RAB7A | TRIT1 |
| CCDC71L | GEMIN7 | MAEA | RABGAPIL | TRMT112 |
| CCDC94 | GGPS1 | MAF | RAC2 | TRMT44 |
| CCDC97 | GIMAP4 | MAFB | RACK1 | TRMT61A |
| CCL1 | GIMAP5 | MAFF | RAD23A | TROVE2 |
| CCL11 | GIMAP6 | MAFG | RAD50 | TRPC4AP |
| CCL13 | GIMAP7 | MAK | RAD51 | TSC22D3 |
| CCL14 | GIMAP8 | MAL | RAF1 | TSC22D4 |
| CCL15 | GIPC1 | MALT1 | RAFTLIN | TSEN34 |
| CCL16 | GK | MAMDC4 | RAI1 | TSHZ2 |
| CCL17 | GK3P | MAML1 | RALB | TSPAN13 |
| CCL18 | GLA | MAN1A1 | RANGAP1 | TSPAN2 |
| CCL19 | GLB1 | MAN1A2 | RAP1A | TSPAN31 |
| CCL2 | GLCCI1 | MAN2B2 | RAPGEF3 | TSPAN5 |
| CCL20 | GLDC | MANBAL | RARA | TSPO |
| CCL21 | GLG1 | MANEA | RARG | TSR3 |
| CCL22 | GLIPR1 | MANSC1 | RARRES3 | TSSC4 |
| CCL23 | GLO1 | MAOA | RASA4 | TST |
| CCL24 | GLRX5 | MAP1LC3A | RASGRP1 | TSTA3 |
| CCL25 | GLS | MAP1LC3B | RASGRP4 | TTC 17 |
| CCL26 | GLT25D1 | MAP2K2 | RASSF2 | TTC22 |
| CCL27 | GLTPD1 | MAP2K3 | RASSF5 | TTC27 |
| CCL28 | GLTSCR2 | MAP2K4 | RB1CC1 | TTC7B |
| CCL3/L1/L3 | GMEB2 | MAP2K7 | RBCK1 | TTC9C |
| CCL4 | GMFG | MAP3K1 | RBM10 | TTLL11 |
| CCL4/L1/L2 | GMIP | MAP3K11 | RBM15 | TTYH3 |
| CCL5 | GMNN | MAP3K3 | RBM15B | TUBA1A |
| CCL7 | GMPR | MAP3K5 | RBM23 | TUBA1B |
| CCL8 | GNA11 | MAP3K7 | RBM26 | TUBB1 |
| CCNA1 | GNA12 | MAP3K8 | RBM7 | TUSC2 |
| CCNA2 | GNA15 | MAP4K4 | RBMS1 | TWF2 |
| CCNB1 | GNAQ | MAPK1 | RBP7 | TXK |
| CCNB1IP1 | GNB2 | MAPK13 | RBPJ | TXN |
| CCNB2 | GNG11 | MAPK14 | RC3H2 | TXNIP |
| CCNC | GNG5 | MAPK8 | RCBTB2 | TYK2 |

(continued)

| Gene | Gene | Gene | Gene | Gene |
|------|------|------|------|------|
| CCND3 | GNG7 | MAPK8IP2 | RCE1 | TYMS |
| CCNG2 | GNLY | MAPK9 | RDX | TYROBP |
| CCNK | GNPTG | MAPKAPK2 | REL | U2AF1L4 |
| CCNT2 | GNS | MAPRE2 | RELA | UBA52 |
| CCNY | GOLGA7 | MAR4 | RELB | UBE2D1 |
| CCR1 | GOLPH3 | MARCKS | RELL2 | UBE2D2 |
| CCR10 | GOLPH3L | MARCKSL1 | RELT | UBE2D3 |
| CCR2 | GOT2 | MARCO | REPIN1 | UBE2F |
| CCR3 | GP9 | MARK3 | REPS1 | UBE2H |
| CCR4 | GPAA1 | MAST3 | RERE | UBE2J1 |
| CCR5 | GPBAR1 | MAT2B | RETN | UBE2J2 |
| CCR6 | GPBP1L1 | MATK | REXO2 | UBE2L6 |
| CCR7 | GPI | MAVS | RFC1 | UBE2N |
| CCR8 | GPN3 | MAX | RFESD | UBE2Q2 |
| CCR9 | GPR137 | MBD1 | RFX1 | UBE2S |
| CCRL2 | GPR137B | MBIP | RGL4 | UBFD1 |
| CD14 | GPR162 | MBNL3 | RGMA | UBN1 |
| CD151 | GPR171 | MBOAT7 | RGS1 | UBP1 |
| CD163 | GPR18 | MBP | RGS14 | UBQLN2 |
| CD177 | GPR25 | MCL1 | RGS16 | UBXN2B |
| CD19 | GPR56 | MCTP1 | RGS19 | UCN |
| CD1E | GPR65 | MCTP2 | RGS2 | UFM1 |
| CD2 | GPR84 | MDC1 | RGS3 | UFSP1 |
| CD209 | GPR97 | MDFIC | RHBDD3 | UGCG |
| CD22 | GPS2 | MDH1 | RHBDF2 | ULK1 |
| CD24 | GPSM1 | MDK | RHBDL1 | ULK2 |
| CD244 | GPSM3 | MDM2 | RHOB | UNC93B1 |
| CD247 | GPX3 | MED13 | RHOG | UNKL |
| CD27 | GPX7 | MED15 | RHOH | UPB1 |
| CD274 | GRAMD1B | MED17 | RILP | UPP1 |
| CD276 | GRAMD1C | MEF2A | RIN2 | USF1 |
| CD28 | GRAP2 | MEF2D | RIN3 | USF2 |
| CD300A | GRB10 | MEFV | RINL | USP10 |
| CD300C | GRB2 | MERTK | RIOK2 | USP15 |
| CD36 | GRIN3B | MESDC1 | RIPK1 | USP18 |
| CD37 | GRINA | METAP1 | RIPK2 | USP21 |
| CD38 | GRK5 | METRN | RIPK3 | USP30-AS1 |
| CD3D | GRWD1 | METTL13 | RIT1 | USP34 |
| CD3E | GSDMD | METTL3 | RMND1 | USP4 |

(continued)

| Gene | Gene | Gene | Gene | Gene |
|------|------|------|------|------|
| CD3G | GSK3B | METTL5 | RNASE1 | UTP14A |
| CD4 | GSPT1 | METTL7B | RNASE2 | UTRN |
| CD40 | GSR | MEX3D | RNASE6 | VAMP2 |
| CD40LG | GSTM1 | MFHAS1 | RNASEL | VAMP3 |
| CD44 | GSTM4 | MFSD12 | RNASET2 | VASN |
| CD45R0 | GSTO1 | MFSD7 | RNF10 | VASP |
| CD45RA | GUCD1 | MGAM | RNF11 | VAV3 |
| CD45RB | GUCY1A1 | MGAT1 | RNF114 | VCAM1 |
| CD48 | GUCY1B1 | MGAT2 | RNF130 | VEGFA |
| CD5 | GUK1 | MGEA5 | RNF135 | VENTX |
| CD52 | GYPA | MGST3 | RNF141 | VEZF1 |
| CD55 | GYPB | MIB2 | RNF146 | VEZT |
| CD59 | GYPC | MICA | RNF170 | VNN1 |
| CD6 | GYPE | MICAL1 | RNF19B | VNN3 |
| CD63 | GZMA | MICAL2 | RNF213 | VOPP1 |
| CD68 | GZMB | MICB | RNF31 | VPREB3 |
| CD69 | GZMH | MID1IP1 | RNF5 | VPS13A |
| CD7 | GZMK | MIEN1 | RNFT1 | VPS13B |
| CD70 | H1F0 | MIER2 | RNMT | VPS13C |
| CD74 | H3F3B | MIF | RNPEPL1 | VPS37A |
| CD79A | HAAO | MIIP | ROCK1 | VPS8 |
| CD79B | HACL1 | MINA | RP54X | VPS9D1 |
| CD80 | HAGH | MINPP1 | RPGRIP1 | VRK3 |
| CD81 | HAGHL | MIPEPP3 | RPIA | VSIG4 |
| CD82 | HAL | MIR1287 | RPL10A | VSIR |
| CD84 | HAMP | MIR1909 | RPL15 | VWF |
| CD86 | HAPLN3 | MIR4489 | RPL17 | WARS |
| CD8A | HAVCR2 | MIR5187 | RPL22 | WAS |
| CD8B | HBD | MIR658 | RPL6 | WASF2 |
| CD93 | HBM | MIR671 | RPL9 | WASH2P |
| CD96 | HBQ1 | MIR718 | RPP25 | WASH3P |
| CD97 | HBZ | MIR937 | RPP25L | WBP2 |
| CDA | HCAR3 | MKI67 | RPS14 | WDFY1 |
| CDC25A | HCK | MKLN1 | RPS4X | WDFY3 |
| CDC26 | HCLS 1 | MKNK 1 | RPS4Y1 | WDR24 |
| CDC34 | HCST | MKNK2 | RPS6KA1 | WDR37 |
| CDC42BPG | HDAC4 | MKRN1 | RPS6KA3 | WDR47 |
| CDC42EP2 | HDAC7 | MLKL | RPS6KA4 | WDR70 |
| CDC42EP4 | HDC | MLLT1 | RPS6KA5 | WDR75 |

(continued)

| Gene | Gene | Gene | Gene | Gene |
|---|---|---|---|---|
| CDH1 | HDHD1A | MLLT10 | RPS6KB1 | WIPF1 |
| CDIPT | HEATR1 | MLLT6 | RPUSD1 | WIPI1 |
| CDK1 | HEBP2 | MMD | RPUSD4 | WRAP73 |
| CDK2AP2 | HELZ2 | MME | RRAGC | WSB2 |
| CDK4 | HEMK1 | MMP17 | RRM2 | XAF1 |
| CDK5RAP2 | HERC5 | MMP25 | RRNAD1 | XBP1 |
| CDK6 | HERC6 | MMP8 | RRP12 | XCL1/2 |
| CDKN1B | HESX1 | MMP9 | RSAD2 | XCR1 |
| CDKN1C | HEXDC | MMRN1 | RSG1 | XK |
| CDPF1 | HFE | MOB3C | RTN1 | XKR8 |
| CDS2 | HGS | MORC3 | RTN3 | XPC |
| CEACAM1 | HGSNAT | MOSC1 | RTN4 | XPO4 |
| CEACAM3 | HHEX | MOSPD2 | RTP4 | XPO6 |
| CEACAM4 | HIAT1 | MOV10 | RUNX2 | YDJC |
| CEACAM6 | HIATL1 | MPC 1 | RUNX3 | YEATS4 |
| CEACAM8 | HIBCH | MPLKIP | RXRA | YIPF2 |
| CEBPA | HIC1 | MPO | RYBP | YIPF4 |
| CEBPB | HIC2 | MPPE1 | RYK | YJEFN3 |
| CEBPD | HIF1A | MPST | S100A12 | YKT6 |
| CEBPE | HINT1 | MPV17L2 | S100A9 | YPEL1 |
| CEBPG | HIP 1 | MPZL1 | S100B | YPEL5 |
| CECR1 | HIPK2 | MPZL2 | S100P | YTHDC2 |
| CECR5 | HIST1H1C | MPZL3 | S100PBP | YTHDF1 |
| CELF6 | HIST1H2AA | MRC1 | S1PR1 | YTHDF3 |
| CENPK | HIST1H2AJ | MRPL12 | SAFB2 | YWHAQ |
| CEP170 | HIST1H2BD | MRPL3 | SAMD1 | ZADH2 |
| CEP 192 | HIST1H2BG | MRPL34 | SAMD9 | ZAP70 |
| CEP55 | HIST1H2BJ | MRPL41 | SAMD9L | ZBED5 |
| CEP68 | HIST1H2BM | MRPL43 | SAMHD1 | ZBP1 |
| CEP97 | HIST1H3A | MRPL44 | SAMSN1 | ZBTB16 |
| CETP | HIST1H3B | MRPS10 | SAP130 | ZBTB18 |
| CFD | HIST1H3C | MRPS35 | SAP30 | ZBTB22 |
| CFLAR | HIST1H3H | MRS2 | SATB1 | ZBTB42 |
| CGAS | HIST1H3I | MS4A1 | SAYSD1 | ZBTB47 |
| CHD3 | HIST1H3J | MS4A2 | SBF2 | ZBTB7B |
| CHFR | HIST1H4C | MS4A4A | SBNO2 | ZBTB9 |
| CHI3L1 | HIST1H4E | MS4A7 | SCAMP4 | ZC3HAV1 |
| CHIC2 | HIST1H4H | MSL1 | SCAND1 | ZDHHC17 |
| CHKB-CPT1B | HIST1H4L | MSRA | SCARB2 | ZDHHC18 |

(continued)

| Gene | Gene | Gene | Gene | Gene |
|------|------|------|------|------|
| CHMP1A | HIST2H2AA3 | MSRB1 | SCARF1 | ZDHHC19 |
| CHMP1B | HIST2H2AC | MST1P2 | SCO1 | ZDHHC24 |
| CHMP4B | HIST2H2BE | MTIE | SCYL1 | ZDHHC3 |
| CHMP6 | HIST2H2BF | MTIG | SDCCAG3 | ZDHHC7 |
| CHMP7 | HK3 | MTIL | SDF2L1 | ZDHHC8 |
| CHN2 | HLA-A | MTIM | SDF4 | ZFAND3 |
| CHST11 | HLA-B | MT2A | SDHC | ZFAND5 |
| CHST12 | HLA-C | MTCH1 | SDHD | ZFC3H1 |
| CHST2 | HLA-DMA | MTF1 | SDPR | ZFP36 |
| CHSY1 | HLA-DMB | MTHFS | SEC24A | ZFP36L1 |
| CHTF8 | HLA-DOB | MTMR11 | SEC61A1 | ZFP36L2 |
| CHTOP | HLA-DPA1 | MTMR3 | SEC62 | ZFPL1 |
| CHUK | HLA-DPB1 | MTO1 | SECTM1 | ZFPM1 |
| CIAPIN1 | HLA-DQA | MTOR | SEH1L | ZFYVE16 |
| CIITA | HLA-DQA1 | MTRR | SELE | ZFYVE21 |
| CISD3 | HLA-DQB1 | MUL1 | SELENBP1 | ZHX2 |
| CISH | HLA-DRA | MVB12A | SELENOP | ZMAT5 |
| CIT | HLA-DRB | MVP | SELENOS | ZMIZ1 |
| CITED2 | HLA-DRB1 | MX1 | SELL | ZMYND11 |
| CKAP2 | HLA-DRB3 | MX2 | SELP | ZNF143 |
| CKS2 | HLA-DRB4 | MXD1 | SEM1 | ZNF148 |
| CLASRP | HLA-DRB5 | MXD3 | SEMA4A | ZNF200 |
| CLC | HLA-DRB6 | MXD4 | SEMA4B | ZNF213 |
| CLEC10A | HLA-E | MXI1 | SEMA4D | ZNF266 |
| CLEC1B | HLA-F-AS1 | MYC | SEMA6B | ZNF274 |
| CLEC2B | HLTF | MYCL1 | SEMA7A | ZNF276 |
| CLEC4A | HLX | MYD88 | SEPHS2 | ZNF28 |
| CLEC4D | HMBS | MYH9 | SEPP1 | ZNF281 |
| CLEC4E | HMG20B | MYL9 | SEPW1 | ZNF292 |
| CLEC5A | HMGB1 | MYO9A | SERBP1 | ZNF296 |
| CLEC7A | HMGB2 | MYOF | SERINC3 | ZNF319 |
| CLIC2 | HMHA1 | MZF1 | SERINC5 | ZNF341 |
| CLIC3 | HMMR | N4BP1 | SERP1 | ZNF354A |
| CLIP4 | HMOX1 | NA | SERPINA1 | ZNF408 |
| CLK2 | HNF1A | NAB1 | SERPINB1 | ZNF467 |
| CLK3 | HNRNPA0 | NACA | SERPINB2 | ZNF469 |
| CLK4 | HOOK2 | NACC2 | SERPING1 | ZNF496 |
| CLOCK | HOPX | NADK | SERTAD1 | ZNF503 |
| CLPP | HP | NAE1 | SERTAD2 | ZNF507 |

(continued)

| Gene | Gene | Gene | Gene | Gene |
|---|---|---|---|---|
| CLU | HPCAL1 | NAGS | SERTAD3 | ZNF513 |
| CLUAP1 | HPGD | NAIF1 | SESN1 | ZNF524 |
| CMTM5 | HPN | NAMPT | SETD1B | ZNF562 |
| CNDP2 | HPS1 | NAPA | SETD2 | ZNF576 |
| CNNM3 | HPS6 | NAPSB | SETD8 | ZNF579 |
| CNOT3 | HPSE | NARF | SETX | ZNF587 |
| CNOT7 | HRB2 | NARFL | SF3A2 | ZNF608 |
| CNPY3 | HRH4 | NCAPH2 | SF3B4 | ZNF618 |
| CNTNAP2 | HS2ST1 | NCBP1 | SFRS9 | ZNF646 |
| COASY | FISD11B1 | NCBP2 | SFT2D1 | ZNF672 |
| COL11A2 | HSD17B1 | NCF1 | SFT2D3 | ZNF703 |
| COL17A1 | HSP90AA1 | NCF2 | SFXN1 | ZNF706 |
| COPS7A | HSP90AB1 | NCF4 | SGMS2 | ZNF710 |
| COPS7B | HSP90B1 | NCK2 | SGSH | ZNF740 |
| COQ9 | HSPA6 | NCKAP5L | SGSM3 | ZNF747 |
| COTL1 | HSPA7 | NCOA1 | SGTA | ZNF775 |
| COX15 | HSPA8 | NCOA4 | SH2B3 | ZNF804A |
| CPA3 | HSPB1 | NCOA5 | SH2D1A | ZNF862 |
| CPD | HSPC159 | NCOA6 | SH2D1B | ZNRF2 |
| CPM | HSPE1 | NCR1 | SH2D3C | ZRANB1 |
| CPNE5 | HSPH1 | NCR3 | SH2D4A | ZSCAN18 |
| CPVL | HTRA2 | NDE1 | SH3BP2 | ZVX |
| CR1 | HVCN1 | NDEL1 | SH3BP5L | ZXDB |
| CR2 | ICAM1 | NOFIP1 | SH3GLB1 | ZXDC |
| CREB1 | ICAM2 | NDOR1 | SH3GLB2 | ZYX |
| ICAM5 | ICAM3 | NDST2 | SH3PXD2A | SH3PXD2B |
| DDX6 | PDE4B | RAPGEF1 | TMEM19 | ZBTB33 |

In some embodiments, the systems and methods for determining an infectious disease state in a subject disclosed herein comprise obtaining attribute values from a biological sample of the subject for a plurality of genes, wherein the genes comprise one or more of LY6E, IRF9, ITGAM, and PSTPIP2 selected from Table 8. In some embodiments, the genes comprise any two selected from LY6E, IRF9, ITGAM, and PSTPIP2. In some embodiments, the two genes are LY6E and IRF9, LY6E and ITGAM, LY6E and PSTPIP2, IRF9 and ITGAM, IRF9 and PSTPIP2, or ITGAM and PSTPIP2. In some embodiments, the genes comprise any three genes selected from LY6E, IRF9, ITGAM, and PSTPIP2. In some embodiments, the three genes are (i) LY6E, IRF9, and ITGAM, (ii) LY6E, IRF9, and PSTPIP2, (iii) LY6E, ITGAM, and PSTPIP2, (iv) IRF9, ITGAM, and PSTPIP2. In some embodiments, the genes comprise all four of LY6E, IRF9, ITGAM, and PSTPIP2. In some embodiments, the attribute values of the genes are the mRNA abundance levels or the gene expression. In some embodiments, there can be optionally one or more additional genes in the plurality of genes.

In some embodiments, the plurality of genes comprises at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, at least 45, at least 46, at least 47, or at least 48 genes selected from Table 8. In some embodiments, the plurality of genes comprises at least 10, at least 20, at least 30, at least 40, at least 50, at least 60, at least 70, at least 80, at least 90, or at least 100 genes selected from Table 8. In some embodiments, the plurality of genes comprises at least 100, at least 200, at least 300, at least 400, at least 500, at least 600, at least 700, at least 800, at least 900, or at least 1000 genes selected from Table 8.

In some embodiments, the plurality of genes comprises no more than 2000, no more than 1000, no more than 900, no more than 800, no more than 700, no more than 600, no more than 500, no more than 400, no more than 300, no more than 200, no more than 100, no more than 90, no more than 80, no more than 70, no more than 60, no more than 50, no more than 40, or no more than 30 genes selected from Table 8. In some embodiments, the plurality of genes comprises between 5 and 10, between 2 and 50, between 10 and 200, between 20 and 500, between 10 and 80, between 30 and 100, between 100 and 1000, between 300 and 2000, or between 1000 and 2000 genes selected from Table 8. In some embodiments, the plurality of genes includes between 15 genes and 50 genes selected from Table 8. In some embodiments, the plurality of genes includes between 15 genes and 40 genes selected from Table 8. In some embodiments, the plurality of genes includes between 15 genes and 30 genes selected from Table 8. In some embodiments, the plurality of genes includes between 20 genes and 50 genes selected from Table 8. In some embodiments, the plurality of genes includes between 20 genes and 40 genes selected from Table 8. In some embodiments, the plurality of genes includes between 20 genes and 30 genes selected from Table 8. In some embodiments, the plurality of genes includes between 25 genes and 50 genes selected from Table 8. In some embodiments, the plurality of genes includes between 25 genes and 40 genes selected from Table 8. In some embodiments, the plurality of genes includes between 25 genes and 35 genes selected from Table 8. In some embodiments, the plurality of genes includes between 25 genes and 30 genes selected from Table 8. In some embodiments, the plurality of genes falls within another range starting no lower than 10 genes selected from Table 8 and ending no higher than 2000 genes selected from Table 8. In some embodiments, the plurality of genes falls within another range starting no lower than 2 genes selected from Table 8 and ending no higher than 2000 genes selected from Table 8.

In some embodiments, the plurality of genes comprising one or more genes selected from Table 8 comprise any of the embodiments for genes (*e.g.*, biomarkers) disclosed herein, as described above in the sections entitled "Biomarkers" and "Measurement of Biomarkers."

*Embodiments Integrating Additional Biomarkers*

In some embodiments, a method for determining an infectious disease state in a subject is provided that integrates at least an improvement in a method for obtaining and using a classifier, as described above in the sections entitled "Selection of Configurations" and "Classifiers and Hyperparameters," and an improvement in a plurality of genes (*e.g.*, biomarkers) for detection of attribute values, as described above in the sections entitled "Additional Biomarkers" and "Measurement of Biomarkers."

Accordingly, in one embodiment, a method is provided for determining an infectious disease state of a subject, the method comprising obtaining a training dataset including labels for infectious disease states and respective attribute values for a plurality of genes listed in Table 8, obtained from biological samples of a plurality of training subjects and performing a classifier selection process as described above in the sections entitled "Selection of Configurations" and "Training Classifiers." In some embodiments, the training data set includes respective attribute values for at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 30, at least 40, at least 50, at least 100, at least 250, at least 500, at least 1000, at least 2000, at least 3000, or all of the genes listed in Table 8. In some embodiments, the training data set includes respective attribute values for one or more genes not listed in Table 8.

In another embodiment of the present disclosure, a method is provided for determining an infectious disease state of a test subject, the method comprising obtaining a dataset having attribute values for a plurality of genes listed in Table 8 from a biological sample of the test subject, and, responsive to inputting the plurality of attribute values to a classifier, obtaining a determination as to whether the test subject has an infectious disease state selected from infected with a bacteria, infected with a virus, and not-infected, as described above in the section entitled "Determining Infectious Disease States." In some embodiments, the dataset includes respective attribute values for at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 25, at least 30, at least 40, at least 50, at least 60, at least 70, at least 80, at least 90, at least 100, or more of the genes listed in Table 8. In some embodiments, the dataset includes respective attribute values for one or more genes not listed in Table 8.

Accordingly, in one embodiment, a method is provided for determining an infectious disease state of a test subject, the method comprising obtaining a dataset having attribute values for a plurality of genes listed in Table 8 from a biological sample of the test subject, and, responsive to inputting the plurality of attribute values to a classifier, obtaining a determination as to whether the test subject has an infectious disease state selected from infected with a bacteria, infected with a virus, and not-infected, as described above in the section entitled "Determining Infectious Disease States," where the classifier is obtained by performing a method comprising obtaining a training dataset including labels for infectious disease states and respective attribute values for the plurality of genes obtained from biological samples of a plurality of training subjects and performing a classifier selection process as described above in the sections entitled "Selection of Configurations" and "Training Classifiers." In some embodiments, the dataset includes respective attribute values for at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 25, at least 30, at least 40, at least 50, at least 60, at least 70, at least 80, at least 90, at least 100, or more of the genes listed in Table 8. In some embodiments, the dataset includes respective attribute values for one or more genes not listed in Table 8.

In some embodiments, the classifier is any classifier, as described above in the section entitled "Classifiers and Hyperparameters," and the plurality of genes comprises from 2 to 25 genes for determining an infectious disease state selected from Table 8, as described in the above section entitled "Additional Biomarkers." In some embodiments, the classifier is any classifier, as described above in the section entitled "Classifiers and Hyperparameters," and the plurality of genes comprises from 2 to 25, from 5 to 50, from 10 to 150, from 25 to 500, or from 50 to 1000 genes selected from Table 8, as described in the above section entitled "Additional Biomarkers." In some embodiments, the plurality of genes comprises any number of genes selected from Table 8, as described in the above section entitled "Additional Biomarkers." In some embodiments, the plurality of genes includes one or more genes not listed in Table 8.

In some embodiments, the classifier is any classifier, as described above in the section entitled "Classifiers and Hyperparameters," the plurality of attribute values is measured, for the plurality of genes, using isothermal amplification from a biological sample comprising blood, as described in the above sections entitled "Biomarkers" and "Measurement of Biomarkers," and the plurality of genes comprises from 2 to 25 genes for determining an infectious disease state selected from Table 8, as described in the above section entitled "Additional Biomarkers." In some embodiments, the classifier is any classifier, as described above in the section entitled "Classifiers and Hyperparameters," the plurality of attribute values is measured, for the plurality of genes, using isothermal amplification from a biological sample comprising blood, as described in the above sections entitled "Biomarkers" and "Measurement of Biomarkers," and the plurality of genes comprises from 5 to 50, from 10 to 150, from 25 to 500, or from 50 to 1000 genes selected from Table 8, as described in the above section entitled "Additional Biomarkers." In some embodiments, the plurality of genes comprises any number of genes selected from Table 8, as described in the above section entitled "Additional Biomarkers." In some embodiments, the plurality of genes includes one or more genes not listed in Table 8.

In some embodiments, the classifier is a neural network, as described above in the section entitled "Classifiers and Hyperparameters," and the plurality of genes comprises from 2 to 25 genes for determining an infectious disease state selected from Table 8, as described in the above section entitled "Additional Biomarkers." In some embodiments, the classifier is a neural network, as described above in the section entitled "Classifiers and Hyperparameters," and the plurality of genes comprises from 2 to 25, from 5 to 50, from 10 to 150, from 25 to 500, or from 50 to 1000 genes selected from Table 8, as described in the above section entitled "Additional Biomarkers." In some embodiments, the plurality of genes comprises any number of genes selected from Table 8, as described in the above section entitled "Additional Biomarkers." In some embodiments, the plurality of genes includes one or more genes not listed in Table 8.

In some embodiments, the classifier is a neural network, as described above in the section entitled "Classifiers and Hyperparameters," the plurality of attribute values is measured, for the plurality of genes, using isothermal amplification from a biological sample comprising blood, as described in the above sections entitled "Biomarkers" and "Measurement of Biomarkers," and the plurality of genes comprises from 2 to 25 genes for determining an infectious disease state selected from Table 8, as described in the above section entitled "Additional Biomarkers." In some embodiments, the classifier is a neural network, as described above in the section entitled "Classifiers and Hyperparameters," the plurality of attribute values is measured, for the plurality of genes, using isothermal amplification from a biological sample comprising blood, as described in the above sections entitled "Biomarkers" and "Measurement of Biomarkers," and the plurality of genes comprises from 2 to 25, from 5 to 50, from 10 to 150, from 25 to 500, or from 50 to 1000 genes selected from Table 8, as described in the above section entitled "Additional Biomarkers." In some embodiments, the plurality of genes comprises any number of genes selected from Table 8, as described in the above section entitled "Additional Biomarkers." In some embodiments, the plurality of genes includes one or more genes not listed in Table 8.

In some embodiments, the classifier is a neural network comprising a plurality of hyperparameters selected using a configuration selection process (*e.g.*, a hyperband method), as described above in the section entitled "Selection of Configurations," and the plurality of genes comprises from 2 to 25 genes for determining an infectious disease state selected from Table 8, as described in the above section entitled "Additional Biomarkers." In some embodiments, the classifier is a neural network comprising a plurality of hyperparameters selected using a configuration selection process (*e.g.,* a hyperband method), as described above in the section entitled "Selection of Configurations," and the plurality of genes comprises from 2 to 25, from 5 to 50, from 10 to 150, from 25 to 500, or from 50 to 1000 genes selected from Table 8, as described in the above section entitled "Additional Biomarkers." In some embodiments, the plurality of genes comprises any number of genes selected from Table 8, as described in the above section entitled "Additional Biomarkers." In some embodiments, the plurality of genes includes one or more genes not listed in Table 8.

In some embodiments, the classifier is a neural network comprising a plurality of hyperparameters selected using a configuration selection process *(e.g.,* a hyperband method), as described above in the section entitled "Selection of Configurations," the plurality of attribute values is measured, for the plurality of genes, using isothermal amplification from a biological sample comprising blood, as described in the above sections entitled "Biomarkers" and "Measurement of Biomarkers," and the plurality of genes comprises from 2 to 25 genes for determining an infectious disease state selected from Table 8, as described in the above section entitled "Additional Biomarkers." In some embodiments, the classifier is a neural network comprising a plurality of hyperparameters selected using a configuration selection process *(e.g.,* a hyperband method), as described above in the section entitled "Selection of Configurations," the plurality of attribute values is measured, for the plurality of genes, using isothermal amplification from a biological sample comprising blood, as described in the above sections entitled "Biomarkers" and "Measurement of Biomarkers," and the plurality of genes comprises from 2 to 25, from 5 to 50, from 10 to 150, from 25 to 500, or from 50 to 1000 genes selected from Table 8, as described in the above section entitled "Additional Biomarkers." In some embodiments, the plurality of genes comprises any number of genes selected from Table 8, as described in the above section entitled "Additional Biomarkers." In some embodiments, the plurality of genes includes one or more genes not listed in Table 8.

In some embodiments, the classifier is an ensemble classifier, as described above in the section entitled "Selection of Configurations," and the plurality of genes comprises from 2 to 25 genes for determining an infectious disease state selected from Table 8, as described in the above section entitled "Additional Biomarkers." In some embodiments, the classifier is an ensemble classifier, as described above in the section entitled "Selection of Configurations," and the plurality of genes comprises from 2 to 25, from 5 to 50, from 10 to 150, from 25 to 500, or from 50 to 1000 genes selected from Table 8, as described in the above section entitled "Additional Biomarkers." In some embodiments, the plurality of genes comprises any number of genes selected from Table 8, as described in the above section entitled "Additional Biomarkers." In some embodiments, the plurality of genes includes one or more genes not listed in Table 8.

In some embodiments, the classifier is an ensemble classifier, as described above in the section entitled "Selection of Configurations," the plurality of attribute values is measured, for the plurality of genes, using isothermal amplification from a biological sample comprising blood, as described in the above sections entitled "Biomarkers" and "Measurement of Biomarkers," and the plurality of genes comprises from 2 to 25 genes for determining an infectious disease state selected from Table 8, as described in the above section entitled "Additional Biomarkers." In some embodiments, the classifier is an ensemble classifier, as described above in the section entitled "Selection of Configurations," the plurality of attribute values is measured, for the plurality of genes, using isothermal amplification from a biological sample comprising blood, as described in the above sections entitled "Biomarkers" and "Measurement of Biomarkers," and the plurality of genes comprises from 2 to 25, from 5 to 50, from 10 to 150, from 25 to 500, or from 50 to 1000 genes selected from Table 8, as described in the above section entitled "Additional Biomarkers." In some embodiments, the plurality of genes comprises any number of genes selected from Table 8, as described in the above section entitled "Additional Biomarkers." In some embodiments, the plurality of genes includes one or more genes not listed in Table 8.

In some embodiments, the classifier is an ensemble classifier comprising a plurality of neural networks, as described above in the section entitled "Selection of Configurations," and the plurality of genes comprises from 2 to 25 genes for determining an infectious disease state selected from Table 8, as described in the above section entitled "Additional Biomarkers." In some embodiments, the classifier is an ensemble classifier comprising a plurality of neural networks, as described above in the section entitled "Selection of Configurations," and the plurality of genes comprises from 2 to 25, from 5 to 50, from 10 to 150, from 25 to 500, or from 50 to 1000 genes selected from Table 8, as described in the above section entitled "Additional Biomarkers." In some embodiments, the plurality of genes comprises any number of genes selected from Table 8, as described in the above section entitled "Additional Biomarkers." In some embodiments, the plurality of genes includes one or more genes not listed in Table 8.

In some embodiments, the classifier is an ensemble classifier comprising a plurality of neural networks, as described above in the section entitled "Selection of Configurations," the plurality of attribute values is measured, for the plurality of genes, using isothermal amplification from a biological sample comprising blood, as described in the above sections entitled "Biomarkers" and "Measurement of Biomarkers," and the plurality of genes comprises from 2 to 25 genes for determining an infectious disease state selected from Table 8, as described in the above section entitled "Additional Biomarkers." In some embodiments, the classifier is an ensemble classifier comprising a plurality of neural networks, as described above in the section entitled "Selection of Configurations," the plurality of attribute values is measured, for the plurality of genes, using isothermal amplification from a biological sample comprising blood, as described in the above sections entitled "Biomarkers" and "Measurement of Biomarkers," and the plurality of genes comprises from 2 to 25, from 5 to 50, from 10 to 150, from 25 to 500, or from 50 to 1000 genes selected from Table 8, as described in the above section entitled "Additional Biomarkers." In some embodiments, the plurality of genes comprises any number of genes selected from Table 8, as described in the above section entitled "Additional Biomarkers." In some embodiments, the plurality of genes includes one or more genes not listed in Table 8.

In some embodiments, the classifier is an ensemble classifier comprising a plurality of hyperparameters selected using a configuration selection process, as described above in the section entitled "Selection of Configurations," and the plurality of genes comprises from 2 to 25 genes for determining an infectious disease state selected from Table 8, as described in the above section entitled "Additional Biomarkers." In some embodiments, the classifier is an ensemble classifier comprising a plurality of hyperparameters selected using a configuration selection process, as described above in the section entitled "Selection of Configurations," and the plurality of genes comprises from 2 to 25, from 5 to 50, from 10 to 150, from 25 to 500, or from 50 to 1000 genes selected from Table 8, as described in the above section entitled "Additional Biomarkers." In some embodiments, the plurality of genes comprises any number of genes selected from Table 8, as described in the above section entitled "Additional Biomarkers." In some embodiments, the plurality of genes includes one or more genes not listed in Table 8.

In some embodiments, the classifier is an ensemble classifier comprising a plurality of hyperparameters selected using a configuration selection process, as described above in the section entitled "Selection of Configurations," the plurality of attribute values is measured, for the plurality of genes, using isothermal amplification from a biological sample comprising blood, as described in the above sections entitled "Biomarkers" and "Measurement of Biomarkers," and the plurality of genes comprises from 2 to 25 genes for determining an infectious disease state selected from Table 8, as described in the above section entitled "Additional Biomarkers." In some embodiments, the classifier is an ensemble classifier comprising a plurality of hyperparameters selected using a configuration selection process, as described above in the section entitled "Selection of Configurations," the plurality of attribute values is measured, for the plurality of genes, using isothermal amplification from a biological sample comprising blood, as described in the above sections entitled "Biomarkers" and "Measurement of Biomarkers," and the plurality of genes comprises from 2 to 25, from 5 to 50, from 10 to 150, from 25 to 500, or from 50 to 1000 genes selected from Table 8, as described in the above section entitled "Additional Biomarkers." In some embodiments, the plurality of genes comprises any number of genes selected from Table 8, as described in the above section entitled "Additional Biomarkers." In some embodiments, the plurality of genes includes one or more genes not listed in Table 8.

In some embodiments, the classifier is an ensemble classifier comprising a plurality of neural networks, each neural network comprising a plurality of hyperparameters selected using a configuration selection process, as described above in the section entitled "Selection of Configurations," and the plurality of genes comprises from 2 to 25 genes for determining an infectious disease state selected from Table 8, as described in the above section entitled "Additional Biomarkers." In some embodiments, the classifier is an ensemble classifier comprising a plurality of neural networks, each neural network comprising a plurality of hyperparameters selected using a configuration selection process, as described above in the section entitled "Selection of Configurations," and the plurality of genes comprises from 2 to 25, from 5 to 50, from 10 to 150, from 25 to 500, or from 50 to 1000 genes selected from Table 8, as described in the above section entitled "Additional Biomarkers." In some embodiments, the plurality of genes comprises any number of genes selected from Table 8, as described in the above section entitled "Additional Biomarkers." In some embodiments, the plurality of genes includes one or more genes not listed in Table 8.

In some embodiments, the classifier is an ensemble classifier comprising a plurality of neural networks, each neural network comprising a plurality of hyperparameters selected using a configuration selection process, as described above in the section entitled "Selection of Configurations," the plurality of attribute values is measured, for the plurality of genes, using isothermal amplification from a biological sample comprising blood, as described in the above sections entitled "Biomarkers" and "Measurement of Biomarkers," and the plurality of genes comprises from 2 to 25 genes for determining an infectious disease state selected from Table 8, as described in the above section entitled "Additional Biomarkers." In some embodiments, the classifier is an ensemble classifier comprising a plurality of neural networks, each neural network comprising a plurality of hyperparameters selected using a configuration selection process, as described above in the section entitled "Selection of Configurations," the plurality of attribute values is measured, for the plurality of genes, using isothermal amplification from a biological sample comprising blood, as described in the above sections entitled "Biomarkers" and "Measurement of Biomarkers," and the plurality of genes comprises from 2 to 25, from 5 to 50, from 10 to 150, from 25 to 500, or from 50 to 1000 genes selected from Table 8, as described in the above section entitled "Additional Biomarkers." In some embodiments, the plurality of genes comprises any number of genes selected from Table 8, as described in the above section entitled "Additional Biomarkers." In some embodiments, the plurality of genes includes one or more genes not listed in Table 8.

Another aspect of the present disclosure provides a composition including a plurality of amplification primers for determining an infectious disease state of a subject, the plurality of amplification primers comprising, for each respective gene in a plurality of genes, a respective forward amplification primer and a respective reverse amplification primer as described in the above section entitled "Compositions," where the plurality of genes comprises one or more genes selected from Table 8, as described in the above section entitled "Additional Biomarkers." In some embodiments, the plurality of genes includes one or more genes not listed in Table 8.

In some embodiments, the plurality of genes comprises from 2 to 25 genes for determining an infectious disease state selected from Table 8, as described in the above section entitled "Additional Biomarkers." In some embodiments, the plurality of genes comprises from 2 to 25, from 5 to 50, from 10 to 150, from 25 to 500, or from 50 to 1000 genes selected from Table 8, as described in the above section entitled "Additional Biomarkers." In some embodiments, the plurality of genes comprises any number of genes selected from Table 8, as described in the above section entitled "Additional Biomarkers." In some embodiments, the plurality of genes includes one or more genes not listed in Table 8.

Another aspect of the present disclosure provides a kit including agents for determining an infectious disease state of a subject, including a plurality of amplification primers comprising, for each respective gene in a plurality of genes, a respective forward amplification primer and a respective reverse amplification primer as described in the above section entitled "Kits," where the plurality of genes comprises one or more genes selected from Table 8, as described in the above section entitled "Additional Biomarkers." In some embodiments, the plurality of genes includes one or more genes not listed in Table 8.

In some embodiments, the plurality of genes comprises from 2 to 25 genes for determining an infectious disease state selected from Table 8, as described in the above section entitled "Additional Biomarkers." In some embodiments, the plurality of genes comprises from 2 to 25, from 5 to 50, from 10 to 150, from 25 to 500, or from 50 to 1000 genes selected from Table 8, as described in the above section entitled "Additional Biomarkers." In some embodiments, the plurality of genes comprises any number of genes selected from Table 8, as described in the above section entitled "Additional Biomarkers." In some embodiments, the plurality of genes includes one or more genes not listed in Table 8.

Another aspect of the present disclosure provides a plurality of conjugated nucleic acid probes for determining an infectious disease state of a subject, the plurality of conjugated nucleic acid probes including, for each respective gene in a plurality of genes, a respective nucleic acid probe comprising a respective nucleic acid conjugated to a non-nucleic acid detection moiety, where the respective nucleic acid is complementary to the respective gene, and where the plurality of genes comprises one or more genes selected from Table 8, as described in the above section entitled "Additional Biomarkers." In some embodiments, the plurality of genes includes one or more genes not listed in Table 8.

In some embodiments, the plurality of genes comprises from 2 to 25 genes for determining an infectious disease state selected from Table 8, as described in the above section entitled "Additional Biomarkers." In some embodiments, the plurality of genes comprises, from 2 to 25, from 5 to 50, from 10 to 150, from 25 to 500, or from 50 to 1000 genes selected from Table 8, as described in the above section entitled "Additional Biomarkers." In some embodiments, the plurality of genes comprises any number of genes selected from Table 8, as described in the above section entitled "Additional Biomarkers." In some embodiments, the plurality of genes includes one or more genes not listed in Table 8.

*Examples*

EXAMPLE 1 - Example System for Determining Infectious Disease States

HostDx Sepsis or InSep is a rapid (e.g., under 30 minutes), point-of-care (POC) test for use in patients in the continuum of critical care from the emergency room to the intensive care unit and wards as an aid to physicians in determining whether a patient has an acute bacterial infection; whether a patient has an acute viral infection; and the severity of the condition, in accordance with an embodiment of the present disclosure.

This test, which delivers three results, is intended to aid physicians in patient level of care and treatment decisions in conjunction with standard of care. The HostDx Sepsis or InSep product is a system comprising a cartridge (*e.g.*, for single and/or multiple sample testing) and an instrument with embedded software and one or more classification algorithms (*e.g.*, classifiers), which process the data and deliver the three results.

The HostDx Sepsis or InSep test relies on determining the relative abundance of a predetermined set of informative mRNA biomarkers expressed in leukocytes found in patient whole blood. In some instances, the test has a duration of no longer than 30 minutes to complete, including sample preparation and biomarker quantitation. In some such embodiments, shorter durations for testing minimize sample and reagent volume requirements, minimize the size and cost of assay consumables, and rely on common sample collection techniques to simplify process uptake, thus enabling a more efficient, cost-effective workflow in point-of-care and/or hospital environments.

For example, Figure 5 illustrates an example system for determining infectious disease states, including a POC test system comprising a cartridge and an instrument for performing rapid, highmultiplex diagnostics in under 30 minutes. For example, in some embodiments, the system performs measurements *(e.g.,* measuring gene expression, *e.g.,* mRNA abundance) and/or analysis *(e.g.,* determination of an infectious disease state) of one or more targets (*e.g.*, biomarkers), using less than 2 minutes hands-on time and/or less than 30 minutes turnaround time. The example system illustrated in Figure 5 further includes one or more cartridges (*e.g.*, an outpatient and/or hospital cartridge). In some embodiments, the system comprises an outpatient cartridge (*e.g.*, a fingerstick cartridge) that is used to collect the sample from a subject for analysis by the instrument. In some embodiments, the system comprises a hospital cartridge that is used to collect the sample, at a hospital or testing facility, for analysis by the instrument. In some embodiments, a cartridge is used to accept a sample directly from a subject (*e.g.*, without pipetting and/or without an intermediate transfer container). In some embodiments, a cartridge comprises one or more reagents for performing measurements (*e.g.*, measuring gene expression, *e.g.,* mRNA abundance) of one or more targets *(e.g.,* biomarkers). In some embodiments, the one or more targets comprises one or more genes in a plurality of genes *(e.g.,* as listed in Table 1 and Table 2). For example, an embodiment of a cartridge illustrated in Figure 5 performs measurements and/or analysis of between 1 and 70 targets. In some embodiments, a system as illustrated in Figure 5 *(e.g.,* comprising an instrument and one or more cartridges) is provided as a kit. In some embodiments, each cartridge is separately provided as a corresponding kit.

EXAMPLE 2 - Optimizing Biomarker Selection

As described above, qRT-LAMP provides a rapid technology for measuring the relative abundance of biomarkers (*e.g.*, mRNA biomarkers expressed in human leukocytes) that can be used in the diagnosis and prognosis of sepsis and the discrimination between bacterial and viral etiologies. However, limitations in the analytical performance of qRT-LAMP means that certain biomarkers are not amenable for measurement using this technology in point-of-care applications where time and volume limitations impose constraints on the amount of sample material that can be interrogated. We therefore defined the performance characteristics of qRT-LAMP technology and leveraged this data to identify an improved set of biomarkers that can be accurately measured by LAMP and demonstrate comparable and/or improved performance relative to currently available sets of biomarkers.

A critical challenge for methods of determining infectious disease states (*e.g.*, using the InSep application) is the need to measure a high number of informative biomarkers in parallel. Because LAMP technology is difficult and expensive to multiplex, we have chosen an approach of parallelization of large numbers of amplification reactions. This approach generally involves sample material being split many times prior to performing abundance measurements, meaning that the balance between sample input and the sensitivity of amplification assays may be difficult, depending on i) the abundance of informative biomarkers per volume of sample, ii) the amount of sample that can be reasonably processed, and iii) the amount of each biomarker needed to ensure measurements are made within the quantitative dynamic range of the assays. A second key challenge is the precision of the isothermal amplification technology and the ability to discriminate between relatively small effect sizes observed for changes in expression of the selected set of informative biomarkers.

To address these challenges in the context of optimizing biomarker selection, the following approach was taken:

First, the analytical performance characteristics of the isothermal amplification system were defined using homogenous, contrived control material to identify potential areas of concern with respect to the challenges described above.

Second, an empirical analysis of real-world samples was conducted, and the performance of the qRT-LAMP technology was assessed in comparison to a gold standard reference technology.

Third, based on insights gained in analytical performance testing, an analysis of failure modes was performed to identify means of improving agreement between the two technologies (*e.g.*, qRT-LAMP and a reference technology) through selection of biomarkers more amenable to measurement by qRT-LAMP.

Fourth, using constraints defined based on the above performance testing, an optimized set of biomarkers was selected for determination of infectious disease states (*e.g.*, a biomarker test panel for HostDx Sepsis or InSep) that was predicted to improve agreement between measurements made by qRT-LAMP and reference technologies.

*Materials and Methods.*

As used herein, the term "limit of blank" (LOB) is defined as the mean signal observed in an assay containing no analyte plus three times the standard deviation calculated across the population of observations.

As used herein, the term "limit of quantification" (LOQ) is defined as the lowest total amount of analyte input per assay well that will produce a fluorescent signal with threshold time that (a) exhibits precision of < 10% coefficient of variation (CV) and (b) falls within an input range over which the relationship between time to threshold (Tt) and Log10 input is robustly linear.

As used herein, the term "limit of detection" (LOD) is defined as the lowest total amount of analyte input per assay well that will produce a signal that is reliably distinguishable from blank.

As used herein, the term "time to threshold" (Tt) refers to the amount of time increments (*e.g.*, measured in 20 second cycles) required for a LAMP assay to generate enough amplicon to induce sufficient fluorescent signal to cross a pre-defined fluorescence intensity threshold.

As used herein, the term "count" refers to the number of molecules of an informative biomarker identified by the NanoString nCounter SPRINT Profiler instrument.

*Sample Processing by Qiacube (Reference Technology).* We have developed a sample preparation pipeline using a modified version of the commercially available RNeasy Micro total RNA extraction kit executed on the automated QIAcube instrument (Qiagen). Briefly, human whole blood stabilized in a PAXgene blood RNA tube is allowed to reach room temperature, and a 1 mL aliquot is transferred to a processing tube. A 1 mL aliquot of 1x PBS, pH 7.5 is added to the blood sample, and mixed by inversion. The sample is centrifuged at 3000 x g for 10 minutes to pellet precipitated RNA.

Supernatant is discarded and the pellet is resuspended in 2 mL of nuclease-free water. The sample is centrifuged at 3000 x g for 10 minutes, and the supernatant is discarded. The sample is resuspended in 350 $\mu$L of buffer RLT Plus included with the RNeasy kit. The sample is then loaded onto the Qiacube and a modified version of the RNeasy Micro extraction protocol is performed to purify the RNA. The RNA is eluted in 14 $\mu$L of nuclease-free water to maximize final concentration.

*Fluorescent Dye-based RNA Quantitation.* RNA quantitation is performed using the Quant-iT RNA Assay Kit and Qubit 4 Fluorimeter (ThermoFisher). The Quant-iT technology is based on an intercalating fluorescent dye that specifically recognizes RNA and not DNA. The dye is moderately resistant to inhibition by common chemicals and biologics that are carried through a sample preparation process and therefore less prone to error due to confounding signal than UV/Vis spectroscopy. Quantitation is executed per the manufacturer's protocol. As assay master mix is generated by mixing 199 $\mu$L of Quant-iT RNA buffer with 1 $\mu$L of dye solution per sample to be tested. A 1 $\mu$L RNA sample is then diluted into 199 $\mu$L of the Quant-iT assay master mix for measurement, and fluorescent results are read using the RNA High Sensitivity assay setting on the Qubit 4. The instrument is calibrated to each preparation of the Quant-iT assay master mix.

*Analysis by NanoString nCounter SPRINT Profiler (Reference Technology).* At least 150 ng of total RNA isolated from human specimens is combined with a capture and reporter probe cocktail that is designed and supplied by NanoString. Each probe comprises a 50-base pair (bp) segment of the target mRNA biomarker sequence that is specific to that biomarker. These probes are hybridized to target biomarkers by incubation at 65 °C for 16 hours in a proprietary hybridization buffer also supplied by NanoString. After hybridization is complete, samples are incubated at 4 °C. Post hybridization, samples are further diluted with the addition of nuclease-free water per the manufacturer's protocol. Samples are then loaded into a NanoString SPRINT cartridge and placed in the nCounter SPRINT Profiler for analysis. Results are exported by the instrument as RCC files, which are analyzed using the nSolver 4.0 software provided by NanoString. The abundance of each target transcript is reported as "counts." Each count represents a single instance of the instrument identifying a molecular barcode corresponding to a given target biomarker.

*Loop-mediated Isothermal Amplification (LAMP).* Standard LAMP assays, in accordance with some embodiments of the present disclosure, are carried out in 20 $\mu$L reaction volumes in standard 96-well PCR plates. The reaction mixture contains 5x assay buffer {250 mM Tris, pH 8.3, 450 mM KCl, 0.5% Triton X-100}, 8 mM MgSO$_4$, 0.8 M Betaine, 1.4 mM dNTP mix, 4 $\mu$M SYTO9 dye (ThermoFisher), 8 U GspSSD2.0 polymerase (Optigene), and 2 U of WarmStart RTx reverse transcriptase (NEB). Assay primers are added such that FIP and BIP primers are at a final concentration of 1.6 $\mu$M, F3 and B3 primers are at a final concentration of 200 $\mu$M, and rate enhancing primers are at a final concentration of 400 $\mu$M. A 1 $\mu$L sample aliquot is added for each reaction, and nuclease-free water is added to bring the final reaction volume to 20 $\mu$L. Real-time amplification and fluorescent monitoring are carried out on QuantStudio5/6 Real-time PCR instruments (ThermoFisher). Assays are brought to 65 °C and the temperature is maintained throughout the duration of the assay (20-30 minutes for the proposed application). Fluorescent readings are performed every 20 seconds; each 20 second increment is considered a "cycle," although no temperature cycling takes place in the reaction. The time required to reach a predetermined fluorescent threshold is reported in terms of these cycle times, with each 20 second cycle considered 1 "Tt." LAMP technologies are further described above and illustrated in Figure 4.

*In Vitro RNA Transcription (IVT).* IVT reactions are performed using the HiScribe T7 High Yield RNA Synthesis kit (NEB) per the manufacturer's protocol. Reactions are templated with 50 ng of synthetic, double-stranded DNA (dsDNA) obtained commercially (IDT, available online at idtdna.com). Templates contain a T7 promotor sequence at the 5' terminus of the sense strand, followed by 0.5-1.5 kB of sequence to be transcribed, and are provided blunt-ended. Reaction are allowed to proceed at 37 °C between 2-16 hours (overnight) in a forced air shaker/incubator. After transcription, RNA transcripts are purified from residual assay material using the RNA Clean and Concentrator-5 kit (Zymo Research) per the manufacturer's protocol. RNA transcripts are eluted into 50 $\mu$L of nuclease-free water. Transcripts are quantitated using both the Qubit 4 Fluorimeter and UV/Vis spectroscopy.

*Rapid RNA Extraction for Point-of-care Application.* Rapid, centrifugation-free extraction of total RNA from a human whole blood sample stabilized in PAXgene Blood RNA tubes is carried out using the Agencourt RNAdvance Blood Kit (Beckman Coulter) with a modified protocol. A 1.5 mL aliquot of stabilized blood sample is transferred to a 5 mL tube. 50 U of Qiagen Protease is added to the sample, followed by 1.2 mL of Agencourt Lysis reagent. Reagents are mixed by inversion, then incubated at 55 °C for 2 minutes. The sample is removed from heat, then 1875 $\mu$L of Bind 1 (SPRI beads)/Isopropanol solution {75 $\mu$L of Agencourt Bind 1 reagent, 1800 $\mu$L of 100% Isopropanol} is added. Reagents are mixed with the sample by pipetting thoroughly, then incubated for 1 minute at room temperature. A magnet is then applied to collect the SPRI beads, after which the supernatant is removed and discarded. The SPRI beads are resuspended in 800 $\mu$L of Agencourt Wash reagent and mixed by pipetting. A magnetic is applied to collect the SPRI beads and the supernatant is removed. This procedure is repeated for an additional 2 rounds of washing using 70% ethanol in place of the Agencourt Wash reagent. After washing is complete, bound nucleic acid is eluted by resuspending the SPRI beads in nuclease-free water. A magnet is applied to collect the beads and the supernatant containing purified total RNA is removed and retained. Samples are quantitated via Qubit 4 Fluorimeter.

*Reference Technologies.* The NanoString nCounter SPRINT Profiler was selected as a reference technology against which to evaluate the performance of rapid mRNA quantitation by qRT-LAMP. For mRNA expression analysis by the NanoString instrument, total RNA extraction from patient whole blood samples collected in PAXgene Blood RNA tubes is performed using the commercially available RNeasy Micro (Qiagen) extraction kit in a semi-automated protocol executed on the QIAcube instrument (Qiagen). This total RNA extraction system is also considered a reference method for the purposes of point-of-care device development.

*Optimizing Biomarker Selection for Detection by LAMP.*

Even with well-developed analytical performance characteristics, it can be difficult to predict assay performance in the context of a rapid, point-of-care system, and especially in the context of genuine specimens. Predicting performance is further complicated by the fact that the output from patient sample preparation is total RNA, which is a mixture of rRNA, tRNA, and all cellular mRNA transcripts present at unknown abundance. Thus, in some instances, it is difficult to translate the limits of quantitation and blank, and the linear dynamic range determined analytically in terms of copy number per well into total RNA by mass, as the abundance of target RNA transcripts is not constant per mass of total RNA.

Using reference technologies (*e.g.*, as described above), it is possible to estimate the relative number of copies per mass of total RNA. However, because the efficiencies and biases of these technologies differ from those used in point-of-care assay systems, in some instances, absolute quantitation would nevertheless include calibration to quantified control material and reliance on empirical comparison of the two techniques. Rather than developing a complex and error-prone calibration system, we next carried out a direct comparative analysis of the two assay systems using real patient specimens. We then used our knowledge of analytical performance criteria to evaluate results from this study and draw conclusions about means to improve the accuracy of qRT-LAMP measurements relative to the reference technology.

*Accuracy of LAMP Measurements Relative to Reference Technology.*

Reference gene expression data for all patient samples described here was generated using reference technologies described in the Materials and Methods. This data was used as a comparator to assess performance of qRT-LAMP mRNA expression profiling measurements. This analysis was carried out by measuring 32 biomarkers comprising an initial set of biomarkers (*e.g.*, InSep targets) in a cohort of 60 patient samples comprising whole blood collected into PAXgene Blood RNA tubes and representing multiple infection classes - healthy, bacterial, viral, high likelihood of sepsis, and high likelihood of severe infection (*e.g.*, as defined in the InSep diagnostic classifier algorithm).

*Patient Sample Cohort Description and Selection Rationale.*

Samples of whole blood stabilized in PAXgene mRNA Blood tubes were used to evaluate transcriptomic profiles across 29 informative markers and 3 housekeeping genes using the reference technologies described in the Materials and Methods. In an embodiment, samples in the study cohort would be selected to maximize the marker abundance space interrogated by both technologies; in other words, each biomarker would be represented at, minimally, low, medium and high abundance levels in samples to be tested. Although we did not formally evaluate our entire sample bank to optimize for these criteria (as this would be computationally and resource intensive), we attempted to rationally maximize the abundance space covered by selecting samples that generate extreme InSep scores (*e.g.*, very high and very low likelihood of bacterial infection or very high and very low severity of infection) based on application of an early version of the InSep classifier algorithm BVN1 to mRNA expression data generated using reference technologies. A breakdown of sample classifications and the number of samples selected within each classification is shown in Table 3.

Table 3: Sample classifications and approximate numbers of specified samples to be run.

| Patient Sample Classifications | |
| --- | --- |
| Sample Type | Minimum Quantity |
| Healthy Volunteer | 20 |
| Strong Positive Fever Score | 10 |
| Strong Negative Fever Score | 10 |
| High Mortality/Severity Score | 10 |
| High Sepsis Score | 10 |

*Results of Correlation-based Accuracy Analysis.*

Total RNA extraction and mRNA abundance measurements by qRT-LAMP were carried out as described in the Materials and Methods. Briefly, total RNA was extracted from 1.5 mL of a specimen of human whole blood collected in PAXgene Blood RNA tubes per the manufacturer's protocol. Total RNA extraction was accomplished using an SPRI-based RNA isolation protocol. A portion of the total RNA was set aside to replicate microfluidic loss anticipated in a point of care device. A sample of this RNA was used for quantitation by Qubit (ThermoFisher). Purified total RNA was then distributed evenly across qRT-LAMP assay wells. All 32 biomarkers (29 informative markers and 3 housekeeping genes) were measured in triplicate, meaning 96 individual measurements were performed using each total RNA sample. By testing non-normalized sample inputs, we hoped to better understand the distribution of total RNA mass and abundance of individual biomarker mRNA templates that would likely be observed in a point of care scenario.

The accuracy of qRT-LAMP mRNA abundance measurements relative to the gold standard nCounter SPRINT Profiler was assessed by determining the Pearson correlation coefficient between measurements made by each technology on a gene-by-gene basis across all samples from a pre-selected cohort. To compare LAMP measurements in log scale to reference measurements in linear scale, reference results were Log10 transformed. For both technologies, measurements made for informative biomarkers were normalized to the geometric mean of measurements made for the housekeeping genes KPNA6, RREB1 and YWHAB to correct for differences in total RNA input. Correlation coefficients were then determined for each informative biomarker across all samples in the cohort.

As provided in Table 4, Pearson coefficients determined for the 32 markers ranged from 0.04 to 0.92, with a median correlation coefficient of 0.615 and mean correlation coefficient +/-StdDev of 0.588 +/- 0.243. We interpreted the distribution of performance to be indicative of systemic differences between qRT-LAMP and nCounter measurements. We hypothesized that correlation of the assay measurements may be related to characteristics of the markers coupled with limitations in qRT-LAMP precision. We next investigated potential correlations between marker performance, analytical performance characteristics of qRT-LAMP and characteristics of the biomarkers being evaluated.

Table 4: Pearson correlation coefficients determined between qRT-LAMP and nCounter measurements made for 32 informative biomarkers measured in 60 whole blood samples.

| Gene-by-Gene Correlation Analysis Between LAMP and nCounter | | | | | | | |
|---|---|---|---|---|---|---|---|
| Marker | Pearson R | Marker | Pearson R | Marker | Pearson R | Marker | Pearson R |
| BATF | 0.36 | GNA15 | 0.63 | KIAA1370 | 0.73 | RPGRIP1 | 0.33 |
| C11orf74 | 0.34 | GPAA1 | 0.45 | KPNA6 | 0.43 | RREB1 | 0.47 |
| C3AR1 | 0.50 | HIF1A | 0.86 | LAX1 | 0.85 | SEPP1 | 0.28 |
| CD163 | 0.86 | HK3 | 0.92 | LY86 | 0.86 | TGFBI | 0.80 |
| CEACAM1 | 0.85 | HLA-DPB1 | 0.74 | MTCH1 | 0.02 | TNIP1 | 0.52 |
| CIT | 0.51 | IFI27 | 0.90 | NMRK1 | 0.58 | TST | 0.04 |
| CTSB | 0.71 | JUP | 0.63 | PER1 | 0.64 | YWHAB | 0.29 |
| DEFA4 | 0.74 | KCNJ2 | 0.83 | RGS1 | 0.53 | ZDHHC19 | 0.60 |

*Defining Biomarker Selection Criteria.*

*Marker Abundance*

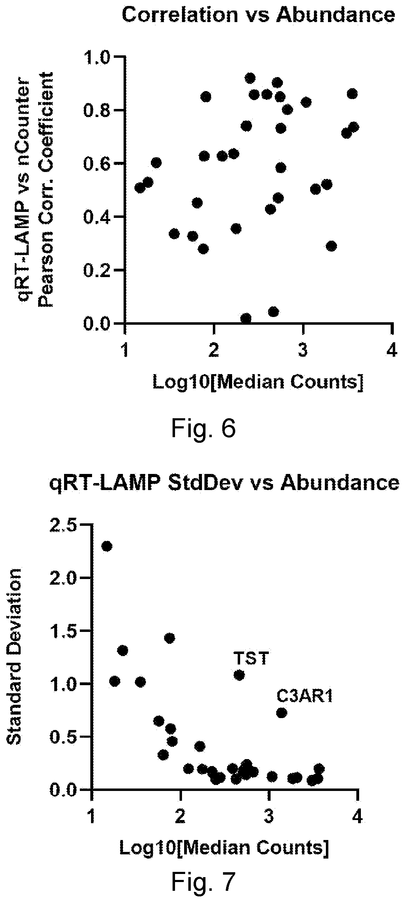

Analytical performance analyses showed that the precision of qRT-LAMP measurements is related to the initial abundance of the template being measured by the assay. LAMP assays demonstrate a limit of quantitation between $10^2$ and $10^4$ copies per well in input titration experiments, with measurements made for mRNA template input levels below LOQ demonstrating significantly increased variability and therefore lower assay resolution. We therefore hypothesized that one rationale for poor correlation observed with certain biomarkers may be a result of LAMP measurements occurring below the LOQ for these biomarkers. We therefore evaluated the correlation between template abundance as measured by the reference technology and the performance of each biomarker, using the Pearson R as our performance metric. Figure 6 is a plot describing the relationship between the accuracy of qRT-LAMP measurements as assessed by correlation to measurements made on the NanoString nCounter SPRINT Profiler and the median abundance of each biomarker across all samples within the study cohort as determined using the nCounter. The Pearson correlation between assay performance and marker abundance was determined to be R = 0.24, indicating a weak relationship between these metrics. We therefore determined that template abundance was not the key driver of concordance between LAMP and nCounter measurements in this experiment.

We also looked to this data as a means of calibrating qRT-LAMP LOQs to template abundance as measured by the reference technology. Analytical performance analyses showed that variance of all assay increases dramatically near the LOQ, therefore, we evaluated the relationship between variance in qRT-LAMP measurements and marker abundance measured by the nCounter SPRINT Profiler. Figure 7 is a plot describing the relationship between the precision of qRT-LAMP measurements as assessed by determining the standard deviation across n=3 technical replicates and the median abundance of each biomarker across all samples within the study cohort as determined using the nCounter. We found that biomarkers with median abundance levels below $10^2$ copies per 150 ng total RNA load as measured by the reference technology show significantly higher levels of variability in qRT-LAMP measurements, suggesting that $10^2$ counts per 150 ng as determined by the reference technology may equate to $10^2$-$10^3$ cpw as assessed in IVT experiments. We therefore also determined that optimizing marker selection or sample input to ensure marker abundance > 100 copies per 150 ng RNA input by nCounter for 95% of samples will likely improve measurement precision and by extension accuracy relative to the reference technology.

*Marker Dynamic Range*

We next tested whether the dynamic range of marker abundance was related to assay performance. In some instances, the need for an assay to have sufficient dynamic range to be measured accurately is related to the resolution of the assay in question over the RNA input range being tested. For example, if the dynamic range of marker abundance in our selected sample cohort is low (< 10-fold change across all samples), and that marker is being measured near LOQ, qRT-LAMP measurements are unlikely to be sufficiently precise to resolve differences across samples.

To test this hypothesis, we evaluated the relationship between biomarker dynamic range and assay performance. We defined the dynamic range of a biomarker as the fold difference between the 95th and 5th percentiles of counts for a given marker as measured across all samples in the cohort by the reference technology. Figure 8 illustrates a plot describing the relationship between the accuracy of qRT-LAMP measurements as assessed by correlation to measurements made on the NanoString nCounter SPRINT Profiler and the dynamic range of RNA template input copy number observed across samples within the study cohort as determined by nCounter SPRINT. The dynamic range was determined by calculating the ratio between the 90th and 10th percentile values for transcript abundance across all samples for each biomarker. Although we did not observe a robust relationship between these metrics (*e.g.*, linear or otherwise), it is clear that markers with lower performance also tend to be those with lower dynamic range; indeed, the 7 markers with a measured correlation of LAMP to nCounter < 0.40 are all markers that exhibit a < 10-fold dynamic range in measurements made by the nCounter SPRINT Profiler. This suggests that, in addition to selecting markers of higher abundance, maximizing marker dynamic range should improve agreement between LAMP and nCounter measurements.

*Setting Constraints for Alternative Biomarker Selection*

The relationships observed between marker performance (*e.g.*, correlation between qRT-LAMP and reference technology measurements) and marker abundance or dynamic range as measured by the reference technology are unfortunately not robust; therefore, no obvious thresholds presented themselves in terms of ensuring high accuracy of qRT-LAMP measurements. Data strongly suggested that measurements made on markers with median abundance < 100 copies per 150 ng will show a marked increase in variance, although two outliers with higher variance at higher abundance were observed. To maximize the likelihood that measurements will fall within the linear dynamic range and exhibit low variance, we therefore set a criterion of 100 < median counts observed per 150 ng of total RNA input across all samples tested by NanoString nCounter SPRINT Profiler.

To set a threshold for marker dynamic range, we took a combined approach of (a) searching the empirical data for a meaningful cutoff, and (b) estimating expected assay resolution based on variability observed for technical replicates in this cohort. To achieve (a), we sorted biomarkers based on median abundance and searched for a point below which the accuracy metric did not meet a desired value. We found that below a dynamic range of 4-fold, no markers achieved a correlation of R > 0.75. Further, we calculated the mean variance (*e.g.*, standard deviation) across all measurements made for each and used this value to estimate the mean resolution across all qRT-LAMP assays. The values from which these calculations were performed can be found in Table 5. Given the mean observed variance of 0.45 Tt, we calculated a 95% confidence interval of ± 0.88 Tt, which implies a range of 1.76 Tt for each measurement. Applying this to our calculated fold-change per amplicon cycle, we found a mean resolution of about 4.6 across all assays. We therefore set our second criterion for marker selection as 4-fold < the fold difference between the 95th and 5th percentiles of counts across all samples tested to date by NanoString nCounter SPRINT Profiler.

Table 5: The median abundance across all samples for each biomarker as measured by the reference technology was calculated and listed under "Median Abundance." All LAMP measurements were performed in triplicate and the standard deviation across triplicate measurements was calculated for each marker for each sample. The mean of standard deviations across all samples was calculated for each marker and is listed under "Mean Variance Across All Samples." Slopes of linear fit models determined in linearity studies performed as part of analytical performance characterization are listed under "Slope." From the slope (efficiency) of each assay, the fold-change in amplicon copy number per LAMP measurement cycle (e.g., each Tt) was calculated and is listed under "Fold-change in Amplicon per LAMP Cycle."

| qRT-LAMP Assay Performance Criteria for Resolution Requirements | | | | |
|---|---|---|---|---|
| Marker | Median Abundance | Mean Variance Across All Measurements | Slope | Fold-change in Amplicon per LAMP Cycle |
| CIT | 1.17 | 2.30 | -3.34 | 1.99 |
| RGS1 | 1.26 | 1.03 | -2.46 | 2.55 |
| ZDHHC19 | 1.35 | 1.31 | -2.03 | 3.11 |
| C11 orf74 | 1.55 | 1.02 | -2.15 | 2.92 |
| RPGRIP1 | 1.76 | 0.65 | -3.27 | 2.02 |
| HIF1A | 1.81 | 0.33 | -3.17 | 2.07 |
| SEPP1 | 1.88 | 1.43 | -2.52 | 2.49 |
| KCNJ2 | 1.89 | 0.58 | -5.87 | 1.48 |
| LAX1 | 1.91 | 0.46 | -2.75 | 2.31 |
| GPAA1 | 2.09 | 0.20 | -3.25 | 2.03 |
| PER1 | 2.22 | 0.41 | -2.65 | 2.38 |
| BATF | 2.25 | 0.19 | -2.37 | 2.64 |
| MTCH1 | 2.36 | 0.17 | -2.55 | 2.47 |
| DEFA4 | 2.36 | 0.16 | -3.20 | 2.05 |
| HLA-DPB1 | 2.40 | 0.10 | -2.83 | 2.25 |
| CD163 | 2.45 | 0.11 | -2.44 | 2.57 |
| LY86 | 2.59 | 0.20 | -2.14 | 2.93 |
| KPNA6 | 2.63 | 0.10 | -2.95 | 2.18 |
| TST | 2.67 | 1.08 | -2.57 | 2.45 |
| JUP | 2.71 | 0.15 | -2.63 | 2.40 |
| RREB1 | 2.72 | 0.19 | -3.03 | 2.14 |
| CEACAM1 | 2.74 | 0.14 | -2.58 | 2.44 |
| GNA15 | 2.75 | 0.24 | -3.28 | 2.02 |
| NMRK1 | 2.75 | 0.21 | -3.75 | 1.85 |
| TGFBI | 2.82 | 0.17 | -3.46 | 1.95 |
| KIAA1370 | 3.03 | 0.12 | -2.48 | 2.53 |
| C3AR1 | 3.14 | 0.73 | -2.41 | 2.60 |
| TNIP1 | 3.27 | 0.10 | -2.96 | 2.18 |
| YWHAB | 3.32 | 0.11 | -2.61 | 2.42 |
| CTSB | 3.49 | 0.09 | -2.55 | 2.47 |
| HK3 | 3.55 | 0.10 | -2.24 | 2.80 |
| IFI27 | 3.57 | 0.20 | -2.59 | 2.43 |

(continued)

| qRT-LAMP Assay Performance Criteria for Resolution Requirements | | | | |
|---|---|---|---|---|
| Marker | Median Abundance | Mean Variance Across All Measurements | Slope | Fold-change in Amplicon per LAMP Cycle |
| Mean | 2.45 | 0.45 | -2.85 | 2.35 |

EXAMPLE 3 - Identifying Alternative Biomarker Sets

*Down-selecting Biomarkers*

To identify alternative marker sets, counts for all markers as measured by the reference technologies across samples prospectively collected or commercially obtained were curated for samples evaluated using a single NanoString nCounter SPRINT Profiler capture and reporter code set designated CS3. For each biomarker, the median, 5th, and 95th percentiles of abundance were calculated, and from these data the dynamic range of abundance for each biomarker was also calculated (counts at 95th percentile divided by counts at 5th percentile). These results were evaluated against selection criteria determined from empirical analyses of qRT-LAMP assay performance.

To be measured accurately and quantitatively across different cohorts, the biomarkers were constrained to also exhibit a minimum 4-fold dynamic range as measured across all samples. To ensure the markers of selection meet both constraints, markers with less than 400 copies (minimum 100 copies*4 fold-change) at 95th percentiles were first excluded to ensure sufficient abundance that can be detected by RT-LAMP in different cohorts. Next, markers with lower than 4-fold dynamic change between the 95th and 5th percentiles were further excluded to minimize the number of markers with limited resolution. From this 2-step exclusion selection method, 27 alternative markers were identified. 19 out of these 27 candidates with five-fold or high dynamic change were ranked as Tier 1, while the remaining 8, with dynamic change lower than five-fold, were ranked as Tier 2. Subsequently, two markers (CD24 and SUCLG2) failed gDNA screening and were removed. This process resulted in the final list of 25 Tier 1 and Tier 2 candidate markers.

The original set of 29 markers (*e.g.*, described above in Example 2) were evaluated using the same criteria. 23 markers with both 95th percentile > 400 copies and 95th/5th fold change > 4 were identified and combined with the 25 alternative markers to generate a 48-candidate pool for down-selection. The 48 candidate genes (*e.g.*, biomarkers) are provided herein as Table 1 (above).

*Selecting Optimized Alternative Marker Set using Machine Learning*

We applied machine learning to identify 29 markers for use in determining infectious disease states (*e.g.,* on the InSep cartridge). The process used the pool of 48 markers which satisfied the assay-based criteria described above and produced a final list of 29 markers estimated to provide optimized clinical diagnostic performance for determining infectious disease states (*e.g.*, in the InSep classifiers).

The selection of 29 markers proceeded in two phases. In Phase I, we used a forward selection method, a logistic regression (LOGR) model and random hyperparameter search to choose an initial set of markers. In Phase II, we used a forward selection method, a multi-layer perceptron model, a Bayesian Hyperparameter Optimization and expert judgement to choose additional markers for a total of 29. The rationale for this approach and the descriptions of individual steps within the 3 phases are provided in greater detail below.

We used logistic regression in Phase I due to competitive performance on our datasets, and low computational complexity (fast training) of LOGR. We reasoned that the initial set of genes will comprise genes with relatively strong signal, and therefore be detectable by a generic competitive machine learning algorithm. LOGR was selected based on a balanced trade-off between accuracy and complexity. We further reasoned that tuning the set of markers to the target size of 29 would comprise using a highly accurate classifier because the signal from the additional markers is gradually weakening. To that end, Phase II used forward selection with a multi-layer perceptron classifier, which has to date yielded highly accurate models for classification of infections using host response data, and therefore was most likely to uncover the additional informative markers. Phase II involved human input because the weaker signal of the final 10 markers was validated by additional evaluation of multiple target metrics. Generally, simultaneous assessment of multiple metrics is not amenable to automation using generic computer optimization algorithms because they require a single loss (criterion) function.

Phase I used the following variant of the forward-selection algorithm:

Input: empty marker set M and full set of candidate markers F

(continued)

| |
|---|
| • Repeat for remaining markers (*e.g.*, markers in F \ M) |
| o Add one marker |
| o Repeat for 100 logistic regression hyperconfigurations |
| ▪ Repeat over 100 splits of 5-fold random CV |
| • For each fold |
| o Train LOGR on the training set, compute probabilities for the validation set |
| • Pool validation set probabilities, calculate AUC |
| ▪ Average AUC over 100 splits |
| o Calculate 95-th percentile of AUCs (over 100 hyperconfigurations) |
| o Stop if no improvement to AUC is achieved by adding new markers |
| o Add marker with best 95-th percentile of AUCs to M |
| Output: minimal list M of markers which maximize AUC |

Phase II used the following variant of the forward-selection algorithm, with human input:

| |
|---|
| Input: marker set M and full set of candidate markers F |
| • Repeat for remaining markers (*e.g.*, markers in R = F \ M) |
| o For each marker in R |
| ▪ Add one marker to the training and validation sets |
| ▪ Select best MLP model using training set and cross-validation |
| ▪ Apply the model to the validation set |
| ▪ Record training (cross-validation) AUC and validation AUC - these statistics are a function of the marker |
| o Select best marker in R (human input) and move it to M |
| o Stop if M has 29 markers |
| Output: minimal list of markers which achieve best tradeoff of diagnostic performance and robustness |

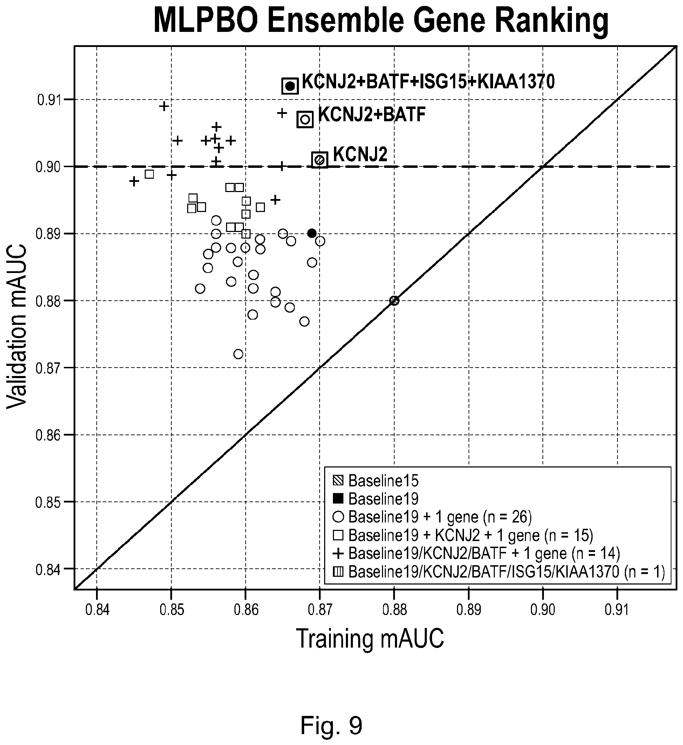

Phase I yielded 19 genes. Phase II yielded an additional 10 genes, for a total of 29 genes (e.g., biomarkers), provided herein as Table 2 (above). An intermediate step in Phase II is illustrated in Figure 9. Figure 9 illustrates an intermediate snapshot of Phase II of marker selection by machine learning. The x-axis is the cross-validation AUC for best model found by Bayesian Hyperparameter Optimization using features comprising current marker set plus one marker at a time. The y-axis is the AUC of that model applied to validation set. For example, the blue dots represent training and validation AUCs for feature sets consisting of the 19 markers found in Phase I, plus one of the markers in the remaining set of markers. With expert input, KCNJ2 was added to current marker set and the process repeated for the remaining set of markers (e.g., "KCNJ2+" markers shown in boxes). Other additions to marker sets, based on expert input, are indicated by their respective groupings (e.g., KCNJ2/BATF and/or KCNJ2/BATF/ISG15/KIAA1370).

The diagnostic performance metrics of a neural network classifier developed using the markers listed in Table 2 are shown below in Table 6. Notably, the replacement of the initial set of original 29 markers (e.g., as described above in Example 2) with markers swapped using the methods described in this Example (above) did not decrease the overall predictive performance of the bacterial/viral/noninfected classifier *(e.g.,* the InSep classifier), as judged by a combination of the clinically relevant metrics.

Table 6: Clinical diagnostic performance metrics for the swapped 29 markers. The classifier used was an ensemble of multi-layer perceptron models, selected based on a balanced trade-off between mAUROC in training data (cross-validation) and validation.

| Metric | Training (cross-validation) | Validation |
|---|---|---|
| mAUROC | 0.867 | 0.889 |
| Bacterial LR- | 0.075 | 0.044 |
| Bacterial fraction 1 [%] | 18.2 | 14.9 |
| Bacterial band 1 sensitivity [%] | 98.1 | 98.3 |
| Bacterial LR+ | 7.5 | 14 |
| Bacterial fraction 4 [%] | 24.3 | 40.8 |
| Bacterial band 4 specificity [%] | 92.2 | 95.6 |
| Viral LR- | 0.074 | 0.071 |
| Viral fraction 1 [%] | 25.0 | 33.4 |
| Viral band 1 sensitivity [%] | 97.3 | 96.6 |
| Viral LR+ | 10 | 16 |
| Viral fraction 4 [%] | 28.6 | 22.5 |
| Viral band 4 specificity [%] | 92.8 | 96.1 |

*Summary of Results.*

In accordance with the methods and results described above in Examples 2 and 3, in some embodiments, qRT-LAMP assays can be designed to be highly selective against primer-dimer or intraassay amplification, and against amplification of genomic DNA (gDNA). Additionally, qRT-LAMP assays exhibit a log-linear relationship between the number of target nucleic acid copies present at reaction initiation and the time required to achieve generation of a predetermined quantity of amplicons as assessed by measuring the signal generated by an intercalating fluorescent dye. However, this relationship breaks down, in some cases, at template input levels near or below the limit of quantitation for a given assay. For example, limits of quantitation fall between $10^2$ and $10^3$ copies for most qRT-LAMP assays tested here. Notably, this is somewhat higher than observed for qRT-PCR and imposes a more stringent constraint on sample input requirements for these assays.

As shown herein, in some embodiments, qRT-LAMP assay precision is relatively constant within the linear dynamic range of the assay but increases near the limit of quantitation. For example, qRT-LAMP assays exhibit characteristic efficiencies, which are inversely related to the resolution of the assay; error introduced in the measurement process or from instrumentation will be more impactful for assays with high efficiency. In some instances, resolution limitations of qRT-LAMP assays may be as low or as high as two-fold for input levels well within the linear dynamic range of a moderately efficient assay but fall off dramatically as imprecision and assay efficiency increase. Thus, the accuracy of qRT-LAMP measurements relative to reference technologies varies widely across informative biomarkers when measured in a cohort of patient samples.

For example, in some implementations, biomarkers of very low abundance (*e.g.*, less than 100 copies per 150 ng of total RNA as assessed by the reference technologies) typically fall near or below the limit of quantitation for qRT-LAMP assays measuring total RNA after rapid sample preparation *(e.g.,* for 500 μL stabilized whole blood per 32 individual biomarker measurements). In some instances, a key feature in predicting likely agreement between technologies is the dynamic range of biomarker abundance (*e.g.*, the fold-change between the highest and lowest expression levels of the biomarker) across a given cohort. For example, in some instances, based on observed technical precision of qRT-LAMP assays when measuring patient samples, in conjunction with their measured efficiencies, most biomarkers with < 4-fold dynamic range will not be resolvable by LAMP.

Based on the above constraints determined by evaluating performance in patient samples, a subset of biomarkers likely to be amenable to measurement by qRT-LAMP was selected for a rapid workflow using 500 μL of stabilized whole blood. Subsequent machine learning-based down-selection of qRT-LAMP favorable biomarkers was used to identify an optimized set of biomarkers (*e.g.*, as listed in Table 1 and Table 2) with clinical performance comparable to the original set of markers.

EXAMPLE 4 - Performance Measures for Methods of Determining Infectious Disease States

*Performance Measures using mAUC.*

A classification model was obtained in accordance with the systems and methods provided herein and assayed for comparative performance against a plurality of existing state-of-the-art classifiers, including commercial classifiers, in the field of diagnosing infections. Existing classifiers used for performance comparisons included H2O Driverless AI, DataRobot, Gaussian Process Classifiers, AutoGluon, Hyperband Random Cross-Validation (CV), Hyperband Grouped CV, Random Search, logistic regression (LOGR), XGBoost, Radial Basis Function (RBF) Network, Light Gradient Boosting Machine (LGBM), Support Vector Machine (SVM) and Bayesian Hyperparameter Optimization, among others. The results of performance for each model were evaluated using the validation mAUC (mean area under curve) and are presented in Table 7 (ND: no data; NA: not applicable, *e.g.*, where respective method does not compute metric).

Table 7: Performance Comparison

| Classifier Method | Training mAUC | Validation mAUC |
|---|---|---|
| Hyperband Grouped CV | 0.867 | 0.872 |
| AutoGluon | ND | ND |
| Gaussian Process Classifier | NA | 0.863 |
| Hyperband Random CV | 0.964 | 0.860 |
| DataRobot | 0.820 | 0.845 |
| LOGR | 0.816 | 0.875 |
| XGBoost | 0.815 | 0.852 |
| RBF | 0.810 | 0.815 |
| LGBM | 0.806 | 0.832 |
| H20 Driverless AI | 0.805 | 0.853 |
| SVM | 0.789 | 0.853 |

*Performance Measures using Bin Measures.*

In some embodiments, a classifier for determining infectious disease states, such as the HostDx Sepsis test, generates class probabilities for bacterial, viral and non-infected classes, in accordance with an embodiment of the present disclosure. In some embodiments, the classifier generates a severity score. The following describes example implementations for measuring performance of the former type of classifier, which generates the three probabilities (bacterial, viral and non-infected). In some such embodiments, the test assigns each sample to one of four bacterial bins, using bacterial probability, and one of four viral bins, using viral probability. For most of this discussion we shall focus on the bacterial bins. The viral bins can be analyzed equivalently. To simplify discussion, when convenient we shall also refer to bacterial samples as Positive (POS), and viral+non-infected as Negative (NEG). Also assume total number of samples equals N.

The bacterial bins are labeled B1, B2, B3 and B4. B1 is the "low" bin and B4 is the "high" bin. The bins are defined by thresholds BT1, BT2 and BT3 (in this section, these are considered to be given numbers in [0, 1]; for derivation of the thresholds, see the "Optimizing Thresholds" section, below). Samples whose bacterial probability is < BT1 are assigned to B1. Samples whose bacterial probability is in [BT1, BT2) are assigned to B2. Samples whose bacterial probability is in [BT2, BT3] are assigned to B3. The remaining samples, whose bacterial probability is > BT3, are assigned to B4. Intuitively, the classifier assigns samples it deems unlikely to be bacterial to B 1; and it assigns samples it deems likely to be bacterial to B4. The remaining samples are in essence deemed "indeterminate" as far as the classifier is concerned.

In some instances, a suitable classifier would assign all NEG samples to B1, and all POS samples to B4. The bin measure is designed to quantify how close we are to this paradigm. Thus, if all POS samples are assigned by the classifier to B4, and all NEG samples to B1, the measure should be equal to 1; conversely, if all POS samples are assigned to B1, and all NEG samples to B4, the measure should equal 0.

A measure which satisfies these conditions can be formulated as follows:

- count how many NEG samples are assigned to B1 (b 1_neg). Equivalently, these are persons not having a disease (bacterial infection) testing negative. Should be large, ideally #NEG
  -

$$P1 = b1\_neg/\#NEG$$

- count how many POS samples are assigned to B4 (b4_pos). Equivalently, these are persons having a disease testing positive. Should be large, ideally #POS
-

$$P2 = b4\_pos/\#POS$$

-

$$bacterial\_bm = (P1+P2)/2$$

This is the BM for bacterial score. Equivalently, one may calculate the viral_bm, for viral score. Both bacterial and viral BM are independently useful. For a summary measure, one may consider the overall BM, defined as the mean of the two: bm = (bacterial_bm+viral _bm)/2

*Likelihoods*

This section defines how to calculate likelihood ratios (abbreviated: likelihoods). Each bin has an associated likelihood. Likelihood for B1 is called "negative likelihood ratio" (LR-) and likelihood for B4 is called "positive likelihood ratio" (LR+). We use the formulation: "the probability of a person who has the disease testing negative divided by the probability of a person who does not have the disease testing negative." This formulation uses the same probabilities already used in the definition of the BM measure above. In some instances, other formulations for likelihoods are based on sensitivity and specificity.

Given this formulation, and given the bin thresholds BT1, BT3, the LR- computation is:

- count POS samples assigned to B1. The count is b1_pos

-

$$P1 = b1\_pos/\#POS$$

- count NEG samples assigned to B1. The count is b1_neg

-

$$P2 = b1\_neg/\#NEG$$

-

$$LR- = P1/P2$$

LR+ computation is based on "the probability of a person who has the disease testing positive divided by the probability of a person who does not have the disease testing positive":

- count POS samples assigned to B4. The count is b4_pos

-

$$P1 = b4\_pos/\#POS$$

- count NEG samples assigned to B4. The count is b4_neg

-

$$P2 = b4\_neg/\#NEG$$

-

$$LR+ = P1/P2$$

This way we can compute LR- and LR+ given the thresholds BT1, BT3. Per expert guidance, in some instances, LR- is < 0.05, and LR+ is > 10.

*Three-class Sensitivity and Specificity*

Besides likelihood ratios, the sensitivity and specificity for 3-class situation are also sometimes of interest. Sensitivity and specificity can be described as follows:

Considering bacterial bin 1 sensitivity first, we use bacterial probability and bin 1 threshold to assign samples into POS1 class and NEG1 class (the suffix 1 indicates bin 1). A sample is assigned to POS1 if the bacterial probability is less than the bin 1 threshold. The POS1 class in this context is "non-bacterial" (because we are analyzing bacterial bin 1, so being "positive" for this bin means non-bacterial). The NEG1 is bacterial. Therefore, to form truth vector, we assign POS1 truth to non-bacterial and NEG1 to bacterial. Assume the total number of actual POS1 (non-bacterial) is #POS 1 and assume the number of non-bacterial assigned to bin 1 is s1. Then bacterial bin 1 sensitivity is s1/#POS1.

For bacterial bin 4, we calculate specificity. Again, we use bacterial probability and bin 4 threshold to assign samples into POS4 and NEG4 class. A sample is assigned to POS4 if bacterial probability is greater than the bin 4 threshold. POS4 in this context is bacterial, and NEG4 is non-bacterial, so the truth corresponds to "real" truth, meaning POS4 truth is bacterial, and NEG4 truth is non-bacterial. Assume the number of actual NEG4 samples is #NEG4 and assume the number of NEG4 samples assigned to NEG4 is s4. Then the bacterial bin 4 specificity is s4/#NEG4.

*Optimizing Thresholds*

The previous sections assume that the thresholds are given. This section defines how to calculate optimal thresholds given the truths and the predicted probabilities. Typically, the thresholds are determined by analyzing the pooled cross-validation probabilities of the training data. They are then locked and the classifier, along with the thresholds, applied to the test data.

The threshold optimization is based on likelihoods. In short, we seek to create bins B1 and B4 which are as large as possible, while keeping the likelihoods within given bounds (defined by the domain experts). The reason is that bins B1 and B4 are clinically actionable, because they tell the physician she can be fairly confident about bacterial infection or lack thereof.

Per expert guidance, LR- is < 0.05, and LR+ is > 10.

The thresholds are optimized as follows:

- sort probabilities. Set threshold to midpoints between probabilities, one midpoint at a time, and compute LR- and LR+ for each threshold.

- for BT1:

- remove all thresholds for which LR- >= 0.05

- among remaining thresholds, select the greatest one. This is BT1.

- for BT3:

- remove all thresholds for which LR+ <= 10

- among remaining thresholds, select the smallest one. This is BT3.

Once we have the optimal BT1 and BT3, we can compute bacterial_bm, viral_bm, b1_neg, b4_pos and bm for any set of probabilities, using the procedure in section "Bin measure."

*Performance Measures using bm_ftaction1, bm_fraction4*

In some instances, bm_fraction1 and bm_fraction4 are more useful, and in particular closer to HostDx Sepsis test customer requirements, than the BM. The measures are defined for each class (bacterial, viral and non-infected). For simplicity, we discuss the bacterial bm_fraction1 and bm_fraction4.

$$\text{bm\_fraction1} = (\text{b1\_neg} + \text{b1\_pos})/(\#NEG + \#POS)$$

$$\text{bm\_fraction4} = (\text{b4\_neg} + \text{b4\_pos})/(\#NEG + \#POS)$$

In words, bm_fraction1 is the proportion of all samples (POS and NEG) assigned to B1. bm_fraction4 is the proportion of all samples assigned to B4. bm_fraction1+bm_fraction4 is the proportion of all samples assigned to B1 or B4. This is a statistic which can be referred to such that the bacterial result shall have the following criteria: lowest band shall have a Likelihood Ratio of <1; highest band shall have a Likelihood Ratio of >5; and at least 50% of results will fall into either the lowest or the highest band. The condition that "at least 50% of results will fall into either the lowest or the highest band" means that bm_fraction1+bm_fraction4 for bacterial score shall be at least 50%. In some instances, similar requirements will apply to B1 and B4 for the viral score.

EXAMPLE 5 - Other Biomarker Sets

Classification models with different biomarker sets of the systems and methods provided herein were assayed for comparative performance. In this example, classification models comprising 2, 3, 4, and 5 gene combinations of LY6E, IRF9, ITGAM, and PSTPIP2 were assayed for diagnostic power (e.g., area under the curve (AUC)) in distinguishing bacterial infections, viral infections, and non-infected subjects in 38 datasets comprising 2976 samples. Logistic regression models were evaluated using a 75/25 train/test split, where each model was trained using 75% of the samples and then AUC was calculated for the predicted probabilities of the remaining 25% of the samples. The AUCs for 11 different classification models comprising 2, 3, or 4 gene combinations of LY6E, IRF9, ITGAM, and PSTPIP2 are shown in Table 10. All of the classification models of Table 10 have AUCs greater than 0.65 and a majority of the models have AUCs greater than 0.7.

Table 10: Performance of 2, 3, and 4 gene classification models

| Gene 1 | Gene 2 | Gene 3 | Gene 4 | AUC |
|--------|--------|--------|--------|-----|
| PSTPIP2 | IRF9 | - | - | 0.69547 |
| PSTPIP2 | LY6E | - | - | 0.751496 |
| PSTPIP2 | ITGAM | - | - | 0.676343 |
| IRF9 | LY6E | - | - | 0.762419 |
| IRF9 | ITGAM | - | - | 0.742915 |
| LY6E | ITGAM | - | - | 0.756273 |
| PSTPIP2 | IRF9 | LY6E | - | 0.746785 |
| PSTPIP2 | IRF9 | ITGAM | - | 0.746785 |
| PSTPIP2 | LY6E | ITGAM | - | 0.770034 |
| IRF9 | LY6E | ITGAM | - | 0.787901 |
| PSTPIP2 | IRF9 | LY6E | ITGAM | 0.792424 |

As provided in the systems and methods herein, the classification models provided in Table 10 can comprise one or more optional genes. For example, one additional gene selected from one or more of Tables 1, 2, 8, or 9 can be included in the classification model. To understand how the addition of another gene affects diagnostic power, the AUCs were calculated for exemplary models. For each classification model in Table 10 (e.g., 2, 3, and 4-gene model), 1000 augmented models were created by adding one random gene. That is, each 2-gene model became 1000 3-gene models, each 3-gene model became 1000 4-gene models, and the 4-gene model became 1000 5-gene models. Figures 12A-12K illustrate the range of AUCs obtained for the augmented models in the same dataset. As shown in Figures 12A-12K, the addition of one gene generally increases the AUC relative to the base case (e.g., without the additional gene) as shown by the bars to the right of the base AUC (dashed line) in each plot. In few instances, the addition of one gene could decrease the AUC relative to the base as shown by the bars to the left of the base AUC in each plot.

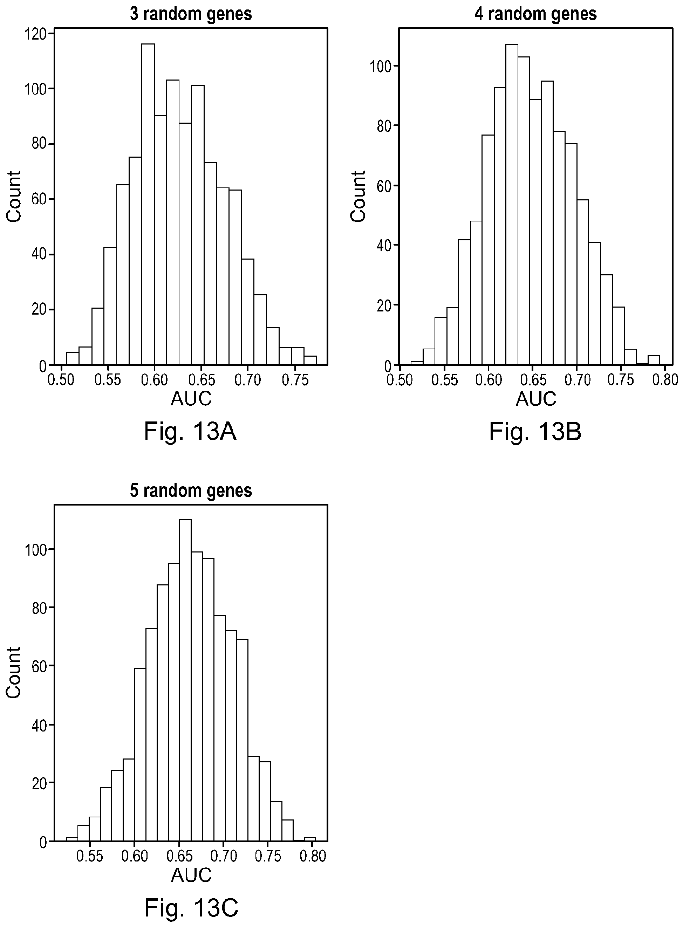

To evaluate the relative performance of these classification models, the AUCs were calculated for 1000 random 3 gene models, 1000 random 4 gene models, and 1000 random 5 gene models. Figures 13A-13C illustrates the ranges of AUC obtained for these 3, 4, and 5 gene classification models with random selections of genes.

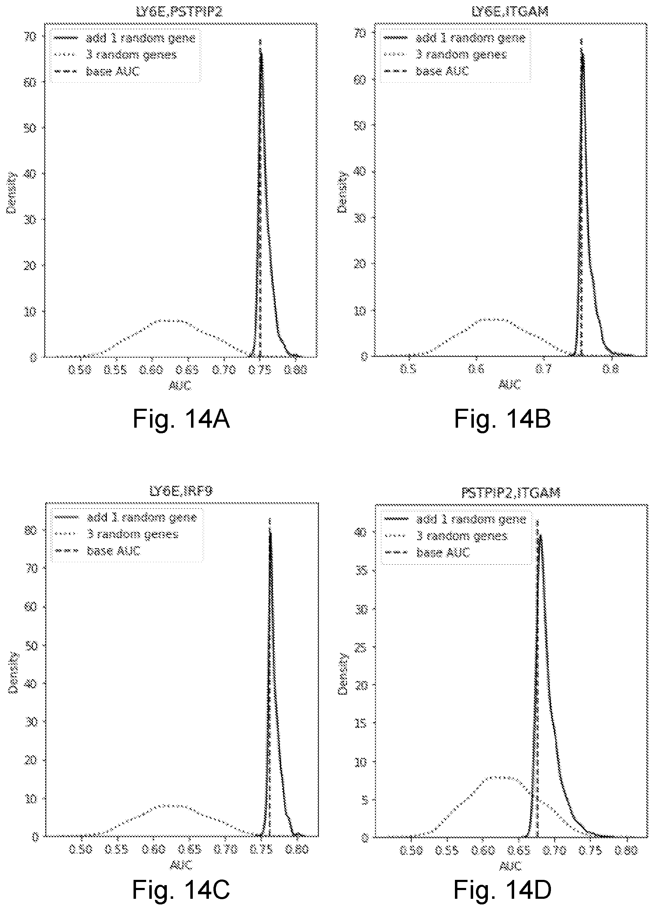

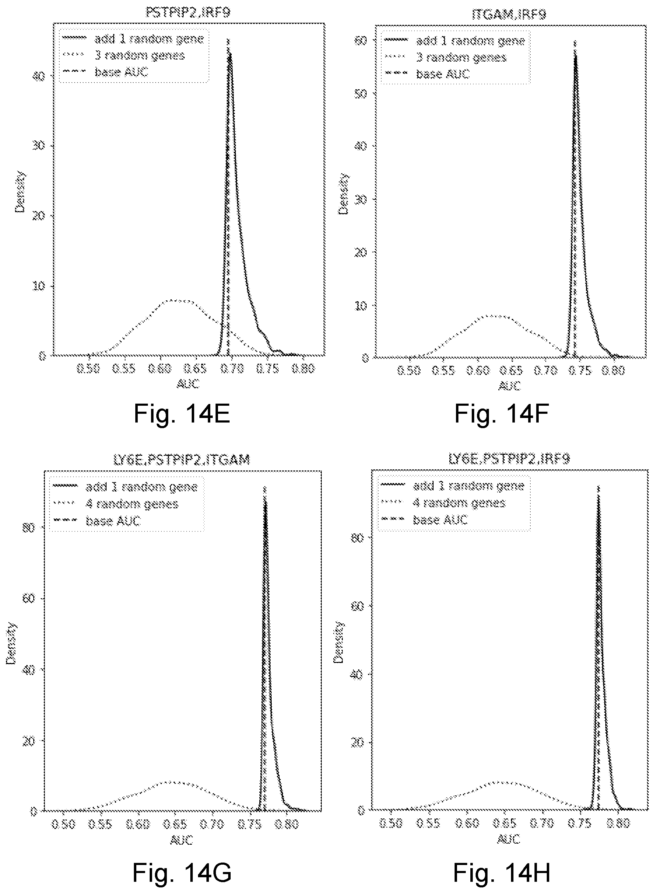

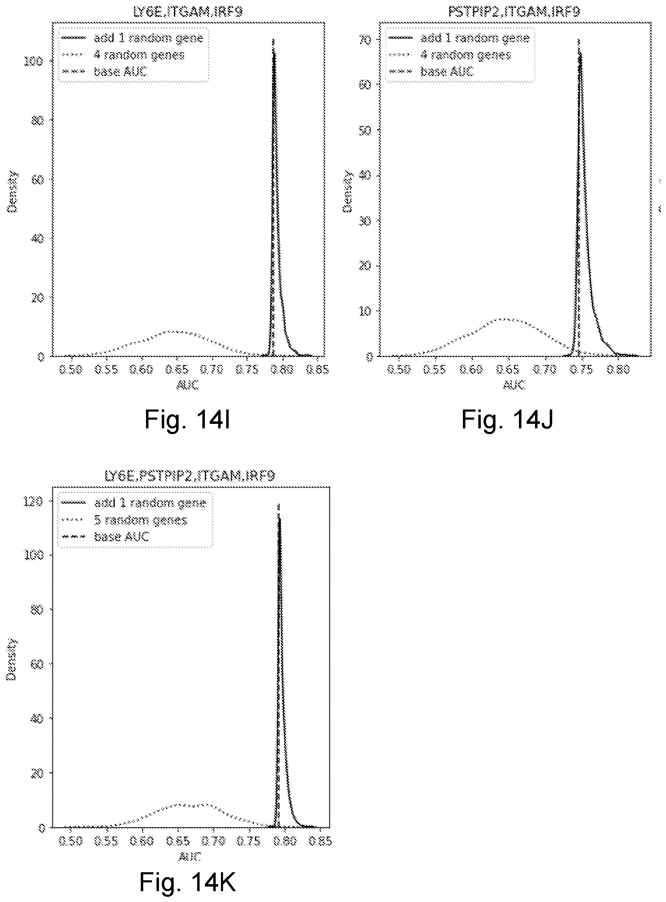

Figures 14A-14K show, respectively, for each of the classification models of Table 10, a combined plot of the base AUC, distribution of AUCs when one gene is added at random (e.g., augmented models, n=1000), and distribution of AUCs for random gene classification models (n=1000). As illustrated in Figure 14, each of the classification models of Table 10 performs better than random gene classification models of the same gene number. Further, the addition of an optional gene tends to increase the diagnostic power, as measured by AUC.

## CONCLUSION

Plural instances may be provided for components, operations or structures described herein as a single instance. Finally, boundaries between various components, operations, and data stores are somewhat arbitrary, and particular operations are illustrated in the context of specific illustrative configurations. Other allocations of functionality are envisioned and may fall within the scope of the implementation(s). In general, structures and functionality presented as separate components in the example configurations may be implemented as a combined structure or component. Similarly, structures and functionality presented as a single component may be implemented as separate components. These and other variations, modifications, additions, and improvements fall within the scope of the implementation(s).

It will also be understood that, although the terms first, second, *etc.* may be used herein to describe various elements, these elements should not be limited by these terms. These terms are only used to distinguish one element from another. For example, a first subject could be termed a second subject, and, similarly, a second subject could be termed a first subject, without departing from the scope of the present disclosure. The first subject and the second subject are both subjects, but they are not the same subject.

As used herein, the term "if" may be construed to mean "when" or "upon" or "in response to determining" or "in response to detecting," depending on the context. Similarly, the phrase "if it is determined" or "if [a stated condition or event] is detected" may be construed to mean "upon determining" or "in response to determining" or "upon detecting (the stated condition or event (" or "in response to detecting (the stated condition or event)," depending on the context.

The foregoing description included example systems, methods, techniques, instruction sequences, and computing machine program products that embody illustrative implementations. For purposes of explanation, numerous specific details were set forth in order to provide an understanding of various implementations of the inventive subject matter. It will be evident, however, to those skilled in the art that implementations of the inventive subject matter may be practiced without these specific details. In general, well-known instruction instances, protocols, structures and techniques have not been shown in detail.

The foregoing description, for purpose of explanation, has been described with reference to specific implementations. However, the illustrative discussions above are not intended to be exhaustive or to limit the implementations to the precise forms disclosed. Many modifications and variations are possible in view of the above teachings. The implementations were chosen and described in order to best explain the principles and their practical applications, to thereby enable others skilled in the art to best utilize the implementations and various implementations with various modifications as are suited to the particular use contemplated.

The following numbered clauses, describing aspects of the invention, are part of the description.

1. A method for obtaining an ensemble classifier for determining an infectious disease state of a subject, the infectious disease state being one or more of infected with a bacteria, infected with a virus, and not-infected, the method comprising:

at a computer system comprising at least one processor and a memory storing at least one program for execution by the at least one processor, the at least one program comprising instructions for:

obtaining a training dataset, wherein the training dataset comprises, in electronic form, for each respective training subject in a plurality of training subjects: (i) a corresponding label for the infectious disease state of the respective training subject and (ii) a respective attribute value for each respective gene in a plurality of genes obtained from a biological sample of the respective training subject, wherein the plurality of training subjects is 100 training subjects or more;

for each respective random seed in a plurality of random seeds, performing a corresponding instance of an outer loop, wherein each corresponding instance of the outer loop is characterized by a respective downsampling rate and a respective maximum iteration rate, the corresponding instance of the outer loop comprising:

A) for each respective initial classifier in a plurality of initial classifiers, using the random seed to pseudo-randomly assign values for each respective hyperparameter in a plurality of hyperparameters for the respective initial classifier, wherein each respective hyperparameter in the plurality of hyperparameters has a respective value selected from a respective plurality of candidate values for the respective hyperparameter, and wherein each respective initial classifier in the plurality of initial classifiers has a corresponding plurality of parameters, and wherein the corresponding plurality of parameters comprises more than 500 parameters;

B) binning the plurality of initial classifiers into a plurality of bins, wherein each bin in the plurality of bins is characterized by a respective initial number of initial classifiers in the plurality of initial classifiers, a respective initial number of iterations, and the downsampling rate,

for each respective bin in the plurality of bins, performing a corresponding inner loop in which an iteration count is initially set to the respective initial number of iterations, comprising:

i) for a number of iterations equal to the iteration count, training each initial classifier in the respective bin in a K-fold cross-validation context, wherein the K-fold cross-validation comprises refining each initial classifier in the respective bin against the training dataset using the values assigned for each respective hyperparameter in the plurality of hyperparameters for the respective initial classifier,

ii) determining, based on the K-fold cross-validation, a corresponding evaluation score for each initial classifier in the respective bin,

iii) removing, from the respective bin, a subset of initial classifiers in accordance with the downsampling rate and the corresponding evaluation score for each initial classifier in the respective bin;

iv) increasing the iteration count as a function of an inverse of the downsampling rate; and

v) repeating the performing i), determining ii), removing iii) and increasing iv) for a number of repetitions that is determined based on a corresponding identity for the respective bin; and

C) selecting, from among all initial classifiers in the plurality of initial classifiers, a corresponding classifier that has the best corresponding evaluation score as representative of the respective random seed in the plurality of random seeds; and

forming the ensemble classifier from the corresponding classifier selected by the selecting C) for each respective random seed in the plurality of random seeds.

2. The method of clause 1, wherein the K-fold cross-validation is performed with a value for K that is between 2 and 20.

3. The method of clause 1, wherein the K-fold cross-validation is performed with a value for K that is between 3 and 8.

4. The method of clause 1, wherein each respective initial classifier in the plurality of initial classifiers is selected from the group consisting of: a neural network algorithm, a support vector machine algorithm, a Naive Bayes algorithm, a nearest neighbor algorithm, a boosted trees algorithm, a random forest algorithm, a decision tree algorithm, a multinomial logistic regression algorithm, a linear model, or a linear regression algorithm.

5. The method of any one of clauses 1-4, wherein the biological sample is a blood sample of the respective training subject.

6. The method of any one of clauses 1-4, wherein the biological sample comprises blood, whole blood, plasma, serum, urine, cerebrospinal fluid, fecal, saliva, sweat, tears, pleural fluid, pericardial fluid, peritoneal fluid, nasal swabs, nasopharyngeal swabs, or oropharyngeal swabs of the respective training subject.

7. The method of any one of clauses 1-6, wherein the downsampling rate is between 1.5 and 6.

8. The method of any one of clauses 1-6, wherein the downsampling rate is 2 or 3.

9. The method of any one of clauses 1-8, wherein the number of bins is between 3 and 25.

10. The method of clause 1, wherein each respective initial classifier in the plurality of initial classifiers is a neural network that comprises:

a corresponding plurality of inputs, wherein each input in the corresponding plurality of inputs is for an attribute value for a gene in the plurality of genes,

a corresponding first hidden layer comprising a corresponding plurality of hidden neurons, wherein each hidden neuron in the corresponding plurality of hidden neurons (i) is fully connected to each input in the plurality of inputs, (ii) is associated with a first activation function type, and (iii) is associated with a corresponding parameter in the corresponding plurality of parameters for the respective neural network, and

one or more corresponding neural network outputs, wherein each respective neural network output in the corresponding one or more neural network outputs (i) directly or indirectly receives, as input, an output of each hidden neuron in the corresponding plurality of hidden neurons, and (ii) is associated with a second activation function type.

11. The method of clause 10, wherein the first activation function type is pseudo-randomly assigned by the using A) from the group consisting of all or a combination of tanh, sigmoid, softmax, Gaussian, Boltzmann-weighted averaging, absolute value, linear, rectified linear unit (ReLU), bounded rectified linear, soft rectified linear, parameterized rectified linear, average, max, min, sign, square, square root, multiquadric, inverse quadratic, inverse multiquadric, polyharmonic spline, and thin-plate spline.

12. The method of clause 10 or 11, wherein the second activation function type is pseudo-randomly assigned by the using A) from the group consisting of all or a combination of tanh, sigmoid, softmax, Gaussian, Boltzmann-weighted averaging, absolute value, linear, rectified linear unit (ReLU), bounded rectified linear, soft rectified linear, parameterized rectified linear, average, max, min, sign, square, square root, multiquadric, inverse quadratic, inverse multiquadric, polyharmonic spline, and thin-plate spline.

13. The method of any one of clauses 10-12, wherein the corresponding plurality of hidden neurons is pseudo-randomly assigned by the using A) to be between 2 and 500 neurons.

14. The method of any one of clauses 10-12, wherein the corresponding plurality of hidden neurons is pseudo-randomly assigned by the using A) to be between 2 and 300 neurons.

15. The method of any one of clauses 10-14, wherein each respective initial classifier in the plurality of initial classifiers is pseudo-randomly assigned by the using A) to have between 1 and 50 hidden layers.

16. The method of any one of clauses 10-14, wherein each respective initial classifier in the plurality of initial classifiers is pseudo-randomly assigned by the using A) to have between 1 and 20 hidden layers.

17. The method of any one of clauses 1-16, wherein the plurality of hyperparameters comprises a regularization hyperparameter that penalizes one or more parameters in the corresponding plurality of parameters, for each respective initial classifier in the plurality of initial classifiers.

18. The method of clause 17, wherein the regularization hyperparameter is pseudo-randomly assigned by the using A) to be an L1 or L2 penalty.

19. The method of any one of clauses 1-18, wherein the plurality of hyperparameters comprises a learning rate.

20. The method of any one of clauses 1-19, wherein each respective initial classifier in the plurality of initial classifiers is assigned a different plurality of values for the respective plurality of hyperparameters.

21. The method of any one of clauses 1-20, wherein the maximum iteration rate for each corresponding instance of the outer loop is between 20 and 1000.

22. The method of any one of clauses 1-21, wherein, for each corresponding instance of the outer loop, the respective initial number of initial classifiers binned B) into each respective bin in the plurality of bins is determined based on the number of bins, the maximum iteration rate, the downsampling rate, and the corresponding identity for the respective bin.

23. The method of any one of clauses 1-22,

wherein the performing K-fold cross-validation further comprises, for each initial classifier in the respective bin, obtaining one or more cross-validation scores based on a performance measure of the respective initial classifier, and the determining a corresponding evaluation score for the respective initial classifier is determined from the one or more cross-validation scores obtained from the K-fold cross-validation;

wherein the K-fold cross-validation is grouped K-fold cross-validation; and

wherein each group in the grouped K-fold cross-validation represents a clinical study.

24. The method of any one of clauses 1-23, wherein the removing iii) further comprises:

ranking each initial classifier in the respective bin based on the corresponding evaluation score, and

removing a number of lowest ranked initial classifiers in accordance with the downsampling rate.

25. The method of any one of clauses 1-24, wherein the number of repetitions in the repeating v) is s+1, wherein s is an identifying value assigned to the respective bin.

26. The method of any one of clauses 1-25, wherein the ensemble classifier is formed by combining a plurality of outputs obtained from the plurality of classifiers selected by the selecting C).

27. The method of any one of clauses 1-26, wherein the corresponding plurality of parameters for each respective initial classifier in the plurality of initial classifiers comprises at least 1000, at least 5000, or at least 10,000 parameters.

28. The method of any one of clauses 1-27, wherein each gene in the plurality of genes is selected for use in a biomarker panel.

29. The method of any one of clauses 1-28, wherein the plurality of genes comprises at least 10 genes selected from Table 1.

30. The method of any one of clauses 1-29, wherein the plurality of genes comprises at least 10 genes selected from Table 2 or Table 9.

31. The method of any one of clauses 1-30, wherein the plurality of genes comprises at least 20 genes selected from Table 1.

32. The method of any one of clauses 1-31, wherein the plurality of genes comprises at least 20 genes selected from Table 2 or Table 9.

33. The method of any one of clauses 1-32, wherein the attribute value for each corresponding gene in the plurality of genes is obtained using real-time quantitative isothermal amplification on one or more nucleic acid molecules in the biological sample of the respective training subject.

34. The method of clause 33, wherein the real-time quantitative isothermal amplification is real-time quantitative loop-mediated isothermal amplification (LAMP).

35. The method of any one of clauses 1-34, wherein the attribute value for each corresponding gene in the plurality of genes is mRNA abundance data.

36. The method of any one of clauses 1-35, wherein the corresponding label for the infectious disease state of the respective training subject comprises an indication selected from the group consisting of: infected with a bacteria, infected with a virus, not-infected, a sepsis status, a severity, an inflammation status, and an outcome.

37. The method of any one of clauses 1-36, further comprising:

obtaining a test dataset comprising, in electronic form, a respective attribute value for each respective gene in the plurality of genes obtained from a biological sample of a test subject, and

using the ensemble classifier to determine the infectious disease state of the test subject, based on at least the plurality of attribute values for the plurality of genes.

38. The method of clause 37, wherein the biological sample is a blood sample of the test subject.

39. The method of clause 37, wherein the biological sample comprises blood, whole blood, plasma, serum, urine, cerebrospinal fluid, fecal, saliva, sweat, tears, pleural fluid, pericardial fluid, peritoneal fluid, nasal swabs, nasopharyngeal swabs, or oropharyngeal swabs of the test subject.

40. The method of any one of clauses 37-39, wherein each gene in the plurality of genes is selected for use in a biomarker panel.

41. The method of any one of clauses 37-40, wherein the plurality of genes comprises at least 10 genes selected from Table 1.

42. The method of any one of clauses 37-41, wherein the plurality of genes comprises at least 10 genes selected from Table 2 or Table 9.

43. The method of any one of clauses 37-42, wherein the plurality of genes comprises at least 20 genes selected from Table 1.

44. The method of any one of clauses 37-43, wherein the plurality of genes comprises at least 20 genes selected from Table 2 or Table 9.

45. The method of any one of clauses 37-44, wherein the attribute value for each corresponding gene in the plurality of genes is obtained using real-time quantitative isothermal amplification on one or more nucleic acid molecules in the biological sample of the test subject.

46. The method of clause 45, wherein the real-time quantitative isothermal amplification is real-time quantitative loop-mediated isothermal amplification (LAMP).

47. The method of any one of clauses 37-46, wherein the attribute value for each corresponding gene in the plurality of genes is mRNA abundance data.

48. The method of any one of clauses 37-47, wherein the infectious disease state determined for the test subject is selected from the group consisting of: infected with a bacteria, infected with a virus, not-infected, sepsis, and severity.

49. The method of any one of clauses 37-48, wherein the infectious disease state determined for the test subject further comprises an indication of whether or not the test subject has the infectious disease state.

50. The method of any one of clauses 37-47, wherein the infectious disease state determined for the test subject comprises (a) an indication of whether the subject has a bacterial infection, (b) an indication of whether the subject has a viral infection, and (c) an indication of the severity of the subject's condition.

51. The method of any one of clauses 37-50, further comprising:

when the infectious disease state determined for the test subject indicates the presence of an infection, administering a first therapeutic regimen tailored for treatment of the subject in the presence of the infection; and when the infectious disease state determined for the test subject indicates the absence of an infection, administering a second therapeutic regimen tailored for treatment of the subject in the absence of the infection.

52. The method of any one of clauses 1-51, wherein the plurality of random seeds comprises between 2 and 100 random seeds.

53. A computer system for obtaining an ensemble classifier for determining an infectious disease state of a subject, the infectious disease state being one or more of infected with a bacteria, infected with a virus, and not-infected, the computer system comprising:

at least one processor; and
a memory storing at least one program for execution by the at least one processor, the at least one program comprising instructions for:

obtaining a training dataset, wherein the training dataset comprises, in electronic form, for each respective training subject in a plurality of training subjects: (i) a corresponding label for the infectious disease state of the respective training subject and (ii) a respective attribute value for each corresponding gene in a plurality of genes obtained from a biological sample of the respective training subject, wherein the plurality of training subjects is 100 training subjects or more;

for each respective random seed in a plurality of random seeds, performing a corresponding instance of an outer loop, wherein each corresponding instance of the outer loop is characterized by a respective downsampling rate and a respective maximum iteration rate, the corresponding instance of the outer loop comprising:

> A) for each respective initial classifier in a plurality of initial classifiers, using the random seed to pseudo-randomly assign values for each respective hyperparameter in a plurality of hyperparameters for the respective initial classifier, wherein each respective hyperparameter in the plurality of hyperparameters has a respective value selected from a respective plurality of candidate values for the respective hyperparameter, and wherein each respective initial classifier in the plurality of initial classifiers has a corresponding plurality of parameters, and wherein the corresponding plurality of parameters comprises more than 500 parameters;
>
> B) binning the plurality of initial classifiers into a plurality of bins, wherein each bin in the plurality of bins is characterized by a respective initial number of initial classifiers in the plurality of initial classifiers, a respective initial number of iterations, and the downsampling rate,
>
> for each respective bin in the plurality of bins, performing a corresponding inner loop in which an iteration count is initially set to the respective initial number of iterations, comprising:
>
>> i) for a number of iterations equal to the iteration count, training each initial classifier in the respective bin in a K-fold cross-validation context, wherein the K-fold cross-validation comprises refining each initial classifier in the respective bin against the training dataset using the values assigned for each respective hyperparameter in the plurality of hyperparameters for the respective initial classifier,
>> ii) determining, based on the K-fold cross-validation, a corresponding evaluation score for each initial classifier in the respective bin,
>> iii) removing, from the respective bin, a subset of initial classifiers in accordance with the downsampling rate and the corresponding evaluation score for each initial classifier in the respective bin;
>> iv) increasing the iteration count as a function of an inverse of the downsampling rate; and
>> v) repeating the performing i), determining ii), removing iii) and increasing iv) for a number of repetitions that is determined based on a corresponding identity for the respective bin; and
>
> C) selecting, from among all initial classifiers in the plurality of initial classifiers, a corresponding classifier that has the best corresponding evaluation score as representative of the respective random seed in the plurality of random seeds; and
>
> forming the ensemble classifier from the corresponding classifier selected by the selecting C) for each respective random seed in the plurality of random seeds.

54. A non-transitory computer-readable storage medium having stored thereon program code instructions that, when executed by a processor, cause the processor to perform a method of obtaining an ensemble classifier for determining an infectious disease state of a subject, the infectious disease state being one or more of infected with a bacteria, infected with a virus, and not-infected, the method comprising:

at a computer system comprising at least one processor and a memory storing at least one program for execution by the at least one processor, the at least one program comprising instructions for:

> obtaining a training dataset, wherein the training dataset comprises, in electronic form, for each respective training subject in a plurality of training subjects: (i) a corresponding label for the infectious disease state of the respective training subject and (ii) a respective attribute value for each corresponding gene in a plurality of genes obtained from a biological sample of the respective training subject, wherein the plurality of training subjects is 100 training subjects or more;
>
> for each respective random seed in a plurality of random seeds, performing a corresponding instance of an outer loop, wherein each corresponding instance of the outer loop is characterized by a respective downsampling rate and a respective maximum iteration rate, the corresponding instance of the outer loop comprising:
>
>> A) for each respective initial classifier in a plurality of initial classifiers, using the random seed to pseudo-randomly assign values for each respective hyperparameter in a plurality of hyperparameters for the respective initial classifier, wherein each respective hyperparameter in the plurality of hyperparameters has a respective value selected from a respective plurality of candidate values for the respective hyperparameter, and wherein each respective initial classifier in the plurality of initial classifiers has a corresponding plurality of parameters, and wherein the corresponding plurality of parameters comprises more than 500 parameters;

B) binning the plurality of initial classifiers into a plurality of bins, wherein each bin in the plurality of bins is characterized by a respective initial number of initial classifiers in the plurality of initial classifiers, a respective initial number of iterations, and the downsampling rate,

for each respective bin in the plurality of bins, performing a corresponding inner loop in which an iteration count is initially set to the respective initial number of iterations, comprising:

> i) for a number of iterations equal to the iteration count, training each initial classifier in the respective bin in a K-fold cross-validation context, wherein the K-fold cross-validation comprises refining each initial classifier in the respective bin against the training dataset using the values assigned for each respective hyperparameter in the plurality of hyperparameters for the respective initial classifier,
> ii) determining, based on the K-fold cross-validation, a corresponding evaluation score for each initial classifier in the respective bin,
> iii) removing, from the respective bin, a subset of initial classifiers in accordance with the downsampling rate and the corresponding evaluation score for each initial classifier in the respective bin;
> iv) increasing the iteration count as a function of an inverse of the downsampling rate; and
> v) repeating the performing i), determining ii), removing iii) and increasing iv) for a number of repetitions that is determined based on a corresponding identity for the respective bin; and

> C) selecting, from among all initial classifiers in the plurality of initial classifiers, a corresponding classifier that has the best corresponding evaluation score as representative of the respective random seed in the plurality of random seeds; and

forming the ensemble classifier from the corresponding classifier selected by the selecting C) for each respective random seed in the plurality of random seeds.

55. A method for determining an infectious disease state of a test subject, the method comprising:
at a computer system comprising at least one processor and a memory storing at least one program for execution by the at least one processor, the at least one program comprising instructions for:

obtaining, in electronic form, a dataset comprising a respective attribute value for each corresponding gene in a plurality of genes obtained from a biological sample of the test subject, thereby obtaining a plurality of attribute values, wherein:

each respective attribute value is determined by a real-time quantitative isothermal amplification on one or more nucleic acid molecules in the biological sample, and
the plurality of genes comprises at least 10 genes selected from one or more of Table 1, Table 2, and Table 9; and

responsive to inputting the plurality of attribute values to a trained ensemble classifier, obtaining, as output from the trained ensemble classifier, a determination as to whether the test subject has an infectious disease state selected from: infected with a bacteria, infected with a virus, and not-infected, wherein the trained ensemble classifier is obtained by performing a method comprising:

obtaining a training dataset, wherein the training dataset comprises, in electronic form, for each respective training subject in a plurality of training subjects: (i) a corresponding label for the infectious disease state of the respective training subject and (ii) a respective attribute value for each corresponding gene in the plurality of genes obtained from a biological sample of the respective training subject, wherein the plurality of training subjects is 100 training subjects or more;

for each respective random seed in a plurality of random seeds, performing a corresponding instance of an outer loop, wherein each corresponding instance of the outer loop is characterized by a respective downsampling rate and a respective maximum iteration rate, the corresponding instance of the outer loop comprising:

A) for each respective initial classifier in a plurality of initial classifiers, using the random seed to pseudo-randomly assign values for each respective hyperparameter in a plurality of hyperparameters for the respective initial classifier, wherein each respective hyperparameter in the plurality of hyperparameters has a respective value selected from a respective plurality of candidate values for the respective hyperparameter, and wherein each respective initial classifier in the plurality of initial classifiers has a corresponding plurality of parameters, and wherein the corresponding plurality of parameters comprises more than 500 parameters;

B) binning the plurality of initial classifiers into a plurality of bins, wherein each bin in the plurality of bins is characterized by a respective initial number of initial classifiers in the plurality of initial classifiers, a respective initial number of iterations, and the downsampling rate,

for each respective bin in the plurality of bins, performing a corresponding inner loop in which an iteration count is initially set to the respective initial number of iterations, comprising:

i) for a number of iterations equal to the iteration count, training each initial classifier in the respective bin in a K-fold cross-validation context, wherein the K-fold cross-validation comprises refining each initial classifier in the respective bin against the training dataset using the values assigned for each respective hyperparameter in the plurality of hyperparameters for the respective initial classifier,

ii) determining, based on the K-fold cross-validation, a corresponding evaluation score for each initial classifier in the respective bin,

iii) removing, from the respective bin, a subset of initial classifiers in accordance with the downsampling rate and the corresponding evaluation score for each initial classifier in the respective bin;

iv) increasing the iteration count as a function of an inverse of the downsampling rate; and

v) repeating the performing i), determining ii), removing iii) and increasing iv) for a number of repetitions that is determined based on a corresponding identity for the respective bin; and

C) selecting, from among all initial classifiers in the plurality of initial classifiers, a corresponding classifier that has the best corresponding evaluation score as representative of the respective random seed in the plurality of random seeds; and

forming the ensemble classifier from the corresponding classifier selected by the selecting C) for each respective random seed in the plurality of random seeds.

56. The method of clause 55, further comprising:

when the infectious disease state determined for the test subject indicates the presence of an infection, administering a first therapeutic regimen tailored for treatment of the subject in the presence of the infection; and

when the infectious disease state determined for the test subject indicates the absence of an infection, administering a second therapeutic regimen tailored for treatment of the subject in the absence of the infection.

57. A computer system for determining an infectious disease state of a test subject, the computer system comprising:

at least one processor; and

a memory storing at least one program for execution by the at least one processor, the at least one program comprising instructions for:

obtaining, in electronic form, a dataset comprising a respective attribute value for each corresponding gene in a plurality of genes obtained from a biological sample of the test subject, thereby obtaining a plurality of attribute values, wherein:

each respective attribute value is determined by a real-time quantitative isothermal amplification on one or more nucleic acid molecules in the biological sample, and

the plurality of genes comprises at least 10 genes selected from one or more of Table 1, Table 2, and Table 9; and

responsive to inputting the plurality of attribute values to a trained ensemble classifier, obtaining, as output from the trained ensemble classifier, a determination as to whether the test subject has an infectious disease state selected from: infected with a bacteria, infected with a virus, and not-infected, wherein the trained ensemble classifier is obtained by performing a method comprising:

obtaining a training dataset, wherein the training dataset comprises, in electronic form, for each respective training subject in a plurality of training subjects: (i) a corresponding label for the infectious disease state of the respective training subject and (ii) a respective attribute value for each corresponding gene in the plurality of genes obtained from a biological sample of the respective training subject, wherein the plurality of training subjects is 100 training subjects or more;

for each respective random seed in a plurality of random seeds, performing a corresponding instance of an outer loop, wherein each corresponding instance of the outer loop is characterized by a respective downsampling rate and a respective maximum iteration rate, the corresponding instance of the outer loop comprising:

A) for each respective initial classifier in a plurality of initial classifiers, using the random seed to pseudo-randomly assign values for each respective hyperparameter in a plurality of hyperparameters for the respective initial classifier, wherein each respective hyperparameter in the plurality of hyperparameters has a respective value selected from a respective plurality of candidate values for the respective hyperparameter, and wherein each respective initial classifier in the plurality of initial classifiers has a corresponding plurality of parameters, and wherein the corresponding plurality of parameters comprises more than 500 parameters;

B) binning the plurality of initial classifiers into a plurality of bins, wherein each bin in the plurality of bins is characterized by a respective initial number of initial classifiers in the plurality of initial classifiers, a respective initial number of iterations, and the downsampling rate,

for each respective bin in the plurality of bins, performing a corresponding inner loop in which an iteration count is initially set to the respective initial number of iterations, comprising:

i) for a number of iterations equal to the iteration count, training each initial classifier in the respective bin in a K-fold cross-validation context, wherein the K-fold cross-validation comprises refining each initial classifier in the respective bin against the training dataset using the values assigned for each respective hyperparameter in the plurality of hyperparameters for the respective initial classifier,

ii) determining, based on the K-fold cross-validation, a corresponding evaluation score for each initial classifier in the respective bin,

iii) removing, from the respective bin, a subset of initial classifiers in accordance with the downsampling rate and the corresponding evaluation score for each initial classifier in the respective bin;

iv) increasing the iteration count as a function of an inverse of the downsampling rate; and

v) repeating the performing i), determining ii), removing iii) and increasing iv) for a number of repetitions that is determined based on a corresponding identity for the respective bin; and

C) selecting, from among all initial classifiers in the plurality of initial classifiers, a corresponding classifier that has the best corresponding evaluation score as representative of the respective random seed in the plurality of random seeds; and

forming the ensemble classifier from the corresponding classifier selected by the selecting C) for each respective random seed in the plurality of random seeds.

58. A non-transitory computer-readable storage medium having stored thereon program code instructions that, when executed by a processor, cause the processor to perform a method of determining an infectious disease state of a test subject, the method comprising:

at a computer system comprising at least one processor and a memory storing at least one program for execution by the at least one processor, the at least one program comprising instructions for:

obtaining, in electronic form, a dataset comprising a respective attribute value for each corresponding gene in a plurality of genes obtained from a biological sample of the test subject, thereby obtaining a plurality of attribute values, wherein:

each respective attribute value is determined by a real-time quantitative isothermal amplification on one or more nucleic acid molecules in the biological sample, and

the plurality of genes comprises at least 10 genes selected from one or more of Table 1, Table 2, and Table 9; and

responsive to inputting the plurality of attribute values to a trained ensemble classifier, obtaining, as output from the trained ensemble classifier, a determination as to whether the test subject has an infectious disease state selected from: infected with a bacteria, infected with a virus, and not-infected, wherein the trained ensemble classifier is obtained by performing a method comprising:

obtaining a training dataset, wherein the training dataset comprises, in electronic form, for each respective training subject in a plurality of training subjects: (i) a corresponding label for the infectious disease state of the respective training subject and (ii) a respective attribute value for each corresponding gene in the plurality of genes obtained from a biological sample of the respective training subject, wherein the plurality of training subjects is 100 training subjects or more;

for each respective random seed in a plurality of random seeds, performing a corresponding instance of an outer loop, wherein each corresponding instance of the outer loop is characterized by a respective downsampling rate and a respective maximum iteration rate, the corresponding instance of the outer loop comprising:

A) for each respective initial classifier in a plurality of initial classifiers, using the random seed to pseudo-randomly assign values for each respective hyperparameter in a plurality of hyperparameters for the respective initial classifier, wherein each respective hyperparameter in the plurality of hyperparameters has a respective value selected from a respective plurality of candidate values for the respective hyperparameter, and wherein each respective initial classifier in the plurality of initial classifiers has a corresponding plurality of parameters, and wherein the corresponding plurality of parameters comprises more than 500 parameters;

B) binning the plurality of initial classifiers into a plurality of bins, wherein each bin in the plurality of bins is characterized by a respective initial number of initial classifiers in the plurality of initial classifiers, a respective initial number of iterations, and the downsampling rate,

for each respective bin in the plurality of bins, performing a corresponding inner loop in which an iteration count is initially set to the respective initial number of iterations, comprising:

i) for a number of iterations equal to the iteration count, training each initial classifier in the respective bin in a K-fold cross-validation context, wherein the K-fold cross-validation comprises refining each initial classifier in the respective bin against the training dataset using the values assigned for each respective hyperparameter in the plurality of hyperparameters for the respective initial classifier,

ii) determining, based on the K-fold cross-validation, a corresponding evaluation score for each initial classifier in the respective bin,

iii) removing, from the respective bin, a subset of initial classifiers in accordance with the downsampling rate and the corresponding evaluation score for each initial classifier in the respective bin;

iv) increasing the iteration count as a function of an inverse of the downsampling rate; and

v) repeating the performing i), determining ii), removing iii) and increasing iv) for a number of repetitions that is determined based on a corresponding identity for the respective bin; and

C) selecting, from among all initial classifiers in the plurality of initial classifiers, a corresponding classifier that has the best corresponding evaluation score as representative of the respective random seed in the plurality of random seeds; and

forming the ensemble classifier from the corresponding classifier selected by the selecting C) for each respective random seed in the plurality of random seeds.

59. A method for determining an infectious disease state of a test subject, the method comprising:
at a computer system comprising at least one processor and a memory storing at least one program for execution by the at least one processor, the at least one program comprising instructions for:

obtaining, in electronic form, a dataset comprising a respective attribute value for each corresponding gene in a plurality of genes obtained from a biological sample of the test subject, thereby obtaining a plurality of attribute values, wherein the plurality of genes comprises at least 20 genes selected from Table 1;

responsive to inputting the plurality of attribute values to a trained classifier, obtaining, as output from the trained classifier, a determination as to whether the test subject has an infectious disease state selected from: infected with a bacteria, infected with a virus, and not-infected.

60. A method for determining an infectious disease state of a test subject, the method comprising:
at a computer system comprising at least one processor and a memory storing at least one program for execution by the at least one processor, the at least one program comprising instructions for:

obtaining, in electronic form, a dataset comprising a respective attribute value for each corresponding gene in a plurality of genes obtained from a biological sample of the test subject, thereby obtaining a plurality of attribute values, wherein the plurality of genes comprises at least 20 genes selected from Table 2 or Table 9;
responsive to inputting the plurality of attribute values to a trained classifier, obtaining, as output from the trained classifier, a determination as to whether the test subject has an infectious disease state selected from: infected with a bacteria, infected with a virus, and not-infected.

61. The method of clause 59 or 60, wherein each gene in the plurality of genes is selected for use in a biomarker panel.

62. The method of any one of clauses 59-61, wherein the plurality of genes comprises at least 29 genes selected from Table 1.

63. The method of any one of clauses 59-62, wherein the plurality of genes comprises at least 29 genes selected from Table 2 or Table 9.

64. The method of any one of clauses 59-63, wherein the biological sample is a blood sample of the test subject.

65. The method of any one of clauses 59-63, wherein the biological sample comprises blood, whole blood, plasma, serum, urine, cerebrospinal fluid, fecal, saliva, sweat, tears, pleural fluid, pericardial fluid, peritoneal fluid, nasal swabs, nasopharyngeal swabs, or oropharyngeal swabs of the test subject.

66. The method of any one of clauses 59-65, wherein the attribute value for each corresponding gene in the plurality of genes is obtained using real-time quantitative isothermal amplification on one or more nucleic acid molecules in the biological sample of the test subject.

67. The method of clause 66, wherein the real-time quantitative isothermal amplification is real-time quantitative loop-mediated isothermal amplification (LAMP).

68. The method of any one of clauses 59-67, wherein the attribute value for each corresponding gene in the plurality of genes is mRNA abundance data.

69. The method of any one of clauses 59-68, wherein the infectious disease state determined for the test subject is further selected from the group consisting of: infected with a bacteria, infected with a virus, not-infected, sepsis, and severity.

70. The method of any one of clauses 59-69, wherein the infectious disease state determined for the test subject further comprises an indication of whether or not the test subject has the infectious disease state.

70a. The method of any one of clauses 59-68, wherein the infectious disease state determined for the test subject comprises (a) an indication of whether the subject has a bacterial infection, (b) an indication of whether the subject has a viral infection, and (c) an indication of the severity of the subject's condition.

71. The method of any one of clauses 59-70, further comprising:

obtaining a reference dataset comprising, in electronic form, a respective attribute value for each corresponding gene in a plurality of genes obtained from a biological sample of a reference subject, wherein the reference subject is matched to the test subject based on a corresponding clinical event time,
using the trained classifier to determine the infectious disease state of the reference subject, based on at least the plurality of attribute values for the plurality of genes in the reference subject, and
comparing the infectious disease state determined for the respective reference subject with the infectious disease state determined for the matched test subject.

72. The method of any one of clauses 59-71, further comprising:

when the infectious disease state determined for the test subject indicates the presence of an infection, administering a first therapeutic regimen tailored for treatment of the subject in the presence of the infection; and when the infectious disease state determined for the test subject indicates the absence of an infection, administering a second therapeutic regimen tailored for treatment of the subject in the absence of the infection.

73. The method of any one of clauses 59-72, wherein each gene in the plurality of genes has an abundance that satisfies an abundance threshold, and wherein the abundance threshold is determined based on a threshold limit of quantitation for the respective gene.

74. The method of clause 73, wherein the threshold limit of quantitation is determined, for each respective gene in the plurality of genes, based on one or more corresponding methods of measurement used to obtain the attribute value for the respective gene.

75. The method of clause 73 or clause 74, wherein the attribute value for each gene in the plurality of genes is obtained using real-time quantitative loop-mediated isothermal amplification (LAMP), and the threshold limit of quantitation is between 10 and 500 copies per 150 ng total RNA load.

76. The method of any one of clauses 59-75, wherein each gene in the plurality of genes has a dynamic range that satisfies a dynamic range threshold.

77. The method of clause 76, wherein the dynamic range threshold is determined, for each respective gene in the plurality of genes, based on one or more corresponding methods of measurement used to obtain the attribute value for the respective gene.

78. The method of clause 76 or clause 77, wherein the attribute value for each gene in the plurality of genes is mRNA abundance data, and the dynamic range threshold is determined based on a fold difference of abundance values for the respective gene, measured across a plurality of samples obtained from a reference cohort.

79. The method of clause 78, wherein the dynamic range threshold is between 2-fold and 40-fold.

80. The method of any one of clauses 59-79, wherein the trained classifier is selected from the group consisting of: a neural network algorithm, a support vector machine algorithm, a Naive Bayes algorithm, a nearest neighbor algorithm, a boosted trees algorithm, a random forest algorithm, a decision tree algorithm, a multinomial logistic regression algorithm, a linear model, or a linear regression algorithm.

81. The method of any one of clauses 59-79, wherein the trained classifier is an ensemble classifier.

82. The method of any one of clauses 59-79, wherein the trained classifier is obtained by a method comprising:

obtaining a training dataset, wherein the training dataset comprises, in electronic form, for each respective training subject in a plurality of training subjects: (i) a corresponding label for the infectious disease state of the respective training subject and (ii) a respective attribute value for each corresponding gene in the plurality of genes obtained from a biological sample of the respective training subject, wherein the plurality of training subjects is 100 training subjects or more;

for each respective random seed in a plurality of random seeds, performing a corresponding instance of an outer loop, wherein each corresponding instance of the outer loop is characterized by a respective downsampling rate and a respective maximum iteration rate, the corresponding instance of the outer loop comprising:

A) for each respective initial classifier in a plurality of initial classifiers, using the random seed to pseudo-randomly assign values for each respective hyperparameter in a plurality of hyperparameters for the respective initial classifier, wherein each respective hyperparameter in the plurality of hyperparameters has a respective value selected from a respective plurality of candidate values for the respective hyperparameter, and wherein each respective initial classifier in the plurality of initial classifiers has a corresponding plurality of parameters, and wherein the corresponding plurality of parameters comprises more than 500 parameters;

B) binning the plurality of initial classifiers into a plurality of bins, wherein each bin in the plurality of bins is characterized by a respective initial number of initial classifiers in the plurality of initial classifiers, a respective initial number of iterations, and the downsampling rate,

for each respective bin in the plurality of bins, performing a corresponding inner loop in which an iteration count is initially set to the respective initial number of iterations, comprising:

i) for a number of iterations equal to the iteration count, training each initial classifier in the respective bin in a K-fold cross-validation context, wherein the K-fold cross-validation comprises refining each initial classifier in the respective bin against the training dataset using the values assigned for each respective hyperparameter in the plurality of hyperparameters for the respective initial classifier,
ii) determining, based on the K-fold cross-validation, a corresponding evaluation score for each initial classifier in the respective bin,
iii) removing, from the respective bin, a subset of initial classifiers in accordance with the downsampling rate and the corresponding evaluation score for each initial classifier in the respective bin;
iv) increasing the iteration count as a function of an inverse of the downsampling rate; and
v) repeating the performing i), determining ii), removing iii) and increasing iv) for a number of repetitions that is determined based on a corresponding identity for the respective bin; and

C) selecting, from among all initial classifiers in the plurality of initial classifiers, a corresponding classifier that has the best corresponding evaluation score as representative of the respective random seed in the plurality of random seeds; and

forming the ensemble classifier from the corresponding classifier selected by the selecting C) for each respective random seed in the plurality of random seeds.

83. The method of clause 82, wherein each respective initial classifier in the plurality of initial classifiers is selected from the group consisting of: a neural network algorithm, a support vector machine algorithm, a Naive Bayes algorithm, a nearest neighbor algorithm, a boosted trees algorithm, a random forest algorithm, a decision tree algorithm, a multinomial logistic regression algorithm, a linear model, or a linear regression algorithm.

84. The method of clause 82 or clause 83, wherein the K-fold cross-validation is performed with a value for K that is between 2 and 20.

85. The method of clause 82 or clause 83, wherein the K-fold cross-validation is performed with a value for K that is between 3 and 8.

86. The method of any one of clauses 82-85, wherein the performing K-fold cross-validation further comprises, for each initial classifier in the respective bin, obtaining one or more cross-validation scores based on a performance measure of the respective initial classifier, and the determining a corresponding evaluation score for the respective initial classifier is determined from the one or more cross-validation scores obtained from the K-fold cross-validation.

87. The method of any one of clauses 82-86, wherein each respective initial classifier in the plurality of initial classifiers is assigned a different plurality of values for the respective plurality of hyperparameters.

88. The method of any one of clauses 82-87, wherein the ensemble classifier is formed by combining a plurality of outputs obtained from the plurality of classifiers selected by the selecting C).

89. The method of any one of clauses 82-88, wherein the plurality of random seeds comprises between 2 and 100 random seeds.

90. The method of any one of clauses 59-89, wherein the plurality of genes comprises no more than 1000 genes.

91. The method of any one of clauses 59-89, wherein the plurality of genes comprises no more than 200 genes.

92. A computer system for determining an infectious disease state of a test subject, the computer system comprising:

at least one processor; and
a memory storing at least one program for execution by the at least one processor, the at least one program comprising instructions for:

obtaining, in electronic form, a dataset comprising a respective attribute value for each corresponding gene in a plurality of genes obtained from a biological sample of the test subject, thereby obtaining a plurality of attribute values, wherein the plurality of genes comprises at least 20 genes selected from one or more of Table 1, Table 2, and Table 9;

responsive to inputting the plurality of attribute values to a trained classifier, obtaining, as output from the trained classifier, a determination as to whether the test subject has an infectious disease state selected from: infected with a bacteria, infected with a virus, and not-infected.

93. A non-transitory computer-readable storage medium having stored thereon program code instructions that, when executed by a processor, cause the processor to perform a method of determining an infectious disease state of a test subject, the method comprising:

at a computer system comprising at least one processor and a memory storing at least one program for execution by the at least one processor, the at least one program comprising instructions for:

obtaining, in electronic form, a dataset comprising a respective attribute value for each corresponding gene in a plurality of genes obtained from a biological sample of the test subject, thereby obtaining a plurality of attribute values, wherein the plurality of genes comprises at least 20 genes selected from one or more of Table 1, Table 2, and Table 9;

responsive to inputting the plurality of attribute values to a trained classifier, obtaining, as output from the trained classifier, a determination as to whether the test subject has an infectious disease state selected from: infected with a bacteria, infected with a virus, and not-infected.

94. A composition comprising a plurality of amplification primers for determining an infectious disease state of a subject, the plurality of amplification primers comprising, for each respective gene in a plurality of genes comprising at least 20 genes selected from Table 1, a respective forward amplification primer and a respective reverse amplification primer, wherein:

the respective forward amplification primer comprises a 3' binding region and a 5' auxiliary region, wherein:

the 3' binding region consists of from 10 to 50 nucleotides and has a sequence that is complementary to a first target sequence in a first strand of the respective gene or a transcript thereof, and
the 5' auxiliary region has a sequence that is not complementary to the sequence of the first strand of the respective gene or a transcript thereof; and

the respective reverse amplification primer comprises a binding region, wherein the binding region consists of from 10 to 50 nucleotides and has a sequence that is complementary to a second target sequence in the second strand of the respective gene or a transcript thereof.

95. A composition comprising a plurality of amplification primers for determining an infectious disease state of a subject, the plurality of amplification primers comprising, for each respective gene in a plurality of genes comprising at least 20 genes selected from Table 2 or Table 9, a respective forward amplification primer and a respective reverse amplification primer, wherein:

the respective forward amplification primer comprises a 3' binding region and a 5' auxiliary region, wherein:

the 3' binding region consists of from 10 to 50 nucleotides and has a sequence that is complementary to a first target sequence in a first strand of the respective gene or a transcript thereof, and
the 5' auxiliary region has a sequence that is not complementary to the sequence of the first strand of the respective gene or a transcript thereof; and

the respective reverse amplification primer comprises a binding region, wherein the binding region consists of from 10 to 50 nucleotides and has a sequence that is complementary to a second target sequence in the second strand of the respective gene or a transcript thereof.

96. The composition of clause 94 or clause 95, wherein each respective amplification primer in the plurality of amplification primers is between 10 and 100 base pairs.

97. The composition of any one of clauses 94-96, wherein each respective amplification primer in the plurality of amplification primers is between 10 and 70 base pairs.

98. The composition of any one of clauses 94-97, wherein, for each respective forward amplification primer in the plurality of amplification primers, the 5' auxiliary region comprises a binding region consisting of from 10 to 50 nucleotides and having a sequencing that is complementary to a third target sequence in the second strand of the respective gene or a transcript thereof.

99. The composition of any one of clauses 94-98, wherein each respective amplification primer in the plurality of amplification primers further comprises an identifier sequence that is common to all or a subset of the amplification primers in the plurality of amplification primers.

100. The composition of any one of clauses 94-99, wherein each respective amplification primer in the plurality of amplification primers is further conjugated to a respective affinity moiety.

101. The composition of any one of clauses 94-100, wherein each gene in the plurality of genes is selected for use in a biomarker panel.

102. The composition of any one of clauses 94-101, wherein the plurality of genes comprises at least 29 genes selected from Table 1.

103. The composition of any one of clauses 94-102, wherein the plurality of genes comprises at least 29 genes selected from Table 2 or Table 9.

104. The composition of any one of clauses 94-103, wherein the plurality of genes comprises no more than 1000 genes.

105. The composition of any one of clauses 94-104, wherein the plurality of genes comprises no more than 200 genes.

106. The composition of any one of clauses 94-105, wherein each gene in the plurality of genes satisfies an abundance threshold based on a measure of abundance for the respective gene in a reference dataset.

107. The composition of clause 106, wherein the abundance threshold is between 10 and 500 copies per 150 ng total RNA load.

108. The composition of any one of clauses 94-107, wherein each gene in the plurality of genes satisfies a dynamic range threshold based on a measure of dynamic range for the respective gene in a reference dataset.

109. The composition of clause 108, wherein the dynamic range threshold is between 2-fold and 40-fold.

110. A kit comprising agents for determining an infectious disease state of a subject, comprising:
a plurality of amplification primers comprising, for each respective gene in a plurality of genes comprising at least 20 genes selected from Table 1, a respective forward amplification primer and a respective reverse amplification primer, wherein:

the respective forward amplification primer comprises a 3' binding region and a 5' auxiliary region, wherein:

the 3' binding region consists of from 10 to 50 nucleotides and has a sequence that is complementary to a first target sequence in a first strand of the respective gene or a transcript thereof, and
the 5' auxiliary region has a sequence that is not complementary to the sequence of the first strand of the respective gene or a transcript thereof; and

the respective reverse amplification primer comprises a binding region, wherein the binding region consists of from 10 to 50 nucleotides and has a sequence that is complementary to a second target sequence in the second strand of the respective gene or a transcript thereof.

111. A kit comprising agents for determining an infectious disease state of a subject, comprising: a plurality of amplification primers comprising, for each respective gene in a plurality of genes comprising at least 20 genes selected from Table 2 or Table 9, a respective forward amplification primer and a respective reverse amplification primer, wherein:

the respective forward amplification primer comprises a 3' binding region and a 5' auxiliary region, wherein:

the 3' binding region consists of from 10 to 50 nucleotides and has a sequence that is complementary to a first target sequence in a first strand of the respective gene or a transcript thereof, and

the 5' auxiliary region has a sequence that is not complementary to the sequence of the first strand of the respective gene or a transcript thereof; and

the respective reverse amplification primer comprises a binding region, wherein the binding region consists of from 10 to 50 nucleotides and has a sequence that is complementary to a second target sequence in the second strand of the respective gene or a transcript thereof.

112. The kit of clause 110 or clause 111, further comprising information, in electronic or paper form, comprising instructions for measuring attributes of the plurality of genes in a biological sample of the subject, thereby obtaining a plurality of attribute values for the plurality of genes.

113. The kit of any of clauses 110-112, further comprising information, in electronic or paper form, comprising instructions for using the plurality of attribute values with a trained classifier to determine an infectious disease state of the subject, the infectious disease state selected from the group consisting of: infected with a bacteria, infected with a virus, and not-infected.

114. The kit of any one of clauses 110-113, wherein each gene in the plurality of genes is selected for use in a biomarker panel.

115. The kit of any one of clauses 110-114, wherein the plurality of genes comprises at least 29 genes selected from Table 1.

116. The kit of any one of clauses 110-115, wherein the plurality of genes comprises at least 29 genes selected from Table 2 or Table 9.

117. The kit of any one of clauses 110-116, wherein the plurality of genes comprises no more than 1000 genes.

118. The kit of any one of clauses 110-117, wherein the plurality of genes comprises no more than 200 genes.

119. The kit of any one of clauses 110-118, wherein each gene in the plurality of genes satisfies an abundance threshold based on a measure of abundance for the respective gene in a reference dataset.

120. The kit of clause 119, wherein the abundance threshold is between 10 and 500 copies per 150 ng total RNA load.

121. The kit of any one of clauses 110-120, wherein each gene in the plurality of genes satisfies a dynamic range threshold based on a measure of dynamic range for the respective gene in a reference dataset.

122. The kit of clause 121, wherein the dynamic range threshold is between 2-fold and 40-fold.

123. A plurality of conjugated nucleic acid probes for determining an infectious disease state of a subject, the plurality of conjugated nucleic acid probes comprising, for each respective gene in a plurality of genes comprising at least 20 genes selected from Table 1, a respective nucleic acid probe comprising a respective nucleic acid conjugated to a non-nucleic acid detection moiety, wherein the respective nucleic acid is complementary to the respective gene.

124. A plurality of conjugated nucleic acid probes for determining an infectious disease state of a subject, the plurality of conjugated nucleic acid probes comprising, for each respective gene in a plurality of genes comprising at least 20 genes selected from Table 2 or Table 9, a respective nucleic acid probe comprising a respective nucleic acid conjugated to a non-nucleic acid detection moiety, wherein the respective nucleic acid is complementary to the respective gene.

125. The plurality of conjugated nucleic acid probes of clause 123 or clause 124, wherein each gene in the plurality of genes is selected for use in a biomarker panel.

126. The plurality of conjugated nucleic acid probes of any one of clauses 123-125, wherein the plurality of genes comprises at least 29 genes selected from Table 1.

127. The plurality of conjugated nucleic acid probes of any one of clauses 123-126, wherein the plurality of genes comprises at least 29 genes selected from Table 2 or Table 9.

128. The plurality of conjugated nucleic acid probes of any one of clauses 123-127, wherein the plurality of genes comprises no more than 100 genes.

129. The plurality of conjugated nucleic acid probes of any one of clauses 123-128, wherein the plurality of genes comprises no more than 50 genes.

130. The plurality of conjugated nucleic acid probes of any one of clauses 123-129, wherein each gene in the plurality of genes satisfies an abundance threshold based on a measure of abundance for the respective gene in a reference dataset.

131. The plurality of conjugated nucleic acid probes of clause 130, wherein the abundance threshold is between 10 and 500 copies per 150 ng total RNA load.

132. The plurality of conjugated nucleic acid probes of any one of clauses 123-131, wherein each gene in the plurality of genes satisfies a dynamic range threshold based on a measure of dynamic range for the respective gene in a reference dataset.

133. The plurality of conjugated nucleic acid probes of clause 132, wherein the dynamic range threshold is between 2-fold and 40-fold.

134. A method for determining an infectious disease state of a subject, the method comprising:
at a computer system comprising at least one processor and a memory storing at least one program for execution by the at least one processor, the at least one program comprising instructions for:

obtaining, in electronic form, a dataset comprising respective attribute values for at least two genes selected from Table 8, wherein the attribute value is obtained from a biological sample of the subject;
responsive to inputting the attribute values to a trained classifier, obtaining, as output from the trained classifier, a determination as to whether the subject has an infectious disease state selected from: infected with a bacteria, infected with a virus, and not-infected.

135. The method of clause 134, wherein the at least two genes are selected from LY6E, IRF9, ITGAM, and PSTPIP2.

136. The method of any one of clauses 134-135, wherein the dataset comprises respective attribute values for at least three genes selected from Table 8.

137. The method of clause 136, wherein the at least three genes are selected from LY6E, IRF9, ITGAM, and PSTPIP2.

138. The method of any one of clauses 134-137, wherein the dataset comprises respective attribute values for at least four genes selected from Table 8.

139. The method of clause 138, wherein the at least four genes comprise LY6E, IRF9, ITGAM, and PSTPIP2.

140. The method of clause 139, wherein the dataset comprises a respective attribute value for one additional gene that is not LY6E, IRF9, ITGAM, and PSTPIP2.

141. The method of clause 140, wherein the one additional gene is selected from Table 8.

142. The method of any one of clauses 134-141, wherein the biological sample is a blood sample of the subject.

143. The method of any one of clauses 134-142, wherein the biological sample comprises blood, whole blood, plasma, serum, urine, cerebrospinal fluid, fecal, saliva, sweat, tears, pleural fluid, pericardial fluid, peritoneal fluid, nasal swabs, nasopharyngeal swabs, or oropharyngeal swabs of the subject.

144. The method of any one of clauses 134-143, wherein the attribute value is obtained using real-time polymerase chain reaction (RT-PCR), quantitative RT-PCR (qRT-PCR), or real-time quantitative isothermal amplification on one or more nucleic acid molecules in the biological sample of the subject.

145. The method of clause 144, wherein the real-time quantitative isothermal amplification is real-time quantitative loop-mediated isothermal amplification (LAMP).

146. The method of any one of clauses 134-145, wherein the attribute value is mRNA abundance data.

147. A method for diagnosing a subject suspected of having a bacterial or viral infection, the method comprising:

receiving a biological sample obtained from the subject;
measuring the expression levels of at least two genes selected from Table 8;
determining whether the subject has a bacterial infection or viral infection using the expression levels in a classification model which has been validated in multiple independent cohorts,
wherein the classification model has an area under the receiver operating characteristic (ROC) curve of at least 0.65 in at least one validation cohort.

148. The method of clause 147, wherein the at least two genes are selected from LY6E, IRF9, ITGAM, and PSTPIP2.

149. The method of any one of clauses 147-148, comprising measuring the expression levels of at least three genes selected from Table 8.

150. The method of clause 149, wherein the at least three genes are selected from LY6E, IRF9, ITGAM, and PSTPIP2.

151. The method of any one of clauses 147-150, comprising measuring the expression levels of at least four genes selected from Table 8.

152. The method of clause 151, wherein the at least four genes comprise LY6E, IRF9, ITGAM, and PSTPIP2.

153. The method of any one of clauses 147-152, comprising measuring the expression levels of at least five genes selected from Table 8.

154. The method of any one of clauses 147-153, wherein the classification model has an ROC curve of at least 0.7 in at least one validation cohort.

155. The method of clause 154, wherein the classification model has an ROC curve of at least 0.75 in at least one validation cohort.

156. The method of clause 155, wherein the classification model has an ROC curve of at least 0.8 in at least one validation cohort.

157. The method of any one of clauses 147-156, wherein the biological sample is a blood sample of the subject.

158. The method of any one of clauses 147-157, wherein the biological sample comprises blood, whole blood, plasma, serum, urine, cerebrospinal fluid, fecal, saliva, sweat, tears, pleural fluid, pericardial fluid, peritoneal fluid, nasal swabs, nasopharyngeal swabs, or oropharyngeal swabs of the test subject.

159. The method of any one of clauses 147-158 wherein the expression levels are obtained using real-time polymerase chain reaction (RT-PCR), quantitative RT-PCR (qRT-PCR), or real-time quantitative isothermal amplification on one or more nucleic acid molecules in the biological sample of the subject.

160. The method of clause 159, wherein the real-time quantitative isothermal amplification is real-time quantitative loop-mediated isothermal amplification (LAMP).

161. The method of any one of clauses 47-160, further comprising administering an antibiotic to the subject if the subject is determined to have a bacterial infection.

162. The method of any one of clauses 147-161, further comprising administering an anti-viral agent to the subject if the subject is determined to have a viral infection.

**Claims**

1. A method for diagnosing a subject suspected of having a bacterial or viral infection, the method comprising:

   receiving a biological sample obtained from the subject;
   measuring the expression levels of at least two genes selected from Table 8, wherein the at least two genes are selected from LY6E, ITGAM, PSTPIP2, and IRF9;
   determining whether the subject has a bacterial infection or viral infection using the expression levels in a classification model which has been validated in multiple independent cohorts,
   wherein the classification model has an area under the receiver operating characteristic (ROC) curve of at least 0.65 in at least one validation cohort.

2. The method of claim 1, wherein measuring the expression levels comprises measuring the expression levels of at least three genes selected from Table 8.

3. The method of claim 2, wherein the at least three genes are selected from LY6E, ITGAM, PSTPIP2, and IRF9.

4. The method of any one of claims 1-3, wherein measuring the expression levels comprises measuring the expression levels of at least four genes selected from Table 8.

5. The method of claim 4, wherein the at least four genes comprise LY6E, ITGAM, PSTPIP2, and IRF9.

6. The method of any one of claims 1-5, wherein measuring the expression levels comprises measuring the expression levels of at least five genes selected from Table 8.

7. The method of any one of claims 1-6, wherein the classification model has an ROC curve of at least 0.7 in at least one validation cohort.

8. The method of claim 7, wherein the classification model has an ROC curve of at least 0.75 in at least one validation cohort.

9. The method of claim 8, wherein the classification model has an ROC curve of at least 0.8 in at least one validation cohort.

10. The method of any one of claims 1-9, wherein the biological sample is a blood sample of the subject.

11. The method of any one of claims 1-9, wherein the biological sample comprises blood, whole blood, plasma, serum, urine, cerebrospinal fluid, fecal, saliva, sweat, tears, pleural fluid, pericardial fluid, peritoneal fluid, nasal swabs, nasopharyngeal swabs, or oropharyngeal swabs of the test subject.

12. The method of any one of claims 1-11, wherein the expression levels are obtained using real-time polymerase chain reaction (RT-PCR), quantitative RT-PCR (qRT-PCR), or real-time quantitative isothermal amplification on one or more nucleic acid molecules in the biological sample of the subject.

13. The method of any one of claims 1-11, wherein the expression levels are obtained using real-time quantitative loop-mediated isothermal amplification (LAMP).

14. The method of any one of claims 1-13, wherein the classification model is an ensemble classification model.

15. The method of claim 14, wherein the ensemble classification model outputs a first score that indicates whether the subject does or does not have a bacterial infection, and wherein the ensemble classification model outputs a second score that indicates whether the subject does or does not have a viral infection.

**System 100**

Non-Persistent Memory 111

| Processing core **102** |
|---|

114

| Network interface **104** |
|---|

| Optional operating system | 116 |
|---|---|
| Optional network communication module | 118 |
| Training dataset | 122 |
| Training subject 1 | 124-1 |
| Label 1 | 126-1-1 |
| . : | |
| Label N | 126-1-N |
| Attribute value for Gene 1 | 128-1-1 |
| . : | |
| Attribute value for Gene K | 128-1-K |
| . : | |
| Training subject M | 124-M |
| Test dataset | 130 |
| Test subject 1 | 132-1 |
| Attribute value for Gene 1 | 134-1-1 |
| . : | |
| Attribute value for Gene K | 134-1-K |
| . : | |
| Test subject P | 132-P |
| Classifier construction module | 136 |
| Random seed set | 138 |
| Hyperparameter assignment construct | 140 |
| Validation construct | 142 |
| Evaluation construct | 144 |
| Classification module | 146 |

| Persistent memory 112 |
|---|

User interface

108 — | Display | — 106

| Input |

110

Fig. 1

<u>200</u>

Obtain an ensemble classifier for determining an infectious disease state of a subject, the infectious disease state being one or more of infected with a bacteria, infected with a virus, and not-infected. — 202

Obtain a training dataset. The training dataset comprises, for each respective training subject in a plurality of training subjects, a corresponding label for the infectious disease state of the respective training subject and a respective attribute value for each corresponding gene in a plurality of genes obtained from a biological sample of the respective training subject. — 204

Perform, for each respective random seed in a plurality of random seeds, a corresponding instance of an outer loop. Each corresponding instance of the outer loop is characterized by a respective downsampling rate and a respective maximum iteration rate. — 206

A. For each respective initial classifier in a plurality of initial classifiers, use the random seed to pseudo-randomly assign values for each respective hyperparameter in a plurality of hyperparameters for the respective initial classifier. Each respective hyperparameter in the plurality of hyperparameters has a respective value selected from a respective plurality of candidate values, and each respective initial classifier in the plurality of initial classifiers has a corresponding plurality of parameters. — 208

B. Bin the plurality of initial classifiers into a plurality of bins. Each bin in the plurality of bins is characterized by a respective initial number of initial classifiers in the plurality of initial classifiers, a respective initial number of iterations, and the downsampling rate. For each respective bin in the plurality of bins, perform a corresponding inner loop in which an iteration count is initially set to the respective initial number of iterations. — 210

( A )

Fig. 2A

A

i. Train each initial classifier in the respective bin, for a number of iterations equal to the iteration count, in a K-fold cross-validation context comprising refining each initial classifier in the respective bin against the training dataset using the values assigned for each respective hyperparameter in the plurality of hyperparameters for the respective initial classifier. — 212

ii. Determine, based on the K-fold cross-validation, a corresponding evaluation score for each initial classifier in the respective bin. — 214

iii. Remove, from the respective bin, a subset of initial classifiers in accordance with the downsampling rate and the corresponding evaluation score for each initial classifier in the respective bin. — 216

iv. Increase the iteration count as a function of an inverse of the downsampling rate. — 218

v. Repeat the performing i), determining ii), removing iii) and increasing iv) for a number of repetitions that is determined based on a corresponding identity for the respective bin. — 220

C. Select, from among all initial classifiers in the plurality of initial classifiers, a corresponding classifier that has the best corresponding evaluation score as representative of the respective random seed in the plurality of random seeds. — 222

Form the ensemble classifier from the corresponding classifier selected for each respective random seed in the plurality of random seeds. — 224

Obtain a test dataset comprising a respective attribute value for each corresponding gene in the plurality of genes obtained from a biological sample of a test subject. Use the ensemble classifier to determine the infectious disease state of the test subject, based on at least the plurality of attribute values for the plurality of genes. — 226

Fig. 2B

300

Determine an infectious disease state of a test subject.    / 302

Obtain a dataset comprising, for each corresponding gene in a plurality of genes, a respective attribute value obtained from a biological sample of the test subject, thus obtaining a plurality of attribute values. The plurality of genes comprises at least 20 genes selected from Table 1 and/or at least 20 genes selected from Table 2.    / 304

Responsive to inputting the plurality of attribute values to a trained classifier, obtain, as output from the trained classifier, a determination as to whether the test subject has an infectious disease state selected from: infected with a bacteria, infected with a virus, and not-infected.    / 306

The classifier is an ensemble classifier.    / 308

When the infectious disease state determined for the test subject indicates the presence of an infection, administer a first therapeutic regimen tailored for treatment of the subject in the presence of the infection.
When the infectious disease state determined for the test subject indicates the absence of an infection, administer a second therapeutic regimen tailored for treatment of the subject in the absence of the infection.    / 310

Fig. 3

Fig. 4

# High-multiplex diagnostics in <30 minutes

**Example System Instrument**
< 2 min hands-on time,
< 30 min turnaround time
Small footprint

Fingerstick cartridge for **outpatients**

Up to 64 targets

**Hospital** cartridge accepts sample directly: no pipetting

Self-contained reagents

Insert cartridge

Fig. 5

## Correlation vs Abundance

Fig. 6

## qRT-LAMP StdDev vs Abundance

Fig. 7

Fig. 8

Fig. 9

| max_iter = 81 | s=4 | | s=3 | | s=2 | | s=1 | | s=0 | |
|---|---|---|---|---|---|---|---|---|---|---|
| eta = 3 | n_i | r_i | n_i | r_i | n_i | r_i | n_i | r_i | n_i | r_i |
| B = 5*max_iter | — — — — | | — — — — | | — — — — | | — — — — | | — — — — | |
| | 81 | 1 | 27 | 3 | 9 | 9 | 6 | 27 | 5 | 81 |
| | 27 | 3 | 9 | 9 | 3 | 27 | 2 | 81 | | |
| | 9 | 9 | 3 | 27 | 1 | 81 | | | | |
| | 3 | 27 | | | | | | | | |
| | 1 | 81 | | | | | | | | |

## Fig. 10

**Test Result**          **Exit Test >**

Sample ID          io23479
Completion Time          09/16/2020   3:38 PM
Operator          janeydoe

**Bacterial Infection**          **Very Unlikely**

7

| v. unlikely | unlikely | possible | v. likely |

0          10          20          30          40

**Viral Infection**          **Very Likely**

37

| v. unlikely | unlikely | possible | v. likely |

0          10          20          30          40

**Severity**          **Likely Low**

6

| likely low | Moderate | likely high |

0          10          20          30          40

Fig. 11

Fig. 12A

Fig. 12B

Fig. 12C

Fig. 12D

## IRF9,ITGAM

Fig. 12E

## LY6E,ITGAM

Fig. 12F

## PSTPIP2,IRF9,LY6E

Fig. 12G

## PSTPIP2,IRF9,ITGAM

Fig. 12H

**PSTPIP2,LY6E,ITGAM**

Fig. 12I

**IRF9,LY6E,ITGAM**

Fig. 12J

**PSTPIP2,IRF9,LY6E,ITGAM**

Fig. 12K

Fig. 13A

Fig. 13B

Fig. 13C

Fig. 14A

Fig. 14B

Fig. 14C

Fig. 14D

Fig. 14E

Fig. 14F

Fig. 14G

Fig. 14H

Fig. 14I

Fig. 14J

Fig. 14K

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 63183927
- US 16096261 B
- US 20190144943 A1
- US 2016022233 W
- WO 2016145426 A1
- US 2017036003 W
- WO 2017214061 A1
- US 2017029468 W
- WO 2018004806 A1
- US 2019015462 W
- WO 2019168622 A1
- US 20190144943 A
- US 41107499
- US 5545522 A
- US 5891636 A
- US 5716785 A
- WO 8810315 A
- WO 8906700 A
- US 8700880 W
- US 8901025 W
- US 3654090 A
- US 3850752 A
- US 4016043 A
- US 20050048542 A1
- US 2019051765 W
- WO 2020061217 A1

### Non-patent literature cited in the description

- **HUANG et al.** *N Engl J Med*, 2018, vol. 379, 236-249
- American College of Chest Physicians/Society of Critical Care Medicine Consensus Conference: Definitions for Sepsis and Organ Failure and Guidelines for the Use of Innovative Therapies in Sepsis. *Crit. Care. Med.*, 1992, vol. 20, 864-874
- American College of Chest Physicians Society of Critical Care Medicine. *Chest*, 1997, vol. 101, 1644-1655
- **DRAGHICI**. Data Analysis Tools for DNA Microarrays. Chapman and Hall/CRC, 2003
- **SIMON et al.** Design and Analysis of DNA Microarray Investigations. Springer, 2004
- Real-Time PCR: Current Technology and Applications. Caister Academic Press, 2009
- A-Z of Quantitative PCR. **BUSTIN**. IUL Biotechnology, No. 5. International University Line, 2004
- **VELCULESCU et al.** *Science*, 1995, vol. 270, 484-487
- **MATSUMURA et al.** *Cell. Microbiol.*, 2005, vol. 7, 11-18
- Serial Analysis of Gene Expression (SAGE): Methods and Protocols (Methods in Molecular Biology). Humana Press, 2008
- **SAMBROOK et al.** Molecular Cloning: A Laboratory Manual. 2001
- **CHIRGWIN et al.** *Biochemistry*, 1979, vol. 18, 5294-5299
- Current Protocols In Molecular Biology. Green Publishing Associates, Inc., John Wiley & Sons, Inc., 1989, vol. III, 13.12.1-13.12.5
- **BONALDO et al.** *Genome Res.*, 1996, vol. 6, 791-806
- **VELCULESCU et al.** *Science*, 1995, vol. 270, 484-7
- **CARULLI et al.** *Journal of Cellular Biochemistry*, 1998 (30/31), 286-96
- **HELD et al.** *Genome Research*, 1996, vol. 6, 986-994
- **NOTOMI et al.** *Nucleic Acids Research*, 2000, vol. 28 (12), E63
- **NIXON et al.** *Bimolecular Detection and Quantitation*, 2014, vol. 2, 4-10
- **SCHULER et al.** *Anal Methods.*, 2016, vol. 8, 2750-2755
- **SCHOEPP et al.** *Set. Transl. Med.*, 2017, vol. 9, 3693
- **JAMIESON et al.** Hyperband: A Novel Bandit-Based Approach to Hyperparameter Optimization. *arxiv.org/abs/1603.06560*
- **AGRESTI**. An Introduction to Categorical Data Analysis. John Wiley & Son, 1996, 103-144
- **VINCENT et al.** Stacked denoising autoencoders: Learning useful representations in a deep network with a local denoising criterion. *J Mach Learn Res*, 2010, vol. 11, 3371-3408
- **LAROCHELLE et al.** Exploring strategies for training deep neural networks. *J Mach Learn Res*, 2009, vol. 10, 1-40
- **HASSOUN**. Fundamentals of Artificial Neural Networks. Massachusetts Institute of Technology, 1995

- **CRISTIANINI** ; **SHAWE-TAYLOR**. An Introduction to Support Vector Machines. Cambridge University Press, 2000
- A training algorithm for optimal margin classifiers. **BOSER et al.** Proceedings of the 5th Annual ACM Workshop on Computational Learning Theory. ACM Press, 1992, 142-152
- **VAPNIK**. Statistical Learning Theory. Wiley, 1998
- **MOUNT**. Bioinformatics: sequence and genome analysis. Cold Spring Harbor Laboratory Press, 2001
- **DUDA**. Pattern Classification. John Wiley & Sons, Inc., 2001, vol. 259, 262-265
- **HASTIE**. The Elements of Statistical Learning. Springer, 2001
- **FUREY et al.** *Bioinformatics*, 2000, vol. 16, 906-914

- **DUDA**. Pattern Classification. John Wiley & Sons, Inc., 2001, 395-396
- **DUDA**. Pattern Classification. John Wiley & Sons, Inc., 2001, 396-408, 411-412
- **HASTIE et al.** The Elements of Statistical Learning. Springer-Verlag, 2001
- Random Forests--Random Features. **BREIMAN**. Technical Report 567. Statistics Department, U.C. Berkeley, September 1999
- **DUDA** ; **HART**. Pattern Classification and Scene Analysis. John Wiley & Sons, Inc., 1973, 211-256
- **FERNANDES et al.** Transfer Learning with Partial Observability Applied to Cervical Cancer Screening. *Pattern Recognition and Image Analysis: 8th Iberian Conference Proceedings*, 2017, 243-250